(19) Europäisches Patentamt
European Patent Office
Office européen des brevets

(11) EP 1 989 174 B1

(12) **EUROPÄISCHE PATENTSCHRIFT**

(45) Veröffentlichungstag und Bekanntmachung des Hinweises auf die Patenterteilung:
**03.08.2011 Patentblatt 2011/31**

(21) Anmeldenummer: **06841030.7**

(22) Anmeldetag: **19.12.2006**

(51) Int Cl.:
*C07C 211/26* (2006.01) *C07C 211/29* (2006.01)
*C07C 211/40* (2006.01) *C07C 233/06* (2006.01)
*C07C 233/13* (2006.01) *C07C 233/18* (2006.01)
*C07C 233/65* (2006.01) *C07C 233/66* (2006.01)
*C07C 233/73* (2006.01) *C07C 251/42* (2006.01)
*C07C 251/44* (2006.01) *C07C 275/26* (2006.01)
*C07C 303/38* (2006.01) *C07C 311/20* (2006.01)
*C07C 335/14* (2006.01)

(86) Internationale Anmeldenummer:
**PCT/EP2006/012224**

(87) Internationale Veröffentlichungsnummer:
**WO 2007/079930 (19.07.2007 Gazette 2007/29)**

(54) **SUBSTITUIERTE CYCLOHEXYLMETHYL-DERIVATE**

SUBSTITUTED CYCLOHEXYLMETHYL DERIVATIVES

DERIVE DE CYCLOHEXYLMETHYLE SUBSTITUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.12.2005 DE 102005061428**

(43) Veröffentlichungstag der Anmeldung:
**12.11.2008 Patentblatt 2008/46**

(73) Patentinhaber: **Grünenthal GmbH**
**52078 Aachen (DE)**

(72) Erfinder:
- **OBERBÖRSCH, Stefan**
  **52074 Aachen (DE)**
- **MERLA, Beatrix**
  **52078 Aachen (DE)**
- **SUNDERMANN, Bernd**
  **52074 Aachen (DE)**
- **ENGLBERGER, Werner**
  **52223 Stolberg (DE)**
- **HENNIES, Hagen-Heinrich**
  **52152 Simmerath (DE)**
- **KLESS, Achim**
  **52074 Aachen (DE)**
- **BLOMS-FUNKE, Petra**
  **51246 Würselen (DE)**
- **KÖGEL, Babette-Yvonne**
  **52379 Langerwehe-Hamich (DE)**
- **GRAUBAUM, Heinz**
  **12557 Berlin (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**EP-A2- 1 043 307** **WO-A-02/066432**
**WO-A-2004/043899**

EP 1 989 174 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft substituierte Cyclohexylmethyl-Derivate, Verfahren zu deren Herstellung, Arzneimittel enthaltend diese Verbindungen und die Verwendung von substituierten Cyclohexylmethyl-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

In WO 0290317 werden Verbindungen, offenbart, bei denen zwei substituierte Amine direkt mit dem Cyclohexanring verknüpft sind, wobei eine Aminomethylgruppe nicht beschrieben wird. Diese Verbindungen eignen sich zur Behandlung von Schmerz.

In EP 1043307 A2, WO 02/066432 A1 sowie WO 04/043899 A1 werden Cyclohexylmethyl-Derivate und deren Verwendung zur Herstellung von Arzneimitteln offenbart, die sich zur Behandlung von Schmerz eignen.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen.

Gegenstand der Erfindung sind daher substituierte Cyclohexylmethyl-Derivate der allgemeinen Formel I, **I**

worin

$R^1$ $C_{1-8}$-Alkyl, jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert; $(CH_2)_mCHN\text{-}OH$, $(CH_2)_nNR^6R^7$ oder $(CH_2)_nOR^8$ bedeutet, wobei n für 0, 1, 2 oder 3 und m für 0, 1 oder 2 steht; oder über eine $C_{1-3}$-Alkylgruppe, die gesättigt oder ungesättigt sein kann, verknüpftes $C(O)OR^9$; $CONR^{10}R^{11}$ bedeutet;

$R^2$ H oder OH bedeutet;

oder $R^1$ und $R^2$ gemeinsam für oder =N-OH stehen;

$R^3$ Aryl oder Heteroaryl, unsubstituiert oder einfach oder mehrfach substituiert; oder einen über eine $C_{1-3}$-Alkylgruppe verknüpften Arylrest, der unsubstituiert oder einfach oder mehrfach substituiert sein kann, bedeutet;

$R^4$ und $R^5$ unabhängig voneinander H; $C_{1-3}$-Alkyl, unsubstituiert, bedeutet, wobei $R^4$ und $R^5$ nicht gleichzeitig H bedeuten, oder die Reste $R^4$ und $R^5$ zusammen $CH_2CH_2OCH_2CH_2$, oder $(CH_2)_{3-6}$ bedeuten;

$R^6$H; $C_{1-8}$-Alkyl, gesättigt oder ungesättigt, verzweigt oder unverzweigt, unsubstituiert oder einfach oder mehrfach substituiert; Aryl, Heteroaryl oder $C_{3-10}$-Cycloalkyl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, bedeutet;

$R^7$ H; $C_{1-8}$-Alkyl, gesättigt oder ungesättigt, verzweigt oder unverzweigt, unsubstituiert oder einfach oder mehrfach substituiert; Aryl, Heteroaryl oder $C_{3-10}$-Cycloalkyl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, bedeutet; $C(O)NR^{10}R^{11}$, $C(S)NR^{10}R^{11}$, $SO_2R^{12}$ oder $C(O)R^{13}$ bedeutet;

$R^8$ H; $C_{1-8}$-Alkyl, jeweils verzweigt oder unverzweigt, gesättigt oder ungesättigt, unsubstituiert oder einfach oder mehrfach substituiert; $C_{3-10}$-Cycloalkyl, gesättigt oder ungesättigt, unsubstituiert oder einfach oder mehrfach substituiert; über eine $C_{1-4}$-Alkylgruppe verknüpften Aryl- oder Heteroarylrest, unsubstituiert oder einfach oder mehrfach substituiert, bedeutet;

$R^9$ H; $C_{1-8}$-Alkyl gesättigt oder ungesättigt, verzweigt oder unverzweigt, unsubstiuiert oder einfach oder mehrfach substituiert, bedeutet;

$R^{10}$ und $R^{11}$ unabhängig voneinander H; $C_{3-10}$-Cycloalkyl, gesättigt oder ungesättigt, unsubstituiert oder einfach oder mehrfach substituiert; Aryl oder Heteroaryl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, bedeutet;

$R^{12}$ Aryl oder Heteroaryl, jeweils unsubstituiert oder einfach oder mehrfach substituiert; $C_{1-8}$-Alkyl, jeweils gesättigt oder ungesättigt, verzweigt oder unverzweigt, unsubstituiert oder einfach oder mehrfach substituiert; $C_{3-10}$-Cycloalkyl, gesättigt oder ungesättigt, unsubstituiert oder einfach oder mehrfach substituiert; oder einen über eine $C_{1-3}$-Alkylkette ver-

knüpften Aryl- oder Heteroarylrest, jeweils unsubstituiert oder einfach oder mehrfach substituiert; bedeutet;

$R^{13}$ $C_{1-8}$-Alkyl, gesättigt oder ungesättigt, verzweigt oder unverzweigt, unsubstituiert oder einfach oder mehrfach substituiert; $C_{3-10}$-Cycloalkyl, Aryl oder Heteroaryl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, wobei die Alkylkette verzweigt oder unverzweigt, unsubstituiert oder einfach oder mehrfach substituiert sein kann; bedeutet;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Verbindungen weisen eine Affinität zum $\mu$-Opioid-Rezeptor auf.

Die Ausdrücke "$C_{1-3}$-Alkyl", "$C_{1-4}$-Alkyl" und "$C_{1-8}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 3 C-Atomen bzw. 1 bis 4 C-Atomen bzw. 1-8 C-Atomen, d.h. $C_{1-3}$-Alkanyle, $C_{2-3}$-Alkenyle und $C_{2-3}$-Alkinyle bzw $C_{1-4}$-Alkanyle, $C_{2-4}$-Alkenyle und $C_{2-4}$-Alkinyle bzw. $C_{1-8}$-Alkanyle, $C_{2-8}$-Alkenyle und $C_{2-8}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, 2-Hexyl, n-Heptyl, n-Octyl, Ethylenyl (Vinyl), Ethinyl, Propenyl ($-CH_2CH=CH_2$, $-CH=CH-CH_3$, $-C(=CH_2)-CH_3$), Propinyl ($-CH-C=CH$, $- C=C-CH_3$), Butenyl, Butinyl, Pentenyl, Pentinyl, Hexenyl, Hexinyl, Heptenyl, Heptinyl, Octenyl und Octinyl umfasst. Besonders vorteihaft sind Methyl, Ethyl, n-Propyl, isoPropyl, 2,2-Dimethylpropyl, n-Butyl, sec-Butyl, iso-Butyl, 3-Pentyl, n-Pentyl, n-Hexyl, .

Der Ausdruck "Cycloalkyl" oder "$C_{3-10}$-Cycloalkyl" bedeutet für die Zwecke dieser Erfindung cyclische Kohlenwasserstoffe mit 3, 4, 5, 6, 7, 8, 9 oder 10 Kohlenstoffatomen, wobei die Kohlenwasserstoffe gesättigt oder ungesättigt (aber nicht aromatisch), unsubstituiert oder ein- oder mehrfach substituiert sein können. Die Ringe können verbrückt wie z. B. in Adamantan oder in Bicyclo[2.2.1]heptan, oder unverbrückt sein. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist $C_{3-10}$-Cycloalkyl aus der Gruppe ausgewählt, die Cyclopropyl, Adamantyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält. Besonders bevorzugt sind Adamantyl, Cyclopentyl und Cyclohexyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein, wie beispielsweise im 2,3-Dihydrobenzofuran. Jeder Aryl-Rest kann unsubstituiert oder einfach oder mehrfach substituiert vorliegen, wobei die ArylSubstituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein, wobei ein einziges Heteroatom im Ringsystem das System als Heteroaryl definiert. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Benzothiadiazolyl, Isoxazoyl, Benzothienyl, Thiazolyl, Pyrazolyl, Furyl, Thienyl und Indolyl.

Der Ausdruck "über $C_{1-3}$-Alkyl gebundenes Aryl oder Heteroaryl" oder "über $C_{1-4}$-Alkyl gebundenes Aryl oder Heteroaryl" bedeuten für die Zwecke der vorliegenden Erfindung, daß $C_{1-3}$-Alkyl, $C_{1-4}$-Alkyl und Aryl bzw. Heteroaryl die oben definierten Bedeutungen haben und der Aryl- bzw. Heteroaryl-Rest über eine $C_{1-3}$-Alkyl-Gruppe oder eine $C_{1-4}$-Alkyl-Gruppe an die Verbindung der allgemeinen Struktur I gebunden ist. Besonders vorteilhaft im Sinne dieser Erfindung sind Benzyl, 1-Phenylpropyl, Diphenylmethyl, Phenethyl, Methylthienyl, 2-Indolylethyl, 1-Methyl-2-indolyl-ethyl und 4-Phenylbutyl.

Im Zusammenhang mit "Alkyl" oder "Cycloalkyl" versteht man unter dem Begriff "substituiert" im Sinne dieser Erfindung die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, O-Phenyl, C(=O)$C_{1-6}$-Alkyl, $CO_2H$, $CO_2$-$C_{1-6}$-Alkyl, Phenyl oder Benzyl, wobei unter mehrfach substituierten Resten solche Reste zu verstehen sind, die entweder an verschiedenen oder an gleichen Atomen mehrfach, z.B. zwei-oder dreifach, substituiert sind, beispielsweise dreifach am gleichen C-Atom wie im Falle von $CF_3$ oder $-CH_2CF_3$ oder an verschiedenen Stellen wie im Falle von-CH(OH)-CH=CH-$CHCl_2$. Die Mehrfachsubstitution kann mit dem gleichen oder mit verschiedenen Substituenten erfolgen. Besonders bevorzugt sind Methyl, Phenyl, 4-Chlorphenyl und $CO_2CH_3$.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, gegebenenfalls auch mit $SO_2$Phenyl oder $SO_2C_{1-6}$-Alkyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" und "Heteroaryl" sind dabei bevorzugte Substituenten $OCH_3$, CN, Cl, Br, $CH_3$, 2,3-Dihydrobenzofuran, S-$CH_3$, F, $NO_2$, n-Propyl, S-$C_2H_5$, Ethyl, $CF_3$, Pyridyl und tert.-Butyl; S-Phenyl, Phenyl und O-Phenyl, wobei die Phenylreste ihrerseits wiederum mit Cl, F, $OCH_3$ CN oder $CH_3$ substituiert sein können. Besonders bevorzugt sind $OCH_3$, CN, Cl, Br, S-(4-Chlorphenyl), $CH_3$, 2,6-Dichlorphenyl, 2,3-Dihydrobenzofuran, Phenyl, S-$CH_3$, F, $NO_2$, n-Propyl, O-(4-Methylphenyl), S-$C_2H_5$, O-(4-Chlorphenyl), Ethyl, $CF_3$, 4-Chlorphenyl, Pyridyl, $SO_2$-i-Propyl, $SO_2$-$CH_3$, $SO_2$-(4-Chlorphenyl) und *tert.*-Butyl.

Es ist bevorzugt, dass $R^1$ $C_{1-8}$-Alkyl, jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert wenn $R^2$ OH bedeutet.

Es ist außerdem bevorzugt, dass $R^1$ $(CH_2)_m$CHN-OH, $(CH_2)_nNR^6R^7$ oder $(CH_2)_nOR^8$ bedeutet, wobei n für 0, 1, 2 oder 3 und m für 0, 1 oder 2 steht; oder über eine $C_{1-3}$-Alkylgruppe, die gesättigt oder ungesättigt sein kann, verknüpftes C(O)$OR^9$; $CONR^{10}R^{11}$ bedeutet, wenn $R^2$ H bedeutet.

Weiterhin ist es bevorzugt, dass $R^1$ und $R^2$ gemeinsam für oder =N-OH stehen.

Bevorzugt im Sinne dieser Erfindung sind substituierte Cyclohexylmethyl-Derivate, worin

$R^1$ $C_{1-8}$-Alkyl, jeweils verzweigt oder unverzweigt, gesättigt oder ungesättigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{12}$-Alkyl, Phenyl oder Benzyl; Aryl oder Heteroaryl, unsubstituiert oder einfach oder mehrfach substituiert mit F, Cl, Br, I, CN, $NH_2$, NH-$G_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$A1kyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl; Cycloalkyl, gesättigt oder ungesättigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl oder Benzyl; einen über eine $C_{1-4}$-Alkylkette verknüpften Aryl- oder Heteroarylrest, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$ Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl;

insbesondere

$R^1$ $C_{1-8}$-Alkyl, jeweils verzweigt oder unverzweigt, gesättigt oder ungesättigt, unsubstituiert oder einfach oder mehrfach substituiert mit Methyl, =O, Phenyl oder

$CO_2CH_3$; Phenyl, Naphthyl, Benzyl, Phenethyl, 2-Pyridyl oder 2-Thienyl, unsubstituiert oder einfach oder mehrfach substituiert mit F, $CH_3$, Cl, tert.-Butyl, Methoxy oder $CF_3$; Cyclohexyl oder Cyclopentyl bedeutet.

Besonders bevorzugt sind substituierte Cyclohexylmethyl-Derivate, bei denen $R^1$ für 2,4-Difluorphenyl, 4-Fluor-3-methylphenyl, Phenyl, 3-Methoxybenzyl, 4-Chlorphenyl, Benzyl, 2-Methylphenyl, 4-tert.-Butylphenyl, Cyclopentyl, 4-Fluorphenyl, Phenethyl, 2-Thienyl, 2,4-Dichlorphenyl, 3-Methoxyphenyl, 4-Methylphenyl, 4-Methoxyphenyl, 3,5-Difluorphenyl, Isopropyl, Butyl, Ethyl, Hexyl, sec-Butyl, 2,4,6-Trimethylphenyl, Pentyl, Propyl, 3-Fluorphenyl, 3,5-Dichlorphenyl, 4-Fluorbenzyl, 4-Chlor-3-trifluormethylphenyl, Cyclohexyl, Isobutyl oder 2,5-Dimethoxyphenyl steht.

Bevorzugt im Sinne dieser Erfindung sind auch substituierte Cyclohexylmethyl-Derivate, worin

$R^3$ Phenyl, Thienyl oder Pyridyl, jeweils unsubstituiert oder einfach oder mehrfach substituiert mit F, Cl, CN, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Arylrest, jeweils unsubstituiert oder einfach oder mehrfach substituiert mit F, Cl, CN, NH-$C_{1-6}$-Alkyl, NH-$C_{1-6}$-Alkyl -OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Akyl-OH, C(=O)$C_{1-6}$-Alkyl, $CO_2H$, $CO_2$-$C_{1-6}$-Alkyl, $CF_3$, $C_{1-6}$-Alkyl, bedeutet;

vorzugsweise

$R^3$ Phenyl oder Thienyl, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Phenylrest, jeweils

unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl, bedeutet;
insbesondere
$R^3$ Phenyl, unsubstituiert oder einfach oder mehrfach substituiert mit F, Cl, OH, $OCH_3$, $CF_3$ oder $CH_3$; Thienyl; oder einen über eine $C_{1-3}$-Alkylkette gebundenen Phenylrest, unsubstituiert oder einfach oder mehrfach substituiert mit F, Cl, CN, OH; $OCH_3$, $CF_3$ oder $CH_3$; bedeutet.

Ganz besonders bevorzugt sind Cyclohexylmethyl-Derivate, worin $R^3$ Phenyl, unsubstituiert oder einfach substituiert mit Cl oder F; Phenethyl oder Thienyl bedeutet.

Bevorzugt sind weiterhin Cyclohexylmethyl-Derivate, worin $R^4$ und $R^5$ für H oder $CH_3$ stehen, wobei $R^4$ und $R^5$ nicht gleichzeitig H bedeuten.

Bevorzugt sind auch substituierte Cyclohexylmethyl-Derivate, worin $R^4$ und $R^5$ zusammen $(CH_2)_{3-6}$ bedeuten.

Außerdem bevorzugt sind substituierte Cyclohexylmethyl-Derivate, worin
$R^6$ H; $C_{1-8}$-Alkyl, gesättigt oder ungesättigt, verzweigt oder unverzweigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl$)_2$, N($C_{1-6}$-Alkyl-OH$)_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl oder Benzyl; Aryl oder Heteroaryl, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl$)_2$, N($C_{1-6}$-Alkyl-OH$)_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl; oder einen über eine $C_{1-4}$-Alkylkette verknüpften Aryl-oder Heteroarylrest, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl$)_2$, N ($C_{1-6}$-Alkyl-OH$)_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, wobei die Alkylkette verzweigt oder unverzweigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl$)_2$, N($C_{1-6}$-Alkyl-OH$)_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O) $C_{1-6}$-Alkyl, $CO_2H$, $CO_2$-$C_{1-6}$-Alkyl, Phenyl oder Benzyl sein kann, bedeutet;
vorzugsweise
$R^6$ H, $C_{1-8}$-Alkyl, gesättigt oder ungesättigt, verzweigt oder unverzweigt, unsubstituiert oder einfach oder mehrfach substituiert mit F, Cl, -CN, SH, $SCH_3$, $OCH_3$, OH, =O, $CO_2C_2H_5$ oder $CO_2CH_3$; Aryl, unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder *tert.*-Butyl; oder einen über eine $C_{1-4}$-Alkylkette verknüpften Aryl- oder Heteroarylrest, jeweils unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder *tert.*-Butyl, wobei die Alkylkette verzweigt oder unverzweigt, unsubstituiert oder einfach oder mehrfach substituiert mit $CO_2C_2H_5$ oder $CO_2CH_3$ sein kann, .bedeutet;
insbesondere
$R^6$ H; Phenyl, unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder *tert.*-Butyl; oder einen über eine $C_{1-4}$-Alkylkette verknüpften Phenyl- oder Indolylrest, jeweils unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl, wobei die Alkylkette verzweigt oder unverzweigt, unsubstituiert oder einfach oder mehrfach substituiert mit $CO_2C_2H_5$ oder $CO_2CH_3$ sein kann, bedeutet.

Ganz besonders bevorzugt sind substituierte Cyclohexylmethyl-Derivate, worin $R^6$ 2-Indolylethyl, Phenethyl, 3-Phenylpropyl, Benzyl, Phenyl, 4-Phenylbutyl, 1-(1H-Indol-3-yl)propan-2-yl, 4 2-(3-Indolyl)propionsäuremethylester, jeweils unsubstituiert oder einfach oder mehrfach substituiert mit F oder $OCH_3$, bedeutet.

Ebenfalls ganz besonders bevorzugt sind substituierte Cyclohexylmethyl-Derivate, bei denen $R^6$ H bedeutet.

Bevorzugt sind außerdem substituierte Cyclohexylmethyl-Derivate, worin
$R^7C(O)R^{13}$ bedeutet.
Weiterhin bevorzugt sind substituierte Cyclohexylmethyl-Derivate, worin $R^8$ H; einen über eine $C_{1-4}$-Alkylgruppe verknüpften Aryl- oder Heteroarylrest, unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl$)_2$, N($C_{1-6}$-Alkyl-OH$)_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, bedeutet;
insbesondere
$R^8$ H; einen über eine $C_{1-4}$-Alkylgruppe verknüpften Phenylrest, unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl, bedeutet.

Besonders bevorzugt sind Cyclohexylmethyl-Derivate, worin $R^8$ Benzyl, unsubstituiert oder einfach oder mehrfach substituiert mit F bedeutet.

Ebenfalls bevorzugt sind substituierte Cyclohexylmethyl-Derivate, worin
$R^9$ $C_{1-8}$-Alkyl, gesättigt oder ungesättigt, verzweigt oder unverzweigt, unsubstituiert oder einfach oder mehrfach substituiert mit F, Cl, -CN, SH, $SCH_3$, $OCH_3$, OH, =O, $CO_2C_2H_5$ oder $CO_2CH_3$; bedeutet;
insbesondere

$R^9$ $C_{1-8}$-Alkyl, verzweigt oder unverzweigt, bedeutet.

Besonders bevorzugt sind substituierte Cyclohexylmethyl-Derivate, worin $R^9$ Ethyl bedeutet.

Bevorzugt sind außerdem substituierte Cyclohexylmethyl-Derivate, worin $R^{10}$ und $R^{11}$ unabhängig voneinander H; C3-10-Cycloalkyl, gesättigt oder ungesättigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl oder Benzyl; Aryl oder Heteroaryl, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{12}$-Alkyl, $CF_3$, $C_{1-6}$-Alkyl; oder einen über eine $C_{1-4}$-Alkylkette verknüpften Aryl- oder Heteroarylrest, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, bedeutet;
insbesondere
$R^{10}$ und $R^{11}$ unabhängig voneinander H; Phenyl, Naphthyl oder einen über eine $C_{1-4}$-Alkylkette verknüpften Phenyl- oder Indolylrest, jeweils unsubstituiert oder substituiert mit F, Cl, Br, CN, $NH_2$, $NO_2$, SH, $SCH_3$, OH, $OCH_3$, $CF_3$, Methyl, Ethyl, Propyl, Butyl oder *tert*.-Butyl.

Besonders bevorzugt sind substituierte Cyclohexylmethyl-Derivate, worin $R^{10}$ und $R^{11}$ unabhängig voneinander H; Naphthyl, Phenyl oder Benzyl, jeweils unsubstituiert oder einfach oder mehrfach substituiert mit $CF_3$, F, $NO_2$ oder Br; oder Cyclohexyl, wobei $R^{10}$ und $R^{11}$ nicht gleichzeitig H bedeuten.

Bevorzugt sind auch substituierte Cyclohexylmethyl-Derivate, worin $R^{12}$ Aryl oder Heteroaryl, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl; $C_{1-8}$-Alkyl, jeweils gesättigt oder ungesättigt, verzweigt oder unverzweigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH; $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl oder Benzyl; oder einen über eine $C_{1-3}$-Alkylkette verknüpften Aryl- oder Heteroarylrest, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl-, $C_{1-8}$-Alkyl; bedeutet;
insbesondere
$R^{12}$ Naphthyl, Phenyl oder Benzyl, jeweils unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder *tert.*-Butyl bedeutet.

Besonders bevorzugt sind substituierte Cyclohexylmethyl-Derivate, worin $R^{12}$ Phenyl, unsubstituiert oder einfach oder mehrfach substituiert mit Cl, $OCH_3$, tert.-Butyl oder $NO_2$, bedeutet.

Bevorzugt sind auch substituierte Cyclohexylmethyl-Derivate, worin $R^{13}$ $C_{1-8}$-Alkyl, gesättigt oder ungesättigt, verzweigt oder unverzweigt, unsubstituiert oder einfach oder mehrfach substituiert mit F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, O-Phenyl, C(=O)$C_{1-6}$-Alkyl, $CO_2H$, $CO_2$-$C_{1-6}$-Alkyl, Phenyl oder Benzyl; $C_{3-10}$-Cycloalkyl, unsubstituiert oder einfach oder mehrfach substituiert mit F, Cl, Br, I, -CN, $NH_2$, $C_{1-6}$-Alkyl, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl oder Benzyl; Aryl oder Heteroaryl, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N ($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, Dihydrobenzofuran, $SO_2$Phenyl oder $SO_2C_{1-6}$-Alkyl; oder einen über eine $C_{1-4}$-Alkylkette verknüpften Aryl- oder Heteroarylrest, jeweils unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O) $C_{1-6}$-Alkyl, $CO_2H$, $CO_2$-$C_{1-6}$-Alkyl, $CF_3$, $C_{1-6}$-Alkyl, $SO_2$Phenyl oder $SO_2C_{1-6}$-Alkyl, wobei die Alkylkette verzweigt oder unverzweigt, unsubstituiert oder einfach oder mehrfach substituiert mit F, Cl, Br, I, -CN, $NH_2$, NH-$C_{1-6}$-Alkyl, NH-$C_{1-8}$-Alkyl-OH, $C_{1-8}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-8}$-Alkyl, $CO_2H$, $CO_2$-$C_{1-6}$-Alkyl, Phenyl oder Benzyl sein kann; bedeutet;
vorzugsweise
$R^{13}$ $C_{14}$-Alkyl, gesättigt oder ungesättigt, verzweigt oder unverzweigt, unsubstituiert oder einfach oder mehrfach substituiert mit F, Cl, -CN, NH-$C_{1-8}$-Alkyl, $C_{1-6}$-Alkyl, N($C_{1-6}$-Alkyl)$_2$, SH, S-$C_{1-6}$-Alkyl, S-Benzyl, O-$C_{1-6}$-Alkyl, OH, =O, O-Benzyl, O-Phenyl, C(=O)$C_{1-6}$-Alkyl, $CO_2H$, $CO_2$-$C_{1-6}$-Alkyl, Phenyl oder Benzyl; $C_{3-10}$-Cycloalkyl, unsubstituiert oder einfach oder mehrfach substituiert mit F, Cl, -CN, NH-$C_{1-6}$-Alkyl, $C_{1-6}$-Alkyl, N($C_{1-6}$-Alkyl)$_2$, SH, S-$C_{1-6}$-Alkyl, $C_{1-6}$-Alkyl,

S-Benzyl, O-$C_{1-6}$-Alkyl, OH, =O, O-Benzyl, C(=O)$C_{1-6}$-Alkyl, $CO_2H$, $CO_2$-$C_{1-6}$-Alkyl, Phenyl oder Benzyl; Aryl oder Heteroaryl, jeweils unsubstituiert oder einfach oder mehrfach substituiert mit F, Cl, Br, CN, $NH_2$, NH-$C_{1-6}$-Alkyl, N($C_{1-6}$Alkyl)$_2$, $NO_2$, SH, Pyridyl, S-$C_{1-6}$-Alkyl, S-Phenyl, OH, $(CH_2)_{0-3}$O-$C_{1-6}$-Alkyl, $C_{1-3}$-Alkyl-$C_{3-6}$-Cycloalkyl, O-$C_{1-6}$Alkyl-OH, O-Phenyl, Phenyl, Benzyl, C(=O)$C_{1-8}$-Alkyl, $CO_2H$, $CO_2$-$C_{1-6}$-Alkyl, $CF_3$, $C_{1-6}$-Alkyl, Dihydrobenzofuran, $SO_2$Phenyl oder $SO_2C_{1-6}$-Alkyl; oder einen über eine $C_{1-4}$-Alkylkette verknüpften Aryl- oder Heteroarylrest, jeweils unsubstituiert oder einfach oder mehrfach substituiert mit F, Cl, Br, CN, $NH_2$, NH-$C_{1-8}$-Alkyl, N($C_{1-6}$Alkyl)$_2$, $NO_2$, SH, Pyridyl, S-$C_{1-6}$-Alkyl, S-Phenyl, OH, O-$C_{1-6}$-Alkyl, O-$C_{1-6}$Nkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, $SO_2$Phenyl oder $SO_2C_{1-6}$-Alkyl, wobei die Alkylkette verzweigt oder unverzweigt, unsubstituiert oder einfach oder mehrfach substituiert mit F, Cl, -CN, SH, S-$C_{1-6}$-Alkyl, S-Benzyl, O-$C_{1-8}$-Alkyl, OH, O-$C_{1-6}$-Alkyl-OH, O-Benzyl, $CO_2$-$C_{1-6}$-Alkyl, Phenyl oder Benzyl sein kann; bedeutet;
insbesondere

$R^{13}$ $C_{14}$-Alkyl, gesättigt oder ungesättigt, verzweigt oder unverzweigt, unsubstituiert oder einfach oder mehrfach substituiert mit F, Cl, -CN, $C_{1-6}$-Alkyl, O-Phenyl, O-Benzyl, SH, S-$C_{1-6}$-Alkyl, O-$C_{1-6}$-Alkyl oder OH; Cyclopentyl, Cyclohexyl oder Adamantyl, unsubstituiert oder einfach oder mehrfach substituiert mit F, Cl, -CN, O-$C_{1-6}$-Alkyl, OH, =O, $C_{1-6}$-Alkyl, $CO_2$-$C_{1-6}$-Alkyl; Phenyl, Naphthyl, Thienyl, Furyl, Oxadiazolyl, Benzothiophenyl, Pyrazolyl, Pyridyl, Thiazolyl, Benzofuranyl, Isoxazolyl oder Benzothiadiazolyl, jeweils unsubstituiert oder einfach oder mehrfach substituiert mit F, Cl, Br, CN, $NH_2$, NH-$C_{1-6}$-Alkyl, N($C_{1-6}$Alkyl)$_2$, $NO_2$, SH, Pyridyl, S-$C_{1-6}$-Alkyl, S-Phenyl, OH, $(CH_2)_{0-3}$O-$C_{1-6}$-Alkyl, $C_{1-3}$-Alkyl-$C_{3-6}$-Cycloalkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, Dihydrobenzofuran, $SO_2$Phenyl, wobei Phenyl mit Cl substituiert sein kann, oder $SO_2C_{1-6}$-Alkyl; oder einen über eine $C_{1-4}$-Alkylkette verknüpften Phenyl- oder Thienylrest, jeweils unsubstituiert oder einfach oder mehrfach substituiert mit F, Cl, Br, CN, $NO_2$, SH, S-$C_{1-6}$-Alkyl, OH, O-$C_{1-6}$-Alkyl, $CF_3$ oder $C_{1-6}$-Alkyl, wobei die Alkylkette verzweigt oder unverzweigt, unsubstituiert oder einfach substituiert mit Phenyl sein kann; bedeutet.

Besonders bevorzugt sind substituierte Cyclohexylmethyl-Derivate, worin $R^{13}$ Methyl, Ethyl, Phenyl, Benzyl, 3-Pentyl, n-Propyl, Benzothienyl, 1-(4-Chlorphenyl)-cyclopentyl, 4-Propylphenyl, 3-Cyanophenyl, 3-Chlorphenyl, 5-Chlor-4-methoxy-thiophen-3-yl, 3-Fluor-5-trifluormethylphenyl, 4-Fluor-5-trifluormethylphenyl, 2-Thienyl, 3,5-Dichlorphenyl, 2,4,5-Trifluorphenyl, 3-Bromphenyl, 4-Methylphenyl, 3-Methoxyphenyl, 2,2-Dimethylpropyl, 2-*tert*-Butyl-5-methyl-pyrazol-3-yl, 2,4-Dimethoxyphenyl, 3-Trifluormethylphenyl, 3,5-Difluorphenyl, 2-Fluor-5-trifluormethylphenyl, 4-Chlorbenzyl, 2-Methoxyphenyl, 2-Methylsulfanyl-3-pyridyl, 3,4,5-Trimethoxyphenyl, 2-Ethylsulfanyl-3-pyridyl, 2-Methyl-5-phenyl-furan-3-yl, 1-Phenoxyethyl, tert.-Butylphenyl, 2-(4-Chlor-phenylsulfanyl)-3-pyridyl, 2-p-Tolyloxy-3-pyridyl, 3-Chlor-4-(sulfonyl-2-propyl)-thiophen-2-yl, 5-Methylisoxazol-3-yl, 5-Brom-3-pyridyl, Naphthyl, 2-Methyl-5-(4-chlor-phenyl)-furan-3-yl, 4-(4-Chlor-phenylsulfonyl)-3-methyl-thiophen-2-yl, 1-Phenylpropyl, Adamantyl, 2-Phenyl-thiazol-4-yl, 4-Methyl-2-phenyl-thiazol-5-yl, 2-(2,3-Dihydro-benzofuran-5-yl)-thiazol-4-yl, 3-Methylphenyl, 3-Chlor-4-methylsulfonyl-thiophen-2-yl, Benzyloxymethyl, Methylthienyl, 4-Brom-2-ethyl-5-methyl-pyrazol-3-yl, 2,5-Dimethylfuryl, 5-Pyridin-2-yl-thiophen, 3-Chlor-4-fluorphenyl, Cyclohexyl, 3-Nitrophenyl, 2,5-Difluorphenyl, 2,6-Difluorphenyl, 2-Trifluormethyl-5-fluor-phenyl, 4-Chlorphenoxy-methyl, 2-Bromphenyl, Cyclopentyl, Benzothiadiazolyl, Diphenylmethyl, 2-Methylphenyl, 3-Methoxybenzyl, 2,4,6-Trichlorphenyl, 2-Butyl, 2-Chlorphenyl, 3,5-Dinitrophenyl, 4-Cyanophenyl, 2,4-Dichlor-5-fluorphenyl, 2-Chlor-3-pyridyl, 4-Nitrophenyl, 2,3,4,5,6-Pentafluorphenyl oder 3-(2,6-Dichlor-phenyl)-5-methyl-isoxazol-4-yl, 5-Chlor-4-methylthiophen-3-yl, 4-Fluorphenyl, 4-Chlorphenyl, 2-Fluorphenyl, 3-Methylphenyl, 3-Bromphenyl, 2,6-Dichlorphenyl, 3,4-Dichlorphenyl, 4-Cyanophenyl, 2,4-Difluorphenyl, 2,4-Dichlorphenyl, 2,4-Dichlor-5-fluorphenyl, 2-Chlorpyridin-3-yl, 3,5-Dimethoxyphenyl, 2,6-Dimethoxyphenyl, 2,3,6-Trifluorphenyl, 2-(4-Chlorphenoxy)-3-pyridyl, 3,4-Difluorphenyl, 2-(2,3-Dihydro-benzofuran-5-yl)-4-methyl-thiazol-5-yl, 3-Methyl-oxadiazolyl, 3-Phenyl-oxadiazolyl, 3-Cyclopropylmethyl-oxadiazolyl, 3-Methoxymethyl-oxadiazolyl oder 2,4-Dimethoxyphenyl bedeutet.

Weiterhin ist es bevorzugt, dass die Reste $R^8$ und $R^9$ nicht H bedeutet.

Es ist darüber hinaus bevorzugt, dass die Reste $R^{10}$ und $R^{11}$ bzw $R^6$ und $R^7$ jeweils nicht gleichzeitig H bedeuten.

Außerdem ist es bevorzugt, dass $R^4$ und $R^5$ nicht H bedeuten.

Ganz besonders bevorzugt sind substituierte Cyclohexylmethylderivate (Untergruppe Oxime, primäre Amine, Alkohole, und Ester) aus der Gruppe

(16) 4-(Dimethylamino-phenyl-methyl)-cyclohexanon-oxim
(17) 4-(Dimethylamino-phenyl-methyl)-cyclohexylamin
(18) 4-[Dimethylamino-(4-fluorphenyl)-methyl]-cyclohexanon-oxim
(19) 4-[Dimethylamino-(4-fluorphenyl)-methyl]-cyclohexylamin
(20) 4-[Dimethylamino-(3-fluorphenyl)-methyl]-cyclohexanon-oxim
(21) 4-[Dimethylamino-(3-fluorphenyl)-methyl]-cyclohexylamin
(22) 4-[(4-Chlorphenyl)-dimethylamino-methyl]-cyclohexanonoxim
(23) 4-[(4-Chlorphenyl)-dimethylamino-methyl]-cyclohexylamin
(24) 4-(Dimethylamino-thiophen-2-yl-methyl)-cyclohexanonoxim
(25) 4-(Dimethylamino-thiophen-2-yl-methyl)-cyclohexylamin

(26) 4-(1-Dimethylamino-3-phenyl-propyl)-cyclohexanon oxim
(27) 4-(1-Dimethylamino-3-phenyl-propyl)-cyclohexylamin
(29) 4-(Dimethylamino-phenyl-methyl)-cyclohexan-carbaldehyd-oxim
(30) [(4-Aminomethyl-cyclohexyl)-phenyl-methyl]-dimethylamin
(32) 4-[Dimethylamino-(4-fluorphenyl)-methyl]-cyclohexanecarbaldehydoxim
(33) [(4-Aminomethyl-cyclohexyl)-(4-fluorphenyl)-methyl]-dimethylamin
(35) 4-[Dimethylamino-(3-fluorphenyl)-methyl]-cyclohexanecarbaldehydoxim
(36) [(4-Aminomethyl-cyclohexyl)-(3-fluorphenyl)-methyl]-dimethylamin
(38) 4-[(4-Chlorphenyl)-dimethylamino-methyl]-cyclohexanecarbaldehydoxim
(39) [(4-Aminomethyl-cyclohexyl)-(4-chlorphenyl)-methyl]-dimethylamin
(41) 4-(Dimethylamino-thiophen-2-yl-methyl)-cyclohexanecarbaldehydoxim
(42) [(4-Aminomethyl-cyclohexyl)-thiophen-2-yl-methyl]-dimethylamin
(44) 4-(1-Dimethylamino-3-phenyl-propyl)-cyclohexanecarbaldehydoxim
(45) [1-(4-Aminomethyl-cyclohexyl)-3-phenyl-propyl]-dimethylamin
(47) [4-(Dimethylamino-phenyl-methyl)-cyclohexyl]-acetaldehyd-oxim
(48) 2-[4-Dimethylamino-phenyl-methyl)-cyclohexyl]-ethylamin
(50) {4-[Dimethylamino-(4-fluorphenyl)-methyl]-cyclohexyl}-acetaldehydoxim
(51) 2-{4-[Dimethylamino-(4-fluorphenyl)-methyl]-cyclohexyl}-ethylamin
(53) {4-[Dimethylamino-(3-fluorphenyl)-methyl]-cyclohexyl}-acetaldehydoxim
(54) 2-{4-[Dimethylamino-(3-fluorphenyl)-methyl]-cyclohexyl}-ethylamin
(56) {4-[Dimethylamino-(4-chlorphenyl)-methyl]-cyclohexyl}-acetaldehydoxim
(66) 2-{4-[Dimethylamino-(4-chlorphenyl)-methyl]-cyclohexyl}-ethylamin
(68) 2-(4-((dimethylamino)(thiophen-2-yl)methyl)cyclohexyl)acetaldehydoxim
(69) 2-[4-(Dimethylamino-thiophen-2-yl-methyl)-cyclohexyl]-ethylamin
(71) [4-(1-Dimethylamino-3-phenyl-propyl)-cyclohexyl]-acetaldehydeoxim
(72) {1-[4-(2-Amino-ethyl)-cyclohexyl]-3-phenyl-propyl}-dimethylamin
(111) 4-[Dimethylamino-phenyl-methyl]-cyclohexanol
(112) 4-[Dimethylamino-(4-fluorphenyl)-methyl]-cyclohexanol
(113) 4-[Dimethylamino-(3-fluorphenyl)-methyl]-cyclohexanol
(114) 4-[(4-Chlorphenyl)-dimethylamino-methyl]-cyclohexanol
(115) 4-(Dimethylamino-thiophen-2-yl-methyl)-cyclohexanol
(116) 4-(1-Dimethylamino-3-phenyl-propyl)-cyclohexanol
(117) [4-(Dimethylamino-phenyl-methyl)-cyclohexyl]-methanol
(118) {4-[Dimethylamino-(4-fluorphenyl)-methyl]-cyclohexyl}-methanol
(119) {4-[Dimethylamino-(3-fluorphenyl)-methyl]-cyclohexyl}-methanol
(120) {4-[(4-Chlorphenyl)-dimethylamino-methyl]-cyclohexyl}-methanol
(121) [4-(Dimethylamino-thiophen-2-yl-methyl)-cyclohexyl]-methanol
(122) [4-(1-Dimethylamino-3-phenyl-propyl)-cyclohexyl]-methanol
(123) [4-(Dimethylamino-phenyl-methyl)-cyclohexyliden]-essigsäure-ethylester
(124) [4-(Dimethylamino-phenyl-methyl)-cyclohexyl]-essigsäure-ethylester
(125) 2-[4-(Dimethylamino-phenyl-methyl)-cyclohexyl]-ethanol
(126) {4-[Dimethylamino-(4-fluorphenyl)-methyl]-cyclohexyliden}-essigsäure-ethylester
(127) {4-[Dimethylamino-(4-fluorphenyl)-methyl]-cyclohexyl}-essigsäure-ethylester
(128) 2-{4-[Dimethylamino-(4-fluorphenyl)-methyl]-cyclohexyl}-ethanol
(129) {4-[Dimethylamino-(3-fluorphenyl)-methyl]-cyclohexyliden}essigsäure-ethylester
(130) {4-[Dimethylamino-(3-fluorphenyl)-methyl]-cyclohexyl}-essigsäure-ethylester
(131) 2-{4-[Dimethylamino-(3-fluorphenyl)-methyl]-cyclohexyl}-ethanol
(132) 3-[4-(Dimethylamino-phenyl-methyl)-cyclohexyl]-acrylsäure-ethylester
(133) 3-[4-(Dimethylamino-phenyl-methyl)-cyclohexyl]-propionsäureethylester
(134) 3-[4-(Dimethylamino-phenyl-methyl)-cyclohexyl]-propan-1-ol
(135) 3-{4-[Dimethylamino-(4-fluorphenyl)-methyl]-cyclohexyl}-acrylsäureethylester
(136) 3-{4-[Dimethylamino-(4-fluorphenyl)-methyl]-cyclohexyl}-propionsäureethyl-ester
(137) 3-{4-[Dimethylamino-(4-fluorphenyl)-methyl]-cyclohexyl}-propan-1-ol
(138) 3-{4-[Dimethylamino-(3-fluorphenyl)-methyl]-cyclohexyl}-acrylsäure-ethylester
(139) 3-{4-[Dimethylamino-(3-fluorphenyl)-methyl]-cyclohexyl}-propionsäureethyl-ester
(140) 3-{4-[Dimethylamino-(3-fluorphenyl)-methyl]-cyclohexyl}-propan-1-ol
(141) 3-[4-(1-Dimethylamino-3-phenyl-propyl)-cyclohexyl]-acrylsäureethylester
(142) 3-[4-(1-Dimethylamino-3-phenyl-propyl)-cyclohexyl]-propionsäureethylester

(143) 3-[4-(1-Dimethylamino-3-phenyl-propyl)-cyclohexyl]-propan-1-ol

Weiterhin ganz besonders bevorzugt sind substituierte Cyclohexylmethylderivate (Untergruppe Amide, sekundäre und tertiäre Amine, Hamstoffe, Grignardprodukte und Ether) aus der Gruppe

(73) 1-(4-((Dimethylamino)(phenyl)methyl)cyclohexyl)-3-(naphthalen-1-yl)hamstoff
(74) 1-(2,4-Difluorphenyl)-3-(4-((dimethylamino)(phenyl)methyl)cyclohexyl)harnstoff Hydrochlorid
(75) 1-(4-((Dimethylamino)(phenyl)methyl)cyclohexyl)-3-(3-(trifluormethyl)phenyl)harnstoff Hydrochlorid
(76) 1-(4-((Dimethylamino)(phenyl)methyl)cyclohexyl)-3-(2-nitrophenyl)harnstoff Hydrochlorid
(77) 1-(3-Bromphenyl)-3-(4-((dimethylamino)(phenyl)methyl)cyclohexyl)harnstoff Hydrochlorid
(78) 1-(4-((Dimethylamino)(phenyl)methyl)cyclohexyl)-3-phenylharnstoff Hydrochlorid
(79) 1-Benzyl-3-(4-((dimethylamino)(phenyl)methyl)cyclohexyl)harnstoff
(80) 1-Cyclohexyl-3-(4-((dimethylamino)(phenyl)methyl)cyclohexyl)harnstoff
(81) 1-(4-Bromphenyl)-3-(4-((dimethylamino)(phenyl)methyl)cyclohexyl)harnstoff
(82) 1-(4-((Dimethylamino)(phenyl)methyl)cyclohexyl)-3-(4-methoxyphenyl)harnstoff
(83) N-(2-(1H-Indol-3-yl)ethyl)-4-((dimethylamino)(phenyl)methyl)cyclohexanamin hydrochlorid
(84) 4-((Dimethylamino)(phenyl)methyl)-N-phenethylcyclohexanamin Hydrochlorid
(85) 4-((Dimethylamino)(phenyl)methyl)-N-(3-phenylpropyl)cyclohexanamin Dihydrochlorid
(86) N-Benzyl-4-((dimethylamino)(phenyl)methyl)cyclohexanamin Hydrochlorid
(87) 4-((Dimethylamino)(phenyl)methyl)-N-(4-phenylbutyl)cyclohexanamin Hydrochlorid
(88) N-(1-(1H-Indol-3-yl)propan-2-yl)-4-((dimethylamino)(phenyl)methyl)-cyclohexanamin Hydrochlorid
(89) N-(4-((Dimethylamino)(phenyl)methyl)cyclohexyl)-4-methoxybenzenamin Hydrochlorid
(90) 4-((Dimethylamino)(phenyl)methyl)-N-(4-methoxybenzyl)cyclohexanamin Dihydrochlorid
(91) 4-((Dimethylamino)(phenyl)methyl)-N-(4-fluorbenzyl)cyclohexanamin Hydrochlorid
(92) N-(4-((Dimethylamino)(phenyl)methyl)cyclohexyl)benzenamin Hydrochlorid
(93) N-(4-((Dimethylamino)(phenyl)methyl)cyclohexyl)-2-ethylbutanamid Hydrochlorid
(94) N-(4-((Dimethylamino)(phenyl)methyl)cyclohexyl)benzamid Hydrochlorid
(95) N-(4-((Dimethylamino)(phenyl)methyl)cyclohexyl)-N-(3-phenylpropyl)acetamid Hydrochlorid
(96) N-(4-((Dimethylamino)(phenyl)methyl)cyclohexyl)-N-phenylacetamid Hydrochlorid
(97) N-(4-((Dimethylamino)(phenyl)methyl)cyclohexyl)-N-(4-phenylbutyl)propionamid Hydrochlorid
(98) N-(4-((Dimethylamino)(phenyl)methyl)cyclohexyl)-N-(4-phenylbutyl)acetamid Hydrochlorid
(99) N-(4-((Dimethylamino)(phenyl)methyl)cyclohexyl)-N-(4-methoxyphenyl)acetamid Hydrochlorid
(100) N-Benzyl-N-(4-((dimethylamino)(phenyl)methyl)cyclohexyl)acetamid Hydrochlorid
(101) N-Benzyl-N-(4-((dimethylamino)(phenyl)methyl)cyclohexyl)-2-ethylbutanamid Hydrochlorid
(102) N-Benzyl-N-(4-((dimethylamino)(phenyl)methyl)cyclohexyl)butyramid Hydrochlorid
(103) N-Benzyl-N-(4-((dimethylamino)(phenyl)methyl)cyclohexyl)-4-fluorbenzamid Hydrochlorid
(104) N-Benzyl-N-(4-((dimethylamino)(phenyl)methyl)cyclohexyl)benzamid Hydrochlorid
(105) N-(4-((Dimethylamino)(phenyl)methyl)cyclohexyl)-2-ethyl-N-phenylbutanamid Hydrochlorid
(106) 4-Chlor-N-(4-((dimethylamino)(phenyl)methyl)cyclohexyl)benzolsulfonamid Hydrochlorid
(107) N-(4-((Dimethylamino)(phenyl)methyl)cyclohexyl)-4-methoxybenzolsulfonamid Hydrochlorid
(108) 4-tert-Butyl-N-(4-((dimethylamino)(phenyl)methyl)cyclohexyl)benzolsulfonamid Hydrochlorid
(109) N-(4-((Dimethylamino)(phenyl)methyl)cyclohexyl)-2-nitrobenzolsulfonamid Hydrochlorid
(110) N-(4-((Dimethylamino)(phenyl)methyl)cyclohexyl)benzolsulfonamid Hydrochlorid
(144) 4-(benzyloxy)cyclohexyl)-N,N-dimethyl(phenyl)methanamin Hydrochlorid
(145) 4-(4-Fluorbenzyloxy)cyclohexyl)-N,N-dimethyl(phenyl)methanamin Hydrochlorid
(146) trans-N,N-dimethyl(4-phenethylcyclohexyl)(phenyl)methanamin Hydrochlorid
(147) 1-Benzyl-4-((dimethylamino)(phenyl)methyl)cyclohexanol Hydrochlorid
(148) 4-((dimethylamino)(phenyl)methyl)-1-(4-fluorbenzyl)cyclohexanol Hydrochlorid
(149) 1-(2,5-Dimethoxyphenyl)-4-((dimethylamino)(phenyl)methyl)cyclohexanol
(150) 4-((Dimethylamino)(phenyl)methyl)-1-(4-fluor-3-methylphenyl)cyclohexanol
(151) 4-(Dimethylamino-phenyl-methyl)-1-(4-fluor-3-methyl-phenyl)-cyclohexanol
(152) 1-Benzyl-4-[dimethylamino-(3-fluor-phenyl)-methyl]-cyclohexanol
(153) 4-[Dimethylamino-(3-fluor-phenyl)-methyl]-1-phenethyl-cyclohexanol
(154) 4-[Dimethylamino-(3-fluor-phenyl)-methyl]-1-pentyl-cyclohexanol
(155) 1-(3,5-Dichlor-phenyl)-4-[dimethylamino-(3-fluor-phenyl)-methyl]-cyclohexanol
(156) 4-[Dimethylamino-(3-fluor-phenyl)-methyl]-1-(3-methoxy-benzyl)-cyclohexanol
(157) 1-(4-Chlor-3-trifluormethyl-phenyl)-4-[dimethylamino-(3-fluor-phenyl)-methyl]-cyclohexanol
(158) 4-[(4-Chlor-phenyl)-dimethylamino-methyl]-1-phenyl-cyclohexanol

(159) 1-Benzyl-4-[(4-chlor-phenyl)-dimethylamino-methyl]-cyclohexanol
(160) 4-[(4-Chlor-phenyl)-dimethylamino-methyl]-1-(4-fluor-3-methyl-phenyl)-cyclohexanol
(161) 4-[(4-Chlor-phenyl)-dimethylamino-methyl]-1-o-tolyl-cyclohexanol
(162) 4-[(4-Chlor-phenyl)-dimethylamino-methyl]-1-(4-fluor-phenyl)-cyclohexanol
(163) 4-[(4-Chlor-phenyl)-dimethylamino-methyl]-1-phenethyl-cyclohexanol
(164) 4-[(4-Chlor-phenyl)-dimethylamino-methyl]-1-(3-methoxy-phenyl)-cyclohexanol
(165) 4-[(4-Chlor-phenyl)-dimethylamino-methyl]-1-p-tolyl-cyclohexanol
(166) 4-[(4-Chlor-phenyl)-dimethylamino-methyl]-1-(3,5-difluor-phenyl)-cyclohexanol
(167) 1-Butyl-4-[(4-chlor-phenyl)-dimethylamino-methyl]-cyclohexanol
(168) 4-[(4-Chlor-phenyl)-dimethylamino-methyl]-1-hexyl-cyclohexanol
(169) 4-[(4-Chlor-phenyl)-dimethylamino-methyl]-1-pentyl-cyclohexanol (polareres Diastereomer)
(170) 4-[(4-Chlor-phenyl)-dimethylamino-methyl]-1-pentyl-cyclohexanol (unpolareres Diastereomer)
(171) 4-[(4-Chlor-phenyl)-dimethylamino-methyl]-1-(3-fluor-phenyl)-cyclohexanol
(172) 4-[(4-Chlor-phenyl)-dimethylamino-methyl]-1-(4-fluor-benzyl)-cyclohexanol
(173) 4-[(4-Chlor-phenyl)-dimethylamino-methyl]-1-(3-methoxy-benzyl)-cyclohexanol
(174) Methyl 2-(4-((dimethylamino)(phenyl)methyl)cyclohexylamino)-3-(1H-indol-3-yl)propanoat (polareres Diastereomer)
(175) Methyl 2-(4-((dimethylamino)(phenyl)methyl)cyclohexylamino)-3-(1H-indol-3-yl)propanoat (unpolareres Diastereomer)
(176) N-(4-((Dimethylamino)(phenyl)methyl)cyclohexyl)-N-(4-methoxybenzyl)acetamid
(177) N-(1-(1H-indol-3-yl)propan-2-yl)-N-(4-(dimethylamino)(phenyl)methyl)cyclohexyl)acetamid (polareres Diastereomer)
(178) N-(1-(1H-indol-3-yl)propan-2-yl)-N-(4-((dimethylamino)(phenyl)methyl)cyclohexyl)acetamid (unpolareres Diastereomer)
(179) N-(4-((Dimethylamino)(phenyl)methyl)cyclohexyl)-N-(4-fluorbenzyl)acetamid
(180) N-(4-((Dimethylamino)(phenyl)methyl)cyclohexyl)-N-phenylbutyramid
(181) N-(2-(1H-indol-3-yl)ethyl)-N-(4-((dimethylamino)(phenyl)methyl)-cyclohexyl)butyramid
(182) N-(2-(1H-indol-3-yl)ethyl)-N-(4-((dimethylamino)(phenyl)methyl)cyclohexyl)acetamid
(183) Benzo[b]thiophen-3-carbonsäure-[4-(dimethylamino-phenyl-methyl)-cyclohexyl]-amid
(184) 1-(4-Chlor-phenyl)-cyclopentancarbonsäure-[4-(dimethylamino-phenyl-methyl)-cyclohexyl]-amid
(185) N-[4-(Dimethylamino-thiophen-2-yl-methyl)-cyclohexyl]-4-propyl-benzamid
(186) 3-Cyano-N-[4-(dimethylamino-thiophen-2-yl-methyl)-cyclohexyl]-benzamid
(187) 3-Chlor-N-[4-(dimethylamino-phenyl-methyl)-cyclohexyl]-benzamid
(188) 5-Chlor-4-methoxy-thiophen-3-carbonsäure-[4-(dimethylamino-phenyl-methyl)-cyclohexyl]-amid
(189) 3,4-Dichlor-N-[4-(dimethylamino-phenyl-methyl)-cyclohexyl]-benzamid
(190) N-[4-(Dimethylamino-phenyl-methyl)-cyclohexyl]-3-fluor-5-trifluormethyl-benzamid
(191) 5-Chlor-4-methoxy-thiophen-3-carbonsäure-{4-[dimethylamino-(3-fluor-phenyl)-methyl]-cyclohexyl}-amid
(192) N-[4-(Dimethylamino-thiophen-2-yl-methyl)-cyclohexyl]-4-fluor-3-trifluormethyl-benzamid
(193) Thiophen-2-corbonsäure-[4-(dimethylamino-thiophen-2-yl-methyl)-cyclohexyl]-amid
(194) 3,5-Dichlor-N-[4-(dimethylamino-thiophen-2-yl-methyl)-cyclohexyl]-benzamid
(195) 5-Chlor-4-methoxy-thiophen-3-carbonsäure-[4-(dimethylamino-thiophen-2-yl-methyl)-cyclohexyl]-amid
(196) N-[4-(Dimethylamino-phenyl-methyl)-cyclohexyl]-2,4,5-trifluor-benzamid
(197) 3-Brom-N-[4-(dimethylamino-phenyl-methyl)-cyclohexyl]-benzamid
(198) N-[4-(Dimethylamino-thiophen-2-yl-methyl)-cyclohexyl]-4-methyl-benzamid
(199) N-[4-(Dimethylamino-thiophen-2-yl-methyl)-cyclohexyl]-3-methoxy-benzamid
(200) N-[4-(Dimethylamino-thiophen-2-yl-methyl)-cyclohexyl]-3,3-dimethyl-butyramid
(201) 2-tert-Butyl-5-methyl-2H-pyrazol-3-ccarbonsäure-[4-(dimethylamino-thiophen-2-yl-methyl)-cyclohexyl]-amid
(202) N-[4-(Dimethylamino-thiophen-2-yl-methyl)-cyclohexyl]-2,4-dimethoxy-benzamid
(203) N-[4-(Dimethylamino-phenyl-methyl)cyclohexyl]-3-trifluormethyl-benzamid
(204) N-{4-[Dimethylamino-(3-fluor-phenyl)-methyl]-cyclohexyl}-3,5-difluor-benzamid
(205) N-[4-(Dimethylamino-phenyl-methyl)-cyclohexyl]-2-fluor-5-trifluormethyl-benzamid
(206) 2-(4-Chlor-phenyl)-N-[4-(dimethylamino-phenyl-methyl)-cyclohexyl]-acetamid
(207) N-[4-(Dimethylamino-thiophen-2-yl-methyl)-cyclohexyl]-2-methoxy-benzamid
(208) N-[4-(Dimethylamino-thiophen-2-yl-methyl)-cyclohexyl]-2-methylsulfanyl-nicotinamid
(209) 3,4-Dichlor-N-{4-[dimethylamino-(3-fluor-phenyl)-methyl]-cyclohexyl}-benzamid
(210) N-[4-(1-Dimethylamino-3-phenyl-propyl)-cyclohexyl]-4-fluor-3-trifluormethyl-benzamid (polareres Diastereomer)
(211) 5-Chlor-4-methoxy-thiophen-3-carbonsäure-{4-[dimethylamino-(4-fluor-phenyl)-methyl]-cyclohexyl}-amid

(212) N-[4-(Dimethylamino-thiophen-2-yl-methyl)-cyclohexyl]-3,4,5-trimethoxy-benzamid
(213) N-[4-(Dimethylamino-thiophen-2-yl-methyl)-cyclohexyl]-2-ethylsulfanyl-nicotinamid
(214) 2-Methyl-5-phenyl-furan-3-carbonsäure-[4-(dimethylamino-phenyl-methyl)-cyclohexyl]-amid
(215) N-{4-[Dimethylamino-(3-fluor-phenyl)-methyl]-cyclohexyl}-2-phenoxy-propionamid unpolareres Diastereomer)
(216) N-[4-(Dimethylamino-phenyl-methyl)-cyclohexyl]-2,4-dimethoxy-benzamid
(217) 4-tert-Butyl-N-[4-(dimethylamino-phenyl-methyl)-cyclohexyl]-benzamid
(218) 2-(4-Chlor-phenylsulfanyl)-N-[4-(dimethylamino-thiophen-2-yl-methyl)-cyclohexyl]-nicotinamid
(219) 2-(4-Chlor-phenyl)-N-[4-(dimethylamino-thiophen-2-yl-methyl)-cyclohexyl]-acetamid
(220) N-[4-(Dimethylamino-thiophen-2-yl-methyl)-cyclohexyl]-2-p-tolyloxy-nicotinamid
(221) 3-Chlor-4-(propan-2-sulfonyl)-thiophen-2-carbonsäure-[4-(dimethylamino-thiophen-2-yl-methyl)-cyclohexyl]-amid
(222) N-{4-[Dimethylamino-(3-fluor-phenyl)-methyl]-cyclohexyl}-2-phenoxy-propionamid (polareres Diastereomer)
(223) 2-tert-Butyl-5-methyl-2H-pyrazol-3-carbonsäure-[4-(dimethylamino-phenyl-methyl)-cyclohexyl]-amid
(224) 5-Methyl-isoxazol-3-carbonsäure-[4-(dimethylamino-thiophen-2-yl-methyl)-cyclohexyl]-amid
(225) 5-Brom-N-[4-(dimethylamino-thiophen-2-yl-methyl)-cyclohexyl]-nicotinamid
(226) Naphthyl-1-carbonsäure-[4-(dimethylamino-phenyl-methyl)-cyclohexyl]-amid
(227) N-[4-(1-Dimethylamino-3-phenyl-propyl)-cyclohexyl]-4-fluor-3-trifluormethyl-benzamid (unpolareres Diastereomer)
(228) N-[4-(Dimethylamino-phenyl-methyl)-cyclohexyl]-3,3-dimethyl-butyramid (polareres Diastereomer)
(229) 5-(4-Chlor-phenyl)-2-methyl-furan-3-carbonsäure-[4-(dimethylamino-thiophen-2-yl-methyl)-cyclohexyl]-amid
(230) N-[4-(Dimethylamino-phenyl-methyl)-cyclohexyl]-2-phenoxy-propionamid
(231) Benzo[b]thiophen-2-carbonsäure-{4-[dimethylamino-(3-fluor-phenyl)-methyl]-cyclohexyl}-amid
(232) 5-(4-Chlor-phenyl)-2-methyl-furan-3-carbonsäure-[4-(dimethytamino-phenyl-methyl)-cyclohexyl]-amid
(233) 4-(4-Chlor-benzenesulfonyl)-3-methyl-thiophen-2-carbonsäure-[4-(dimethylamino-phenyl-methyl)-cyclohexyl]-amid
(234) N-[4-(Dimethylamino-phenyl-methyl)-cyclohexyl]-2-phenyl-butyramid (unpolareres Diastereomer)
(235) 5-Brom-N-[4-(dimethylamino-phenyl-methyl)-cyclohexyl]-nicotinamid
(236) Adamantan-1-carbonsäure-[4-(dimethylamino-phenyl-methyl)-cyclohexyl]-amid
(237) 2-Phenyl-thiazol-4--carbonsäure-[4-(dimethylamino-phenyl-methyl)-cyclohexyl]-amid
(238) 4-Methyl-2-phenyl-thiazol-5--carbonsäure-[4-(dimethylamino-phenyl-methyl)-cyclohexyl]-amid
(239) 2-(2,3-Dihydro-benzofuran-5-yl)-thiazol-4-carbonsäure-[4-(dimethylamino-thiophen-2-yl-methyl)-cyclohexyl]-amid
(240) N-{4-[Dimethylamino-(3-fluor-phenyl)-methyl]-cyclohexyl}-2-phenyl-acetamid
(241) 3-Chlor-N-[4-(1-dimethylamino-3-phenyl-propyl)-cyclohexyl]-benzamid
(242) N-[4-(Dimethylamino-phenyl-methyl)-cyclohexyl]-4-methyl-benzamid
(243) 3,5-Dichlor-N-{4-[dimethylamino-(3-fluor-phenyl)-methyl]-cyclohexyl}-benzamid
(244) N-[4-(Dimethylamino-phenyl-methyl)-cyclohexyl]-2,3,6-trifluor-benzamid
(245) Thiophen-2-carbonsäure-[4-(dimethylamino-phenyl-methyl)-cyclohexyl]-amid (unpolareres Diastereomer)
(246) N-[4-(Dimethylamino-phenyl-methyl)-cyclohexyl]-3,3-dimethyl-butyramid (unpolareres Diastereomer)
(247) 2-tert-Butyl-5-methyl-2H-pyrazol-3-carbonsäure-[4-(dimethylamino-phenyl-methyl)-cyclohexyl]-amid
(248) N-[4-(1-Dimethylamino-3-phenyl-propyl)-cyclohexyl]-3-methyl-benzamid
(249) Thiophen-2-carbonsäure-[4-(dimethylamino-phenyl-methyl)-cyclohexyl]-amid (polareres Diastereomer)
(250) N-[4-(Dimethylamino-phenyl-methyl)-cyclohexyl]-2-phenyl-butyramid (polareres Diastereomer)
(251) N-{4-[Dimethylamino-(3-fluor-phenyl)-methyl]-cyclohexyl}-3,3-dimethyl-butyramid
(252) 3-Chlor-4-methanesulfonyl-thiophen-2-carbonsäure-[4-(dimethylamino-phenyl-methyl)-cyclohexyl]-amid
(253) 4-(4-Chlor-benzenesulfonyl)-3-methyl-thiophen-2-carbonsäure-[4-(dimethylamino-phenyl-methyl)-cyclohexyl]-amid
(254) 2-Benzyloxy-N-[4-(dimethylamino-phenyl-methyl)-cyclohexyl]-acetamid
(255) N-[4-(Dimethylamino-phenyl-methyl)-cyclohexyl]-2-thiophen-2-yl-acetamid
(256) 4-Methyl-2-phenyl-thiazol-5-carbonsäure-{4-[dimethylamino-(3-fluor-phenyl)-methyl]-cyclohexyl}-amid
(257) 2-(4-Chlor-phenoxy)-N-[4-(dimethylamino-thiophen-2-yl-methyl)-cyclohexyl]-nicotinamid
(258) N-[4-(1-Dimethylamino-3-phenyl-propyl)-cyclohexyl]-4-fluor-benzamid
(259) 5-Brom-N-[4-(dimethylamino-phenyl-methyl)-cyclohexyl]-nicotinamid
(260) 4-Brom-2-ethyl-5-methyl-2H-pyrazol-3-ccarbonsäure-[4-(dimethylamino-thiophen-2-yl-methyl)-cyclohexyl]-amid
(261) 3-Cyano-N-[4-(dimethylamino-phenyl-methyl)-cyclohexyl]-benzamid
(262) N-{4-[Dimethylamino-(4-fluor-phenyl)-methyl]-cyclohexyl}-4-fluor-benzamid

(263) 3-Brom-N-{4-[dimethylamino-(4-fluor-phenyl)-methyl]-cyclohexyl}-benzamid
(264) 2-Phenyl-thiazol-4-carbonsäure-{4-[dimethylamino-(3-fluor-phenyl)-methyl]-cyclohexyl}-amid
(265) 2,5-Dimethyl-furan-3-carbonsäure-{4-[dimethylamino-(3-fluor-phenyl)-methyl]-cyclohexyl}-amid
(266) 2-Methyl-5-phenyl-furan-3-carbonsäure-{4-[dimethylamino-(3-fluor-phenyl)-methyl]-cyclohexyl}-amid
(267) 5-Pyridin-2-yl-thiophen-2-carbonsäure-{4-[dimethylamino-(3-fluor-phenyl)-methyl]-cyclohexyl}-amid
(268) 4-Brom-2-ethyl-5-methyl-2H-pyrazol-3-carbonsäure-[4-(dimethylamino-phenyl-methyl)-cyclohexyl]-amid
(269) 3-Chlor-N-{4-[dimethylamino-(3-fluor-phenyl)-methyl]-cyclohexyl}-4-fluor-benzamid
(270) 3,4-Dichlor-N-{4-[dimethylamino-(4-fluor-phenyl)-methyl]-cyclohexyl}-benzamid
(271) N-{4-[Dimethylamino-(4-fluor-phenyl)-methyl]-cyclohexyl}-2,4,5-trifluor-benzamid
(272) Cyclohexancarbonsäure-[4-(dimethylamino-phenyl-methyl)-cyclohexyl]-amid
(273) N-{4-[Dimethylamino-(3-fluor-phenyl)-methyl]-cyclohexyl}-2-phenyl-butyramid
(274) 2-(4-Chlor-phenyl)-N-{4-[dimethylamino-(3-fluor-phenyl)-methyl]-cyclohexyl}-acetamid
(275) N-[4-(Dimethylamino-phenyl-methyl)-cyclohexyl]-3-nitro-benzamid
(276) N-[4-(1-Dimethylamino-3-phenyl-propyl)-cyclohexyl]-2,5-difluor-benzamid
(277) 3-Brom-N-[4-(1-dimethylamino-3-phenyl-propyl)-cyclohexyl]-benzamid
(278) N-{4-[Dimethylamino-(3-fluor-phenyl)-methyl]-cyclohexyl}-2,6-difluor-benzamid
(279) 2,5-Dimethyl-furan-3-carbonsäure-[4-(dimethylamino-phenyl-methyl)-cyclohexyl]-amid
(280) 3-Chlor-N-{4-[dimethylamino-(4-fluor-phenyl)-methyl]-cyclohexyl}-4-fluor-benzamid
(281) N-[4-(Dimethylamino-phenyl-methyl)-cyclohexyl]-5-fluor-2-trifluormethyl-benzamid
(282) 5-Methyl-isoxazol-3-carbonsäure-[4-(dimethylamino-phenyl-methyl)-cyclohexyl]-amid
(283) 2-(2,3-Dihydro-benzofuran-5-yl)-4-methyl-thiazol-5-carbonsäure-[4-(dimethylamino-thiophen-2-yl-methyl)-cyclohexyl]-amid
(284) 2-(4-Chlor-phenoxy)-N-[4-(dimethylamino-phenyl-methyl)-cyclohexyl]-acetamid
(285) 5-(4-Chlor-phenyl)-2-methyl-furan-3-carbonsäure-{4-[dimethylamino-(3-fluorphenyl)-methyl]-cyclohexyl}-amid
(286) 2-Brom-N-[4-(dimethylamino-phenyl-methyl)-cyclohexyl]-benzamid
(287) N-[4-(Dimethylamino-phenyl-methyl)-cyclohexyl]-2,6-dimethoxy-benzamid
(288) Cyclopentancarbonsäure-[4-(dimethylamino-phenyl-methyl)-cyclohexyl]-amid
(289) 2-(2,3-Dihydro-benzofuran-5-yl)-thiazol-4-carbonsäure-[4-(dimethylaminophenyl-methyl)-cyclohexyl]-amid
(290) Benzo[1,2,5]thiadiazol-5-carbonsäure-{4-[dimethylamino-(3-fluor-phenyl)-methyl]-cyclohexyl}amid
(291) N-[4-(Dimethylamino-thiophen-2-yl-methyl)-cyclohexylmethyl]-2-thiophen-2-yl-acetamid
(292) Benzo[b]thiophen-3-carbonsäure-[4-(dimethylamino-phenyl-methyl)-cyclohexylmethyl]-amid
(293) 5-Chlor-4-methoxy-thiophen-3-carbonsäure-[4-(dimethylamino-phenyl-methyl)-cyclohexylmethyl]-amid
(294) N-[4-(Dimethylamino-phenyl-methyl)-cyclohexylmethyl]-3,4-difluor-benzamid (unpolareres Diastereomer)
(295) N-{4-[Dimethylamino-(3-fluor-phenyl)-methyl]-cyclohexylmethyl}-3,3-dimethyl-butyramid
(296) 2-Brom-N-[4-(dimethylamino-thiophen-2-yl-methyl)-cyclohexylmethyl]-benzamid
(297) N-[4-(Dimethylamino-thiophen-2-yl-methyl)-cyclohexylmethyl]-2,2-diphenyl-acetamid
(298) N-[4-(Dimethylamino-phenyl-methyl)-cyclohexylmethyl]-3,3-dimethyl-butyramid
(299) N-[4-(Dimethylamino-phenyl-methyl)-cyclohexylmethyl]-2-methylsulfanyl-nicotinamid
(300) N-[4-(Dimethylamino-thiophen-2-yl-methyl)-cyclohexylmethyl]-2,6-dimethoxy-benzamid
(301) N-[4-(Dimethylamino-thiophen-2-yl-methyl)-cyclohexylmethyl]-3,3-dimethyl-butyramid
(302) Benzo[b]thiophen-3-carbonsäure-[4-(dimethylamino-thiophen-2-yl-methyl)-cyclohexylmethyl]-amid
(303) 5-Chlor-4-methoxy-thiophen-3-carbonsäure-{4-[dimethylamino-(3-fluor-phenyl)-methyl]-cyclohexylmethyl}-amid
(304) N-[4-(Dimethylamino-thiophen-2-yl-methyl)-cyclohexylmethyl]-2-phenoxy-propionamid
(305) N-[4-(Dimethylamino-thiophen-2-yl-methyl)-cyclohexylmethyl]-2-methoxy-benzamid
(306) N-[4-(Dimethylamino-phenyl-methyl)-cyclohexylmethyl]-2-phenyl-acetamid
(307) 3-Brom-N-[4-(dimethylamino-thiophen-2-yl-methyl)-cyclohexylmethyl]-benzamid
(308) N-[4-(Dimethylamino-thiophen-2-yl-methyl)-cyclohexylmethyl]-3-fluor-5-trifluormethyl-benzamid
(309) N-{4-[(4-Chlor-phenyl)-dimethylamino-methyl]-cyclohexylmethyl}-3,3-dimethyl-butyramid
(310) N-[4-(Dimethylamino-phenyl-methyl)-cyclohexylmethyl]-2-ethylsulfanyl-nicotinamid
(311) 2-(4-Chlor-phenyl)-N-[4-(dimethylamino-thiophen-2-yl-methyl)-cyclohexylmethyl]-acetamid
(312) N-{4-[(4-Chlor-phenyl)-dimethylamino-methyl]-cyclohexylmethyl}-2,2-diphenyl-acetamid
(313) N-[4-(Dimethylamino-thiophen-2-yl-methyl)-cyclohexylmethyl]-2,6-difluor-benzamid
(314) Benzo[b]thiophen-3-carbonsäure-{4-[dimethylamino-(3-fluor-phenyl)-methyl]-cyclohexylmethyl}-amid
(315) N-{4-[(4-Chlor-phenyl)-dimethylamino-methyl]-cyclohexylmethyl}-2-methylsulfanyl-nicotinamid
(316) N-{4-[(4-Chlor-phenyl)-dimethylamino-methyl]-cyclohexylmethyl}-2-thiophen-2-yl-acetamid
(317) N-[4-(Dimethylamino-phenyl-methyl)-cyclohexylmethyl]-2-methyl-benzamid (unpolareres Diastereomer)

(318) 1-(4-Chlor-phenyl)-cyclopentan-carbonsäure-[4-(dimethylamino-phenyl-methyl)-cyclohexylmethyl]-amid
(319) N-{4-[Dimethylamino-(3-fluor-phenyl)-methyl]-cyclohexylmethyl}-2-phenyl-acetamid (polareres Diastereomer)
(320) N-{4-[(4-Chlor-phenyl)-dimethylamino-methyl]-cyclohexylmethyl}-2-(3-methoxyphenyl)-acetamid
(321) N-[4-(Dimethylamino-phenyl-methyl)-cyclohexylmethyl]-2-phenyl-butyramid
(322) N-{4-[Dimethylamino-(4-fluor-phenyl)-methyl]-cyclohexylmethyl}-3,3-dimethyl-butyramid
(323) N-[4-(Dimethylamino-phenyl-methyl)-cyclohexylmethyl]-2-phenoxy-propionamid
(324) 2-(4-Chlor-phenyl)-N-[4-(dimethylamino-phenyl-methyl)-cyclohexylmethyl]-acetamid
(325) N-[4-(Dimethylamino-phenyl-methyl)-cyclohexylmethyl]-2-methyl-benzamid (polareres Diastereomer)
(326) N-[4-(Dimethylamino-phenyl-methyl)-cyclohexylmethyl]-3-trifluormethyl-benzamid (unpolareres Diastereomer)
(327) 1-(4-Chlor-phenyl)-cyclopentan-carbonsäure-{4-[dimethylamino-(3-fluor-phenyl)-methyl]-cyclohexylmethyl}-amid
(328) Thiophen-2-carbonsäure-[4-(dimethylamino-phenyl-methyl)-cyclohexylmethyl]-amid
(329) 3,5-Dichlor-N-[4-(dimethylamino-phenyl-methyl)-cyclohexylmethyl]-benzamid
(330) 2-Methyl-5-phenyl-furan-3-carbonsäure-{4-[(4-chlor-phenyl)-dimethylaminomethyl]-cyclohexylmethyl}-amid
(331) 3-Chlor-N-[4-(dimethylamino-phenyl-methyl)-cyclohexylmethyl]-benzamid
(332) N-{4-[Dimethylamino-(3-fluor-phenyl)-methyl]-cyclohexylmethyl}-2-phenoxy-propionamid (polareres Diastereomer)
(333) N-[4-(Dimethylamino-phenyl-methyl)-cyclohexylmethyl]-3-trifluormethyl-b enzamid (polareres Diastereomer)
(334) Thiophen-2-carbonsäure-{4-[dimethylamino-(3-fluor-phenyl)-methyl]-cyclohexylmethyl}-amid (polareres Diastereomer)
(335) 2-Phenyl-thiazol-4-carbonsäure-[4-(dimethylamino-phenyl-methyl)-cyclohexylmethyl]-amid
(336) Benzo[b]thiophen-3-carbonsäure-{4-[(4-chlor-phenyl)-dimethylamino-methyl]-cyclohexylmethyl}-amid (unpolareres Diastereomer)
(337) N-{4-[Dimethylamino-(3-fluor-phenyl)-methyl]-cyclohexylmethyl}-2-p-tolyloxy-nicotinamid
(338) 2,4,6-Trichlor-N-[4-(dimethylamino-phenyl-methyl)-cyclohexylmethyl]-benzamid
(339) 1-(4-Chlor-phenyl)-cyclopentan-carbonsäure-[4-(dimethylamino-phenyl-methyl)-cyclohexylmethyl]-amid
(340) Thiophen-2-carbonsäure-{4-[dimethylamino-(3-fluor-phenyl)-methyl]-cyclohexylmethyl}-amid (unpolareres Diastereomer)
(341) N-{4-[Dimethylamino-(3-fluor-phenyl)-methyl]-cyclohexylmethyl}-2-phenoxy-propionamid (unpolareres Diastereomer)
(342) N-{4-[Dimethylamino-(3-fluor-phenyl)-methyl]-cyclohexylmethyl}-2-methyl-butyramid (polareres Diastereomer)
(343) N-{4-[Dimethylamino-(3-fluor-phenyl)-methyl]-cyclohexylmethyl}-2-thiophen-2-yl-acetamid
(344) Benzo[b]thiophen-3-carbonsäure-{4-[dimethylamino-(4-fluor-phenyl)-methyl]-cyclohexylmethyl}-amid
(345) 2-Methyl-5-phenyl-furan-3-carbonsäure-[4-(dimethylamino-phenyl-methyl)-cyclohexylmethyl]-amid
(346) N-{4-[Dimethylamino-(3-fluor-phenyl)-methyl]-cyclohexylmethyl}-2-phenoxy-propionamid
(347) 3-Cyano-N-[4-(dimethylamino-thiophen-2-yl-methyl)-cyclohexylmethyl]-benzamid
(348) N-{4-[Dimethylamino-(3-fluor-phenyl)-methyl]-cyclohexylmethyl}-2-phenyl-acetamid (unpolareres Diastereomer)
(349) N-[4-(Dimethylamino-phenyl-methyl)-cyclohexylmethyl]-2-(3-methoxy-phenyl)-acetamid
(350) N-{4-[Dimethylamino-(3-fluor-phenyl)-methyl]-cyclohexylmethyl}-4-fluor-3-t rifluormethyl-benzamid
(351) N-{4-[(4-Chlor-phenyl)-dimethylamino-methyl]-cyclohexylmethyl}-2-ethylsulfanyl-nicotinamid
(352) N-[4-(Dimethylamino-thiophen-2-yl-methyl)-cyclohexylmethyl]-2-p-tolyloxy-nicotinamid (polareres Diastereomer)
(353) 2-Methyl-5-phenyl-furan-3-carbonsäure-{4-[dimethylamino-(3-fluor-phenyl)-methyl]-cyclohexylmethyl}-amid
(354) 2-Chlor-N-{4-[(4-chlor-phenyl)-dimethylamino-methyl]-cyclohexylmethyl}-benzamid
(355) 2-Chlor-N-[4-(dimethylamino-phenyl-methyl)-cyclohexylmethyl]-nicotinamid
(356) N-[4-(Dimethylamino-thiophen-2-yl-methyl)-cyclohexylmethyl]-4-propyl-benzamid
(357) N-[4-(Dimethylamino-phenyl-methyl)-cyclohexylmethyl]-3,4-difluor-benzamid (polareres Diastereomer)
(358) 3-Brom-N-[4-(dimethylamino-phenyl-methyl)-cyclohexylmethyl]-benzamid
(359) N-{4-[Dimethylamino-(4-fluor-phenyl)-methyl]-cyclohexylmethyl}-2-thiophen-2-yl-acetamid
(360) 2-(4-Chlor-phenoxy)-N-[4-(dimethylamino-phenyl-methyl)-cyclohexylmethyl]-nicotinamid (unpolareres Diastereomer)
(361) 2,4-Dichlor-N-[4-(dimethylamino-phenyl-methyl)-cyclohexylmethyl]-benzamid
(362) N-[4-(Dimethylamino-phenyl-methyl)-cyclohexylmethyl]-3-methyl-benzamid
(363) 2-Brom-N-{4-[dimethylamino-(3-fluor-phenyl)-methyl]-cyclohexylmethyl}-benzamid

(364) N-{4-[Dimethylamino-(3-fluor-phenyl)-methyl]-cyclohexylmethyl}-3-trifluormethyl-benzamid
(365) 2-Phenyl-thiazol-4-carbonsäure-{4-[dimethylamino-(3-fluor-phenyl)-methyl]-cyclohexylmethyl}-amid
(366) 2-tert-Butyl-5-methyl-2H-pyrazol-3-carbonsäure-[4-(dimethylamino-thiophen-2-yl-methyl)-cyclohexylmethyl]-amid
(367) 3-Chlor-4-(propan-2-sulfonyl)-thiophen-2-carbonsäure-[4-(dimethylamino-thiophen-2-yl-methyl)-cyclohexylmethyl]-amid
(368) N-{4-[Dimethylamino-(3-fluor-phenyl)-methyl]-cyclohexylmethyl}-2-methoxy-benzamid
(369) N-{4-[(4-Chlor-phenyl)-dimethylamino-methyl]-cyclohexylmethyl}-3-trifluormethyl-benzamid
(370) 1-(4-Chlor-phenyl)-cyclopentan-carbonsäure-{4-[dimethylamino-(4-fluor-phenyl)-methyl]-cyclohexylmethyl}-amid
(371) 3,5-Dichlor-N-{4-[dimethylamino-(3-fluor-phenyl)-methyl]-cyclohexylmethyl}-benzamid
(372) Benzo[b]thiophen-3-carbonsäure-{4-[(4-chlor-phenyl)-dimethylamino-methyl]-cyclohexylmethyl}-amid (polarere Diastereomer)
(373) 2-Methyl-5-phenyl-furan-3-carbonsäure {4-[dimethylamino-(3-fluor-phenyl)-methyl]-cyclohexylmethyl}amid
(374) 2-(4-Chlor-phenylsulfanyl)-N-[4-(dimethylamino-phenyl-methyl)-cyclohexylmethyl]-nicotinamid
(375) 4-Brom-2-ethyl-5-methyl-2H-pyrazol-3-carbonsäure-[4-(dimethylamino-thiophen-2-yl-methyl)-cyclohexylmethyl]-amid
(376) 5-Chlor-4-methoxy-thiophen-3-carbonsäure-{4-[dimethylamino-(4-fluor-phenyl)-methyl]-cyclohexylmethyl}-amid
(378) N-{4-[Dimethylamino-(3-fluor-phenyl)-methyl]-cyclohexylmethyl}-2-methyl-butyramid (unpolareres Diastereomer)
(379) 5-Methyl-isoxazol-3-carbonsäure-[4-(dimethylamino-phenyl-methyl)-cyclohexylmethyl]-amid
(380) Benzo[b]thiophen-3-carbonsäure-[4-(1-dimethylamino-3-phenyl-propyl)-cyclohexylmethyl]-amid
(381) N-{4-[Dimethylamino-(4-fluor-phenyl)-methyl]-cyclohexylmethyl}-2-methylsulfanyl-nicotinamid
(382) N-{4-[(4-Chlor-phenyl)-dimethylamino-methyl]-cyclohexylmethyl}-2-p-tolyloxy-nicotinamid
(383) 2-(4-Chlor-phenoxy)-N-[4-(dimethylamino-phenyl-methyl)-cyclohexylmethyl]-nicotinamid (polareres Diastereomer)
(384) N-{4-[Dimethylamino-(3-fluor-phenyl)-methyl]-cyclohexylmethyl}-2-methyl-benzamid
(385) 5-Methyl-isoxazol-3-carbonsäure-{4-[(4-chlor-phenyl)-dimethylamino-methyl]-cyclohexylmethyl}-amid
(386) 5-Brom-N-[4-(dimethylamino-phenyl-methyl)-cyclohexylmethyl]-nicotinamid
(387) N-[4-(Dimethylamino-phenyl-methyl)-cyclohexylmethyl]-2-methyl-butyramid
(388) N-{4-[Dimethylamino-(3-fluor-phenyl)-methyl]-cyclohexylmethyl}-2-ethylsulfanyl-nicotinamid
(389) N-[4-(Dimethylamino-thiophen-2-yl-methyl)-cyclohexylmethyl]-2-p-tolyloxy-nicotinamid (unpolareres Diastereomer)
(390) 4-Brom-2-ethyl-5-methyl-2H-pyrazol-3-carbonsäure-[4-(dimethylamino-phenyl-methyl)-cyclohexylmethyl]-amid
(391) N-{4-[(4-Chlor-phenyl)-dimethylamino-methyl]-cyclohexylmethyl}-2-phenoxy-propionamid
(392) N-[4-(Dimethylamino-phenyl-methyl)-cyclohexylmethyl]-3,5-dinitro-benzamid
(393) N-{4-[(4-Chlor-phenyl)-dimethylamino-methyl]-cyclohexylmethyl}-3-methoxy-benzamid
(394) 2-Brom-N-{4-[(4-chlor-phenyl)-dimethylamino-methyl]-cyclohexylmethyl}-benzamid
(395) 2-Brom-N-(2-{4-[(4-chlor-phenyl)-dimethylamino-methyl]-cyclohexyl}-ethyl)-benzamid
(396) 2-Brom-N-(2-{4-[dimethylamino-(3-fluor-phenyl)-methyl]-cyclohexyl}-ethyl)-benzamid
(397) 3-Chlor-N-{2-[4-(dimethylamino-phenyl-methyl)-cyclohexyl]-ethyl}-benzamid (polareres Diastereomer)
(398) 3-Chlor-N-{2-[4-(dimethylamino-phenyl-methyl)-cyclohexyl]-ethyl}-benzamid (unpolareres Diastereomer)
(399) 3-Chlor-N-(2-{4-[(4-chlor-phenyl)-dimethylamino-methyl]-cyclohexyl}-ethyl)-benzamid
(400) 3-Chlor-N-(2-{4-[dimethylamino-(3-fluor-phenyl)-methyl]-cyclohexyl}-ethyl)-benzamid (unpolareres Diastereomer)
(401) 3-Chlor-N-(2-{4-[dimethylamino-(3-fluor-phenyl)-methyl]-cyclohexyl}-ethyl)-benzamid (polareres Diastereomer)
(402) 2-Chlor-N-{2-[4-(dimethylamino-phenyl-methyl)-cyclohexyl]-ethyl}-benzamid
(403) 2-Chlor-N-(2-{4-[(4-chlor-phenyl)-dimethylamino-methyl]-cyclohexyl}-ethyl)-benzamid
(404) 2-Chlor-N-(2-{4-[dimethylamino-(3-fluor-phenyl)-methyl]-cyclohexyl}-ethyl)-benzamid
(405) 4-Chlor-N-{2-[4-(dimethylamino-phenyl-methyl)-cyclohexyl]-ethyl}-benzamid
(406) 4-Chlor-N-(2-{4-[dimethylamino-(3-fluor-phenyl)-methyl]-cyclohexyl}-ethyl)-benzamid
(407) N-{2-[4-(Dimethylamino-phenyl-methyl)-cyclohexyl]-ethyl}-4-fluor-benzamid
(408) N-(2-{4-[(4-Chlor-phenyl)-dimethylamino-methyl]-cyclohexyl}-ethyl)-4-fluor-benzamid
(409) N-(2-{4-[Dimethylamino-(3-fluor-phenyl)-methyl]-cyclohexyl}-ethyl)-4-fluor-benzamid
(410) N-{2-[4-(Dimethylamino-phenyl-methyl)-cyclohexyl]-ethyl}-2-fluor-benzamid

(411) N-(2-{4-[Dimethylamino-(3-fluor-phenyl)-methyl]-cyclohexyl}-ethyl)-2-fluor-benzamid
(412) N-(2-{4-[Dimethylamino-(3-fluor-phenyl)-methyl]-cyclohexyl}-ethyl)-3-methyl-benzamid
(413) 2,6-Dichlor-N-{2-[4-(dimethylamino-phenyl-methyl)-cyclohexyl]-ethyl}-benzamid
(414) N-{2-[4-(Dimethylamino-phenyl-methyl)-cyclohexyl]-ethyl}-2-methoxy-benzamid
(415) N-(2-{4-[Dimethylamino-(3-fluor-phenyl)-methyl]-cyclohexyl}-ethyl)-2-methoxy-benzamid
(416) 3,4-Dichlor-N-{2-[4-(dimethylamino-phenyl-methyl)-cyclohexyl]-ethyl}-benzamid
(417) N-{2-[4-(Dimethylamino-phenyl-methyl)-cyclohexyl]-ethyl}-2-methyl-benzamid (polareres Diastereomer)
(418) N-{2-[4-(Dimethylamino-phenyl-methyl)-cyclohexyl]-ethyl}-2-methyl-benzamid (unpolareres Diastereomer)
(419) N-(2-{4-[(4-Chlor-phenyl)-dimethylamino-methyl]-cyclohexyl}-ethyl)-2-methyl-benzamid
(420) N-(2-{4-[Dimethylamino-(3-fluor-phenyl)-methyl]-cyclohexyl}-ethyl)-2-methyl-benzamid
(421) 4-Cyano-N-{2-[4-(dimethylamino-phenyl-methyl)-cyclohexyl]-ethyl}benzamid
(422) 3-Chlor-N-(2-{4-[dimethylamino-(4-fluor-phenyl)-methyl]-cyclohexyl}ethyl)-benzamid (polareres Diastzereomer)
(423) 3-Chlor-N-(2-{4-[dimethylamino-(4-fluor-phenyl)-methyl]-cyclohexyl}-ethyl)-benzamid (unpolareres Diastereomer)
(424) 3-Chlor-N-{2-[4-(dimethylamino-thiophen-2-yl-methyl)-cyclohexyl]-ethyl}-benzamid
(425) 2-Chlor-N-{2-[4-(dimethylamino-thiophen-2-yl-methyl)-cyclohexyl]-ethyl}-benzamid
(426) N-{2-[4-(Dimethylamino-thiophen-2-yl-methyl)-cyclohexyl]-ethyl}-4-fluor-benzamid
(427) N-(2-{4-[Dimethylamino-(4-fluor-phenyl)-methyl]-cyclohexyl}-ethyl)-2-fluor-benzamid
(428) N-{2-[4-(Dimethylamino-thiophen-2-yl-methyl)-cyclohexyl]-ethyl}-2-fluor-benzamid
(429) N-(2-{4-[Dimethylamino-(4-fluor-phenyl)-methyl]-cyclohexyl}-ethyl)-3-methyl-benzamid
(430) N-{2-[4-(Dimethylamino-thiophen-2-yl-methyl)-cyclohexyl]-ethyl}-3-methyl-benzamid
(431) 2,6-Dichlor-N-{2-[4-(dimethylamino-thiophen-2-yl-methyl)-cyclohexyl]-ethyl}-benzamid
(432) N-(2-{4-[Dimethylamino-(4-fluor-phenyl)-methyl]-cyclohexyl}-ethyl)-2-methoxy-benzamid
(433) N-{2-[4-(Dimethylamino-phenyl-methyl)-cyclohexyl]-ethyl}-3,5-difluor-benzamid
(434) N-(2-{4-[(4-Chlor-phenyl)-dimethylamino-methyl]-cyclohexyl}-ethyl)-3,5-difluor-benzamid
(435) N-(2-{4-[Dimethylamino-(3-fluor-phenyl)-methyl]-cyclohexyl}-ethyl)-3,5-difluor-benzamid
(436) N-(2-{4-[(4-Chlor-phenyl)-dimethylamino-methyl]-cyclohexyl}-ethyl)-2,4-difluor-benzamid
(437) 2,4-Dichlor-N-(2-{4-[(4-chlor-phenyl)-dimethylamino-methyl]-cyclohexyl}-ethyl)-5-fluor-benzamid
(438) 2,4-Dichlor-N-(2-{4-[dimethylamino-(3-fluor-phenyl)-methyl]-cyclohexyl}-ethyl)-5-fluor-benzamid (polareres Diastereomer)
(439) 2,4-Dichlor-N-(2-{4-[dimethylamino-(3-fluor-phenyl)-methyl]-cyclohexyl}-ethyl)-5-fluor-benzamid (unpolareres Diastereomer)
(440) 2,4-Dichlor-N-{2-[4-(dimethylamino-thiophen-2-yl-methyl)-cyclohexyl]-ethyl}-5-fluor-benzamid
(441) 2-Chlor-N-(2-{4-[(4-chlor-phenyl)-dimethylamino-methyl]-cyclohexyl}-ethyl)-nicotinamid
(442) Naphthalen-2-carbonsäure(2-{4-[dimethylamino-(4-fluor-phenyl)-methyl]-cyclohexyl}-ethyl)-amid
(443) N-{2-[4-(Dimethylamino-thiophen-2-yl-methyl)-cyclohexyl]-ethyl}-4-propyl-benzamid
(444) N-{2-[4-(Dimethylamino-phenyl-methyl)-cyclohexyl]-ethyl}-3,4-difluor-benzamid
(445) N-(2-{4-[(4-Chlor-phenyl)-dimethylamino-methyl]-cyclohexyl}-ethyl)-3,4-difluor-benzamid
(446) N-{2-[4-(Dimethylamino-thiophen-2-yl-methyl)-cyclohexyl]-ethyl}-3,4-difluor-benzamid
(447) N-(2-{4-[Dimethylamino-(4-fluor-phenyl)-methyl]-cyclohexyl}-ethyl)-3-methoxy-benzamid
(448) N-{2-[4-(Dimethylamino-phenyl-methyl)-cyclohexyl]-ethyl}-2,2-diphenyl-acetamid
(449) 1-(4-Chlor-phenyl)-cyclopentan-carbonsäure-{2-[4-(dimethylamino-phenyl-methyl)-cyclohexyl]-ethyl}-amid
(450) 2-Benzyloxy-N-{2-[4-(dimethylamino-phenyl-methyl)-cyclohexyl]-ethyl}-acetamid
(451) N-{2-[4-(Dimethylamino-phenyl-methyl)-cyclohexyl]-ethyl}-2-phenyl-acetamid
(452) Thiophen-2-carbonsäure-{2-[4-(dimethylamino-phenyl-methyl)-cyclohexyl]-ethyl}-amid
(453) N-(2-{4-[Dimethylamino-(3-fluor-phenyl)-methyl]-cyclohexyl}-ethyl)-2-(3-methoxy-phenyl)-acetamid
(454) N-{2-[4-(Dimethylamino-thiophen-2-yl-methyl)-cyclohexyl]-ethyl}-2-(3-methoxyphenyl)-acetamid
(455) N-(2-{4-[(4-Chlor-phenyl)-dimethylamino-methyl]-cyclohexyl}-ethyl)-2-phenyl-butyramid
(456) N-{2-[4-(Dimethylamino-thiophen-2-yl-methyl)-cyclohexyl]-ethyl}-2-phenyl-butyramid
(457) Benzo[b]thiophen-2-carbonsäure-{2-[4-(dimethylamino-phenyl-methyl)-cyclohexyl]-ethyl}-amid
(458) N-{2-[4-(Dimethylamino-phenyl-methyl)-cyclohexyl]-ethyl}-4-nitro-benzamid
(459) 3-Brom-N-(2-{4-[dimethylamino-(4-fluor-phenyl)-methyl]-cyclohexyl}-ethyl)-benzamid
(460) N-{2-[4-(Dimethylamino-phenyl-methyl)-cyclohexyl]-ethyl}-2,3,4,5,6-pentafluor-benzamid
(461) N-{2-[4-(Dimethylamino-phenyl-methyl)-cyclohexyl]-ethyl}-2,6-difluor-benzamid
(462) N-(2-{4-[Dimethylamino-(3-fluor-phenyl)-methyl]-cyclohexyl}-ethyl)-2,6-difluor-benzamid
(463) 2-Phenyl-thiazol-4-carbonsäure-{2-[4-(dimethylamino-phenyl-methyl)-cyclohexyl]-ethyl}-amid
(464) 2-Phenyl-thiazol-4-carbonsäure-(2-{4-[dimethylamino-(4-fluor-phenyl)-methyl]-cyclohexyl}-ethyl)-amid

(465) Benzo[b]thiophen-3-carbonsäure-{2-[4-(dimethylamino-thiophen-2-yl-methyl)-cyclohexyl]-ethyl}-amid
(466) N-{2-[4-(Dimethylamino-phenyl-methyl)-cyclohexyl]-ethyl}-2-methylsulfanyl-nicotinamid
(467) 2-Methyl-5-phenyl-furan-3-carbonsäure-{2-[4-(dimethylamino-phenyl-methyl)-cyclohexyl]-ethyl}-amid
(468) 2-(2,3-Dihydro-benzofuran-5-yl)-thiazol-4-carbonsäure-{2-(4-(dimethylaminophenyl-methyl)-cyclohexyl)-ethyl}-amid
(469) 3-(2,6-Dichlor-phenyl)-5-methyl-isoxazol-4--carbonsäure-{2-[4-(dimethylamino-thiophen-2-yl-methyl)-cyclohexyl]-ethyl}-amid
(470) 2-(4-Chlor-phenylsulfanyl)-N-{2-[4-(dimethylamino-phenyl-methyl)-cyclohexyl]-ethyl}-nicotinamid (polareres Diastereomer)
(471) 2-(4-Chlor-phenylsulfanyl)-N-{2-[4-(dimethylamino-phenyl-methyl)-cyclohexyl]-ethyl}-nicotinamid (unpolareres Diastereomer)
(472) Benzo[1,2,3]thiadiazol-5-carbonsäure-{2-[4-(dimethylamino-phenyl-methyl)-cyclohexyl]-ethyl}-amid
(473) 5-Brom-N-{2-[4-(dimethylamino-phenyl-methyl)-cyclohexyl]-ethyl}-nicotinamid
(474) 5-Chlor-4-methoxy-thiophen-3-carbonsäure-{2-[4-(dimethylamino-phenyl-methyl)-cyclohexyl]-ethyl}-amid
(475) 5-Chlor-4-methoxy-thiophen-3-carbonsäure-(2-{4-[dimethylamino-(4-fluorphenyl)-methyl]-cyclohexyl}-ethyl)-amid
(476) 5-Chlor-4-methoxy-thiophen-3-carbonsäure-{2-[4-(1-dimethylamino-3-phenylpropyl)-cyclohexyl]-ethyl}-amid
(477) 3-Cyano-N-{2-[4-(dimethylamino-phenyl-methyl)-cyclohexyl]-ethyl}-benzamid
(478) N-{2-[4-(Dimethylamino-phenyl-methyl)-cyclohexyl]-ethyl}-2,4-dimethoxy-benzamid
(479) 2-Chlor-N-((4-((dimethylamino)(phenyl)methyl)cyclohexyl)methyl)benzamid
(480) N-((4-((Dimethylamino)(phenyl)methyl)cyclohexyl)methyl)-4-fluorbenzamid
(481) N-(2-(4-((Dimethylamino)(phenyl)methyl)cyclohexyl)ethyl)-4-fluorbenzamid
(482) N-((4-((Dimethylamino)(phenyl)methyl)cyclohexyl)methyl)-2-fluorbenzamid
(483) N-((4-((Dimethylamino)(phenyl)methyl)cyclohexyl)methyl)-3-methylbenzamid
(484) N-((4-((Dimethylamino)(phenyl)methyl)cyclohexyl)methyl)-2-methoxybenzamid
(485) N-(2-(4-((dimethylamino)(phenyl)methyl)cyclohexyl)ethyl)-3,5-dimethoxybenzamid
(486) N-((4-((Dimethylamino)(3-fluorphenyl)methyl)cyclohexyl)methyl)-2,6-dimethoxybenzamid
(487) N-((4-((Dimethylamino)(phenyl)methyl)cyclohexyl)methyl)-2,4-difluorbenzamid
(488) N-((4-((Dimethylamino)(thiophen-2-yl)methyl)cyclohexyl)methyl)-3-methoxybenzamid
(489) N-((4-((Dimethylamino)(thiophen-2-yl)methyl)cyclohexyl)methyl)-3,4,5-trimethoxybenzamid
(490) 4-((Dimethylamino)(phenyl)methyl)-1-(4-fluor-3-methylphenyl)cyclohexanol

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Ein weiterer Gegenstand der Erfindung ist ein Verfahren zur Herstellung eines erfindungsgemäßen Cyclohexylmethyl-Derivates. Dabei schützt man die Ketofunktion von 4-Oxo-cyclohexancarbonsäureester,

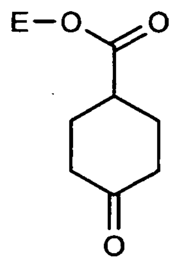

wobei E für einen $C_{1-6}$-Alkylrest, vorzugsweise Ethyl, steht nach dem Fachmann bekannten Methoden,

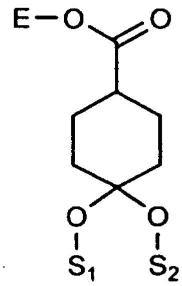

C

Wobei $S^1$ und $S^2$ jeweils für eine Schutzgruppe stehen, vorzugsweise einen Ring bilden und zusammen für $-CH_2-CH_2-$ stehen. Der Ester C wird mit einem Reduktionsmittel, beispielsweise Diisobutylaluminiumhydrid zum Aldehyd D

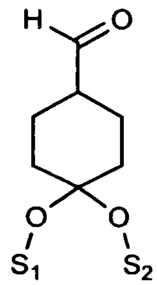

D

reduziert. Durch Zugabe eines Amins der allgemeinen Formel $R^5R^6NH$ und eines Cyanids, beispielsweise KCN oder NaCN, wird der Aldehyd D unter Zugabe einer Säure, beispielsweise Salzsäure, in einem organischen Lösungsmittel, beispielsweise Methanol oder Ethanol, zum Nitril E umgesetzt.

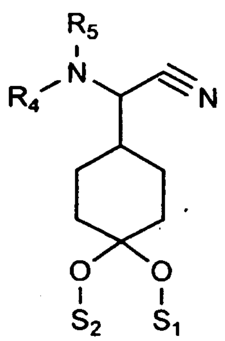

E

Das Nitril E wird mit einem Grignard-Reagenz der allgemeinen Formel $R^2MgHal$, wobei Hal für Br, Cl oder I steht, oder einer metallorganischen Verbindung der allgemeinen Formel $R^2Li$ in einem organischen Lösungsmittel, beispielsweise Diethylether, Dioxan oder Tetrahydrofuran, zu einer Verbindung der allgemeinen Formel F umgesetzt.

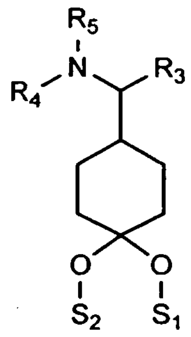

**F**

Die Schutzgruppen werden nach den üblichen Methoden abgespalten, wobei man das Keton G erhält.

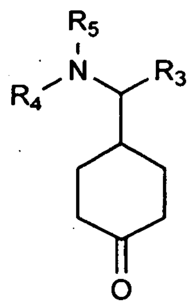

**G**

Der erfindungsgemäße Aldehyd H

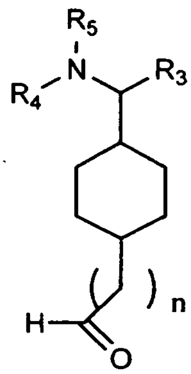

**H**

kann durch Umsetzung des Ketons **G** mit (Methoxymethyl)triphenylphosphonium-chlorid und einer starken Base, beispielsweise Kalium-tert-butylat, bei einer Temperatur von -20°C und +30°C, erhalten werden, wobei der Reaktionsschritt gegebenenfalls für Verbindungen mit n > 0 wiederholt werden kann.

Das Keton **G** bzw. die Aldehyde **H** können durch Umsetzung mit Hydroxylamin Hydrochlorid in einem organischen Lösungsmittel, beispielsweise Ethanol oder Methanol, unter Zugabe einer Base, beispielsweise einem basischen Ionenaustauscher Amberlyst zu Oximen der allgemeinen Formel **K** umgesetzt werden. Durch Umsetzung mit einem Reduktionsmittel, beispielsweise $LiAlH_4$ können die Amine der allgemeinen Formel **L** erhalten werden

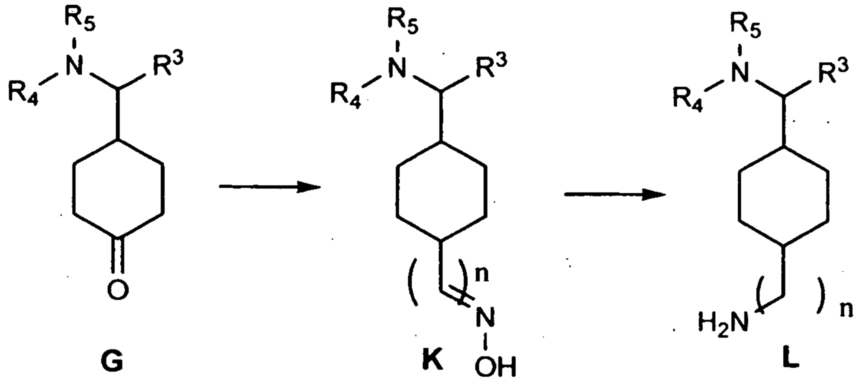

Erfindungsgemäße Substanzen der allgemeinen Formel L können zu weiteren erfindungsgemäßen Substanzen, bei denen $R^1$ $(CH_2)_nNHC(O)R^{13}$ bedeutet, nach den folgenden Methoden hergestellt werden:

Grundsätzlich sind zur Darstellung der Substanzen die vielfältigen, dem Fachmann bekannten Methoden zur Herstellung von Amiden geeignet. Das erfindungsgemäße Verfahren beruht bevorzugt darauf, substituierte Cyclohexylmethyl-Derivate der allgemeinen Formel L mit geeigneten Carbonsäuren und/oder Carbonsäurederivaten, insbesondere Carbonsäurechloriden oder - bromiden, zu verknüpfen und so in erfindungsgemäße Verbindungen, bei denen $R^1$ $(CH_2)_nNHC(O)R^{13}$ zu überführen. Bei Umsetzungen mit Säurechloriden und - bromiden werden polare oder unpolare aprotischen Lösungsmitteln eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base und als Lösungsmittel geeignet. Vorzugsweise werden Säurechloride mit Aminen bei -30 bis +40 °C in Dichlormethan oder Chloroform in Gegenwart von Triethylamin oder Pyridin und ggf. katalytischer Mengen DMAP umgesetzt.

Für die Umsetzung von Carbonsäuren mit einem substituierten Cyclohexylmethyl-Derivat der allgemeinen Formel L steht ebenfalls die gesamte Bandbreite der dem Fachmann bekannten Methoden zur Herstellung von Amiden zur Verfügung. Vorteilhaft ist dabei der Einsatz organischer oder anorganischer wasserentziehender Mittel wie z.B. Molsieb, Magnesiumsulfat, Schwefelsäure oder Carbodiimiden wie DCC oder DIC, letztere ggf. in Gegenwart von HOBt. Auch diese Umsetzungen werden vorzugsweise in polaren oder unpolaren aprotischen Lösungsmitteln bei Temperaturen zwischen -30 und +110 °C, bevorzugt -10 und +40 °C durchgeführt. Gegebenenfalls werden anschließend die Schutzgruppen abgespalten.

Erfindungsgemäße Substanzen der allgemeinen Formel L können zu weiteren erfindungsgemäßen Substanzen, bei denen $R^1$ $(CH_2)_nNHC(O)NR^{10}R^{11}$ bzw $(CH_2)_nNHC(S)NR^{10}R^{11}$ bedeutet, nach den folgenden Methoden hergestellt werden: Grundsätzlich sind zur Darstellung der Substanzen die vielfältigen, dem Fachmann bekannten Methoden zur Herstellung von Harnstoffen und Thioharnstoffen geeignet. Das erfindungsgemäße Verfahren beruht bevorzugt darauf, substituierte Cyclohexylmethyl-Derivate der allgemeinen Formel L in einem Reaktionsmedium mit geeigneten Isocyanaten der allgemeinen Formel $R^{10}$-N=C=O bzw. Isothiocyanaten der allgemeinen Formel $R^{10}$-N=C=S, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin $R^1$ $(CH_2)_nNHC(O)NR^{10}R^{11}$ oder $(CH_2)_nNHC(S)NR^{10}R^{11}$ bedeutet, und diese ggf. gereinigt und/oder isoliert wird. Diese Verbindungen, bei denen $R^{11}$ H bedeutet, können ggf in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes, beispielsweise Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-$R^{11}$, worin LG für eine Abgangsgruppe, bevorzugt für ein Halogen-Atom, besonders bevorzugt für ein Chloratom steht, und $R^{11}$ die vorstehend genannte Bedeutung mit Ausnahme von Wasserstoff hat, zu wenigstens einer Verbindung der allgemeinen Formel I umgesetzt wird, worin $R^1$ $(CH_2)_nNHC(O)NR^{10}R^{11}$ oder $(CH_2)_nNHC(S)NR^{10}R^{11}$ bedeutet, wobei $R^{11}$ nicht H bedeutet, und diese ggf. gereinigt und/oder isoliert wird.

Zur Herstellung von erfindungsgemäßen Sulfonsäureamiden der allgemeinen Formel I, wobe R1

$(CH_2)_nNHSO_2R^{12}$ bedeutet, steht grundsätzlich die gesamte Bandbreite der dem Fachmann bekannten Methoden zur Herstellung von Sulfonsäureamiden zur Verfügung. Bevorzugt werden die erfindungsgemäßen Verbindungen nach dem folgenden Verfahren hergestellt:

Das erfindungsgemäße Verfahren beruht bevorzugt darauf, substituierte Cyclohexylmethyl-Derivate der allgemeinen Formel **L** mit geeigneten Sulfonsäurederivaten, insbesondere Sulfonsäurechloriden, zu verknüpfen und so in erfindungsgemäße Verbindungen, bei denen $R^1$ $(CH_2)NHSO_2R^{12}$ zu überführen. Bei der Umsetzung von Aminen der allgemeinen Formel **L** werden polare oder unpolare aprotischen Lösungsmitteln eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base und als Lösungsmittel geeignet. Vorzugsweise werden Sulfonsäurechloride mit Aminen bei -30 bis +40 °C in Dichlormethan oder Chloroform in Gegenwart von Triethylamin oder Pyridin und ggf. katalytischer Mengen DMAP umgesetzt.

Das Keton **G** bzw. die Aldehyde **H** können durch Umsetzung mit einem Reduktionsmittel, beispielsweise Natriumborhydrid, zu erfindungsgemäßen Verbindungen umgesetzt werden, bei denen $R^1$ $(CH_2)_nOH$ bedeutet.

Ein Phosphonoessigsäureester, vorzugsweise Phosphonoessigsäure-trimethylester oder Phosphonoessigsäure-triethylester, wird zunächst mit einer starken Base, vorzugsweise Kalium-tert.butylat, Natriumhydrid oder Butyllithium, dann mit einem Keton der allgemeinen Formel G oder einem Aldehyd H oder umgesetzt. Dabei entstehen die erfindungsgemäßen $\alpha,\beta$-ungesättigte Ester.

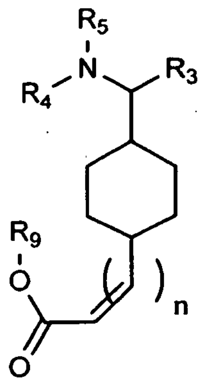

Die Ester können mit einer geeigneten wässrigen, basischen Lösung, bevorzugt mit Kaliumhydroxid- oder Lithiumhydroxid-Lösung, bei RT oder leicht erhöhter Temperatur zu den korrespondierenden Carbonsäuren hydrolysiert werden.

Die Doppelbindung kann gegebenenfalls auch reduziert werden. Hierbei wird nach dem erste Schritt, der Umsetzung mit dem Phosphonoessigsäureester, die Doppelbindung nach literaturbekannten Methoden reduziert, bevorzugt durch heterogene, katalytische Hydrierung an Palladium- oder Platin-Katalysatoren oder durch homogen katalysierte Hydrierung mit Rhodium-Katalysatoren, jeweils bei Temperaturen zwischen RT und 60°C und unter Wasserstoff-Drücken zwischen 1 bar und 6 bar, besonders bevorzugt bei RT unter einem Wasserstoffdruck zwischen 2 und 3 bar an Palladium auf Kohle. Anschließend wird wie oben beschrieben mit der Esterhydrolyse weiterverfahren. Die Ester können mit einem Reduktionsmittel, beispielsweise $LiAlH_4$, zu den entsprechenden Alkoholen reduziert werden.

Die erfindungsgemäßen Verbindungen, bei denen $R^1$ für $(CH_2)_nOR^8$ steht, können aus den Alkoholen in einem Reaktionsmedium unter Zugabe einer Base, beispielsweise NaH, durch Umsetzung mit einer Verbindung der allgemeinen Formel $R^8Hal$, wobei Hal bevorzugt für Cl steht, erhalten werden.

Für die Herstellung von erfindungsgemäßen Verbindungen, bei denen $R^1$ für $(CH_2)_nC(O)NR^{10}R^{11}$ steht, steht grundsätzlich die gesamte Bandbreite der dem Fachmann bekannten Methoden zur Herstellung von Säureamiden zur Verfügung. Bevorzugt werden die erfindungsgemäßen Verbindungen nach dem folgenden Verfahren hergestellt:

Carbonsäuren der allgemeinen Formel **J**

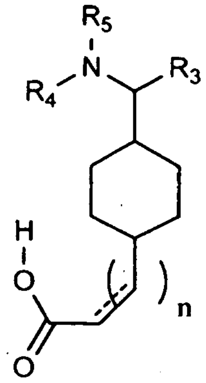

J

werden in Gegenwart wasserentziehender Mittel mit einem primären oder sekundären Aminen Amid umgesetzt. Es kann vorteilhaft sein, die Carbonsäurefunktion Cyclohexylmethylderivats vor der Darstellung des Amids durch Überführung in ein Carbonsäureäquivalent (z.B. Säurechlorid oder Aktivester) zu aktivieren.

Bei Umsetzungen mit Säurechloriden werden polare oder unpolare aprotische Lösungsmittel eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base wie auch als Lösungsmittel geeignet. Vorzugsweise werden Säurechloride mit Aminen bei -10 und +40 °C in Dichlormethan oder Chloroform in Gegenwart von Triethylamin oder Pyridin und ggf. katalytischer Mengen DMAP umgesetzt.

Für die Umsetzung der Carbonsäurefunktion mit einem weiteren Amin steht die gesamte Bandbreite der dem Fachmann bekannten Methoden zur Herstellung von Amiden zur Verfügung. Vorteilhaft ist dabei der Einsatz organischer oder anorganischer wasserentziehender Mittel wie z.B. Molsieb, Magnesiumsulfat, Schwefelsäure oder Carbodiimiden wie DCC oder DIC, letztere ggf. in Gegenwart von HOBt (1-Hydroxybenzotriazol). Auch diese Umsetzungen werden vorzugsweise in polaren oder unpolaren aprotischen Lösungsmitteln bei Temperaturen zwischen -20 und +110 °C, bevorzugt -10 und +40 °C durchgeführt.

Zur Herstellung von erfindungsgemäßen Verbindungen der allgemeinen Formel **I**, wobei $R^1$ $(CH_2)_nNHR^6$ bedeutet, steht grundsätzlich die gesamte Bandbreite der dem Fachmann bekannten Methoden zur reduktiven Aminierung zur Verfügung.

Bevorzugt werden die erfindungsgemäßen Verbindungen nach dem folgenden Verfahren hergestellt:

> Das Keton **G** bzw. die Aldehyde **H** werden in polaren, aprotischen Lösungsmitteln, bespielsweise THF gelöst und zunächst mit dem entsprechenden Aminen der allgemeinen Formel $NH_2R^6$ versetzt. Nach Zugabe von Eisessig liefert die Umsetzung mit geeigneten Reduktionsmitteln, beispielsweise Natriumborhydrid, die erfindungsgemäßen Verbindungen.

Für die Herstellung der erfindungsgemäßen Verbindungen, bei denen $R^2$ OH und $R^1$ $C_{1-8}$-Alkyl, jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert bedeutet (= $R^{1a}$), werden Ketone der allgemeinen Formel G mit metallorganischen Verbindungen der allgemeinen Formel $R^{1a}MgHal$ mit Hal = Cl oder Br bzw. $R^{1a}Li$ unter Kühlung auf -30 bis +10°C in einem organischen Lösungsmittel, beispielsweise Diethylether oder THF, umgesetzt.

Alternativ kann auch ein Aryliodid in einem organischen Lösungsmittel, beispielsweise THF, vorgelegt werden, bei einer Temperatur zwischen -30°C und 0°mit Isopropylmagnesiumchlorid-Lsg. versetzt und nach einer Rührzeit von mindestens 10 min mit dem Keton der allgemeinen Formel **G** zu Verbindungen der allgemeinen Formel I, worin $R^1$ Aryl bedeutet, umgesetzt werden.

Die gegebenenfalls bei den Synthesen anfallenden Diastereomeren können nach dem Fachmann bekannten Methoden zur Trennung von Diastereomeren getrennt werden, z. B. durch Chromatographie, insbesondere an Kieselgel, Normalphase oder Umkehrphase. Besonders geeignet zur Trennung der Diastereomeren ist RP-HPLC (mobile Phase Acetonitril/Wasser oder Methanol/Wasser).

Es hat sich gezeigt, dass die erfindungsgemäßen Substanzen nicht nur an den μ-Opioid-Rezeptor binden, sondern auch die Serotonin- und Noradrenalin-Wiederaufnahme hemmen. Noradrenalin- und Serotonin-Wiederaufnahmehemmer haben eine antidepressive und anxiolytische Wirkung, sind jedoch auch geeignet zur Behandlung von

Schmerz (Analgesics - from Chemistry and Pharmacology to Clinical Application, Wiley 2002, S. 265-284)

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes Cyclohexylmethyl-Derivat, sowie gegebenenfalls geeignete Zusatz- und/oder Hilfsstoffe und/oder gegebenenfalls weitere Wirkstoffe.

Überraschenderweise zeigen auch die Zwischenprodukte bei der Synthese der Amide, sekundären und tertiären Amide, der Grignardprodukte, Ether, Harnstoffe und Thioharnstoffe, nämlich die Oxime, Ester, primären Amine und Alkohole bereits Wirksamkeit und eignen sich daher als pharmazeutische Wirkstoffe in Arzneimitteln.

Die erfindungsgemäßen Arzneimittel enthalten neben mindestens einem erfindungsgemäßen substituierten Cyclohexylmethyl-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße

Cyclohexylmethyl-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Cyclohexylmethyl-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen Cyclohexylmethyl-Derivats appliziert.

In einer bevorzugten Form des Arzneimittels liegt ein enthaltenes erfindungsgemäßes Cyclohexylmethyl-Derivat als reines Diastereomer und/oder Enantiomer, als Razemat oder als nicht-äquimolare oder äquimolare Mischung der Diastereomere und/oder Enantiomere vor.

Ein weiterer Gegenstand der Erfindung ist die Verwendung eines erfindungsgemäßen Cyclohexylmethyl-Derivats zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen Cyclohexylmethyl-Derivats zur Herstellung eines Arzneimittels zur Behandlung von Depressionen und/oder zur Anxiolyse.

Die substituierten Cyclohexylmethyl-Derivate der allgemeinen Formel I eignen sich auch zur Behandlung von Harninkontinenz, Diarrhöe, Pruritus, Alkohol- und Drogenmißbrauch, Medikamentenabhängigkeit und Antriebslosigkeit.

Gegenstand der Erfindung ist daher auch die Verwendung eines substituierten Cyclohexylmethyl-Derivates der allgemeinen Formel I zur Herstellung eines

Arzneimittels zur Behandlung von von Harninkontinenz, Diarrhöe, Pruritus, Alkohol-und Drogenmißbrauch, Medikamentenabhängigkeit und Antriebslosigkeit.

Besonders bevorzugt werden die erfindungsgemäßen substituierten Cyclohexylmethyl-Derivate, die zur Herstellung eines Arzneimittels zur Behandlung von Schmerz, insbesondere von akutem, neuropathischem oder chronischem Schmerz, von Depressionen und/oder zur Anxiolyse, zur Behandlung von Harninkontinenz, Diarrhöe, Pruritus, Alkohol- und Drogenmißbrauch, Medikamentenabhängigkeit und Antriebslosigkeit verwendet werden, ausgewählt aus folgender Gruppe:

(16) 4-(Dimethylamino-phenyl-methyl)-cyclohexanon-oxim
(17) 4-(Dimethylamino-phenyl-methyl)-cyclohexylamin
(18) 4-[Dimethylamino-(4-fluorphenyl)-methyl]-cyclohexanon-oxim
(19) 4-[Dimethylamino-(4-fluorphenyl)-methyl]-cyclohexylamin
(20) 4-[Dimethylamino-(3-fluorphenyl)-methyl]-cyclohexanon-oxim
(21) 4-[Dimethylamino-(3-fluorphenyl)-methyl]-cyclohexylamin
(22) 4-[(4-Chlorphenyl)-dimethylamino-methyl]-cyclohexanonoxim
(23) 4-[(4-Chlorphenyl)-dimethylamino-methyl]-cyclohexylamin
(24) 4-(Dimethylamino-thiophen-2-yl-methyl)-cyclohexanonoxim
(25) 4-(Dimethylamino-thiophen-2-yl-methyl)-cyclohexylamin
(26) 4-(1-Dimethylamino-3-phenyl-propyl)-cyclohexanon oxim
(27) 4-(1-Dimethylamino-3-phenyl-propyl)-cyclohexylamin

(29) 4-(Dimethylamino-phenyl-methyl)-cyclohexan-carbaldehyd-oxim
(30) [(4-Aminomethyl-cyclohexyl)-phenyl-methyl]-dimethylamin
(32) 4-[Dimethylamino-(4-fluorphenyl)-methyl]-cyclohexanecarbaldehydoxim
(33) [(4-Aminomethyl-cyclohexyl)-(4-fluorphenyl)-methyl]-dimethylamin
(35) 4-[Dimethylamino-(3-fluorphenyl)-methyl]-cyclohexanecarbaldehydoxim
(36) [(4-Aminomethyl-cyclohexyl)-(3-fluorphenyl)-methyl]-dimethylamin
(38) 4-[(4-Chlorphenyl)-dimethylamino-methyl]-cyclohexanecarbaldehydoxim
(39) [(4-Aminomethyl-cyclohexyl)-(4-chlorphenyl)-methyl]-dimethylamin
(41) 4-(Dimethylamino-thiophen-2-yl-methyl)-cyclohexanecarbaldehydoxim
(42) [(4-Aminomethyl-cyclohexyl)-thiophen-2-yl-methyl]-dimethylamin
(44) 4-(1-Dimethylamino-3-phenyl-propyl)-cyclohexanecarbaldehydoxim
(45) [1-(4-Aminomethyl-cyclohexyl)-3-phenyl-propyl]-dimethylamin
(47) [4-(Dimethylamino-phenyl-methyl)-cyclohexyl]-acetaldehyd-oxim
(48) 2-[4-Dimethylamino-phenyl-methyl)-cyclohexyl]-ethylamin
(50) {4-[Dimethylamino-(4-fluorphenyl)-methyl]-cyclohexyl}-acetaldehydoxim
(51) 2-{4-[Dimethylamino-(4-fluorphenyl)-methyl]-cyclohexyl}-ethylamin
(53) {4-[Dimethylamino-(3-fluorphenyl)-methyl]-cyclohexyl}-acetaldehydoxim
(54) 2-{4-[Dimethylamino-(3-fluorphenyl)-methyl]-cyclohexyl}-ethylamin
(56) {4-[Dimethylamino-(4-chlorphenyl)-methyl]-cyclohexyl}-acetaldehydoxim
(66) 2-{4-[Dimethylamino-(4-chlorphenyl)-methyl]-cyclohexyl}-ethylamin
(68) 2-(4-((dimethylamino)(thiophen-2-yl)methyl)cyclohexyl)acetaldehydoxim
(69) 2-[4-(Dimethylamino-thiophen-2-yl-methyl)-cyclohexyl]-ethylamin
(71) [4-(1-Dimethylamino-3-phenyl-propyl)-cyclohexyl]-acetaldehydeoxim
(72) {1-[4-(2-Amino-ethyl)-cyclohexyl]-3-phenyl-propyl}-dimethylamin
(111) 4-[Dimethylamino-phenyl-methyl]-cyclohexanol
(112) 4-[Dimethylamino-(4-fluorphenyl)-methyl]-cyclohexanol
(113) 4-[Dimethylamino-(3-fluorphenyl)-methyl]-cyclohexanol
(114) 4-[(4-Chlorphenyl)-dimethylamino-methyl]-cyclohexanol
(115) 4-(Dimethylamino-thiophen-2-yl-methyl)-cydohexanol
(116) 4-(1-Dimethylamino-3-phenyl-propyl)-cyclohexanol
(117) [4-(Dimethylamino-phenyl-methyl)-cyclohexyl]-methanol
(118) {4-[Dimethylamino-(4-fluorphenyl)-methyl]-cyclohexyl}-methanol
(119) {4-[Dimethylamino-(3-fluorphenyl)-methyl]-cyclohexyl}-methanol
(120) {4-[(4-Chlorphenyl)-dimethylamino-methyl]-cyclohexyl}-methanol
(121) [4-(Dimethylamino-thiophen-2-yl-methyl)-cyclohexyl]-methanol
(122) [4-(1-Dimethylamino-3-phenyl-propyl)-cyclohexyl]-methanol
(123) [4-(Dimethylamino-phenyl-methyl)-cyclohexyliden]-essigsäure-ethylester
(124) [4-(Dimethylamino-phenyl-methyl)-cyclohexyl]-essigsäure-ethylester
(125) 2-[4-(Dimethylamino-phenyl-methyl)-cyclohexyl]-ethanol
(126) (4-[Dimethylamino-(4-fluorphenyl)-methyl]-cyclohexyliden)-essigsäure-ethylester
(127) {4-[Dimethylamino-(4-fluorphenyl)-methyl]-cyclohexyl}-essigsäure-ethylester
(128) 2-{4-[Dimethylamino-(4-fluorphenyl)-methyl]-cyclohexyl}-ethanol
(129) {4-[Dimethylamino-(3-fluorphenyl)-methyl]-cyclohexyliden}-essigsäure-ethylester
(130) {4-[Dimethylamino-(3-fluorphenyl)-methyl]-cyclohexyl}-essigsäure-ethylester
(131) 2-{4-[Dimethylamino-(3-fluorphenyl)-methyl]-cyclohexyl}-ethanol
(132) 3-[4-(Dimethylamino-phenyl-methyl)-cyclohexyl]-acrylsäure-ethylester
(133) 3-[4-(Dimethylamino-phenyl-methyl)-cyclohexyl]-propionsäureethylester
(134) 3-[4-(Dimethylamino-phenyl-methyl)-cydohexyl]-propan-1-ol
(135) 3-{4-[Dimethylamino-(4-fluorphenyl)-methyl]-cyclohexyl}-acrylsäureethylester
(136) 3-{4-[Dimethylamino-(4-fluorphenyl)-methyl]-cyclohexyl}-propionsäureethyl-ester
(137) 3-{4-[Dimethylamino-(4-fluorphenyl)-methyl]-cyclohexyl}-propan-1-ol
(138) 3-{4-[Dimethylamino-(3-fluorphenyl)-methyl]-cyclohexyl}-acrylsäure-ethylester
(139) 3-{4-[Dimethylamino-(3-fluorphenyl)-methyl]-cyclohexyl}propionsäureethyl-ester
(140) 3-{4-[Dimethylamino-(3-fluorphenyl)-methyl]-cyclohexyl}-propan-1-ol
(141) 3-[4-(1-Dimethylamino-3-phenyl-propyl)-cyclohexyl]-acrylsäureethylester
(142) 3-[4-(1-Dimethylamino-3-phenyl-propyl)-cyclohexyl]-propionsäureethylester
(143) 3-[4-(1-Dimethylamino-3-phenyl-propyl)-cyclohexyl]-propan-1-ol
(73) 1-(4-((Dimethylamino)(phenyl)methyl)cyclohexyl)-3-(naphthalen-1-yl)hamstoff

(74) 1-(2,4-Difluorphenyl)-3-(4-((dimethylamino)(phenyl)methyl)cyclohexyl)harnstoff Hydrochlorid
(75) 1-(4-((Dimethylamino)(phenyl)methyl)cyclohexyl)-3-(3-(trifluormethyl)phenyl)harnstoff Hydrochlorid
(76) 1-(4-((Dimethylamino)(phenyl)methyl)cyclohexyl)-3-(2-nitrophenyl)harnstoff Hydrochlorid
(77) 1-(3-Bromphenyl)-3-(4-((dimethylamino)(phenyl)methyl)cyclohexyl)harnstoff Hydrochlorid
(78) 1-(4-((Dimethylamino)(phenyl)methyl)cyclohexyl)-3-phenylhamstoff Hydrochlorid
(79) 1-Benzyl-3-(4-((dimethylamino)(phenyl)methyl)cyclohexyl)harnstoff
(80) 1-Cyclohexyl-3-(4-((dimethylamino)(phenyl)methyl)cyclohexyl)hamstoff
(81) 1-(4-Bromphenyl)-3-(4-((dimethylamino)(phenyl)methyl)cyclohexyl)harnstoff
(82) 1-(4-((Dimethylamino)(phenyl)methyl)cyclohexyl)-3-(4-methoxyphenyl)harnstoff
(83) N-(2-(1H-Indol-3-yl)ethyl)-4-((dimethylamino)(phenyl)methyl)cyclohexanamin hydrochlorid
(84) 4-((Dimethylamino)(phenyl)methyl)-N-phenethylcyclohexanamin Hydrochlorid
(85) 4-((Dimethylamino)(phenyl)methyl)-N-(3-phenylpropyl)cyclohexanamin Dihydrochlorid
(86) N-Benzyl-4-((dimethylamino)(phenyl)methyl)cyclohexanamin Hydrochlorid
(87) 4-((Dimethylamino)(phenyl)methyl)-N-(4-phenylbutyl)cyclohexanamin Hydrochlorid
(88) N-(1-(1H-Indol-3-yl)propan-2-yl)-4-((dimethylamino)(phenyl)methyl)-cyclohexanamin Hydrochlorid
(89) N-(4-((Dimethylamino)(phenyl)methyl)cyclohexyl)-4-methoxybenzenamin Hydrochlorid
(90) 4-((Dimethylamino)(phenyl)methyl)-N-(4-methoxybenzyl)cyclohexanamin Dihydrochlorid
(91) 4-((Dimethylamino)(phenyl)methyl)-N-(4-fluorbenzyl)cyclohexanamin Hydrochlorid
(92) N-(4-((Dimethylamino)(phenyl)methyl)cyclohexyl)benzenamin Hydrochlorid
(93) N-(4-((Dimethylamino)(phenyl)methyl)cyclohexyl)-2-ethylbutanamid Hydrochlorid
(94) N-(4-((Dimethylamino)(phenyl)methyl)cyclohexyl)benzamid Hydrochlorid
(95) N-(4-((Dimethylamino)(phenyl)methyl)cyclohexyl)-N-(3-phenylpropyl)acetamid Hydrochlorid
(96) N-(4-((Dimethylamino)(phenyl)methyl)cyclohexyl)-N-phenylacetamid Hydrochlorid
(97) N-(4-((Dimethylamino)(phenyl)methyl)cyclohexyl)-N-(4-phenylbutyl)propionamid Hydrochlorid
(98) N-(4-((Dimethylamino)(phenyl)methyl)cyclohexyl)-N-(4-phenylbutyl)acetamid Hydrochlorid
(99) N-(4-((Dimethylamino)(phenyl)methyl)cyclohexyl)-N-(4-methoxyphenyl)acetamid Hydrochlorid
(100) N-Benzyl-N-(4-((dimethylamino)(phenyl)methyl)cyclohexyl)acetamid Hydrochlorid
(101) N-Benzyl-N-(4-((dimethylamino)(phenyl)methyl)cyclohexyl)-2-ethylbutanamid Hydrochlorid
(102) N-Benzyl-N-(4-((dimethylamino)(phenyl)methyl)cyclohexyl)butyramid Hydrochlorid
(103) N-Benzyl-N-(4-((dimethylamino)(phenyl)methyl)cyclohexyl)-4-fluorbenzamid Hydrochlorid
(104) N-Benzyl-N-(4-((dimethylamino)(phenyl)methyl)cyclohexyl)benzamid Hydrochlorid
(105) N-(4-((Dimethylamino)(phenyl)methyl)cyclohexyl)-2-ethyl-N-phenylbutanamid Hydrochlorid
(106) 4-Chlor-N-(4-((dimethylamino)(phenyl)methyl)cyclohexyl)benzolsulfonamid . Hydrochlorid
(107) N-(4-((Dimethylamino)(phenyl)methyl)cyclohexyl)-4-methoxybenzolsulfonamid Hydrochlorid
(108) 4-tert-Butyl-N-(4-((dimethylamino)(phenyl)methyl)cyclohexyl)benzolsulfonamid Hydrochlorid
(109) N-(4-((Dimethylamino)(phenyl)methyl)cyclohexyl)-2-nitrobenzolsulfonamid Hydrochlorid
(110) N-(4-((Dimethylamino)(phenyl)methyl)cyclohexyl)benzolsulfonamid Hydrochlorid
(144) 4-(benzyloxy)cyclohexyl)-N,N-dimethyl(phenyl)methanamin Hydrochlorid
(145) 4-(4-Fluorbenzyloxy)cyclohexyl)-N,N-dimethyl(phenyl)methanamin Hydrochlorid
(146) trans-N,N-dimethyl(4-phenethylcyclohexyl)(phenyl)methanamin Hydrochlorid
(147) 1-Benzyl-4-((dimethylamino)(phenyl)methyl)cyclohexanol Hydrochlorid
(148) 4-((dimethylamino)(phenyl)methyl)-1-(4-fluorbenzyl)cyclohexanol Hydrochlorid
(149) 1-(2,5-Dimethoxyphenyl)-4-((dimethylamino)(phenyl)methyl)cyclohexanol
(150) 4-((Dimethylamino)(phenyl)methyl)-1-(4-fluor-3-methylphenyl)cyclohexanol
(151) 4-(Dimethylamino-phenyl-methyl)-1-(4-fluor-3-methyl-phenyl)-cyclohexanol
(152) 1-Benzyl-4-[dimethylamino-(3-fluor-phenyl)-methyl]-cyclohexanol
(153) 4-[Dimethylamino-(3-fluor-phenyl)-methyl]-1-phenethyl-cyclohexanol
(154) 4-[Dimethylamino-(3-fluor-phenyl)-methyl]-1-pentyl-cyclohexanol
(155) 1-(3,5-Dichlor-phenyl)-4-[dimethylamino-(3-fluor-phenyl)-methyl]-cyclohexanol
(156) 4-[Dimethylamino-(3-fluor-phenyl)-methyl]-1-(3-methoxy-benzyl)-cyclohexanol
(157) 1-(4-Chlor-3-trifluormethyl-phenyl)-4-[dimethylamino-(3-fluor-phenyl)-methyl]-cyclohexanol
(158) 4-[(4-Chlor-phenyl)-dimethylamino-methyl]-1-phenyl-cyclohexanol
(159) 1-Benzyl-4-[(4-chlor-phenyl)-dimethylamino-methyl]-cyclohexanol
(160) 4-[(4-Chlor-phenylpdimethylamino-methyl]-1-(4-fluor-3-methyl-phenyl)-cyclohexanol
(161) 4-[(4-Chlor-phenyl)-dimethylamino-methyl]-1-o-tolyl-cyclohexanol
(162) 4-[(4-Chlor-phenyl)-dimethylamino-methyl]-1-(4-fluor-phenyl)-cyclohexanol
(163) 4-[(4-Chlor-phenyl)-dimethylamino-methyl]-1-phenethyl-cyclohexanol
(164) 4-[(4-Chlor-phenyl)-dimethylamino-methyl]-1-(3-methoxy-phenyl)-cyclohexanol

(165) 4-[(4-Chlor-phenyl)-dimethylamino-methyl]-1-p-tolyl-cyclohexanol
(166) 4-[(4-Chlor-phenyl)-dimethylamino-methyl]-1-(3,5-difluor-phenyl)-cyclohexanol
(167) 1-Butyl-4-[(4-chlor-phenyl)-dimethylamino-methyl]-cyclohexanol
(168) 4-[(4-Chlor-phenyl)-dimethylamino-methyl]-1-hexyl-cyclohexanol
(169) 4-[(4-Chlor-phenyl)-dimethylamino-methyl]-1-pentyl-cyclohexanol (polareres Diastereomer)
(170) 4-[(4-Chlor-phenyl)-dimethylamino-methyl]-1-pentyl-cyclohexanol (unpolareres Diastereomer)
(171) 4-[(4-Chlor-phenyl)-dimethylamino-methyl]-1-(3-fluor-phenyl)-cyclohexanol
(172) 4-[(4-Chlor-phenyl)-dimethylamino-methyl]-1-(4-fluor-benzyl)-cyclohexanol
(173) 4-[(4-Chlor-phenyl)-dimethylamino-methyl]-1-(3-methoxy-benzyl)-cyclohexanol
(174) Methyl 2-(4-((dimethylamino)(phenyl)methyl)cyclohexylamino)-3-(1 H-indol-3-yl)propanoat (polareres Diastereomer)
(175) Methyl2-(4-((dimethylamino)(phenyl)methyl)cyclohexylamino)-3-(1H-indol-3-yl)propanoat (unpolareres Diastereomer)
(176) N-(4-((Dimethylamino)(phenyl)methyl)cyclohexyl)-N-(4-methoxybenzyl)acetamid
(177) N-(1-(1H-indol-3-yl)propan-2-yl)-N-(4-(dimethylamino)(phenyl)methyl)cyclohexyl)acetamid (polareres Diastereomer)
(178) N-(1-(1H-indol-3-yl)propan-2-yl)-N-(4-((dimethylamino)(phenyl)methyl)cyclohexyl)acetamid (unpolareres Diastereomer)
(179) N-(4-((Dimethylamino)(phenyl)methyl)cyclohexyl)-N-(4-fluorbenzyl)acetamid
(180) N-(4-((Dimethylamino)(phenyl)methyl)cyclohexyl)-N-phenylbutyramid
(181) N-(2-(1 H-indol-3-yl)ethyl)-N-(4-((dimethylamino)(phenyl)methyl)-cyclohexyl)butyramid
(182) N-(2-(1H-indol-3-yl)ethyl)-N-(4-((dimethylamino)(phenyl)methyl)cyclohexyl)acetamid
(183) Benzo[b]thiophen-3-carbonsäure-[4-(dimethylamino-phenyl-methyl)-cyclohexyl]-amid
(184) 1-(4-Chlor-phenyl)-cyclopentancarbonsäure-[4-(dimethylamino-phenyl-methyl)-cyclohexyl]-amid
(185) N-[4-(Dimethylamino-thiophen-2-yl-methyl)-cyclohexyl]-4-propyl-benzamid
(186) 3-Cyano-N-[4-(dimethylamino-thiophen-2-yl-methyl)-cyclohexyl]-benzamid
(187) 3-Chlor-N-[4-(dimethylamino-phenyl-methyl)-cyclohexyl]-benzamid
(188) 5-Chlor-4-methoxy-thiophen-3-carbonsäure-[4-(dimethylamino-phenyl-methyl)-cyclohexyl]-amid
(189) 3,4-Dichlor-N-[4-(dimethylamino-phenyl-methyl)-cyclohexyl]-benzamid
(190) N-[4-(Dimethylamino-phenyl-methy)-cyclohexyl]-3-fluor-5-trifluormethyl-benzamid
(191) 5-Chlor-4-methoxy-thiophen-3-carbonsäure-{4-[dimethylamino-(3-fluor-phenyl)-methyl]-cyclohexyl}-amid
(192) N-[4-(Dimethylamino-thiophen-2-yl-methyl)-cyclohexyl]-4-fluor-3-trifluormethyl-benzamid
(193) Thiophen-2-corbonsäure-[4-(dimethylamino-thiophen-2-yl-methyl)-cyclohexyl]-amid
(194) 3,5-Dichlor-N-[4-(dimethylamino-thiophen-2-yl-methyl)-cyclohexyl]-benzamid
(195) 5-Chlor-4-methoxy-thiophen-3-carbonsäure-[4-(dimethylamino-thiophen-2-yl-methyl)-cyclohexyl]-amid
(196) N-[4-(Dimethylamino-phenyl-methyl)-cyclohexyl]-2,4,5-trifluor-benzamid
(197) 3-Brom-N-[4-(dimethylamino-phenyl-methyl)-cyclohexyl]-benzamid
(198) N-[4-(Dimethylamino-thiophen-2-yl-methyl)-cyclohexyl]-4-methyl-benzamid
(199) N-[4-(Dimethylamino-thiophen-2-yl-methyl)-cyclohexyl]-3-methoxy-benzamid
(200) N-[4-(Dimethylamino-thiophen-2-yl-methyl)-cyclohexyl]-3,3-dimethyl-butyramid
(201) 2-tert-Butyl-5-methyl-2H-pyrazol-3-ccarbonsäure-[4-(dimethylamino-thiophen-2-yl-methyl)-cyclohexyl]-amid
(202) N-[4-(Dimethylamino-thiophen-2-yl-methyl)-cyclohexyl]-2,4-dimethoxy-benzamid
(203) N-[4-(Dimethylamino-phenyl-methyl)-cyclohexyl]-3-trifluormethyl-benzamid
(204) N-{4-[Dimethylamino-(3-fluor-phenyl)-methyl]-cyclohexyl}-3,5-difluor-benzamid
(205) N-[4-(Dimethylamino-phenyl-methyl)-cyclohexyl]-2-fluor-5-trifluormethyl-benzamid
(206) 2-(4-Chlor-phenyl)-N-[4-(dimethylamino-phenyl-methyl)-cyclohexyl]-acetamid
(207) N-[4-(Dimethylamino-thiophen-2-yl-methyl)-cyclohexyl]-2-methoxy-benzamid
(208) N-[4-(Dimethylamino-thiophen-2-yl-methyl)-cyclohexyl]-2-methylsulfanyl-nicotinamid
(209) 3,4-Dichlor-N-{4-[dimethylamino-(3-fluor-phenyl)-methyl]-cyclohexyl}-benzamid
(210) N-[4-(1-Dimethylamino-3-phenyl-propyl)-cyclohexyl]-4-fluor-3-trifluormethyl-benzamid (polareres Diastereomer)
(211) 5-Chlor-4-methoxy-thiophen-3-carbonsäure-{4-[dimethylamino-(4-fluor-phenyl)-methyl]-cyclohexyl}-amid
(212) N-[4-(Dimethylamino-thiophen-2-yl-methyl)-cyclohexyl]-3,4,5-trimethoxy-benzamid
(213) N-[4-(Dimethylamino-thiophen-2-yl-methyl)-cyclohexyl]-2-ethylsulfanyl-nicotinamid
(214) 2-Methyl-5-phenyl-furan-3-carbonsäure-[4-(dimethylamino-phenyl-methyl)-cyclohexyl]-amid
(215) N-{4-[Dimethylamino-(3-fluor-phenyl)-methyl]-cyclohexyl}-2-phenoxy-propionamid unpolareres Diastereomer)
(216) N-[4-(Dimethylamino-phenyl-methyl)-cyclohexyl]-2,4-dimethoxy-benzamid

(217) 4-tert-Butyl-N-[4-(dimethylamino-phenyl-methyl)-cyclohexyl]-benzamid
(218) 2-(4-Chlor-phenylsulfanyl)-N-[4-(dimethylamino-thiophen-2-yl-methyl)-cyclohexyl]-nicotinamid
(219) 2-(4-Chlor-phenyl)-N-[4-(dimethylamino-thiophen-2-yl-methyl)-cyclohexyl]-acetamid
(220) N-[4-(Dimethylamino-thiophen-2-yl-methyl)-cyclohexyl]-2-p-tolyloxy-nicotinamid
(221) 3-Chlor-4-(propan-2-sulfonyl)-thiophen-2-carbonsäure-[4-(dimethylamino-thiophen-2-yl-methyl)-cyclohexyl]-amid
(222) N-{4-[Dimethylamino-(3-fluor-phenyl)-methyl]-cyclohexyl}-2-phenoxy-propionamid (polareres Diastereomer)
(223) 2-tert-Butyl-5-methyl-2H-pyrazol-3-carbonsäure-[4-(dimethylamino-phenyl-methyl)-cyclohexyl]-amid
(224) 5-Methyl-isoxazol-3-carbonsäure-[4-(dimethylamino-thiophen-2-yl-methyl)-cyclohexyl]-amid
(225) 5-Brom-N-[4-(dimethylamino-thiophen-2-yl-methyl)-cyclohexyl]-nicotinamid
(226) Naphthyl-1-carbonsäure-[4-(dimethylamino-phenyl-methyl)-cyclohexyl]-amid
(227) N-[4-(1-Dimethylamino-3-phenyl-propyl)-cyclohexyl]-4-fluor-3-trifluormethyl-benzamid (unpolareres Diastereomer)
(228) N-[4-(Dimethylamino-phenyl-methyl)-cyclohexyl]-3,3-dimethyl-butyramid (polareres Diastereomer)
(229) 5-(4-Chlor-phenyl)-2-methyl-furan-3-carbonsäure-[4-(dimethylamino-thiophen-2-yl-methyl)-cyclohexyl]-amid
(230) N-[4-(Dimethylamino-phenyl-methyl)-cyclohexyl]-2-phenoxy-propionamid
(231) Benzo[b]thiophen-2-carbonsäure-{4-[dimethylamino-(3-fluor-phenyl)-methyl]-cyclohexyl}-amid
(232) 5-(4-Chlor-phenyl)-2-methyl-furan-3-carbonsäure-[4-(dimethylamino-phenyl-methyl)-cyclohexyl]-amid
(233) 4-(4-Chlor-benzenesulfonyl)-3-methyl-thiophen-2-carbonsäure-[4-(dimethylamino-phenyl-methyl)-cyclohexyl]-amid
(234) N-[4-(Dimethylamino-phenyl-methyl)-cyclohexyl]-2-phenyl-butyramid (unpolareres Diastereomer)
(235) 5-Brom-N-[4-(dimethylamino-phenyl-methyl)-cyclohexyl]-nicotinamid
(236) Adamantan-1-carbonsäure-[4-(dimethylamino-phenyl-methyl)-cyclohexyl]-amid
(237) 2-Phenyl-thiazol-4-carbonsäure-[4-(dimethylamino-phenyl-methyl)-cyclohexyl]-amid
(238) 4-Methyl-2-phenyl-thiazol-5-carbonsäure-[4-(dimethylamino-phenyl-methyl)-cyclohexyl]-amid
(239) 2-(2,3-Dihydro-benzofuran-5-yl)-thiazol-4-carbonsäure-[4-(dimethylamino-thiophen-2-yl-methyl)-cyclohexyl]-amid
(240) N-{4-[Dimethylamino-(3-fluor-phenyl)-methyl]-cyclohexyl}-2-phenyl-acetamid
(241) 3-Chlor-N-[4-(1-dimethylamino-3-phenyl-propyl)-cyclohexyl]-benzamid
(242) N-[4-(Dimethylamino-phenyl-methyl)-cyclohexyl]-4-methyl-benzamid
(243) 3,5-Dichlor-N-{4-[dimethylamino-(3-fluor-phenyl)-methyl]-cyclohexyl}-benzamid
(244) N-[4-(Dimethylamino-phenyl-methyl)-cyclohexyl]-2,3,6-trifluor-benzamid
(245) Thiophen-2-carbonsäure-[4-(dimethylamino-phenyl-methyl)-cyclohexyl]-amid (unpolareres Diastereomer)
(246) N-[4-(Dimethylamino-phenyl-methyl)-cyclohexyl]-3,3-dimethyl-butyramid (unpolareres Diastereomer)
(247) 2-tert-Butyl-5-methyl-2H-pyrazol-3-carbonsäure-[4-(dimethylamino-phenyl-methyl)-cyclohexyl]-amid
(248) N-[4-(1-Dimethylamino-3-phenyl-propyl)-cyclohexyl]-3-methyl-benzamid
(249) Thiophen-2-carbonsäure-[4-(dimethylamino-phenyl-methyl)-cyclohexyl]-amid (polareres Diastereomer)
(250) N-[4-(Dimethylamino-phenyl-methyl)-cyclohexyl]-2-phenyl-butyramid (polareres Diastereomer)
(251) N-{4-[Dimethylamino-(3-fluor-phenyl)-methyl]-cyclohexyl}-3,3-dimethyl-butyramid
(252) 3-Chlor-4-methanesulfonyl-thiophen-2-carbonsäure-[4-(dimethylamino-phenyl-methyl)-cyclohexyl]-amid
(253) 4-(4-Chlor-benzenesulfonyl)-3-methyl-thiophen-2-carbonsäure-[4-(dimethylamino-phenyl-methyl)-cyclohexyl]-amid
(254) 2-Benzyloxy-N-[4-(dimethylamino-phenyl-methyl)-cyclohexyl]-acetamid
(255) N-[4-(Dimethylamino-phenyl-methyl)-cydohexyl]-2-thiophen-2-yl-acetamid
(256) 4-Methyl-2-phenyl-thiazol-5-carbonsäure-{4-[dimethylamino-(3-fluor-phenyl)-methyl]-cyclohexyl}-amid
(257) 2-(4-Chlor-phenoxy)-N-[4-(dimethylamino-thiophen-2-yl-methyl)-cyclohexyl]-nicotinamid
(258) N-[4-(1-Dimethylamino-3-phenyl-propyl)-cyclohexyl]-4-fluor-benzamid
(259) 5-Brom-N-[4-(dimethylamino-phenyl-methyl)-cyclohexyl]-nicotinamid
(260) 4-Brom-2-ethyl-5-methyl-2H-pyrazol-3-ccarbonsäure-[4-(dimethylamino-thiophen-2-yl-methyl)-cyclohexyl]-amid
(261) 3-Cyano-N-[4-(dimethylamino-phenyl-methyl)-cyclohexyl]-benzamid
(262) N-{4-[Dimethylamino-(4-fluor-phenyl)-methyl]-cyclohexyl}-4-fluor-benzamid
(263) 3-Brom-N-{4-[dimethylamino-(4-fluor-phenyl)-methyl]-cyclohexyl}-benzamid
(264) 2-Phenyl-thiazol-4-carbonsäure-{4-[dimethylamino-(3-fluor-phenyl)-methyl]-cyclohexyl}-amid
(265) 2,5-Dimethyl-furan-3-carbonsäure-{4-[dimethylamino-(3-fluor-phenyl)-methyl]-cyclohexyl}-amid
(266) 2-Methyl-5-phenyl-furan-3-carbonsäure-{4-[dimethylamino-(3-fluor-phenyl)-methyl]-cyclohexyl}-amid
(267) 5-Pyridin-2-yl-thiophen-2-carbonsäure-{4-[dimethylamino-(3-fluor-phenyl)-methyl]-cyclohexyl}-amid
(268) 4-Brom-2-ethyl-5-methyl-2H-pyrazol-3-carbonsäure-[4-(dimethylamino-phenyl-methyl)-cyclohexyl]-amid

(269) 3-Chlor-N-{4-[dimethylamino-(3-fluor-phenyl)-methyl]-cyclohexyl}-4-fluor-benzamid
(270) 3,4-Dichlor-N-{4-[dimethylamino-(4-fluor-phenyl)-methyl]-cydohexyl}-benzamid
(271) N-{4-[Dimethylamino-(4-fluor-phenyl)-methyl]-cyctohexyl}-2,4,5-trifluor-benzamid
(272) Cyclohexancarbonsäure-[4-(dimethylamino-phenyl-methyl)-cyclohexyl]-amid
(273) N-{4-[Dimethylamino-(3-fluor-phenyl)-methyl]-cyclohexyl}-2-phenyl-butyramid
(274) 2-(4-Chlor-phenyl)-N-{4-[dimethylamino-(3-fluor-phenyl)-methyl]-cyclohexyl}-acetamid
(275) N-[4-(Dimethylamino-phenyl-methyl)-cyclohexyl]-3-nitro-benzamid
(276) N-[4-(1-Dimethylamino-3-phenyl-propyl)-cyclohexyl]-2,5-difluor-benzamid
(277) 3-Brom-N-[4-(1-dimethylamino-3-phenyl-propyl)-cyclohexyl]-benzamid
(278) N-{4-[Dimethylamino-(3-fluor-phenyl)-methyl]-cyclohexyl}-2,6-difluor-benzamid
(279) 2,5-Dimethyl-furan-3-carbonsäure-[4-(dimethylamino-phenyl-methyl)-cyclohexyl]-amid
(280) 3-Chlor-N-{4-[dimethylamino-(4-fluor-phenyl)-methyl]-cyclohexyl}-4-fluor-benzamid
(281) N-[4-(Dimethylamino-phenyl-methyl)-cyclohexyl]-5-fluor-2-trifluormethyl-benzamid
(282) 5-Methyl-isoxazol-3-carbonsäure-[4-(dimethylamino-phenyl-methyl)-cyclohexyl]-amid
(283) 2-(2,3-Dihydro-benzofuran-5-yl)-4-methyl-thiazol-5-carbonsäure-[4-(dimethylamino-thiophen-2-yl-methyl)-cyclohexyl]-amid
(284) 2-(4-Chlor-phenoxy)-N-[4-(dimethylamino-phenyl-methyl)-cyclohexyl]-acetamid
(285) 5-(4-Chlor-phenyl)-2-methyl-furan-3-carbonsäure-{4-[dimethylamino-(3-fluorphenyl)-methyl]-cyclohexyl}-amid
(286) 2-Brom-N-[4-(dimethylamino-phenyl-methyl)-cyclohexyl]-benzamid
(287) N-[4-(Dimethylamino-phenyl-methyl)-cyclohexyl]-2,6-dimethoxy-benzamid
(288) Cyclopentancarbonsäure-[4-(dimethylamino-phenyl-methyl)-cyclohexyl]-amid
(289) 2-(2,3-Dihydro-benzofuran-5-yl)-thiazol-4-carbonsäure-[4-(dimethylamino-phenyhmethyl)-cyclohexyl]-amid
(290) Benzo[1,2,5]thiadiazol-5-carbonsäure-{4-[dimethylamino-(3-fluor-phenyl)-methyl]-cyclohexyl}-amid
(291) N-[4-(Dimethylamino-thiophen-2-yl-methyl)-cyclohexylmethyl]-2-thiophen-2-yl-acetamid
(292) Benzo[b]thiophen-3-carbonsäure-[4-(dimethylamino-phenyl-methyl)-cyclohexylmethyl]-amid
(293) 5-Chlor-4-methoxy-thiophen-3-carbonsäure-[4-(dimethylamino-phenyl-methyl)-cyclohexylmethyl]-amid
(294) N-[4-(Dimethylamino-phenyl-methyl)-cyclohexylmethyl]-3,4-difluor-benzamid (unpolareres Diastereomer)
(295) N-{4-[Dimethylamino-(3-fluor-phenyl)-methyl]-cyclohexylmethyl}-3,3-dimethyl-butyramid
(296) 2-Brom-N-[4-(dimethylamino-thiophen-2-yl-methyl)-cyclohexylmethyl]-benzamid
(297) N-[4-(Dimethylamino-thiophen-2-yl-methyl)-cyclohexylmethyl]-2,2-diphenyl-acetamid
(298) N-[4-(Dimethylamino-phenyl-methyl)-cyclohexylmethyl]-3,3-dimethyl-butyramid
(299) N-[4-(Dimethylamino-phenyl-methyl)-cyclohexylmethyl]-2-methylsulfanyl-nicotinamid
(300) N-[4-(Dimethylamino-thiophen-2-yl-methyl)-cyclohexylmethyl]-2,6-dimethoxy-benzamid
(301) N-[4-(Dimethylamino-thiophen-2-yl-methyl)-cyclohexylmethyl]-3,3-dimethyl-butyramid
(302) Benzo[b]thiophen-3-carbonsäure-[4-(dimethylamino-thiophen-2-yl-methyl)-cyclohexylmethyl]-amid
(303) 5-Chlor-4-methoxy-thiophen-3-carbonsäure-{4-[dimethylamino-(3-fluor-phenyl)-methyl]-cyclohexylmethyl}-amid
(304) N-[4-(Dimethylamino-thiophen-2-yl-methyl)-cyclohexylmethyl]-2-phenoxy-propionamid
(305) N-[4-(Dimethylamino-thiophen-2-yl-methyl)-cyclohexylmethyl]-2-methoxy-benzamid
(306) N-[4-(Dimethylamino-phenyl-methyl)-cyclohexylmethyl]-2-phenyl-acetamid
(307) 3-Brom-N-[4-(dimethylamino-thiophen-2-yl-methyl)-cyclohexylmethyl]-benzamid
(308) N-[4-(Dimethylamino-thiophen-2-yl-methyl)-cyclohexylmethyl]-3-fluor-5-trifluormethyl-benzamid
(309) N-{4-[(4-Chlor-phenyl)-dimethylamino-methyl]-cyclohexylmethyl}-3,3-dimethyl-butyramid
(310) N-[4-(Dimethylamino-phenyl-methyl)-cyclohexylmethyl]-2-ethylsulfanyl-nicotinamid
(311) 2-(4-Chlor-phenyl)-N-[4-(dimethylamino-thiophen-2-yl-methyl)-cyclohexylmethyl]-acetamid
(312) N-{4-[(4-Chlor-phenyl)-dimethylamino-methyl]-cyclohexylmethyl}-2,2-diphenyl-acetamid
(313) N-[4-(Dimethylamino-thiophen-2-yl-methyl)-cyclohexylmethyl]-2,6-difluor-benzamid
(314) Benzo[b]thiophen-3-carbonsäure-{4-[dimethylamino-(3-fluor-phenyl)-methyl]-cyclohexylmethyl}-amid
(315) N-{4-[(4-Chlor-phenyl)-dimethylamino-methyl]-cyclohexylmethyl}-2-methylsulfanyl-nicotinamid
(316) N-{4-[(4-Chlor-phenyl)-dimethylamino-methyl]-cyclohexylmethyl}-2-thiophen-2-yl-acetamid
(317) N-[4-(Dimethylamino-phenyl-methyl)-cyclohexylmethyl]-2-methyl-benzamid (unpolareres Diastereomer)
(318) 1-(4-Chlor-phenyl)-cyclopentan-carbonsäure-[4-(dimethylamino-phenyl-methyl)-cyclohexylmethyl]-amid
(319) N-{4-[Dimethylamino-(3-fluor-phenyl)-methyl]-cyclohexylmethyl}-2-phenyl-acetamid (polareres Diastereomer)
(320) N-{4-[(4-Chlor-phenyl)-dimethylamino-methyl]-cyclohexylmethyl}-2-(3-methoxyphenyl)-acetamid
(321) N-[4-(Dimethylamino-phenyl-methyl)-cyclohexylmethyl]-2-phenyl-butyramid
(322) N-{4-[Dimethylamino-(4-fluor-phenyl)-methyl]-cyclohexylmethyl}-3,3-dimethyl-butyramid

(323) N-[4-(Dimethylamino-phenyl-methyl)-cyclohexylmethyl]-2-phenoxy-propionamid
(324) 2-(4-Chlor-phenyl)-N-[4-(dimethylamino-phenyl-methyl)-cyclohexylmethyl]-acetamid
(325) N-[4-(Dimethylamino-phenyl-methyl)-cyclohexylmethyl]-2-methyl-benzamid (polareres Diastereomer)
(326) N-[4-(Dimethylamino-phenyl-methyl)-cyclohexylmethyl]-3-trifluormethyl-benzamid (unpolareres Diastereomer)
(327) 1-(4-Chlor-phenyl)-cyclopentan-carbonsäure-{4-[dimethylamino-(3-fluor-phenyl)-methyl]-cyclohexylmethyl}-amid
(328) Thiophen-2-carbonsäure-[4-(dimethylamino-phenyl-methyl)-cyclohexylmethyl]-amid
(329) 3,5-Dichlor-N-[4-(dimethylamino-phenyl-methyl)-cyclohexylmethyl]-benzamid
(330) 2-Methyl-5-phenyl-furan-3-carbonsäure-{4-[(4-chlor-phenyl)-dimethylaminomethyl]-cyclohexylmethyl}-amid
(331) 3-Chlor-N-[4-(dimethylamino-phenyl-methyl)-cyclohexylmethyl]-benzamid
(332) N-{4-[Dimethylamino-(3-fluor-phenyl)-methyl]-cyclohexylmethyl}-2-phenoxy-propionamid (polareres Diastereomer)
(333) N-[4-(Dimethylamino-phenyl-methyl)-cyclohexylmethyl]-3-trifluormethyl-b enzamid (polareres Diastereomer)
(334) Thiophen-2-carbonsäure-{4-[dimethylamino-(3-fluor-phenyl)-methyl]-cyclohexylmethyl}-amid (polareres Diastereomer)
(335) 2-Phenyl-thiazol-4-carbonsäure-[4-(dimethylamino-phenyl-methyl)-cyclohexylmethyl]-amid
(336) Benzo[b]thiophen-3-carbonsäure-{4-[(4-chlor-phenyl)-dimethylamino-methyl]-cyclohexylmethyl}-amid (unpolareres Diastereomer)
(337) N-{4-[Dimethylamino-(3-fluor-phenyl)-methyl]-cyclohexylmethyl}-2-p-tolyloxy-nicotinamid
(338) 2,4,6-Trichlor-N-[4-(dimethylamino-phenyl-methyl)-cyclohexylmethyl]-benzamid
(339) 1-(4-Chlor-phenyl)-cyclopentan-carbonsäure-[4-(dimethylamino-phenyl-methyl)-cyclohexylmethyl]-amid
(340) Thiophen-2-carbonsäure-{4-[dimethylamino-(3-fluor-phenyl)-methyl]-cyclohexylmethyl}-amid (unpolareres Diastereomer)
(341) N-{4-[Dimethylamino-(3-fluor-phenyl)-methyl]-cyclohexylmethyl}-2-phenoxy-propionamid (unpolareres Diastereomer)
(342) N-{4-[Dimethylamino-(3-fluor-phenyl)-methyl]-cyclohexylmethyl}-2-methyl-butyramid (polareres Diastereomer)
(343) N-{4-[Dimethylamino-(3-fluor-phenyl)-methyl]-cyclohexylmethyl}-2-thiophen-2-yl-acetamid
(344) Benzo[b]thiophen-3-carbonsäure-{4-[dimethylamino-(4-fluor-phenyl)-methyl]-cyclohexylmethyl}-amid
(345) 2-Methyl-5-phenyl-furan-3-carbonsäure-[4-(dimethylamino-phenyl-methyl)-cyclohexylmethyl]-amid
(346) N-{4-[Dimethylamino-(3-fluor-phenyl)-methyl]-cyclohexylmethyl}-2-phenoxy-propionamid
(347) 3-Cyano-N-[4-(dimethylamino-thiophen-2-yl-methyl)-cyclohexylmethyl]-benzamid
(348) N-{4-[Dimethylamino-(3-fluor-phenyl)-methyl]-cyclohexylmethyl}-2-phenyl-acetamid (unpolareres Diastereomer)
(349) N-[4-(Dimethylamino-phenyl-methyl)-cyclohexylmethyl]-2-(3-methoxy-phenyl)-acetamid
(350) N-{4-[Dimethylamino-(3-fluor-phenyl)-methyl]-cyclohexylmethyl}-4-fluor-3-t rifluormethyl-benzamid
(351) N-{4-[(4-Chlor-phenyl)-dimethylamino-methyl]-cyclohexylmethyl}-2-ethylsulfanyl-nicotinamid
(352) N-[4-(Dimethylamino-thiophen-2-yl-methyl)-cyclohexylmethyl]-2-p-tolyloxy-nicotinamid (polareres Diastereomer)
(353) 2-Methyl-5-phenyl-furan-3-carbonsäure-{4-[dimethyla.mino-(3-fluor-phenyl)-methyl]-cyclohexylmethyl}-amid
(354) 2-Chlor-N-{4-[(4-chlor-phenyl)-dimethylamino-methyl]-cyclohexylmethyl}-benzamid
(355) 2-Chlor-N-[4-(dimethylamino-phenyl-methyl)-cyclohexylmethyl]-nicotinamid
(356) N-[4-(Dimethylamino-thiophen-2-yl-methyl)-cyclohexylmethyl]-4-propyl-benzamid
(357) N-[4-(Dimethylamino-phenyl-methyl)-cyclohexylmethyl]-3,4-difluor-benzamid (polareres Diastereomer)
(358) 3-Brom-N-[4-(dimethylamino-phenyl-methyl)-cyclohexylmethyl]-benzamid
(359) N-{4-[Dimethylamino-(4-fluor-phenyl)-methyl]-cyctohexylmethyl}-2-thiophen-2-yl-acetamid
(360) 2-(4-Chlor-phenoxy)-N-[4-(dimethylamino-phenyl-methyl)-cyclohexylmethyl]-nicotinamid (unpolareres Diastereomer)
(361) 2,4-Dichlor-N-[4-(dimethylamino-phenyl-methyl)-cyclohexylmethyl]-benzamid
(362) N-[4-(Dimethylamino-phenyl-methyl)-cyclohexylmethyl]-3-methyl-benzamid
(363) 2-Brom-N-{4-[dimethylamino-(3-fluor-phenyl)-methyl]-cyclohexylmethyl}-benzamid
(364) N-{4-[Dimethylamino-(3-fluor-phenyl)-methyl]-cyclohexylmethyl}-3-trifluormethyl-benzamid
(365) 2-Phenyl-thiazol-4-carbonsäure-{4-[dimethylamino-(3-fluorphenyl)-methyl]-cyclohexylmethyl}-amid
(366) 2-tert-Butyl-5-methyl-2H-pyrazol-3-carbonsäure-[4-(dimethylamino-thiophen-2-yl-methylrcyclohexylmethyl]-amid
(367) 3-Chlor-4-(propan-2-sulfonyl)-thiophen-2-carbonsäure-[4-(dimethylamino-thiophen-2-yl-methyl)-cyclohexylmethyl]-amid

(368) N-{4-[Dimethylamino-(3-fluor-phenyl)-methyl]-cyclohexylmethyl}-2-methoxy-benzamid
(369) N-{4-[(4-Chlor-phenyl)-dimethylamino-methyl]-cyclohexylmethyl}-3-trifluormethyl-benzamid
(370) 1-(4-Chlor-phenyl)-cyclopentan-carbonsäure-{4-[dimethylamino-(4-fluor-phenyl)-methyl]-cyclohexylmethyl}-amid
(371) 3,5-Dichlor-N-{4-[dimethylamino-(3-fluor-phenyl)-methyl]-cyclohexylmethyl}-benzamid
(372) Benzo[b]thiophen-3-carbonsäure-{4-[(4-chlor-phenyl)-dimethylamino-methyl]-cyclohexylmethyl}-amid (polarere Diastereomer)
(373) 2-Methyl-5-phenyl-furan-3-carbonsäure {4-[dimethylamino-(3-fluor-phenyl)-methyl]-cyclohexylmethyl}-amid
(374) 2-(4-Chlor-phenylsulfanyl)-N-[4-(dimethylamino-phenyl-methyl)-cyclohexylmethyl]-nicotinamid
(375) 4-Brom-2-ethyl-5-methyl-2H-pyrazol-3-carbonsäure-[4-(dimethylamino-thiophen-2-yl-methyl)-cyclohexylmethyl]-amid
(376) 5-Chlor-4-methoxy-thiophen-3-carbonsäure-{4-[dimethylamino-(4-fluor-phenyl)-methyl]-cyclohexylmethyl}-amid
(378) N-{4-[Dimethylamino-(3-fluor-phenyl)-methyl]-cyclohexylmethyl}-2-methyl-butyramid (unpolareres Diastereomer)
(379) 5-Methyl-isoxazol-3-carbonsäure-[4-(dimethylamino-phenyl-methyl)-cyclohexylmethyl]-amid
(380) Benzo[b]thiophen-3-carbonsäure-[4-(1-dimethylamino-3-phenyl-propyl)-cyclohexylmethyl]-amid
(381) N-{4-[Dimethylamino-(4-fluor-phenyl)-methyl]-cyclohexylmethyl}-2-methylsulfanyl-nicotinamid
(382) N-{4-[(4-Chlor-phenyl)-dimethylamino-methyl]-cyclohexylmethyl}-2-p-tolyloxy-nicotinamid
(383) 2-(4-Chlor-phenoxy)-N-[4-(dimethylamino-phenyl-methyl)-cyclohexylmethyl]-nicotinamid (polareres Diastereomer)
(384) N-{4-[Dimethylamino-(3-fluor-phenyl)-methyl]-cyclohexylmethyl}-2-methyl-benzamid
(385) 5-Methyl-isoxazol-3-carbonsäure-{4-[(4-chlor-phenyl)-dimethylamino-methyl]-cyclohexylmethyl}-amid
(386) 5-Brom-N-[4-(dimethylamino-phenyl-methyl)-cyclohexylinethyl]-nicotinamid
(387) N-[4-(Dimethylamino-phenyl-methyl)-cyclohexylmethyl]-2-methyl-butyramid
(388) N-{4-[Dimethylamino-(3-fluor-phenyl)-methyl]-cyclohexylmethyl}-2-ethylsulfanyl-nicotinamid
(389) N-[4-(Dimethylamino-thiophen-2-yl-methyl)-cyclohexylmethyl]-2-p-tolyloxy-nicotinamid (unpolareres Diastereomer)
(390) 4-Brom-2-ethyl-5-methyl-2H-pyrazol-3-carbonsäure-[4-(dimethylamino-phenyl-methyl)-cyclohexylmethyl]-amid
(391) N-{4-[(4-Chlor-phenyl)-dimethylamino-methyl]-cyclohexylmethyl}-2-phenoxy-propionamid
(392) N-[4-(Dimethylamino-phenyl-methyl)-cyclohexylmethyl]-3,5-dinitro-benzamid
(393) N-{4-[(4-Chlor-phenyl)-dimethylamino-methyl]-cyclohexylmethyl}-3-methoxy-benzamid
(394) 2-Brom-N-{4-[(4-chlor-phenyl)-dimethylamino-methyl]-cyclohexylmethyl}-benzamid
(395) 2-Brom-N-(2-{4-[(4-chlor-phenyl)-dimethylamino-methyl]-cyclohexyl}-ethyl)-benzamid
(396) 2-Brom-N-(2-{4-[dimethylamino-(3-fluor-phenyl)-methyl]-cyclohexyl}-ethyl)-benzamid
(397) 3-Chlor-N-{2-[4-(dimethylamino-phenyl-methyl)-cyclohexyl]-ethyl}-benzamid (polareres Diastereomer)
(398) 3-Chlor-N-{2-[4-(dimethylamino-phenyl-methyl)-cyclohexyl]-ethyl}-benzamid (unpolareres Diastereomer)
(399) 3-Chlor-N-(2-{4-[(4-chlor-phenyl)-dimethylamino-methyl]-cyclohexyl}-ethyl)-benzamid
(400) 3-Chlor-N-(2-{4-[dimethylamino-(3-fluor-phenyl)-methyl]-cyclohexyl}-ethyl)-benzamid (unpolareres Diastereomer)
(401) 3-Chlor-N-(2-{4-[dimethylamino-(3-fluor-phenyl)-methyl]-cyclohexyl}-ethyl)-benzamid (polareres Diastereomer)
(402) 2-Chlor-N-{2-[4-(dimethylamino-phenyl-methyl)-cyclohexyl]-ethyl}-benzamid
(403) 2-Chlor-N-(2-{4-[(4-chlor-phenyl)-dimethylamino-methyl]-cyclohexyl}-ethyl)-benzamid
(404) 2-Chlor-N-(2-{4-[dimethylamino-(3-fluor-phenyl)-methyl]-cyclohexyl}-ethyl)-benzamid
(405) 4-Chlor-N-{2-[4-(dimethylamino-phenyl-methyl)-cyclohexyl]-ethyl}-benzamid
(406) 4-Chlor-N-(2-{4-[dimethylamino-(3-fluor-phenyl)-methyl]-cyclohexyl}-ethyl)-benzamid
(407) N-{2-[4-(Dimethylamino-phenyl-methyl)-cyclohexyl]-ethyl}-4-fluor-benzamid
(408) N-(2-{4-[(4-Chlor-phenyl)-dimethylamino-methyl]-cyclohexyl}-ethyl)-4-fluor-benzamid
(409) N-(2-{4-[Dimethylamino-(3-fluor-phenyl)-methyl]-cyclohexyl}-ethyl)-4-fluor-benzamid
(410) N-{2-[4-(Dimethylamino-phenyl-methyl)-cyclohexyl]-ethyl}-2-fluor-benzamid
(411) N-(2-{4-[Dimethylamino-(3-fluor-phenyl)-methyl]-cyclohexyl}-ethyl)-2-fluor-benzamid
(412) N-(2-{4-[Dimethylamino-(3-fluor-phenyl)-methyl]-cyclohexyl}-ethyl)-3-methyl-benzamid
(413) 2,6-Dichlor-N-{2-[4-(dimethylamino-phenyl-methyl)-cyclohexyl]-ethyl}-benzamid
(414) N-{2-[4-(Dimethylamino-phenyl-methyl)-cyclohexyl]-ethyl}-2-methoxy-benzamid
(415) N-(2-{4-[Dimethylamino-(3-fluor-phenyl)-methyl]-cyclohexyl}-ethyl)-2-methoxy-benzamid
(416) 3,4-Dichlor-N-{2-[4-(dimethylamino-phenyl-methyl)-cyclohexyl]-ethyl}-benzamid

(417) N-{2-[4-(Dimethylamino-phenyl-methyl)-cyclohexyl]-ethyl}-2-methyl-benzamid (polareres Diastereomer)
(418) N-{2-[4-(Dimethylamino-phenyl-methyl)-cyclohexyl]-ethyl}-2-methyl-benzamid (unpolareres Diastereomer)
(419) N-(2-{4-[(4-Chlor-phenyl)-dimethylamino-methyl]-cyclohexyl}-ethyl)-2-methyl-benzamid
(420) N-(2-{4-[Dimethylamino-(3-fluor-phenyl)-methyl]-cyclohexyl}-ethyl)-2-methyl-benzamid
(421) 4-Cyano-N-{2-[4-(dimethylamino-phenyl-methyl)-cyclohexyl]-ethyl}-benzamid (422) 3-Chlor-N-(2-{4-[dimethylamino-(4-fluor-phenyl)-methyl]-cyclohexyl}-ethyl)-benzamid (polareres Diastzereomer)
(423) 3-Chlor-N-(2-{4-[dimethylamino-(4-fluor-phenyl)-methyl]-cyclohexyl}-ethyl)-benzamid (unpolareres Diastereomer)
(424) 3-Chlor-N-{2-[4-(dimethylamino-thiophen-2-yl-methyl)-cyclohexyl]-ethyl}-benzamid
(425) 2-Chlor-N-{2-[4-(dimethylamino-thiophen-2-yl-methyl)-cyclohexyl]-ethyl}-benzamid
(426) N-{2-[4-(Dimethylamino-thiophen-2-yl-methyl)-cyclohexyl]-ethyl}-4-fluor-benzamid
(427) N-(2-{4-[Dimethylamino-(4-fluor-phenyl)-methyt]-cyclohexyl}-ethyl)-2-fluor-benzamid
(428) N-{2-[4-(Dimethylamino-thiophen-2-yl-methyl)-cyclohexyl]-ethyl}-2-fluor-benzamid
(429) N-(2-{4-[Dimethylamino-(4-fluor-phenyl)-methyl]-cyclohexyl}-ethyl)-3-methyl-benzamid
(430) N-{2-[4-(Dimethylamino-thiophen-2-yl-methyl)-cyclohexyl]-ethyl}-3-methyl-benzamid
(431) 2,6-Dichlor-N-{2-[4-(dimethylamino-thiophen-2-yl-methyl)-cyclohexyl]-ethyl}-benzamid
(432) N-(2-{4-[Dimethylamino-(4-fluor-phenyl)-methyl]-cyclohexyl}-ethyl)-2-methoxy-benzamid
(433) N-{2-[4-(Dimethylamino-phenyl-methyl)-cyclohexyl]-ethyl}-3,5-difluor-benzamid
(434) N-(2-{4-[(4-Chlor-phenyl)-dimethylamino-methyl]-cyclohexyl}-ethyl)-3,5-difluor-benzamid
(435) N-(2-{4-[Dimethylamino-(3-fluor-phenyl)-methyl]-cyclohexyl}-ethyl)-3,5-difluor-benzamid
(436) N-(2-{4-[(4-Chlor-phenyl)-dimethylamino-methyl]-cyclohexyl}-ethyl)-2,4-difluor-benzamid
(437) 2,4-Dichlor-N-(2-{4-[(4-chlor-phenyl)-dimethylamino-methyl]-cyclohexyl}-ethyl)-5-fluor-benzamid
(438) 2,4-Dichlor-N-(2-{4-[dimethylamino-(3-fluor-phenyl)-methyl]-cyclohexyl}-ethyl)-5-fluor-benzamid (polareres Diastereomer)
(439) 2,4-Dichlor-N-(2-{4-[dimethylamino-(3-fluor-phenyl)-methyl]-cyclohexyl}-ethyl)-5-fluor-benzamid (unpolareres Diastereomer)
(440) 2,4-Dichlor-N-{2-[4-(dimethylamino-thiophen-2-yl-methyl)-cyclohexyl]-ethyl}-5-fluor-benzamid
(441) 2-Chlor-N-(2-{4-[(4-chlor-phenyl)-dimethylamino-methyl]-cyclohexyl}-ethyl)-nicotinamid
(442) Naphthalen-2-carbonsäure(2-{4-[dimethylamino-(4-fluor-phenyl)-methyl]-cyclohexyl}-ethyl)-amid
(443) N-{2-[4-(Dimethylamino-thiophen-2-yl-methyl)-cyclohexyl]-ethyl}-4-propyl-benzamid
(444) N-{2-[4-(Dimethylamino-phenyl-methyl)-cyclohexyl]-ethyl}-3,4-difluor-benzamid
(445) N-(2-{4-[(4-Chlor-phenyl)-dimethylamino-methyl]-cyclohexyl}-ethyl)-3,4-difluor benzamid
(446) N-{2-[4-(Dimethylamino-thiophen-2-yl-methyl)-cyclohexyl]-ethyl}-3,4-difluor-benzamid
(447) N-(2-{4-[Dimethylamino-(4-fluor-phenyl)-methyl]-cyclohexyl}-ethyl)-3-methoxy-benzamid
(448) N-{2-[4-(Dimethylamino-phenyl-methyl)-cyclohexyl]-ethyl}-2,2-diphenyl-acetamid
(449) 1-(4-Chlor-phenyl)-cyclopentan-carbonsäure-{2-[4-(dimethylamino-phenyl-methyl)-cyclohexyl]-ethyl}-amid
(450) 2-Benzyloxy-N-{2-[4-(dimethylamino-phenyl-methyl)-cyclohexyl]-ethyl}-acetamid
(451) N-{2-[4-(Dimethylamino-phenyl-methyl)-cyclohexyl]-ethyl}-2-phenyl-acetamid
(452) Thiophen-2-carbonsäure-{2-[4-(dimethylamino-phenyl-methyl)-cyclohexyl]-ethyl}-amid
(453) N-(2-{4-[Dimethylamino-(3-fluor-phenyl)-methyl]-cyclohexyl}-ethyl)-2-(3-methoxy-phenyl)-acetamid
(454) N-{2-[4-(Dimethylamino-thiophen-2-yl-methyl)-cyclohexyl]-ethyl}-2-(3-methoxyphenyl)-acetamid
(455) N-(2-{4-[(4-Chlor-phenyl)-dimethylamino-methyl]-cyclohexyl}-ethyl)-2-phenyl-butyramid
(456) N-{2-[4-(Dimethylamino-thiophen-2-yl-methyl)-cyclohexyl]-ethyl}-2-phenyl-butyramid
(457) Benzo[b]thiophen-2-carbonsäure-{2-[4-(dimethylamino-phenyl-methyl)-cyclohexyl]-ethyl}-amid
(458) N-{2-[4-(Dimethylamino-phenyl-methyl)-cyclohexyl]-ethyl}-4-nitro-benzamid
(459) 3-Brom-N-(2-{4-[dimethylamino-(4-fluor-phenyl)-methyl]-cyclohexyl}-ethyl)-benzamid
(460) N-{2-[4-(Dimethylamino-phenyl-methyl)-cyclohexyl]-ethyl}-2,3,4,5,6-pentafluor-benzamid
(461) N-{2-[4-(Dimethylamino-phenyl-methyl)-cyclohexyl]-ethyl}-2,6-difluor-benzamid
(462) N-(2-{4-[Dimethylamino-(3-fluor-phenyl)-methyl]-cyclohexyl}-ethyl)-2,6-difluor-benzamid
(463) 2-Phenyl-thiazol-4-carbonsäure-{2-[4-(dimethylamino-phenyl-methyl)-cyctohexyl]-ethyl}-amid
(464) 2-Phenyl-thiazol-4-carbonsäure-(2-{4-[dimethylamino-(4-fluor-phenyl)-methyl]-cyclohexyl}-ethyl)-amid
(465) Benzo[b]thiophen-3-carbonsäure-{2-[4-(dimethylamino-thiophen-2-yl-methyl)-cyclohexyl]-ethyl}-amid
(466) N-{2-[4-(Dimethylamino-phenyl-methyl)-cyclohexyl]-ethyl}-2-methylsulfanyl-nicotinamid
(467) 2-Methyl-5-phenyl-furan-3-carbonsäure-{2-[4-(dimethylamino-phenyl-methyl)-cyclohexyl]-ethyl}-amid
(468) 2-(2,3-Dihydro-benzofuran-5-yl)-thiazol-4-carbonsäure-{2-[4-(dimethylaminophenyl-methyl)-cyclohexyl]-ethyl}-amid
(469) 3-(2,6-Dichlor-phenyl)-5-methyl-isoxazol-4--carbonsäure-{2-[4-(dimethylamino-thiophen-2-yl-methyl)-cyclohexyl]-ethyl}-amid

(470) 2-(4-Chlor-phenylsulfanyl)-N-{2-[4-(dimethylamino-phenyl-methyl)-cyclohexyl]-ethyl}-nicotinamid (polareres Diastereomer)
(471) 2-(4-Chlor-phenylsulfanyl)-N-{2-[4-(dimethylamino-phenyl-methyl)-cyclohexyl]-ethyl}-nicotinamid (unpolareres Diastereomer)
(472) Benzo[1,2,3]thiadiazol-5-carbonsäure-{2-[4-(dimethylamino-phenyl-methyl)-cyclohexyl]-ethyl}-amid
(473) 5-Brom-N-{2-[4-(dimethylamino-phenyl-methyl)-cyclohexyl]-ethyl}-nicotinamid
(474) 5-Chlor-4-methoxy-thiophen-3-carbonsäure-{2-[4-(dimethylamino-phenyl-methyl)-cyclohexyl]-ethyl}-amid
(475) 5-Chlor-4-methoxy-thiophen-3-carbonsäure-(2-{4-[dimethylamino-(4-fluorphenyl)-methyl]-cyclohexyl}-ethyl)-amid
(476) 5-Chlor-4-methoxy-thiophen-3-carbonsäure-{2-[4-(1-dimethylamino-3-phenylpropyl)-cyclohexyl]-ethyl}-amid
(477) 3-Cyano-N-{2-[4-(dimethylamino-phenyl-methyl)-cyclohexyl]-ethyl}-benzamid
(478) N-{2-[4-(Dimethylamino-phenyl-methyl)-cyclohexyl]-ethyl}-2,4-dimethoxy-benzamid
(479) 2-Chlor-N-((4-((dimethylamino)(phenyl)methyl)cyclohexyl)methyl)benzamid
(480) N-((4-((Dimethylamino)(phenyl)methyl)cyclohexyl)methyl)-4-fluorbenzamid
(481) N-(2-(4-((Dimethylaminoj(phenyl)methyl)cyclohexyl)ethyl)-4-fluorbenzamid
(482) N-((4-((Dimethylamino)(phenyl)methyl)cydohexyl)methyl)-2-fluorbenzamid
(483) N-((4-((Dimethylamino)(phenyl)methyl)cyclohexyl)methyl)-3-methylbenzamid
(484) N-((4-((Dimethylamino)(phenyl)methyl)cyclohexyl)methyl)-2-methoxybenzamid
(485) N-(2-(4-((dimethylamino)(phenyl)methyl)cyclohexyl)ethyl)-3,5-dimethoxybenzamid
(486) N-((4-((Dimethylamino)(3-fluorphenyl)methyl)cyclohexyl)methyl)-2,6-dimethoxybenzamid
(487) N-((4-((Dimethylamino)(phenyl)methyl)cyclohexyl)methyl)-2,4-difluorbenzamid
(488) N-((4-((Dimethylamino)(thiophen-2-yl)methyl)cyclohexyl)methyl)-3-methoxybenzamid
(489) N-((4-((Dimethylamino)(thiophen-2-yl)methyl)cyclohexyl)methyl)-3,4,5-trimethoxybenzamid
(490) 4-((Dimethylamino)(phenyl)methyl)-1-(4-fluor-3-methylphenyl)cyclohexanol
(491) N-Cyclohexyl-2-(4-(2-phenyl-1-(pyrrolidin-1-yl)ethyl)cyclohexyl)acetamid
(492) N-(3-Methoxyphenyl)-2-(4-(2-phenyl-1-(pyrrolidin-1-yl)ethyl)cyclohexyl)acetamid
(493) N-(4-Methoxyphenyl)-2-(4-(piperidin-1-yl(p-tolyl)methyl)cyclohexyliden)acetamid
(494) N-Phenethyl-2-(4-(piperidin-1-yl(p-tolyl)methyl)cyclohexyliden)acetamid
(495) 2-(4-(2-Phenyl-1-(pyrrolidin-1-yl)ethyl)cyclohexyliden)-N-(pyridin-2-ylmethyl)acetamid
(496) N-Benryl-N-methyl-2-(4-(piperidin-1-yl(p-tolyl)methyl)cyclohexyliden)acetamid
(497) 3-Thiophen-2-yl-[1,2,4]oxadiazol-5-carbonsäure [4-(dimethylamino-phenyl-methyl)-cyclohexyl]-amid
(498) 3-Methyl-[1,2,4]oxadiazol-5-carbonsäure {2-[4-(dimethylamino-phenyl-methyl)-cyclohexyl]-ethyl}-amid
(499) 3-Phenyl-[1,2,4]oxadiazol-5-carbonsäure {4-[dimethylamino-(3-fluoro-phenyl)-methyl]-cyclohexyl}-amid
(500) 3-Cyclopropylmethyl-[1,2,4]oxadiazol-5-carbonsäure {4-[dimethylamino-(3-fluoro-phenyl)-methyl]-cyclohexyl}-amid
(501) 3-Methoxymethyl-[1,2,4]oxadiazol-5-carbonsäure {2-[4-(1-dimethylamino-3-phenyl-propyl)-cyclohexyl]-ethyl}-amid

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**Beispiele**

**Synthese der verwendeten Cyclohexanone**

Die Cyclohexanone bilden den Ausgangspunkt für weitere Derivatisierungen.
Die Ketone wurden auf dem unten beschriebenen Weg in einer mehrstufigen Synthese aus dem kommerziell erhältlichen 4-Oxo-cydohexancarbonsäureethylester erhalten. Die Ausbeuten der hergestellten Verbindungen sind nicht optimiert. Alle Temperaturen sind unkorrigiert.

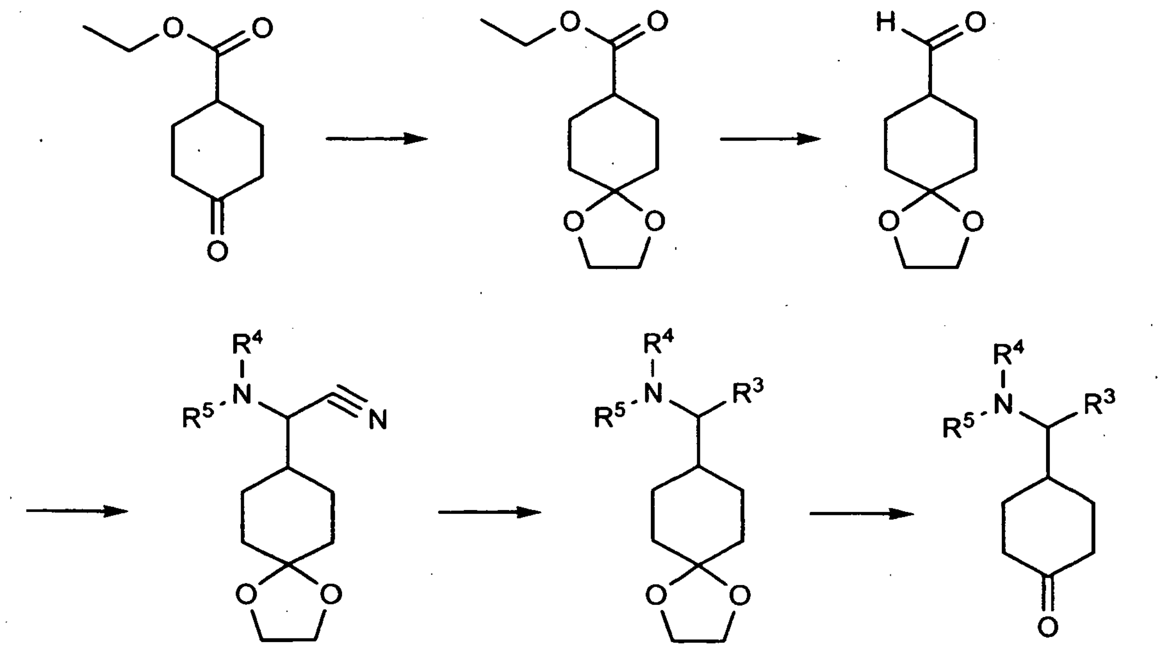

**1,4-Dioxa-spiro[4.5]decan-8-carbonsäure-ethylester 1**

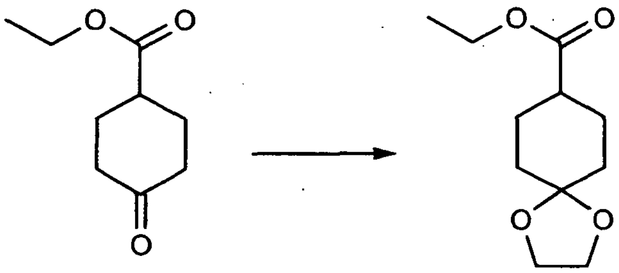

4-Oxo-cyclohexancarbonsäure-ethylester (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet ($Na_2SO_4$), i. Vak. eingeengt und die verbliebene farblose Flüssigkeit ohne Reinigung weiter verarbeitet.
Ausbeute: 66,5 g (100 %)
$^1$H-NMR ($CDCl_3$): 1,24 (t, 3 H); 1,53 (m, 2 H); 1,76 (m, 4 H); 1,92 (m, 2 H); 2,31 (m, 1 H); 3,91 (s, 4 H); 4,11 (q, 2 H).
$^{13}$C-NMR ($CDCl_3$): 14,28 (q); 26,32 (t); 33,76 (t); 41,59 (d); 60,14 (t); 64,21 (t); 107,90 (d); 174,77 (s).

**1,4-Dioxa-spiro[4.5]decane-8-carbaldehyd 2**

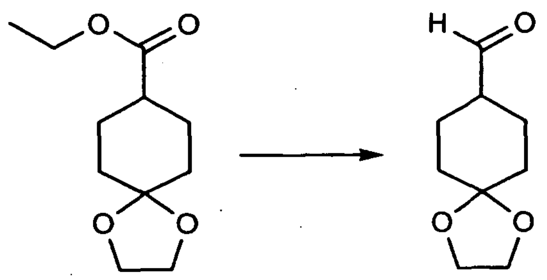

Eine Lösung aus 1,4-Dioxa-spiro[4.5]decan-8-carbonsäureethylester 1 (32,13 g, 150 mmol) in absol.Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Kiesegur wurde zweimal mit Essigester gewaschen, die wäßrige Lösung abgetrennt und zweimal mit Essigester extrahiert: Die vereinigten organischen Extrakte wurden mit gesättigter Natriumchlorid-Lösung gewaschen, über Natriumsulfat getrocknet und i. Vak. eingeengt.
Ausbeute: 24,01 g (94 %), gelbes Öl
$^1$H-NMR ($CDCl_3$): 1,54 (m, 2 H); 1,74 (m, 4 H); 1,91 (m, 2 H); 2,21 (m, 1 H); 3,91 (s, 4 H); 9,60 (s, 1 H).
$^{13}$C-NMR ($CDCl_3$): 23,35 (t); 33,37 (t); 48,18 (d); 64,30 (t); 107,89 (d); 203,51 (s).

**Dimethylamino-(1,4-dioxa-spiro[4.5]dec-8-yl)-acetonitril 3**

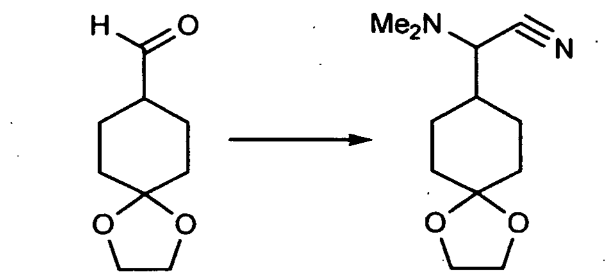

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxa-spiro-[4.5]decan-8-carbaldehyd 2 (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.
Ausbeute: 25,2 g (81 %)
Schmelzpunkt: 48-51 °C.
$^1$H-NMR ($CDCl_3$): 1,23-2,03 (m, 9 H); 2,28 (s, 6 H); 3,16 (d, 1 H); 3,93 (m, 4 H). $^{13}$C-NMR ($CDCl_3$): 26,67 (t); 27,93 (t); 33,87 (t); 36,94 (d); 41.90 (q); 64.30 (t); 64.36 (t); 108.33 (d); 115.94 (s).

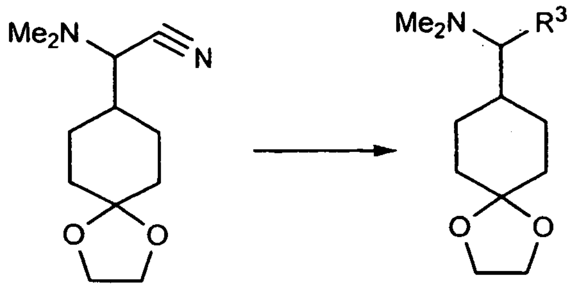

**[(1,4-Dioxa-spiro[4.5]dec-8-yl)-phenyl-methyl]-dimethyl-amin 4 ($R^3$ = Phenyl)**

Eine 25-proz. Lösung von Phenylmagnesiumchlorid (144 ml, 262.5 mmol) in THF wurde unter Argon und Eiskühlung tropfenweise mit einer Lösung des Aminonitrils 3 (23,56 g, 105 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) addiert und mit Diethylether (3 x 100 ml) extrahiert. Die organische Phase wurde mit Wasser und gesättigter Natriumchlorid-Lösung gewaschen, getrocknet ($Na_2SO_4$) und eingeengt.
Ausbeute: 28.9 g (100 %).
$^{13}$C-NMR ($CDCl_3$): 27,05; 28,13; 34,48; 34,57; 36,94 ($C_8$); 41,64 ($N(CH_3)_2$); 64,15; 74,33 (CH); 109,02 ($C_5$); 126,70 ($C_{arom}$); 127,49 ($C_{arom}$); 129,12 ($C_{arom}$); 136,57 ($C_{arom}$).

**[(1,4-Dioxa-spiro[4.5]dec-8-yl)-4-fluorphenyl-methyl]-dimethylamin 5 ($R^3$ = 4-Fluorphenyl)**

Eine 1M Lösung von 4-Fluorphenylmagnesiumbromid in THF (220 ml, 220 mmol) wurde unter Argon und Eiskühlung tropfenweise mit einer Lösung des Aminonitrils 3 (19,89 g, 88 mmol) in absol. THF (160 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet ($Na_2SO_4$) und eingeengt. Ausbeute: 31 g (>100 %)
$^{13}$C-NMR ($CDCl_3$): 26,68 (t); 28,11 (t); 34,43 (t); 34,55 (t); 37,37 (d); 41,68 (q); 64,12 (t); 73,65 (d); 108,88 (d); 114,23 (d); 114,44 (d); 130,27; 130,35; 132,43; 160,36 (s); 162,78 (s).

**[(1,4-Dioxa-spiro[4.5]dec-8-yl)-3-fluorphenyl-methyl]-dimethyl-amin 6 ($R^3$ = 3-Fluorphenyl)**

Eine 1 M Lösung von 3-Fluorphenylmagnesiumbromid in THF (208 ml, 208 mmol) wurde unter Argon und Eiskühlung tropfenweise mit einer Lösung des Aminonitrils 3 (23,45 g, 104 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,33 g (99 %).
$^{1}$H-NMR ($CDCl_3$): 1,12 (m, 1 H); 1,26 (m, 1 H); 1,46 -1,81 (m, 7 H); 2,10 (s, 6 H); 3,10 (d, 1 H); 3,90 (m, 4 H); 6,85 (m, 3 H); 7,27 (m, 1 H).
$^{13}$C-NMR ($CDCl_3$): 26,80 (t); 28,08 (t); 34,48 (t); 34,45 (t); 34,59 (t); 37,26 (d); 41,71 (q); 64,19 (t); 74,04 (t); 108,91 (d); 113,51 (d); 113,71 (d); 115,52 (d); 115,72 (d); 124,83 (d); 128,82 (d); 128,90 (d); 139,66 (s); 161,15 (s); 163,58 (s).

**[(4-Chlorphenyl)-(1,4-dioxa-spiro[4.5]dec-8-yl)-methyl]-dimethyl-amin 7 ($R^3$ = 4-Chlorphenyl)**

Eine 1 M Lösung von 4-Chlorphenylmagnesiumbromid in Ether (200 ml, 200 mmol) wurde unter Argon und Eiskühlung tropfenweise mit einer Lösung des Aminonitrils 3 (22,43 g, 100 mmol) in absol. Ether (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,9 g (100%)
$^{13}$C-NMR ($CDCl_3$): 26,65 (t); 28,11 (t); 34,46 (t); 34,60 (t); 37,28 (d); 41,76 (q); 64,17 (t); 73,80 (d); 108,88 (s); 127,72 (d); 129,53 (d); 132,39 (d); 135,33 (d).

**[(1,4-Dioxa-spiro[4,5]dec-8-yl)-thiophen-2-yl-methyl]-dimethylamin 8 ($R^3$ = 2-Thienyl)**

Eine 1 M Lösung von Thiophen-2-yl-magnesiumbromid in THF (20 ml, 20 mmol) wurde unter Argon und Eiskühlung tropfenweise mit einer Lösung des Aminonitrils 3 (2,24 g, 10 mmol) in absol. THF (10 ml) versetzt und 20 h bei RT gerührt. Zur Aufarbeitung des Reaktionsgemisches wurde unter Eiskühlung gesättigte Ammoniumchlorid-Lösung (10 ml) und Wasser (10 ml) gegeben und mit Diethylether (3 x 10 ml) extrahiert. Die organische Phase wurde mit Wasser und gesättigter Natriumchlorid-Lösung gewaschen, getrocknet und eingeengt.
Ausbeute: 2,8 g (100 %)
$^{13}$C-NMR ($CDCl_3$): 27,72 (t); 27,88 (t); 34,27 (t); 39,28 (d); 41,10 (q); 64,11 (t); 68,89 (d); 108,88 (s); 123,55 (d); 125,88 (d); 127,53 (d); 139,50 (s).

**[1-(1,4-Dioxa-spiro[4.5]dec-8-yl)-3-phenyl-propyl]-dimethylamin 9 ($R^3$ = Phenethyl)**

Eine 1M Lösung von Phenylethylmagnesiumchlorid in THF (242 ml, 242 mmol) wurde unter Argon und Eiskühlung tropfenweise mit einer Lösung des Aminonitrils 3 (21,93 g, 97 mmol) in absol. THF (180 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 34 g (>100 %).
$^{13}$C-NMR ($CDCl_3$): 27,43 (t); 28,95 (t); 29,42 (t); 34,82 (t); 35,40 (t); 38,76 (d); 41,16 (q); 64,17 (t); 67,41 (d); 108,86 (s); 125,41 (d); 127,66 (d); 128,11 (d); 142,69 (s).

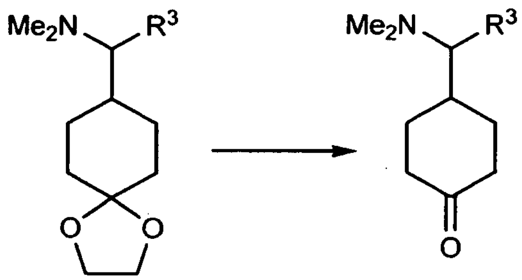

**4-(Dimethylamino-phenyl-methyl)-cyclohexanon 10 ($R^3$ = Phenyl)**

Das Ketal 4 (28,9 g, 0,105 mol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.
Ausbeute: 18.2 g (75%)
$^1$H-NMR ($CDCl_3$): 1,20 (1 H, m); 1,33 (1 H, m); 1,74 (1 H, m); 2,17 (6 H, s, $N(CH_3)_2$); 2,70 (6 H, m); 3,10 (1 H, d, $C_8$-H); 7,07 (2 H, m, $C_{arom}$-H); 7,23 (3 H, m, $C_{arom}$-H). $^{13}$C-NMR ($CDCl_3$): 29,13; 30,56; 36,90 ($C_4$); 40,61; 40,82; 41,89 (N $(CH_3)_2$); 73,79 (CH); 127,05 ($C_{arom}$); 127,67 ($C_{arom}$); 129,00 ($C_{arom}$); 136,13 ($C_{arom}$); 211,79 (C=O).

**4-[Dimethylamino-(4-fluorphenyl)-methyl]-cyclohexanon 11 ($R^3$ = 4-Fluorphenyl)**

Das Rohprodukt des Ketals 5 (26 g, 88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt.
Ausbeute: 21,36 g (98%)
$^{13}$C-NMR ($CDCl_3$): 28,90 (t); 30,48 (t); 37,00 (t); 40,49 (t); 40,72 (t); 41,79 (q); 72,98 (d); 114,42 (d); 114,62 (d); 130,20 (d); 130,28 (d); 131,88 (s); 160,50 (s); 162,93 (s); 211,44 (s).

**4-[Dimethylamino-(3-fluorphenyl)-methyl]-cyclohexanon 12 ($R^3$ = 3-Fluorphenyl)**

Das Ketal 6 (30,3 g, 103 mmol) wurde in Wasser (44 ml) gelöst, mit konz. Salzsäure (64 ml) versetzt und 20

h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloser Feststoff isoliert.
Ausbeute: 22,4 g (87 %)
Schmelzpunkt: 72-75 °C.
$^{13}$C-NMR ($CDCl_3$): 28,97 (t); 30,44 (t); 36,90 (t); 40,52 (t); 40,75 (t); 41,82 (q); 73,37 (d); 113,84; 114,06; 115,42; 115,62; 124,71; 129,03; 129,11; 139,00; 139,06; 161,16; 163,60; 211,40 (s).

**4-[(4-Chlor-phenyl)-dimethylamino-methyl]-cyclohexanon 13 ($R^3$ = 4-Chlorphenyl)**

Das Ketal 7 (30,98 g, 100 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als Öl isoliert.
Ausbeute: 21,9 g (82 %)
$^{13}$C-NMR ($CDCl_3$): 28,88 (t); 30,45 (t); 36,89 (t); 40,49 (t); 40,74 (t); 41,83 (q); 73,12 (d); 127,87 (d); 130,16 (d); 132,75 (d); 13470 (s); 211,35 (s).

**4-(Dimethylamino-thiophen-2-yl-methyl)-cyclohexanon 14 ($R^3$ = 2-Thienyl)**

Das Ketal 8 (2,80 g, 10 mmol) wurde in Wasser (4,4 ml) gelöst, mit konz. Salzsäure (6,4 ml) versetzt und 20 h bei RT gerührt. Das Reaktionsgemisch wurde mit Diethylether (2 x 10 ml) ausgeschüttelt, die wässrige Phase unter Eiskühlung mit 5N NaOH alkalisch gestellt, mit Dichlormethan (3 x 10 ml) extrahiert, getrocknet und eingeengt. Das Keton wurde als Öl isoliert.
Ausbeute: 1,79 g (75 %)
$^{13}$C-NMR ($CDCl_3$): 30,02 (t); 30,18 (t); 38,84 (t); 40,29 (t); 39,28 (d); 41,17 (q); 68,24 (d); 123,88 (d); 126,01 (d); 126,34 (d); 138,77 (d); 211,49 (s).

**4-(1-Dimethylamino-3-phenyl-propyl)-cyclohexanon 15 ($R^3$ = Phenethyl)**

Das Rohprodukt des Ketals 9 (29,6 g, 97 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.
Ausbeute: 16.9 g (58 %)
$^{13}$C-NMR ($CDCl_3$): 29,40 (t); 30,02 (t); 30,97 (t); 35,34 (t); 38,71 (t); 40,79 (t); 41,01 (t); 41,23 (q); 66,65 (d); 125,66 (d); 128,12 (d); 128,19 (d); 142,27 (s); 211,70 (s).

**Synthese der Amino-, Aminomethyl- und Aminoethylcyclohexyle**

Aus den Cyclohexanonderivaten können nun die entsprechenden Amine durch einfache Transformation erhalten werden.

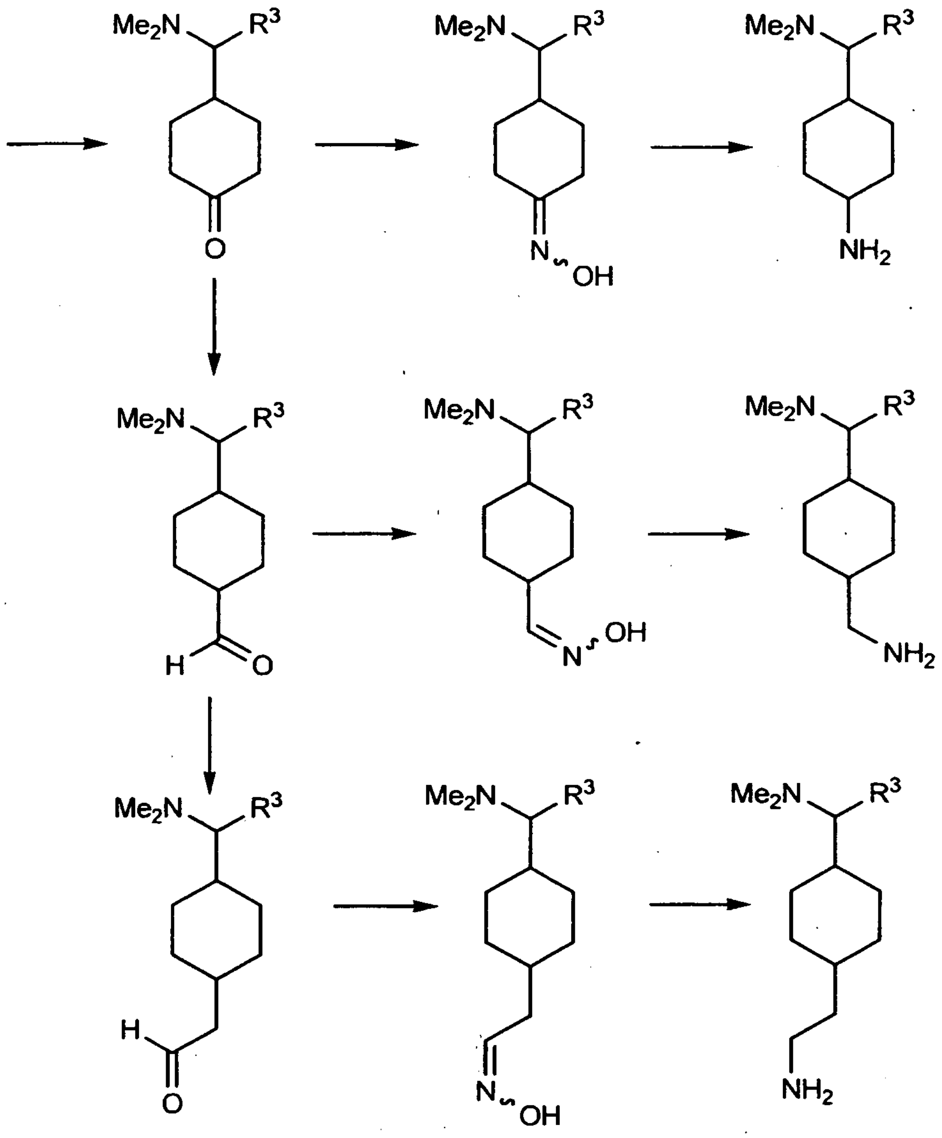

**Synthese der Aminocyclohexane ($R^1 = (CH_2)_nNH_2$, n = 0)**

Die Aminocyclohexane wurden durch zweistufige Reaktionen aus den entsprechend substituierten Cyclohexanonen mit Hydroxylamin Hydrochlorid und anschließender Spaltung mit Lithiumaluminiumhydrid dargestellt.

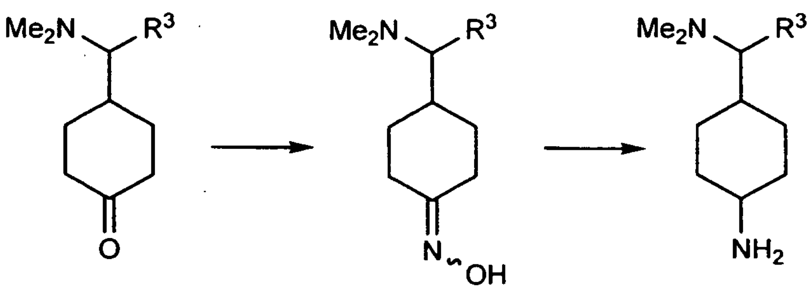

**4-(Dimethylamino-phenyl-methyl)-cyclohexanon-oxim 16 ($R^3$ = Phenyl)**

Das Keton **10** (9,25 g, 40 mmol) und Hydroxylamin Hydrochlorid (4,17 g, 60 mmol) wurden in absol. Ethanol (150 ml) gelöst, mit basischem Ionenaustauscher Amberlyst A21 (28 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essig-ester (3 x 50 ml) extrahiert, die organische Phase über Natriumsulfat getrocknet und i. Vak. eingeengt.
Ausbeute: 9,54 g (97 %)
Schmelzpunkt: 110-115 °C, (farblose Kristalle)
$^{13}$C-NMR ($CDCl_3$): 23,53; 23,70; 27,87; 29,04; 29,48; 30,70; 31,26; 31,40; 37,89 ($C_4$); 42,02 ($N(CH_3)_2$); 74,36 (CH); 126,87 ($C_{arom}$); 127,56 ($C_{arom}$); 129,09 ($C_{arom}$); 136,57 ($C_{arom}$); 160,12 (C=N-O).

**4-(Dimethylamino-phenyl-methyl)-yclohexylamin 17 ($R^3$ = Phenyl)**

Absolutes THF (400 ml) wurde unter Argon mit $LiAlH_4$ (2,92 g, 77 mmol) versetzt, auf 60 °C erwärmt und tropfenweise das Oxim **16** (9,5 g, 38,5 mmol), gelöst in THF (90 ml), zugegeben. Nach 4-stündigem Rühren bei 60 °C wurde unter Eisbadkühlung (10 °C) Wasser (100 ml) zugetropft und die Lösung über Kieselgur abgesaugt. Der Filterrückstand wurde mit THF gewaschen. Das THF wurde i. Vak. entfernt, der Rückstand mit 5N NaOH auf pH 11 eingestellt und mit Essigester (4 x 40 ml) extrahiert. Die organische Phase wurde über Natriumsulfat getrocknet, eingedampft und der Rückstand über eine Kieselgelsäule (300 g) mit MeCN/MeOH/0,5 M $NH_4Cl$ (9:1:1) gereinigt.
Die einzelnen Fraktionen wurden in Wasser und Methylenchlorid gelöst, mit Ammoniak alkalisch gestellt und die wässrige Phase mit $CH_2Cl_2$ (zweimal) extrahiert. Gesamtausbeute: 6,33 g (71 %), Öl
$^{13}$C-NMR ($CDCl_3$): 24,22; 24,80; 28,24; 29,96; 32,39, 32,45; 36,03; 36,58; 36,79; 37,93 ($C_4$); 41,33; 41,89 ($N(CH_3)_2$); 47,42; 50,85; 71,95; 75,22 (CH); 126,52 ($C_{arom}$); 127,29 ($C_{arom}$); 127,33 ($C_{arom}$); 129,04 ($C_{arom}$); 129,11 ($C_{arom}$); 136,22 ($C_{arom}$); 137,03($C_{arom}$).

**4-[Dimethylamino-(4-fluorphenyl)-methyl]-cyclohexanon-oxim 18 ($R^3$ = 4-Fluorphenyl)**

Das Keton 11 (10,68 g, 43 mmol) und Hydroxylamin Hydrochlorid (4,52 g, 65 mmol) wurden in absol. Ethanol (160 ml) gelöst, mit basischem Ionenaustauscher Amberlyst A21 (30 g) versetzt und bei RT über Nacht gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über Natriumsulfat getrocknet und i. Vak. eingeengt.
Ausbeute: 10,49 g (93 %)
$^{13}$C-NMR ($CDCl_3$): 23,76; 23,66; 27,69; 28,87; 29,50; 30,73; 31,22; 31,38; 38,06 ($C_4$); 42,01 ($N(CH_3)_2$); 73,66 (CH); 114,36 ($C_{arom}$); 114,57 ($C_{arom}$); 130,32 ($C_{arom}$); 130,40 ($C_{arom}$); 132,40 ($C_{arom}$); 160,03 (C=N-O); 160,49 ($C_{arom}$); 162,93 ($C_{arom}$).

**4-[Dimethylamino-(4-fluorphenyl)-methyl]-cyclohexylamin 19 ($R^3$ = 4-Fluorphenyl)**

Absolutes THF (435 ml) wurde unter Argon mit $LiAlH_4$ (3,04 g, 82 mmol) versetzt, auf 60 °C erwärmt und tropfenweise das Oxim **18** (10,49 g, 40 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Essigester (4 x 50 ml) extrahiert. Die organische Phase wurde über Natriumsulfat getrocknet, eingedampft und der Rückstand durch Flashchromatographie mit MeCN/MeOH/0,5M $NH_4Cl$ (9:1:1) gereinigt.
Die einzelnen Fraktionen wurden in Wasser und Methylenchlorid gelöst, mit Ammoniak alkalisch gestellt und die wässrige Phase zweimal mit $CH_2Cl_2$ extrahiert. Ausbeute: 6,95 g (70 %), Öl
$^{13}$C-NMR ($CDCl_3$): 24,01; 24,76; 27,99; 29,92; 32,32; 36,26; 36,51; 36,73; 38,07; 41,26 ($C_4$); 41,85 ($N(CH_3)_2$); 47,31; 50,81; 71,25; 74,44 (CH); 114,01($C_{arom}$); 114,08 ($C_{arom}$); 130,20 ($C_{arom}$); 130,27 ($C_{arom}$); 132,02 ($C_{arom}$): 132,85 ($C_{arom}$); 160,22 ($C_{arom}$); 162,64 ($C_{arom}$).

**4-[Dimethylamino-(3-fluorphenyl)-methyl]-cyclohexanon-oxim 20 ($R^3$ = 3-Fluorphenyl)**

Das Keton **12** (10 g, 40 mmol) und Hydroxylamin Hydrochlorid (4,17 g, 60 mmol) wurden in absol. Ethanol (150 ml) gelöst, mit basischem Ionenaustauscher Amberlyst A21 (28 g) versetzt und bei RT über Nacht gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3x 50 ml) extrahiert, die organische Phase über

Natriumsulfat getrocknet und i. Vak. eingeengt.
Ausbeute: 10,05 g (95 %)
$^{13}$C-NMR ($CDCl_3$): 23,48; 23,66; 27,69; 28,87; 29,39; 30,61; 31,18; 31,33; 37,91 ($C_4$); 41,99 ($N(CH_3)_2$); 74,00 (CH); 113,70 ($C_{arom}$); 113,90 ($C_{arom}$); 115,51 ($C_{arom}$); 124,80 ($C_{arom}$); 128,90 ($C_{arom}$); 128,98 ($C_{arom}$); 139,48 ($C_{arom}$); 139,54 ($C_{arom}$); 159,89 (C=N-O); 161,13 ($C_{arom}$); 163,57($C_{arom}$).

**4-[Dimethylamino-(3-fluorphenyl)-methyl]-cyclohexylamin 21 ($R^3$ = 3-Fluorphenyl)**

Absolutes THF (400 ml) wurde unter Argon mit $LiAlH_4$ (2,83 g, 75 mmol) versetzt, auf 60 °C erwärmt und tropfenweise das Oxim **20** (9,86 g, 37,3 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Essigester (4 x 40 ml) extrahiert. Die organische Phase wurde über Natriumsulfat getrocknet, eingedampft und der Rückstand über eine Kieselgelsäule (300 g) mit MeCN/MeOH/0,5 M $NH_4Cl$ (9:1:1) gereinigt.
Die einzelnen Fraktionen wurden in Wasser und Methylenchlorid gelöst, mit Ammoniak alkalisch gestellt und die wässrige Phase zweimal mit $CH_2Cl_2$ extrahiert. Ausbeute: 6,81 g (73 %), Öl
$^{13}$C-NMR ($CDCl_3$): 24,08; 24,69; 28,05; 29,84; 32,33; 32,37; 36,10; 36,48; 36,69; 37,95; 41,27 ($C_4$); 41,85.($N(CH_3)_2$); 47,32; 50,81; 71,63; 74,81 (CH); 113,29 ($C_{arom}$); 115,43 ($C_{arom}$); 124,74 ($C_{arom}$); 128,58 ($C_{arom}$); 139,19 ($C_{arom}$); 139,99 ($C_{arom}$); 160,97 ($C_{arom}$); 163,41 ($C_{arom}$).

**4-[(4-Chlorphenyl)-dimethylamino-methyl]-cyclohexanonoxim 22 ($R^3$ = 4-Chlorphenyl)**

Das Keton **13** (15,76 g, 59,2 mmol) und Hydroxylamin Hydrochlorid (6,25 g, 90 mmol) wurden in absol. Ethanol (200 ml) gelöst, mit basischem Ionenaustauscher Amberlyst A21 (42 g) versetzt und bei RT über Nacht gerührt. Der Ionenaustauscher wurde abfiltriert und mit Ethanol (2x70 ml) gewaschen. Die Lösung wurde eingeengt, der Rückstand mit 5N NaOH auf pH 11 eingestellt, die wässrige Phase mit Essigester (3 x 70 ml) extrahiert, die organische Phase über Natriumsulfat getrocknet und i. Vak. eingeengt.
Ausbeute: 16,6 g (100 %)
$^{13}$C-NMR ($CDCl_3$): 23,46; 23,66; 27,65; 28,81; 29,44; 30,67; 31,21; 31,37; 37,93 ($C_4$); 42,05 ($N(CH_3)_2$); 73,76 (CH); 127,80 ($C_{arom}$); 130,27 ($C_{arom}$); 132,62 ($C_{arom}$); 135,20 159,90 (C=N-O).

**4-[(4-Chlorphenyl)-dimethylamino-methyl]-cyclohexylamin 23 ($R^3$ = 4-Chlorphenyl)**

Absolutes THF (600 ml) wurde unter Argon mit $LiAlH_4$ (4,48 g, 118 mmol) versetzt, auf 60 °C erwärmt und tropfenweise das Oxim **22** (16,6 g, 59 mmol), gelöst in THF (120 ml), zugegeben. Nach vierstündigem Rühren bei 60 °C wurde unter Eisbadkühlung (10 °C) Wasser (150 ml) zugetropft und über Kieselgur abgesaugt. Der Filterrückstand wurde mit THF gewaschen. Das THF im Vakuum entfernt, der Rückstand mit 5N NaOH auf pH 11 eingestellt und mit Essigester (4 x 100 ml) extrahiert. Die organische Phase wurde über Natriumsulfat getrocknet, eingedampft und der Rückstand über eine Kieselgelsäule (400 g) mit MeCN/MeOH/0,5 M $NH_4Cl$ (8:2:1) gereinigt.
Die einzelnen Fraktionen wurden in Wasser und Methylenchlorid gelöst, mit Ammoniak alkalisch gestellt und die wässrige Phase zweimal mit $CH_2Cl_2$ extrahiert. Ausbeute: 12,02 g (76 %), Öl
$^{13}$C-NMR ($CDCl_3$): 24,06; 24,80; 27,99; 29,96; 32,41; 36,24; 36,58; 36,81; 38,06; 41,39 ($C_4$); 42,00 ($N(CH_3)_2$); 47,36; 50,89; 71,51; 74,66 (CH); 127,58($C_{arom}$); 127,65 ($C_{arom}$); 130,30 ($C_{arom}$); 130,35 ($C_{arom}$); 132,27 ($C_{arom}$); 134,94 ($C_{arom}$); 135,80 ($C_{arom}$).

**4-(Dimethylamino-thiophen-2-yl-methyl)-cyclohexanonoxim 24 ($R^3$ = 2-Thiophen)**

Das Keton **14** (9,49 g, 40 mmol) und Hydroxylamin Hydrochlorid (4,17 g, 60 mmol) wurden in absol. Ethanol (150 ml) gelöst, mit basischem Ionenaustauscher Amberlyst A21 (28 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essig-ester (3 x 50 ml) extrahiert, die organische Phase über Natriumsulfat getrocknet und i. Vak. eingeengt.
Ausbeute: 9,21 g (91 %)
Schmelzpunkt: 118-121 °C, gelbe Kristalle

**4-(Dimethylamino-thiophen-2-yl-methyl)-cyclohexylamin 25 ($R^3$ = 2-Thiophen)**

Absolutes THF (300 ml) wurde unter Argon mit $LiAlH_4$ (2,73 g, 72 mmol) versetzt, auf 60 °C erwärmt und

tropfenweise das Oxim **24** (9,08 g, 35,9 mmol), gelöst in THF (80 ml), zugegeben. Nach 4-stündigem Rühren bei 60 °C wurde unter Eisbadkühlung (10 °C) Wasser (80 ml) zugetropft und die Lösung über Kieselgur abgesaugt. Der Filterrückstand wurde mit THF gewaschen. Das THF i. Vak. entfernt, der Rückstand mit 5N NaOH auf pH 11 eingestellt und mit Essigester (3 x 50 ml) extrahiert. Die organische Phase wurde über Natriumsulfat getrocknet, eingedampft und der Rückstand über eine Kieselgelsäule (300 g) mit MeCN/MeOH/0,5M $NH_4Cl$ (8:2:1) gereinigt.
Die einzelnen Fraktionen wurden in Wasser und Methylenchlorid gelöst, mit Ammoniak alkalisch gestellt und die wässrige Phase zweimal mit $CH_2Cl_2$ extrahiert. Gesamtausbeute: 5,66 g (66 %), Öl
$^{13}C$-NMR ($CDCl_3$): 24,81; 24,96; 29,26; 29,76; 32,18; 32,22; 36,46; 36,58; 38,10; 39,99; 40,86; 41,20 ($N(CH_3)_2$); 47,66; 50,80; 64,27; 69,82; 123,43; 125,71; 125,75; 125,95; 126,07; 139,34; 139,79.

**4-(1-Dimethylamino-3-phenyl-propyl)-cyclohexanon oxim 26 ($R^3$ = Phenethyl)**

Das Keton **15** (10,2 g, 40 mmol) und Hydroxylamin Hydrochlorid (4,17 g, 60 mmol) wurden in absol. Ethanol (150 ml) gelöst, mit basischem Ionenaustauscher Amberlyst A21 (28 g) versetzt und bei RT über Nacht gerührt. Der Ionenaustauscher wurde abfiltriert, mit Ethanol (2 x 50 ml) gewaschen, die Lösung eingeengt und der Rückstand mit 5N NaOH auf pH 11 eingestellt. Die wässrige Phase wurde mit Essigester (3 x 50 ml) extrahiert, die organische Phase über Natriumsulfat getrocknet und i. Vak. eingeengt.
Ausbeute: 10,8 g (100 %), Öl
$^{13}C$-NMR ($CDCl_3$): 23,80; 23,96; 28,80; 29,27; 30,00; 31,21; 31,49; 31,58; 35,89 ($C_4$); 39,29; 41,26 ($N(CH_3)_2$); 67,24 (CH); 125,58 ($C_{arom}$); 128,13 ($C_{arom}$); 142,40 ($C_{arom}$); 159,99; 160,04 (C=N-O).

**4-(1-Dimethylamino-3-phenyl-propyl)-cyclohexylamin 27 ($R^3$ = Phenethyl)**

Absolutes THF (435 ml) wurde unter Argon mit $LiAlH_4$ (3,04 g, 82 mmol) versetzt, auf 60 °C erwärmt und tropfenweise das Oxim **26** (11,14 g, 40 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Essigester (4 x 50 ml) extrahiert. Die organische Phase wurde über Natriumsulfat getrocknet, eingedampft und der Rückstand über eine Kieselgelsäule (300 g) mit MeCN/MeOH/0,5M $NH_4Cl$ (9:1:1) und (9:4:1) gereinigt. Die einzelnen Fraktionen wurden in Wasser und Methylenchlorid gelöst, mit Ammoniak alkalisch gestellt und die wässrige Phase zweimal mit $CH_2Cl_2$ extrahiert. Ausbeute: 5,02 g (50 %), Öl
$^{13}C$-NMR ($CDCl_3$): 24,70; 25,36; 29,22; 29,35; 30,42; 32,98; 35,46; 35,72; 36,95; 37,07; 38,89 ($C_4$); 39,32; 41,04; 41,26 ($N(CH_3)_2$); 46,98; 50,85; 66,01; 68,05 (CH); 125,49 ($C_{arom}$); 1,28,11 ($C_{arom}$); 128,14 ($C_{arom}$); 142,75($C_{arom}$).

**Synthese der Aminomethylcyclohexane ($R^1 = (PH_2)_nNH_2$, n = 1)**

Die Aminomethylcyclohexane wurden durch dreistufige Reaktionen aus den entsprechend substituierten Cyclohexanonen über die Stufe der Cyclohexylaldehyde durch Umstzung mit Hydroxylamin Hydrochlorid und anschließender Spaltung mit Lithiumaluminiumhydrid dargestellt.

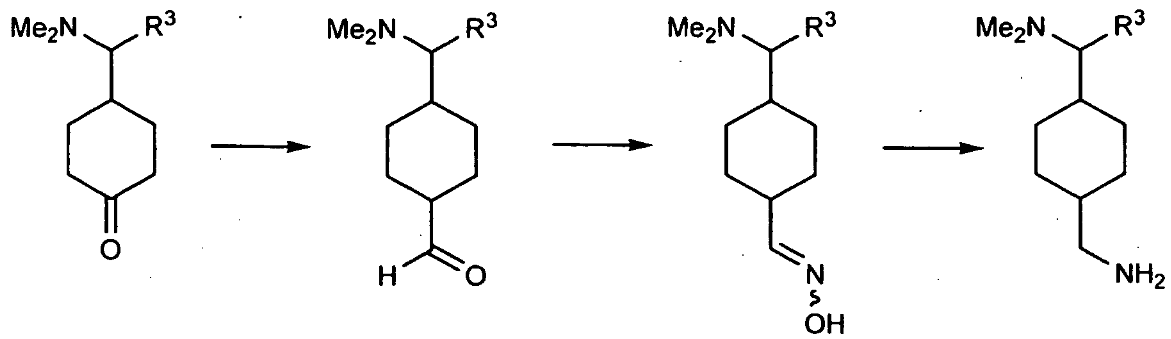

**4-(Dimethylamino-phenyl-methyl)-cyclohexan-carbaldehyd 28 ($R^3$ = Phenyl)**

(Methoxymethyl)triphenylphosphoniumchlorid (31,5 g, 0,092 mol) wurde in absol. THF (150 ml) unter Argon

suspendiert, bei 0 °C tropfenweise mit Kalium-tert-butylat (10, 38 g, 0,092 mol), gelöst in absol. THF (100 ml), versetzt und anschließend 15 min bei 0 °C nachgerührt (Lösung färbte sich tieforange).
Bei RT wurde dann das Keton **10** (14,2 g, 0,061 mol), gelöst in absol. THF (100 ml), zur obigen Lösung zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über $Na_2SO_4$ getrocknet und i. Vak. eingeengt. Das Rohprodukt wurde über eine Kieselgelsäule (300 g) mit Essigester/Cyclohexan (1:1) gereinigt. Ausbeute: 12.2 g (82 %)
$^{13}$C-NMR ($CDCl_3$): 24,01; 24,22; 25,90; 26,06; 26,40; 27,33; 28,21; 29,92;.37,00; 38,19 ($C_4$); 41,51; 41,98; ($N(CH_3)_2$); 47,45; 50,60; 73,37; 75,24 (CH); 126,72 ($C_{arom}$); 126,76 ($C_{arom}$); 127,48 ($C_{arom}$); 129,13 ($C_{arom}$); 136,14 ($C_{arom}$); 136,79 ($C_{arom}$); 204,22; 205.05 (CHO).

**4-(Dimethylamino-phenyl-methyl)-cyclohexan-carbaldehyd-oxim 29 ($R^3$ = Phenyl)**

Der Carbaldehyd **28** (7,36 g, 30 mmol) und Hydroxylamin Hydrochlorid (3,12 g, 45 mmol) wurden in absol. Ethanol (100 ml) gelöst, mit basischem Ionenaustauscher Amberlyst A21 (21 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essig-ester (3 x 50 ml) extrahiert, die organische Phase über Natriumsulfat getrocknet und i. Vak. eingeengt. Ausbeute: 7,81 g (100 %)
$^{13}$C-NMR ($CDCl_3$): 25,83; 26,34; 27,10; 27,55; 28,25; 29,41; 30,12; 30,32; 34,20; 36,45; 36,74; 37,00; 38,19 ($C_4$); 41,37; 41,03; ($N(CH_3)_2$); 72,28; 75,59 (CH); 126,77 ($C_{arom}$); 127,50 ($C_{arom}$); 129,22 ($C_{arom}$); 136,14 ($C_{arom}$); 136,94 ($C_{arom}$); 137,05 ($C_{arom}$); 154,84; 155,55; 156,35.

**[(4-Aminomethyl-cyclohexyl)-phenyl-methyl]-dimethylamin 30 ($R^3$ = Phenyl)**

Absolutes THF (300 ml) wurde unter Argon mit $LiAlH_4$ (2,27 g, 60 mmol) versetzt, auf 60 °C erwärmt und tropfenweise das Oxim **29** (7,81 g, 30 mmol), gelöst in THF (60 ml), zugegeben. Nach 4-stündigem Rühren bei 60 °C wurde unter Eisbadkühlung (10 °C) Wasser (70 ml) zugetropft und die Reaktionslösung über Kieselgur abgesaugt. Der Filterrückstand wurde mit THF gewaschen. Die vereinigten organischen Phasen wurden i. Vak. eingeengt, der Rückstand mit 5N NaOH auf pH 11 eingestellt und mit Essigester (4 x 40 ml) extrahiert. Die organische Phase wurde über Natriumsulfat getrocknet und eingeengt.
Ausbeute: 6,4 g (87 %), Öl
$^{13}$C-NMR ($CDCl_3$): 25,53; 26,03; 26,64; 26,68; 29,06; 30,37; 30,51; 30,67; 30,74; 36,01; 38,83; 38,93; ($C_4$); 41,50; 41,94; ($N(CH_3)_2$); 72,28; 75,59 (CH); 126,77 ($C_{arom}$); 127,50 ($C_{arom}$); 129,22 ($C_{arom}$); 136,14 ($C_{arom}$); 136,94 ($C_{arom}$); 137,05 ($C_{arom}$); 154,84; 155,55; 156,35.

**4-[Dimethylamino-(4-fluorphenyl)-methyl]-cyclohexan-carbaldehyd 31 ($R^3$ = 4-Fluorphenyl)**

(Methoxymethyl)triphenylphosphoniumchlorid (25,7 g, 75 mmol) wurde in absol. THF (100 ml) unter Argon suspendiert, bei 0 °C tropfenweise mit Kalium-tert-butylat (8,42 g, 75 mmol), gelöst in absol. THF (70 ml) versetzt und anschließend 15 min bei 0 °C nachgerührt.
Bei RT wurde dann das Keton **11** (12,44 g, 50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über $Na_2SO_4$ getrocknet und i. Vak. eingeengt. Das Rohprodukt wurde durch Flashchromatographie mit Essigester/Cyclohexan (1:1) gereinigt. Ausbeute: 9,13 g (70 %).
$^1$H-NMR (DMSO, 600 MHz, ausgesuchte Signale): $\delta$ = 1,97 (s, 3 H); 1,99 (s, 3 H); 3,08 (d, 1 H, $J$ = 9,06 Hz); 3,14 (d, 1 H, $J$ = 9,82 Hz); 9,53 (s, 1 H); 9,56 (s, 1 H). $^{13}$C-NMR ($CDCl_3$, beide Diastereomere): $\delta$ = 23,97; 24,21; 25,85; 26,02; 26,17; 27,35; 28,00; 29,90; 37,26; 38,34 ; 41,50; 41,95; 47,36; 50,55; 72,75; 75,84; 114,25; 114,45; 130,33; 130,40; 132,61; 160,41; 162,83; 204,10; 204,93.

**4-[Dimethylamino-(4-fluorphenyl)-methyl]-cyclohexanecarbaldehydoxim 32 ($R^3$ = 4-Fluorphenyl)**

Der Aldehyd **31** (6,50 g, 25 mmol) und Hydroxylamin Hydrochlorid (2,6 g, 37,5 mmol) wurden in absol. Ethanol (80 ml) gelöst, mit basischem Ionenaustauscher Amberlyst A21 (16,5 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essigester (3 x 50 ml) extrahiert, die organischen Phase über Natriumsulfat getrocknet und i. Vak. eingeengt.
Ausbeute: 6,9 g (99 %)

**[(4-Aminomethyl-cyclohexyl)-(4-fluorphenyl)-methyl]-dimethylamin 33 ($R^3$ = 4-Fluorphenyl)**

Absolutes THF (360 ml) wurde unter Argon mit $LiAlH_4$ (1,9 g, 50 mmol) versetzt, auf 60 °C erwärmt und tropfenweise das Oxim **32** (6,9 g, 25 mmol), gelöst in THF (60 ml), zugegeben. Nach 4-stündigem Rühren bei 60 °C wurde unter Eisbadkühlung (10 °C) Wasser (93 ml) zugetropft und die Reaktionslösung über Kieselgur abgesaugt. Der Filterrückstand wurde mit THF gewaschen. Die vereinigten organischen Phasen wurden i. Vak. eingeengt, der Rückstand mit 5N NaOH auf pH 11 eingestellt und dreimal mit Essigester (je 100 ml) extrahiert. Die organische Phase wurde über Natriumsulfat getrocknet und eingeengt.
Ausbeute: 5,4 g (82 %), Öl
$^{13}C$-NMR ($CDCl_3$): 25,25; 25,93; 26,60; 28,75; 30,30; 30,40; 30,67; 36,20; 38,78; 38,93; ($C_4$); 41,24; 41,43 ($N(CH_3)_2$); 48,71; 70,62; 74,69 (CH); 113,97 ($C_{arom}$); 114,04 ($C_{arom}$); 130,24 ($C_{arom}$); 130,31 ($C_{arom}$); 132,94 ($C_{arom}$); 160,19; 162,62; ($C_{arom}$).

**4-[Dimethylamino-(3-fluorphenyl)-methyl]-cyclohexan-carbaldehyd ($R^3$ = 3-Fluorphenyl) 34**

(Methoxymethyl)triphenylphosphoniumchlorid (15,42 g, 45 mmol) wurde in absol. THF (50 ml) unter Argon suspendiert, bei 0 °C tropfenweise mit Kalium-tert-butylat (5,05 g, 45 mmol), gelöst in absol. THF (50 ml), versetzt und anschließend 15 min bei 0 °C nachgerührt.
Bei RT wurde dann das Keton **12** (7,48 g, 0.30 mmol), gelöst in absol. THF (50 ml), zur obigen Lösung zugetropft und über Nacht bei RT gerührt. Unter Eiswasserkühlung wurde tropfenweise mit Wasser (25 ml) und 6N HCl (75 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über $Na_2SO_4$ getrocknet und i. Vak. eingeengt. Das Rohprodukt wurde über durch Flashchromatographie mit Essigester/Cyclohexan (1:1) gereinigt. Ausbeute: 6.55 g (83 %).
Schmelzpunkt: 40-43 °C.
$^1H$-NMR (DMSO, 600 MHz, ausgesuchte Signale): $\delta$ = 1,99 (s, 3 H); 2,01 (s, 3 H); 3,10 (d, 1 H, $J$ = 9,06 Hz); 3,18 (d, 1 H, $J$ = 9,82 Hz); 9,54 (s, 1 H); 9,56 (s, 1 H). $^{13}C$-NMR ($CDCl_3$): 23,93; 24,12; 25,79; 25,95; 26,19; 27,19; 27,99; 29,77; 37,05; 38,16; 41,45; 41,91; 47,30; 50,49; 71,50; 74,78; 113,50; 115,37; 124,78; 128,24; 130,59; 131,24; 131,67; 139,14; 139,76; 160,06; 163,50; 204,01; 204,85.

**4-[Dimethylamino-(3-fluorphenyl)-methyl]-cyclohexan-carbaldehyd-oxim 35 ($R^3$ = 3-Fluorphenyl)**

Der Carbaldehyd **34** (6,32 g, 24 mmol) und Hydroxylamin Hydrochlorid (2,5 g, 36 mmol) wurden in absol. Ethanol (90 ml) gelöst, mit basischem Ionenaustauscher Amberlyst A21 (17 g) versetzt und 3,5 h bei RT gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essigester (3 x 50 ml) extrahiert, die organische Phase über Natriumsulfat getrocknet und im Vakuum eingeengt.
Ausbeute: 6,68 g (100 %)
$^{13}C$-NMR ($CDCl_3$): 25,59; 26,21; 27,38; 28,02; 28,36; 29,27; 29,45; 30,00; 34,14; 35,58; 36,56; 38,19 ($C_4$); 41,33;,41,99; ($N(CH_3)_2$); 72,02; 75,05; 75,19 (CH); 113,55 ($C_{arom}$); 115,62 ($C_{arom}$); 124,88 ($C_{arom}$); 128,78 ($C_{arom}$); 128,86 ($C_{arom}$); 139,84 ($C_{arom}$); 139,90 ($C_{arom}$); 154,38; 155,13; 161,10 ($C_{arom}$); 163,54 ($C_{arom}$).

**[(4-Aminomethyl-cyclohexyl)-(3-fluorphenyl)-methyl]-dimethylamin 36 ($R^3$ = 3-Fluorphenyl)**

Absolutes THF (300 ml) wurde unter Argon mit $LiAlH_4$ (1,82 g, 48 mmol) versetzt, auf 60 °C erwärmt und tropfenweise das Oxim **35** (6,68 g, 24 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Essigester (4 x 40 ml) extrahiert. Die organische Phase wurde über Natriumsulfat getrocknet und eingeengt.
Ausbeute: 5,7 g (90 %), Öl
$^{13}C$-NMR ($CDCl_3$): 25,38; 25,93; 26,44; 28,89; 30,36; 30,45; 30,65; 36,10; 38,87; ($C_4$); 41,33; 41,49; 41,93 ($N(CH_3)_2$); 71,05; 75,11 (CH); 113,94 ($C_{arom}$); 115,53 ($C_{arom}$); 124,86 ($C_{arom}$); 128,59 ($C_{arom}$); 128,67 ($C_{arom}$); 140,14 ($C_{arom}$); 141,21 ($C_{arom}$); 161,03 ($C_{arom}$); 163,46 ($C_{arom}$).

**4-[(4-Chlor-phenyl)-dimethylamino-methyl]-cyclohexancarbaldehyd 37 ($R^3$ = 4-Chlorphenyl)**

(Methoxymethyl)triphenylphosphoniumchlorid (68,55 g, 200 mmol) wurde in absol. THF (200 ml) unter Argon suspendiert, bei 0 °C tropfenweise mit Kalium-tert-butylat (22,44 g, 200 mmol), gelöst in absol. THF (300 ml), versetzt

und anschließend 15 min bei 0 °C nachgerührt.
Bei RT wurde dann das Keton **13** (38 g, 143 mmol), gelöst in absol. THF (200 ml), zur obigen Lösung getropft und über Nacht bei RT gerührt. Unter Eiswasserkühlung wurde tropfenweise mit Wasser (150 ml) und 6N HCl (450 ml) hydrolysiert. Nach 1 h Rühren bei RT wurde mit Ether (10 x 100 ml) extrahiert, die wässrige Phase mit 5N NaOH auf pH 11 gebracht, mit Essigester (3 x 100 ml) ausgeschüttelt, über $Na_2SO_4$ getrocknet und i. Vak. eingeengt. Das Rohprodukt wurde über zwei Kieselgelsäulen (400 g) mit Essigester/Cyclohexan (1:1) gereinigt.
Ausbeute: 32.17 g (80 %).
$^1$H-NMR (DMSO, 600 MHz, ausgesuchte Signale): δ = 1,97 (s, 3 H); 1,99 (s, 3 H); 3,07 (d, 1 H, *J* = 9,07 Hz); 3,14 (d, 1 H, *J* = 9,82 Hz); 9,53 (s, 1 H,; 9,55 (s, 1 H). $^{13}$C-NMR ($CDCl_3$): δ = 23,92; 24,16; 25,80; 25,97; 26,13; 27,25; 27,90; 29,81; 37,08; 38,19; 41,47; 41,96; 47,29; 50,48; 72,81; 74,54 ; 127,65 ; 130,28; 132,40; 134,78; 135,43 ; 203.98; 204.82.

**4-[(4-Chlorphenyl)-dimethylamino-methyl]-cyclohexanecarbaldehydoxim- 38 ($R^3$ = 4-Chlorphenyl)**

Der Carbaldehyd **37** (7,55 g, 27 mmol) und Hydroxylamin Hydrochlorid (2,81 g, 40 mmol) wurden in absol. Ethanol (100 ml) gelöst, mit basischem Ionenaustauscher Amberlyst A21 (19 g) versetzt und 3,5 h bei RT gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essigester (3 x 50 ml) extrahiert, die organische Phase über Natriumsulfat getrocknet und im Vakuum eingeengt.
Ausbeute: 7,57 g (96 %)

**[(4-Aminomethyl-cyclohexyl)-(4-chlorphenyl)-methyl]-dimethylamin 39 ($R^3$ = 4-Chlorphenyl)**

Absolutes THF (300 ml) wurde unter Argon mit $LiAlH_4$ (1,89 g, 50 mmol) versetzt, auf 60 °C erwärmt und tropfenweise das Oxim **38** (7,5 g, 25 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Essigester (4 x 40 ml) extrahiert. Die organische Phase wurde über Natriumsulfat getrocknet und eingeengt.
Ausbeute: 6,3 g (90 %), Öl
$^{13}$C-NMR ($CDCl_3$): 25,22; 25,87; 26,58; 28,70; 30,36; 30,53; 30,59; 36,02; 38,76 ($C_4$); 41,29; 41,39; 41,91 ($N(CH_3)_2$); 45,64; 48,72; 70,72; 74,77 (CH); 127,46 ($C_{arom}$); 127,52 ($C_{arom}$); 130,27 ($C_{arom}$); 132,11 ($C_{arom}$); 132,15 ($C_{arom}$); 134,80 ($C_{arom}$); 135,72 ($C_{arom}$).

**4-(Dimethylamino-thiophen-2-yl-methyl)-cyclohexancarbaldehyd 40 ($R^3$ = 2-Thienyl)**

(Methoxymethyl)triphenylphosphoniumchlorid (20,56 g, 60 mmol) wurde in absol. TH F (70 ml) unter Argon suspendiert, bei 0 °C tropfenweise mit Kalium-tert-butylat (6,73 g, 60 mmol), gelöst in absol. THF (70 ml), versetzt und anschließend 15 min bei 0 °C nachgerührt Bei RT wurde dann das Keton **14** (9,4 g, 40 mmol), gelöst in absol. THF (70 ml), zur obigen Lösung zugetropft und über Nacht bei RT gerührt. Unter Eiswasserkühlung wurde tropfenweise mit Wasser (60 ml) und 6 N HCl (180 ml) hydrolysiert. Nach 1 h Rühren bei RT wurde mit Ether (5 x 50 ml) extrahiert, die wässrige Phase mit 5 N NaOH auf pH 11 gebracht, mit Essigester (3 x 50 ml) ausgeschüttelt, über $Na_2SO_4$ getrocknet und i. Vak. eingeengt. Das Rohprodukt wurde durch Flashchromatographie mit Essigester/Cyclohexan (1:1) gereinigt.
Ausbeute: 7,66 g (77 %).
$^1$H-NMR (DMSO, 600 MHz, ausgesuchte Signale): δ = 2,03 (s, 3 H); 2,05 (s, 3 H); 3,44 (d, 1 H, *J* = 9,82 Hz); 3,52 (d, 1 H, *J* = 10,58 Hz); 9,54 (s, 1 H); 9,58 (s, 1 H). $^{13}$C-NMR ($CDCl_3$): δ = 23,74; 23,83; 25,80; 25,84; 26,98; 27,09; 29,15; 29,68; 39,13; 40,20; 40,98; 41,29 ($N(CH_3)_2$); 47,48; 50,49; 67,81; 69,79; 123,61; 123,70; 125,89; 126,20; 126,24; 139,14; 139,48; 204,07; 204,82.

**4-(Dimethylamino-thiophen-2-yl-methyl)-cyclohexanecarbaldehydoxim 41 ($R^3$ = 2-Thiophen)**

Der Carbaldehyd **40** (7,54 g, 30 mmol) und Hydroxylamin Hydrochlorid (3,12 g, 45 mmol) wurden in absol. Ethanol (100 ml) gelöst, mit basischem Ionenaustauscher Amberlyst A21 (21 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essigester (3 x 50 ml) extrahiert, die organische Phase über Natriumsulfat getrocknet und im Vakuum eingeengt.
Ausbeute: 7,99 g (100 %)

**[(4-Aminomethyl-cyclohexyl)-thiophen-2-yl-methyl]-dimethylamin 42 ($R^3$ = 2-Thiophen)**

Absolutes THF (300 ml) wurde unter Argon mit $LiAlH_4$ (2,27 g, 60 mmol) versetzt, auf 60 °C erwärmt und tropfenweise das Oxim **41** (7,99 g, 30 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Essigester (3 x 50 ml) extrahiert. Die organische Phase wurde über Natriumsulfat getrocknet und eingeengt.
Ausbeute: 6,72 g (89 %), Öl
$^{13}$C-NMR ($CDCl_3$): 25,93; 26,11; 26,24; 26,30; 29,97, 30,34; 30,42; 38,03; 40,65; 40,82; 41,18; 41,34 ($N(CH_3)_2$); 46,19; 48,67; 65,58; 70,06; 123,61; 125,88; 126,23; 140,08.

**4-(1-Dimethylamino-3-phenyl-propyl)-cyclohexan-carbaldehyd 43 ($R^3$ = Phenethyl)**

(Methoxymethyl)triphenylphosphoniumchlorid (20,56 g, 60 mmol) wurde in absol. THF (85 ml) unter Argon suspendiert, bei 0 °C tropfenweise mit Kalium-tert-butylat (6,73 g, 60 mmol), gelöst in absol. THF (70 ml) versetzt und anschließend 15 min bei 0 °C nachgerührt.
Bei RT wurde dann das Keton **15** (10,2 g, 40 mmol), gelöst in absol. THF (60 ml), zur obigen Lösung getropft und über Nacht bei RT gerührt. Unter Eiswasserkühlung wurde tropfenweise mit Wasser (35 ml) und 6N HCl (90 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über $Na_2SO_4$ getrocknet und i. Vak. eingeengt. Das Rohprodukt wurde durch Flashchromatographie mit Essigester / Cyclohexan (1:1) gereinigt.
Ausbeute: 6.73 g (63 %).
$^1$H-NMR (DMSO, 600 MHz, ausgesuchte Signale): $\delta$ = 2,18 (s, 3 H); 2,20 (s, 3 H); 9,54 (s, 1 H); 9,61 (s, 1 H).
$^{13}$C-NMR ($CDCl_3$): $\delta$ = 24,35; 24,49; 26,00; 26,09; 26,85; 27,79; 29,07; 29,13; 35,27; 39,02; 40,98; 41,19; 46,99; 50,33; 66,85; 67,85; 70,54; 71,42; 125,40; 125,44; 128,02; 128,13; 131,15; 131,17 ; 142,45; 204,10; 205,01.

**4-(1-Dimethylamino-3-phenyl-propyl)-cyclohexanecarbaldehydoxim 44 ($R^3$ = Phenethyl)**

Der Aldehyd 43 (6,55 g, 24 mmol) und Hydroxylamin Hydrochlorid (2,5 g, 36 mmol) wurden in absol. Ethanol (90 ml) gelöst, mit basischem Ionenaustauscher Amberlyst A21 (15,6 g) versetzt und über Nacht bei RT gerührt. Der Ionenaustauscher wurde abfiltriert und zweimal mit Ethanol (je 50 ml) gewaschen. Die Lösung wurde eingeengt und der Rückstand mit 5N NaOH auf pH 11 eingestellt. Die wässrige Phase wurde dreimal mit Essigester (je 50 ml) extrahiert, die organische Phase über Natriumsulfat getrocknet und im Vakuum eingeengt.
Ausbeute: 6,90 g (100 %)

**[1-(4-Aminomethyl-cyclohexyl)-3-phenyl-propyl]-dimethylamin 45 ($R^3$ = Phenethyl)**

Absolutes THF (360 ml) wurde unter Argon mit $LiAlH_4$ (1,82 g, 48 mmol) versetzt, auf 60 °C erwärmt und tropfenweise das Oxim **44** (6,90 g, 24 mmol), gelöst in THF (60 ml), dazu gegeben. Nach 4-stündigem Rühren bei 60 °C wurde unter Eisbadkühlung (10 °C) Wasser (90 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Essigester (4 x 40 ml) extrahiert. Die organische Phase wurde über Natriumsulfat getrocknet und eingeengt.
Ausbeute: 5,6 g (85 %), Öl
$^{13}$C-NMR ($CDCl_3$): 25,93; 26,58; 27,09; 29,21; 29,90; 30,32; 30,73; 30,77; 35,38; 35,66; 38,73; ($C_4$); 40,06; 40,90; 41,19 ($N(CH_3)_2$); 48,78; 65,15; 68,22 (CH); 125,36;
127,99; 128,05; 142,69.

**Synthese der Aminoethylcyclohexane ($R^1 = (CH_2)_nNH_2$, n = 2)**

Die Aminoethylcyclohexane wurden durch dreistufige Reaktionen aus den entsprechend substituierten Cyclohexylaldehyde durch Kettenverlängerung (Wittig) und Umsetzung mit Hydroxylamin Hydrochlorid und anschließender Spaltung mit Lithiumaluminiumhydrid dargestellt.

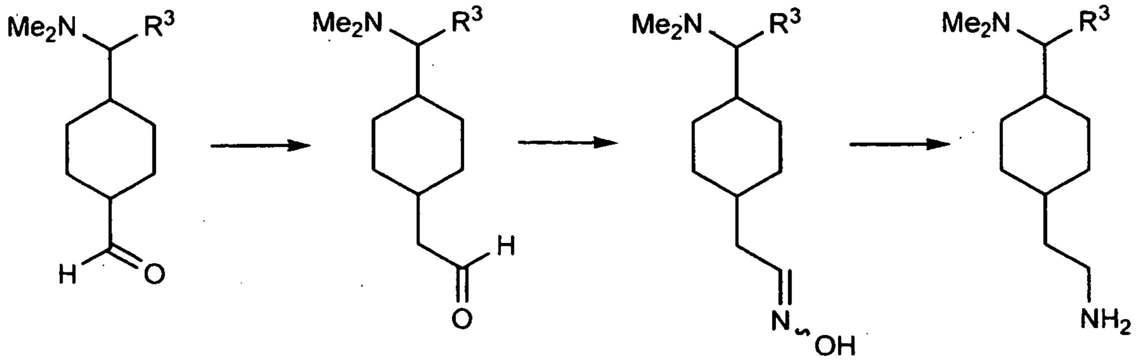

**[4-(Dimethylamino-phenyl-methyl)-cyclohexyl]-acetaldehyd 46 ($R^3$ = Phenyl)**

(Methoxymethyl)triphenylphosphoniumchlorid (38,39 g, 0,112 mol) wurde in absol. THF (150 ml) unter Argon suspendiert, bei 0 °C tropfenweise mit Kalium-tert-butylat (12,56 g, 0,112 mol), gelöst in absol. THF (120 ml), versetzt und anschließend 15 min bei 0 °C nachgerührt (die Lösung färbte sich tieforange).
Bei RT wurde dann der Aldehyd **28** (18,4 g, 0,075 mol), gelöst in absol. THF (120 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde mit Ether (10 x 100 ml) extrahiert. Die wässrige Phase wurde mit 5N NaOH auf pH 11 gebracht, mit Essigester (3 x 80 ml) ausgeschüttelt, über $Na_2SO_4$ getrocknet und im Vakuum eingeengt. Das Rohprodukt wurde durch Flashchromatographie mit Essigester/Cyclohexan (1:1) gereinigt.
Ausbeute: 16,31 g (84 %), Öl
$^{13}$C-NMR ($CDCl_3$): 25,30; 25,92; 29,04; 29,19; 29,74; 30,86; 32,99; 33,02; 35,98; 38,31 ($C_4$); 41,42; 42,06; ($N(CH_3)_2$); 48,04; 51,24; 71,82; 75,47 (CH); 126,64 ($C_{arom}$); 126,68 ($C_{arom}$); 127,39 ($C_{arom}$); 127,46 ($C_{arom}$); 129,15 ($C_{arom}$); 136,20 ($C_{arom}$);
137,11 ($C_{arom}$); 202,27; 202,37 (CHO).

**[4-(Dimethylamino-phenyl-methyl)-cyclohexyl]-acetaldehyd-oxim 47 ($R^3$ = Phenyl)**

Der Carbaldehyd **46** (11,04 g, 42,5 mmol) und Hydroxylamin Hydrochlorid (4,44 g, 64 mmol) wurden in absol. Ethanol (150 ml) gelöst, mit basischem Ionenaustauscher Amberlyst A21 (30 g) versetzt und 4 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über Natriumsulfat getrocknet und i. Vak. eingeengt.
Ausbeute: 11,66 (100 %)
$^{13}$C-NMR ($CDCl_3$): 25,41; 25,57; 28,87; 29,11; 30,92; 30,97; 32,33; 32,99; 33,67; 35,99; 36,10; 38,59 ($C_4$); 41,31; 41,40; 42,11; 42,14 ($N(CH_3)_2$); 71,74; 75,63 (CH); 126,71 ($C_{arom}$); 127,46 ($C_{arom}$); 129,26 ($C_{arom}$); 137,26 ($C_{arom}$); 150,95; 151,37; 151,56 (C=N-O).

**2-[4-Dimethylamino-phenyl-methyl)-cyclohexyl]-ethylamin 48 ($R^3$ = Phenyl)**

Absolutes THF (400 ml) wurde unter Argon mit $LiAlH_4$ (3,22 g, 85 mmol) versetzt, auf 60 °C erwärmt und tropfenweise das Oxim **47** (11,66 g, 42,5 mmol), gelöst in THF (80 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (4 x 50 ml) extrahiert. Die organische Phase wurde über Natriumsulfat getrocknet und eingedampft.
Ausbeute: 9,15 g (83 %), Öl
$^{13}$C-NMR ($CDCl_3$): 25,58; 26,08; 29,16; 29,21; 30,39; 31,10; 32,49; 33,16; 33,33; 35,54; 36,22; 38,80 ($C_4$); 40,32; 41,36; 41,50; 42,11; ($N(CH_3)_2$); 71,77; 75,66 (CH); 126,52 ($C_{arom}$); 127,31 ($C_{arom}$); 127,38 ($C_{arom}$); 129,18 ($C_{arom}$); 139,39 ($C_{arom}$); 137,41 ($C_{arom}$).

**{4-[Dimethylamino-(4-fluorphenyl)-methyl]-cyclohexyl}-acetaldehyd 49 ($R^3$ = 4-Fluorphenyl)**

(Methoxymethyl)triphenylphosphoniumchlorid (43,53 g, 127 mmol) wurde in absol. THF (200 ml) unter Argon suspendiert, bei 0 °C tropfenweise mit Kalium-tert-butylat (14,25 g, 127 mmol), gelöst in absol. THF (130 ml), versetzt und anschließend 15 min bei 0 °C nachgerührt.
Bei RT wurde dann der Aldehyd 31 (22,3 g, 85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Ether (je 100 ml) extrahiert. Die wässrige Phase wurde mit 5N NaOH auf pH 11 gebracht, dreimal mit Essigester (je 100 ml) ausgeschüttelt, über $Na_2SO_4$ getrocknet und i. Vak. eingeengt. Das Rohprodukt wurde durch Flashchromatographie mit Essigester/Cyclohexan (1:1) gereinigt. Ausbeute: 15,8 g (67 %)
$^{13}$C-NMR ($CDCl_3$): δ = 25,08; 25,87; 28,80; 29,10; 29,13; 29,62; 30,82; 32,90; 33,08; 36,19; 38,43; 41,36; 42,01; 47,94; 51,17; 71,11; 74,69; 114,11; 114,20; 114,32; 130,32; 130,40; 132,00; 132,92; 160,31; 162,74; 202,15; 202,23.

**{4-[Dimethylamino-(4-fluorphenyl)-methyl]-cyclohexyl}-acetaldehydoxim 50 ($R^3$ = 4-Fluorphenyl)**

Der Carbaldehyd **49** (5,30 g, 20,0 mmol) und Hydroxylamin Hydrochlorid (2,08 g, 30 mmol) wurden in absol. Ethanol (90 ml) gelöst, mit basischem Ionenaustauscher Amberlyst A21 (14,8 g) versetzt und über Nacht bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 100 ml) extrahiert, die organische Phase über Natriumsulfat getrocknet und i. Vak. eingeengt. Der Rückstand wurde durch Flashchromatographie mit EE/Cyclohexan (2:1) gereinigt.
Ausbeute: 3,50 (60 %)

**2-{4-[Dimethylamino-(4-fluorphenyl)-methyl]-cyclohexyl}-ethylamin 51 ($R^3$ = 4-Fluorphenyl)**

Absolutes THF (450 ml) wurde unter Argon mit $LiAlH_4$ (2,35 g, 62 mmol) versetzt, auf 60 °C erwärmt und tropfenweise das Oxim 50 (9,10 g, 31,0 mmol), gelöst in THF (75 ml), addiert. Nach 4-stündigem Rühren bei 60 °C wurde unter Eisbadkühlung (10 °C) Wasser (116 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (4 x 50 ml) extrahiert. Die organische Phase wurde über Natriumsulfat getrocknet und i. Vak. eingeengt.
Ausbeute: 6,80 g (79 %), Öl
$^{13}$C-NMR ($CDCl_3$): 25,32; 26,03; 28,94; 29,08; 30,37; 31,06; 32,39; 32,90; 33,07; 33,26; 35,50; 37,81; 38,80 39,78 ($C_4$); 41,33; 41,42; 42,09 ($N(CH_3)_2$); 71,11; 74,89 (CH); 114,03; 114,11; 130,32; 130,40; 132,19; 133,18; 133,21; 160,27; 162,69.

**{4-[Dimethylamino-(3-fluorphenyl)-methyl]-cyclohexyl}-acetaldehyd 52 ($R^3$ = 3-Fluorphenyl)**

(Methoxymethyl)triphenylphosphoniumchlorid (26,73 g, 78 mmol) wurde in absol. THF (90 ml) unter Argon suspendiert, bei 0 °C tropfenweise mit Kalium-*tert* butylat (8,75 g, 78 mmol), gelöst in absol. THF (90 ml), versetzt und anschließend 15 min bei 0 °C nachgerührt.
Bei RT wurde dann der Aldehyd **34** (13,69 g, 52 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über $Na_2SO_4$ getrocknet und i. Vak. eingeengt. Das Rohprodukt wurde durch Flashchromatographie mit Essigester/Cyclohexan (1:1) gereinigt.
Ausbeute: 12,61 g (87 %)
$^{13}$C-NMR ($CDCl_3$): δ = 25,19; 25,83; 28,90; 29,06; 29,14; 29,68; 30,77; 32,92; 32,98; 33,10; 36,05; 38,36; 41,39; 42,04; 48,02; 51,20; 71,48; 75,07; 113,43; 113,49; 1.13,64; 113,69; 115,55; 115,76; 124,89; 128,70; 128,78; 128,88; 139,24; 140,08; 140,14; 161,09; 163,52; 202,19; 202,27.

**{4-[Dimethylamino-(3-fluorphenyl)-methyl]-cyclohexyl}-acetaldehydoxim 53 ($R^3$ = 3-Fluorphenyl)**

Der Carbaldehyd **52** (7,18 g, 25,8 mmol) und Hydroxylamin Hydrochlorid (2,71 g, 39 mmol) wurden in absol. Ethanol (90 ml) gelöst, mit basischem Ionenaustauscher Amberlyst A21 (20 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über

Natriumsulfat getrocknet und i. Vak. eingeengt.
Ausbeute: 7,54 (100 %)

**2-{4-[Dimethylamino-(3-fluorphenyl)-methyl]-cyclohexyl}-ethylamin 54 ($R^3$ = 3-Fluorphenyl)**

Absolutes THF (300 ml) wurde unter Argon mit $LiAlH_4$ (1,97 g, 52 mmol) versetzt, auf 60°C erwärmt und tropfenweise das Oxim **53** (7,54 g, 25,8 mmol), gelöst in THF (70 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (3 x 50 ml) extrahiert. Die organische Phase wurde über Natriumsulfat getrocknet und i. Vak. eingeengt.
Ausbeute: 6,3 g (88 %), Öl
$^{13}$C-NMR ($CDCl_3$): 25,28; 25,84; 28,87; 28,98; 30,28; 32,30; 32,93; 33,13; 35,38; 36,16; 37,81; 38,69 ($C_4$); 39,69; 41,20; 41,37; 41,94 ($N(CH_3)_2$); 71,29; 75,11 (CH); 113,14; 113,18; 113,38; 115,41; 115,62; 124,73; 128,44; 128,53; 139,25; 140,27; 140,33; 160,91; 163,34.

**{4-[(4-Chlorphenyl)-dimethylamino-methyl]-cyclohexyl}-acetaldehyd 55 ($R^3$ = 4-Chlorphenyl)**

Methoxymethyl)triphenylphosphoniumchlorid (25,02 g, 73 mmol) wurde in absol. THF (90 ml) unter Argon suspendiert, bei 0 °C tropfenweise mit Kalium-*tert*-butylat (8,19 g, 73 mmol), gelöst in absol. THF (90 ml), versetzt und anschließend 15 min bei 0 °C nachgerührt.
Bei RT wurde dann der Aldehyd **37** (13,86 g, 49 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über $Na_2SO_4$ getrocknet und i. Vak. eingeengt. Das Rohprodukt wurde durch Flashchromatographie mit Essigester/Cyclohexan (1:1) gereinigt. Ausbeute: 12,07 g (84%).
$^{13}$C-NMR ($CDCl_3$): $\delta$ = 25,06; 25,82; 28,74; 29,00; 29,13; 29,60; 30,77; 32,87; 32,94; 33,07; 36,06; 38,32; 41,38; 42,05; 47,95; 51,17; 71,23; 74,80; 127,58; 127,66; 130,31; 132,28; 132,34; 134,81; 135,77; 202,12; 202,20.

**{4-[Dimethylamino-(4-chlorphenyl)-methyl]-cyclohexyl}-acetaldehydoxim 65 ($R^3$ = 4-Chlorphenyl)**

Der Carbaldehyd **55** (6,72 g, 22,8 mmol) und Hydroxylamin Hydrochlorid (2,36 g, 34 mmol) wurden in absol. Ethanol (90 ml) gelöst, mit basischem Ionenaustauscher
Amberlyst A21 (16 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über Natriumsulfat getrocknet und i. Vak. eingeengt.
Ausbeute: 7,04 (100 %)

**2-{4-[Dimethylamino-(4-chlorphenyl)-methyl]-cyclohexyl}-ethylamin 66 ($R^3$ = 4-Chlorphenyl)**

Absolutes THF (300 ml) wurde unter Argon mit $LiAlH_4$ (1,73 g, 45,6 mmol) versetzt, auf 60°C erwärmt und tropfenweise das Oxim **65** (7,04 g, 22,8 mmol), gelöst in THF (60 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (3 x 50 ml) extrahiert. Die organische Phase wurde über Natriumsulfat getrocknet und i. Vak. eingeengt.
Ausbeute: 5,76 g (86 %), Öl
$^{13}$C-NMR ($CDCl_3$): 25,67; 26,35; 29,23; 29,44; 30,74, 31,39; 33,41; 33,61; 35,86; 36,71; 38,20; 39,18; 40,17; 40,67; 41,72; 41,81; 42,50 ($N(CH_3)_2$); 71,59; 75,37; 127,86; 127,95; 130,70; 132,52; 135,38; 136,45.

**{4-[Dimethylamino-thiophen-2-yl-methyl]-cyclohexyl}-acetaldehyd 67 ($R^3$ = 2-Thiophen)**

(Methoxymethyl)triphenylphosphoniumchlorid (28,79 g, 84 mmol) wurde in absol. THF (100 ml) unter Argon suspendiert, bei 0 °C tropfenweise mit Kalium-*tert*-butylat (9,42 g, 84 mmol), gelöst in absol. THF (100 ml), versetzt und anschließend 15 min bei 0 °C nachgerührt.
Bei RT wurde dann der Aldehyd **40** (14,08 g, 56 mmol), gelöst in absol. THF (100 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11

gebracht, dreimal mit Essigester (je 100 ml) ausgeschüttelt, über $Na_2SO_4$ getrocknet und i. Vak. eingeengt. Das Rohprodukt wurde durch Flashchromatographie mit Essigester/Cyclohexan (1:2) gereinigt. Ausbeute: 11,48 g (77%).
$^{13}$C-NMR ($CDCl_3$): δ = 25,80; 25,88; 28,73; 29,95; 30,49, 32,23; 32,76; 37,89; 40,21; 40,88; 41,23; 48,36; 51,09; 66,02; 69,97; 123,19; 123,72; 125,95; 126,31; 139,42; 139,91; 202,61.

**[4-(Dimethylamino-thiophen-2-yl-methyl)-cyclohexyl]-acetaldehydoxim 68 ($R^3$ = 2-Thiophen)**

Der Carbaldehyd **67** (6,3 g, 23,7 mmol) und Hydroxylamin Hydrochlorid (2,5 g, 36 mmol) wurden in absol. Ethanol (90 ml) gelöst, mit basischem Ionenaustauscher Amberlyst A21 (20 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über Natriumsulfat getrocknet und i. Vak. eingeengt.
Ausbeute: 6,64 (100 %)

**2-[4-(Dimethylamino-thiophen-2-yl-methyl)-cyclohexyl]-ethylamin 69 ($R^3$ = 2-Thiophen)**

Absolutes THF (250 ml) wurde unter Argon mit $LiAlH_4$ (1,78 g, 47 mmol) versetzt, auf 60 °C erwärmt und tropfenweise das Oxim **68** (6,64 g, 23,7 mmol), gelöst in THF (60 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (3 x 50 ml) extrahiert. Die organische Phase wurde über Natriumsulfat getrocknet und i. Vak. eingeengt.
Ausbeute: 5,62 g (89 %), Öl
$^{13}$C-NMR ($CDCl_3$): 25,97; 26,13; 28,72; 28,79; 30,15, 30,23; 30,74; 32,61; 32,90; 35,32; 38,22; 39,70; 40,09; 40,69; 40,84; 41,26 ($N(CH_3)_2$); 70,14; 123,56; 123,60; 125,86; 126,21; 126,23; 139,70; 140,24.

**[4-(1-Dimethylamino-3-phenyl-propyl)-cyclohexyl]-acetaldehyd 70 ($R^3$ = Phenethyl)**

(Methoxymethyl)triphenylphosphbniumchlorid (50,3 g, 147 mmol) wurde in absol. THF (150 ml) unter Argon suspendiert, bei 0 °C tropfenweise mit Kalium-*tert*-butylat (16,5 g, 147 mmol), gelöst in absol. THF (140 ml), versetzt und anschließend 15 min bei 0 °C nachgerührt.
Bei RT wurde dann der Aldehyd **43** (27,0 g, 98 mmol), gelöst in absol. THF (150 ml), zugetropft und über Nacht bei RT gerührt. Unter Eiswasserkühlung wurde tropfenweise mit Wasser (102 ml) und 6N HCl (240 ml) hydrolysiert. Nach 1 h Rühren bei RT wurde fünfmal mit Ether (je 200 ml) extrahiert. Die wässrige Phase wurde unter Eiskühlung mit 5 N NaOH auf pH 11 gebracht, dreimal mit Essigester (je 200 ml) ausgeschüttelt, über $Na_2SO_4$ getrocknet und i. Vak. eingeengt. Das Rohprodukt wurde durch Flashchromatographie mit Essigester/Cyclohexan (1: 1) gereinigt. Ausbeute: 18,1 g (64 %)
$^{13}$C-NMR ($CDCl_3$): δ = 25,55; 26,19; 29,04; 29,15; 29,35; 29,85; 31,00; 32,87; 32,68; 33,04; 35,33; 38,49; 40,86; 41,13; 47,51; 51,15; 65,48; 68,09.

**[4-(1-Dimethylamino-3-phenyl-propyl)-cyclohexyl]-acetaldehydeoxim 71 ($R^3$ = Phenethyl)**

Der Carbaldehyd **70** (12,6 g, 44,0 mmol) und Hydroxylamin Hydrochlorid (4,60 g, 66,0 mmol) wurden in absol. Ethanol (200 ml) gelöst, mit basischem Ionenaustauscher Amberlyst A21 (32 g) versetzt und über Nacht bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Essigester (3 x 50 ml) extrahiert, die organische Phase über Natriumsulfat getrocknet und i. Vak. eingeengt.
Ausbeute: 13,3 (100 %)

**{1-[4-(2-Amino-ethyl)-cyclohexyl]-3-phenyl-propyl}-dimethylamin 72 ($R^3$ = Phenethyl)**

Absolutes THF (600 ml) wurde unter Argon mit $LiAlH_4$ (4,25 g, 112 mmol) versetzt, auf 60 °C erwärmt und tropfenweise das Oxim **71** (17,1 g, 56,0 mmol), gelöst in THF (150 ml), addiert. Nach 4-stündigem Rühren bei 60 °C wurde unter Eisbadkühlung (10 °C) Wasser (360 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Essigester (5 x 100 ml) extrahiert. Die organische Phase wurde über Natriumsulfat getrocknet und i. Vak. eingeengt.
Ausbeute: 16,2 g (100 %), Öl
$^{13}$C-NMR ($CDCl_3$): 25,67; 26,44; 29,07; 29,16; 30,05, 30,22; 31,32; 31,80; 33,30; 35,24; 35,37; 37,26; 39,77; 40,30;

40,85; 41,15; 41,40 ($N(CH_3)_2$); 65,61; 68,29; 125,53; 127,68; 128,16; 128,200; 142,91.

**Synthese der Harnstoff-Derivate ($R^1 = (CH_2)_nNHCONHR^{10}$)**

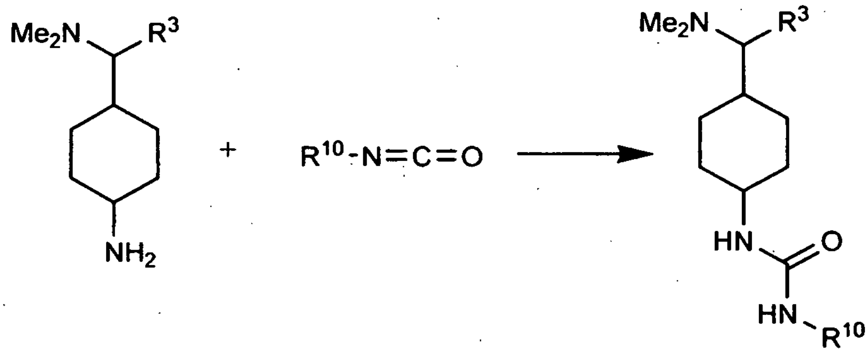

**1-(4-((Dimethylamino)(phenyl)methyl)cyclohexyl)-3-(naphthalen-1-yl)harnstoff 73 ($R^3$ = Phenyl)**

Das Amin **17** (0,5g, 2,15 mmol) wurde in Toluol (21 ml) vorgelegt und unter Rühren mit 1-Naphthylisocyanat (0,36g, 2,15 mmol) versetzt. Anschließend wurde 4h bei 115°C gerührt und anschließend über Nacht bei Raumtemperatur stehengelassen, wobei ein Feststoff ausfiel. Der entstandene Feststoff wurde abfiltriert und mit Essigester gewaschen. Die Mutterlauge wurde am Rotationsverdampfer zur Trockne eingeengt und mit Essigester versetzt. Der hierbei ausgefallene Feststoff wurde abgetrennt, mit Essigester gewaschen und mit der ersten Fällung vereinigt.
$^1$H NMR (600 MHz, DMSO) 0,76 - 1,00 (m, 2 H) 1,05 - 1,31 (m, 2 H) 1,32 - 1,44 (m, 1 H) 1,78 - 2,06 (m, 10 H) 2,06 - 2,16 (m, 1 H) 3,07 (d, *J*=9,06 Hz, 1 H) 6,50 (d, *J*=7,55 Hz, 1 H) 7,16 (d, *J*=7,55 Hz, 2 H) 7,26 (t, *J*=7,18 Hz, 2 H) 7,34 (t, *J*=7,55 Hz, 1 H) 7,39 (t, *J*=7,55 Hz, 1 H) 7,52 (td, *J*=13,60, 6,80 Hz, 3 H) 7,88 (d, *J*=7,55 Hz, 1 H) 8,01 (d, *J*=7,55 Hz, 1 H) 8,05 (d, *J*=8,31 Hz, 1 H) 8,39 (s, 1 H).

**1-(2,4-Difluorphenyl)-3-(4-((dimethylamino)(phenyl)methyl)cyclohexyl) harnstoff Hydrochlorid 74 ($R^3$ = Phenyl)**

Das Amin 17 (0,5g, 2,15 mmol) wurde in Toluol (21 ml) vorgelegt und unter Rühren mit 2,4-Difluorphenylisocyanat (0,33g, 2,15 mmol) versetzt. Anschließend wurde 4h bei 115°C gerührt und anschließend über Nacht bei Raumtemperatur stehengelassen, wobei ein Feststoff ausfiel. Die Reaktionslösung wurde am Rotationsverdampfer zur Trockne eingeengt und durch Flash-Chromatographie gereinigt (Essigester/Methanol 20 : 1).
Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.
$^1$H NMR (600 MHz, DMSO) 0,89 - 0,97 (m, 2 H) 1,10 - 1,17 (m, 1 H) 1,19 - 1,26 (m, 1 H) 1,45 - 1,52 (m, 1 H) 1,81 (d, *J*=12,84 Hz, 1 H) 1,93 (t, *J*=12,84 Hz, 2 H) 2,23 - 2,30 (m, 1 H) 2,57 - 2,62 (m, 3 H) 2,62 - 2,67 (m, 3 H) 3,22 - 3,28 (m, 1 H) 4,22 - 4,27 (m, 1 H) 6,59 (d, *J*=6,80 Hz, 1 H) 6,93 - 6,98 (m, *J*=5,29 Hz, 1 H) 7,20 - 7,25 (m, 1 H) 7,47 - 7,52 (m, 5 H) 8,01 - 8,07 (m, 1 H) 8,13 - 8,18 (m, 1 H) 10,00 (s, 1 H).

**1-(4-((Dimethylamino)(phenyl)methyl)cyclohexyl)-3-(3-(trifluonnethyl)phenyl)-harnstoff Hydrochlorid 75 ($R^3$ = Phenyl)**

Das Amin **17** (0,5g, 2,15 mmol) wurde in Toluol (21 ml) vorgelegt und unter Rühren mit 3-(Trifluormethyl) phenylisocyanat (0,40g, 2,15 mmol) versetzt. Anschließend wurde 4h bei 115°C gerührt und anschließend über Nacht bei Raumtemperatur stehengelassen, wobei ein Feststoff ausfiel. Der entstandene Feststoff wurde abfiltriert und mit Essigester gewaschen. Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.
1 H NMR (600 MHz, DMSO) 0,84 - 1,00 (m, 2 H) 1,11 - 1,34 (m, 2 H) 1,50 - 1,63 (m, 1 H) 1,76 - 1,88 (m, 1 H) 1,89 - 2,01 (m, 2 H) 2,19 - 2,32 (m, 1 H) 2,54 - 2,62 (m, 3 H) 2,63 - 2,72 (m, 3 H) 3,18 - 3,31 (m, 1 H) 4,20 - 4,29 (m, 1 H) 7,10

(t, *J*=7,55 Hz, 1 H) 7,42 - 7,57 (m, 6 H) 7,62 (t, *J*=7,93 Hz, 1 H) 8,03 (d, J=8,31 Hz, 1 H) 8,28 (d, *J*=8,31 Hz, 1 H) 9,30 (s, 1 H), 10,35 (s, 1 H).

**1-(4-((Dimethylamino)(phenyl)methyl)cyclohexyl)-3-(2-nitrophenyl)harnstoff Hydrochlorid 76 ($R^3$ = Phenyl)**

Das Amin 17 (0,5g, 2,15 mmol) wurde in Toluol (21 ml) vorgelegt und unter Rühren mit 2-Nitrophenylisocyanat (0,35g, 2,15 mmol) versetzt. Anschließend wurde 4h bei 115°C gerührt und anschließend über Nacht bei Raumtemperatur stehengelassen, wobei ein Feststoff ausfiel. Die Reaktionslösung wurde am Rotationsverdampfer zur Trockne eingeengt und durch Flash-Chromatographie gereinigt (Essigester/Methanol 20:1).
Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden. 1 H NMR (600 MHz, 0,89 - 0,97 (m, 2 H) 1,13 - 1,20 (m, 1 H) 1,22 - 1,30 (m, 1 H) 1,45 - 1,52 (m, 1 H) 1,79 - 1,85 (m, 1 H) 1,91 -1,98 (m, 2 H) 2,22 - 2,29 (m, 1 H) 2,57 - 2,62 (m, 3 H) 2,63 - 2,67 (m, 3 H) 3,23 - 3,31 (m, 1 H) 4,22 - 4,29 (m, 1 H) 6,39 - 6,45 (m, 1 H) 7,17 - 7,23 (m, 1 H) 7,41 - 7,46 (m, 2 H) 7,47 - 7,53 (m, 5 H) 7,95 (s, 1 H) 8,98 (s, 1 H) 10,01 (s, 1 H).

**1-(3-Bromphenyl)-3-(4-((dimethylamino)(phenyl)methyl)cyclohexyl)harnstoff Hydrochlorid 77 ($R^3$ = Phenyl)**

Das Amin **17** (0,5g, 2,15 mmol) wurde in Toluol (21 ml) vorgelegt und unter Rühren mit 3-Bromphenylisocyanat (0,43g, 2,15 mmol) versetzt, Anschließend wurde 4h bei 115°C gerührt und anschließend über Nacht bei Raumtemperatur stehengelassen, wobei ein Feststoff ausfiel. Die Reaktionslösung wurde am Rotationsverdampfer zur Trockne eingeengt und durch Flash-Chromatographie gereinigt (Essigester/Methanol 20:1).

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden. $^1H$ NMR (600 MHz, DMSO-$d_6$) d ppm 0,85 (t, *J*=7,55 Hz, 2 H) 1,04 - 1,26 (m, 2 H) 1,43 - 1,61 (m, 1 H) 1,67 - 1,80 (m, 1 H) 1,81 - 1,94 (m, 1 H)2,11 - 2,23 (m, 1 H) 2,37 (q, *J*=7,30 Hz, 1 H) 2,48 - 2,55 (m, 3 H) 2,56 - 2,64 (m, 3 H) 3,10 - 3,23 (m, 1 H) 4,19 (t, *J*=6,04 Hz, 1 H) 6,36 - 6,48 (m, 1 H) 6,96 (d, *J*=7,55 Hz, 1 H) 7,08 (t, *J*=7,93 Hz, 1 H) 7,14 (d, 1 H) 7,31 - 7,58 (m, *J*=5,29 Hz, 4 H) 7,71 (s,1 H) 8,93 (s, 1 H); 10,28 (s, 1 H).

**1-(4-((Dimethylamino)(phenyl)methyl)cyclohexyl)-3-phenylharnstoff Hydrochlorid 78 ($R^3$ = Phenyl)**

Das Amin **17** (0,5g, 2,15 mmol) wurde in Toluol (21 ml) vorgelegt und unter Rühren mit Phenylisocyanat (0,43g, 2,15 mmol) versetzt. Anschließend wurde 4h bei 115°C gerührt und anschließend über Nacht bei Raumtemperatur stehengelassen, wobei ein Feststoff ausfiel. Der Feststoff wurde durch Flash-Chromatographie gereinigt (Essigester/ Methanol 20 : 1).

**1-Benzyl-3-(4-((dimethylamino)(phenyl)methyl)cyclohexyl)harnstoff 79 ($R^3$ = Phenyl)**

Das Amin **17** (0,35g, 1,5 mmol) wurde in Toluol (15 ml) vorgelegt und unter Rühren mit Benzylisocyanat (0,20g, 1,5 mmol) versetzt. Anschließend wurde 4h bei 115°C gerührt und anschließend über Nacht bei Raumtemperatur stehengelassen. Die Reaktionslösung wurde am Rotationsverdampfer zur Trockne eingeengt und mit Essigster versetzt. Der entstandene Feststoff wurde abgesaugt und mit Essigester gewaschen.

**1-Cyclohexyl-3-(4-((dimethylamino)(phenyl)methyl)cyclohexyl)harnstoff 80 ($R^3$ = Phenyl)**

Das Amin **17** (0,35g, 1,5 mmol) wurde in Toluol (15 ml) vorgelegt und unter Rühren mit Cyclohexylisocyanat (0,19g, 1,5 mmol) versetzt. Anschließend wurde 4h bei 115°C gerührt und anschließend über Nacht bei Raumtemperatur stehengelassen. Die Reaktionslösung wurde am Rotationsverdampfer zur Trockne eingeengt und mit Essigster versetzt. Der entstandene Feststoff wurde abgesaugt und mit Essigester gewaschen.

**1-(4-Bromphenyl)-3-(4-((dimethylamino)(phenyl)methyl)cyclohexyl)harnstoff 81 ($R^3$ = Phenyl)**

Das Amin **17** (0,35g, 1,5 mmol) wurde in Toluol (15 ml) vorgelegt und unter Rühren mit 4-Bromphenylisocyanat (0,30g, 1,5 mmol) versetzt. Anschließend wurde 4h bei 115°C gerührt und anschließend über Nacht bei Raumtemperatur stehengelassen. Die Reaktionslösung wurde am Rotationsverdampfer zur Trockne eingeengt und mit Essigster versetzt. Der entstandene Feststoff wurde abgesaugt und mit Essigester gewaschen.

**1-(4-((Dimethylamino)(phenyl)methyl)cyclohexyl)-3-(4-methoxyphenyl)harnstoff 82 ($R^3$ = Phenyl)**

Das Amin **17** (0,35g, 1,5 mmol) wurde in Toluol (15 ml) vorgelegt und unter Rühren mit 4-Methoxyphenylisocyanat (0,22g, 1,5 mmol) versetzt. Anschließend wurde 4h bei 115°C gerührt und anschließend über Nacht bei Raumtemperatur stehengelassen. Die Reaktionslösung wurde am Rotationsverdampfer zur Trockne eingeengt und mit Essigster versetzt. Der entstandene Feststoff wurde abgesaugt und mit Essigester gewaschen.

**Synthese der Thioharnstoff-Derivate ($R^1 = (CH_2)_nNHCSNHR^{10}$)**

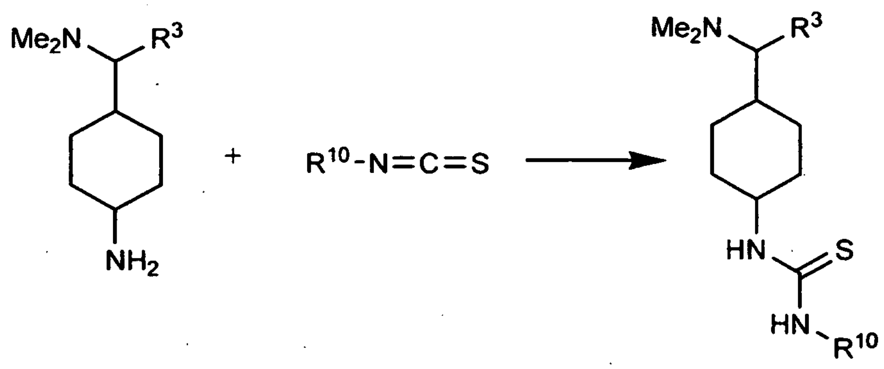

Die Thioharnstoff-Derivate werden analog der beschriebenen Methoden der Harnstoff-Derivat-Synthese erhalten, wobei statt Isocyanaten die entsprechenden Isothiocyanate eingesetzt werden.

**Reduktive Aminierung der primären Amine ($R^1 = (CH_2)_nNR^6R^7$)**

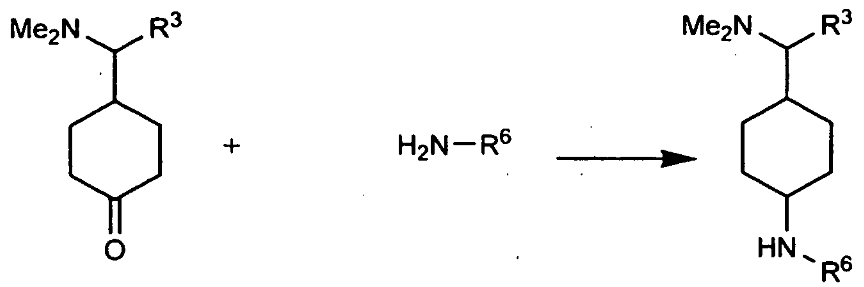

**N-(2-(1H-Indol-3-yl)ethyl)-4-((dimethylamino)(phenyl)methyl)cyclohexanamin hydrochlorid 83 ($R^3$ = Phenyl)**

Das Keton **10** (1,0g, 4,3 mmol) wurde in THF (40 ml) vorlegen und unter Eisbadkühlung mit Tryptamin (0,69g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.

**4-((Dimethylamino)(phenyl)methyl)-N-phenethylcyclohexanamin Hydrochlorid 84 ($R^3$ = Phenyl)**

Das Keton **10** (1,0 g, 4,3 mmol) wurde in THF (40 ml) vorlegen und unter Eisbadkühlung mit Phenethylamin (0,52 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.

**4-((Dimethylamino)(phenyl)methyl)-N-(3-phenylpropyl)cyclohexanamin Dihydrochlorid 85 ($R^3$ = Phenyl)**

Das Keton **10** (1,0 g, 4,3 mmol) wurde in THF (40 ml) vorlegen und unter Eisbadkühlung mit 3-Phenylpropylamin (0,58 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-Benzyl-4-((dimethylamino)(phenyl)methyl)cyclohexanamin Hydrochlorid 86 ($R^3$ = Phenyl)**

Das Keton **10** (1,0 g, 4,3 mmol) wurde in THF (40 ml) vorlegen und unter Eisbadkühlung mit Benzylamin (0,46 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.

**4-((Dimethylamino)(phenyl)methyl)-N-(4-phenylbutyl)cyclohexanamin Hydrochlorid 87 ($R^3$ = Phenyl)**

Das Keton **10** (1,0 g, 4,3 mmol) wurde in THF (40 ml) vorlegen und unter Eisbadkühlung mit Butylphenylamin (0,65 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(1-(1 H-Indol-3-yl)propan-2-yl)-4-((dimethylamino)(phenyl)methyl) cyclohexanamin Hydrochlorid 88 ($R^3$ = Phenyl)**

Das Keton **10** (1,3 g, 5,6 mmol) wurde in THF (40 ml) vorlegen und unter Eisbadkühlung mit DL-alpha-Methyltryptamin (0,98 g, 5,6 mmol) versetzt. Anschließend wurde Eisessig (0,48 ml, 8,4 mmol) zutropfen. Nach 15min rühren wurde Natriumtriacetoxyborhydrid (1,67g, 7,9 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet. Das Produkt wurde in Methylethylketon (1 ml) gelöst und dann mit Wasser (0,01 ml/ 1 mmol) und Trimethylchlorsilan (1,3 ml/ 1mmol) versetzt. Nach Absaugen

und waschen mit Ether erhielt man weiße Kristalle, die im Vakuum getrocknet wurden. $^{13}C$ NMR (600 MHz, DMSO) 7,57; 15,20; 21,33; 21,90; 22,41; 23,22; 23,29; 24,99; 25,13; 28,46; 28,59; 29,27; 31,11; 31,18; 35,97; 43,38; 50,05; 50,12; 50,65; 50,70; 67,50; 67,55; 108,87; 111,45; 118,20; 118,46; 120,99; 124,01; 127,06; 128,93; 129,75; 130,29; 136,09.

**N-(4-((Dimethylamino)(phenyl)methyl)cyclohexyl)-4-methoxybenzenamin Hydrochlorid 89 ($R^3$ = Phenyl)**

Das Keton **10** (1,0 g, 4,3 mmol) wurde in THF (40 ml) vorlegen und unter Eisbadkühlung mit p-Anisidin (0,53 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**4-((Dimethylamino)(phenyl)methyl)-N-(4-methoxybenzyl)cyclohexanamin Dihydrochlorid 90 ($R^3$ = Phenyl)**

Das Keton **10** (1,0 g, 4,3 mmol) wurde in THF (40 ml) vorlegen und unter Eisbadkühlung mit 4-Methoxybenzylamin (0,59 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgS0_4$ getrocknet.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden. **4-((Dimethylamino)(phenyl)methyl)-N-(4-fluorbenzyl)cyclohexanamin Hydrochlorid 91 ($R^3$ = Phenyl, $R^{12}$ = 4-Fluorbenzyl)**

Das Keton **10** (1,0 g, 4,3 mmol) wurde in THF (40 ml) vorlegen und unter Eisbadkühlung mit 4-Fluorbenzylamin (0,54 g, 4,3 mmol) versetzt. Anschließend wurde Eisessig (0,37 ml, 6,6 mmol) zutropfen. Nach 15min rühren wurde Natriumtriacetoxyborhydrid (1,28g, 6,1 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(4-((Dimethylamino)(phenyl)methyl)cyclohexyl)benzenamin Hydrochlorid 92 ($R^3$ = Phenyl)**

Das Keton **10** (3,95 g, 17,1 mmol) wurde in THF (60 ml) vorlegen und unter Eisbadkühlung mit Anilin (1,59 g, 17,1 mmol) versetzt. Anschließend wurde Eisessig (1,46 ml, 25,6 mmol) zutropfen. Nach 15min rühren wurde Natriumtriacetoxyborhydrid (5,07g, 23,9 mmol) portionsweise zügig hinzugefügt und der Reaktionsansatz über Nacht bei RT gerührt. Zur Aufarbeitung wurde zunächst vorsichtig bei 15°C mit $NaHCO_3$ hydrolysiert und die gelbe Suspension anschließend mit Ether extrahiert. Die vereinigten organischen Phasen wurden zunächst mit Wasser und anschließend mit gesättigter NaCl gewaschen und über $MgSO_4$ getrocknet.

Das Rohprodukt wurde mittels Flash-Chromatographie (Diethylether) aufgereinigt. Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

Synthesevorschrift zur automatisierten Synthese

In ein trockenes Gewindeglas mit Septumkappe wurden bei RT 140μmol Triacetoxyborhydrid-Harz (0,078g, 140μmol Triacetoxyborhydrid) eingewogen und mit THF (1ml) versetzt. Anschließend wurden zuerst eine Lösung des Cyclohexanon-Derivats (100 μmol, 1 ml, 0,1 M in THF) und dann eine Lösung des Amins (100 μmol, 1ml, 0,1 M in THF) zugegeben. Die Reaktionslösung wurde für 16h bei RT im Synthesis 1 Solid von Heidolph geschüttelt.

Zur Aufarbeitung wurden die Ansätze über die Filtriereinheit abgesaugt, das Harz mit 1,5 ml THF nachgespült

und das Filtrat im Anschluss in der GeneVac aufkonzentriert.

Analog wurden Methyl 2-(4-((dimethylamino)(phenyl)methyl)cyclohexylamino)-3-(1 H-indol-3yl)propanoat, polareres Diastereomer 174 und unpolareres Diastereomer 175 hergestellt

**Amidierung der primären Amine ($R^1$ = $(CH_2)_nNHCOR^{13}$)**

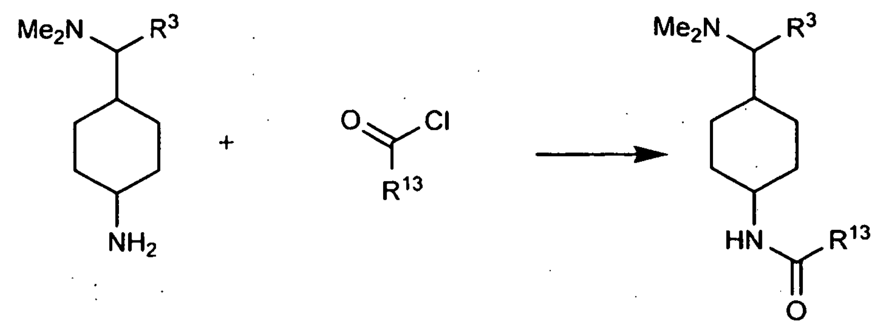

**N-(4-((Dimethylamino)(phenyl)methyl)cyclohexyl)-2-ethylbutanamid Hydrochlorid 93 ($R^3$ = Phenyl)**

Das Amin **17** (0,43g, 1,9 mmol) wurden in DCM (2 ml) gelöst und mit einer Spatelspitze DMAP versetzt. Anschließend wurde 2-Ethylbutylchlorid (0,27g, 2,0 mmol) bei -10°C zugetropft und der Reaktionsansatz über Nacht bei RT nachrühren gelassen.

Zur Suspension wurden 2ml 5N KOH gegeben und anschließend mit DCM extrahiert. Die vereinigten organischen Phasen wurden mit gesättigter NaCl-Lsg. gewaschen, über $Na_2SO_4$ getrocknet und einengt.

Die Aufreinigung erfolgte Säulenchromatographisch (Laufmittel: Essigester/Methanol: 20-1)

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(4-((Dimethylamino)(phenyl)methyl)cyclohexyl)benzamid Hydrochlorid 94 ($R^3$ = Phenyl)**

Das Amin **17** (0,40g, 1,7 mmol) wurden in DCM (2 ml) gelöst und mit einer Spatelspitze DMAP versetzt. Anschließend wurde Benzoylchlorid (0,27g, 1,9 mmol) bei =10°C zugetropft und der Reaktionsansatz über Nacht bei RT nachrühren gelassen.

Zur Suspension wurden 2ml 5N KOH gegeben und anschließend mit DCM extrahiert. Die vereinigten organischen Phasen wurden mit gesättigter NaCl-Lsg. gewaschen, über $Na_2SO_4$ getrocknet und einengt.

Die Aufreinigung erfolgte Säulenchromatographisch (laufmittel: Essigester/Methanol: 20:1)

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**Synthesevorschrift für die automatisierte Synthese**

In einem trockenen Gewindeglas mit Septumkappe werden bei RT eine Lösung des Amins (100μmol, 1 ml, 0,1 M in Pyridin) vorgelegt und mit 100μmol Triethylamin-Lösung (versetzt mit DMAP: 1 mg/10ml Lösung) (100 μmol, 1ml, 0,1 M in Pyridin) sowie mit Säurechlorid-Derivat (300 μmol, 1 ml, 0,3 M in Pyridin) versetzt. Die Reaktionslösung wurde 24h bei RT gerührt. Anschließend wurden bei RT 3 ml Dichlormethan zugegeben und mit 9,5%iger $NaHCO_3$-Lösung (1 ml) versetzt. Die Lösung wurde 30 min. durchmischt.

Die Phasen wurden getrennt. Die wäßrige Phase wurde mit DCM (2 ml) versetzt und 15 min. im Spinreaktor intensiv durchmischt. Nach dem Zentrifugieren wurde die organische Phase abgetrennt und mit der ersten Fraktion vereinigt. Die wäßrige Phase wurde analog ein zweites Mal mit DCM extrahiert. Anschließend wurden die vereinigten, organischen Phasen über eine $MgSO_4$-Kartusche getrocknet und eingeengt.

Es wurden so die folgenden Beispiele synthetisiert. Die Analytik erfolgte über HPLC-MS (ESI). In allen hier aufgeführten Fällen wurde die Masse als M +1 gefunden:

| Nr. | Name | Masse |
|---|---|---|
| 176 | N-(4-((Dimethylamino)(phenyl)methyl)cyclohexyl)-N-(4-methoxybenzyl)acetamid | 394,26 |
| 177 | N-(1-(1H-indol-3-yl)propan-2-yl)-N-(4-((dimethyl-amino)(phenyl)methyl)cyclohexyl)acetamid (polareres Diastereomer) | 431,29 |
| 178 | N-(1-(1H-indol-3-yl)propan-2-yl)-N-(4-((dimethyl-amino)(phenyl)methyl)cyclohexyl)acetamid (unpolareres Diastereomer) | 431,29 |
| 179 | N-(4-((Dimethylamino)(phenyl)methyl)cyclohexyl)-N-(4-fluorbenzyl)acetamid | 382,24 |
| 180 | N-(4-((Dimethylamino)(phenyl)methyl)cyclohexyl)-N-phenylbutyramid | 378,27 |
| 181 | N-(2-(1 H-indol-3-yl)ethyl)-N-(4-((dimethylamino)(phenyl)methyl)cyclohexyl)butyramid | 445,31 |
| 182 | N-(2-(1 H-indol-3-yl)ethyl)-N-(4-((dimethylamino)(phenyl)methyl)cyclohexyl)acetamid | 417,28 |
| 183 | Benzo[b]thiophen-3-carbonsäure-[4-(dimethylamino-phenyl-methyl)-cyclohexyl]-amid | 392,19 |
| 184 | 1-(4-Chlor-phenyl)-cyclopentancarbonsäure-[4-(dimethy)amino-phenyl-methyl)-cyclohexyl]-amid | 438,24 |
| 185 | N-[4-(Dimethylamino-thiophen-2-yl-methyl)-cyclohexyl]-4-propyl-benzamid | 384,22 |
| 186 | 3-Cyano-N-[4-(dimethylamino-thiophen-2-yl-methyl)-cyclohexyl]-benzamid | 367,17 |
| 187 | 3-Chlor-N-[4-(dimethylamino-phenyl-methyl)-cyclohexyl]-benzamid | 370,18 |
| 188 | 5-Chlor-4-methoxy-thiophen-3-carbonsäure-[4-(dimethylamino-phenyl-methyl)-cyclohexyl]-amid | 406,15 |
| 189 | 3,4-Dichlor-N-[4-(dimethy)amino-phenyl-methyl)-cyclohexyl]-benzamid | 404,14 |
| 190 | N-[4-(Dimethylamino-phenyl-methyl)-cyclohexyl]-3-fluor-5-trifluormethyl-benzamid | 422,20 |
| 191 | 5-Chlor-4-methoxy-thiophen-3-carbonsäure-{4-[dimethylamino-(3-fluor-phenyl)-methyl]-cyclohexyl}-amid | 424,14 |
| 192 | N-[4-(Dimethylamino-thiophen-2-yl-methyl)-cyclohexyl]-4-fluor-3-trifluormethyl-benzamid | 428,15 |
| 193 | Thiophen-2-carbonsäure-[4-(dimethylamino-thiophen-2-yl-methyl)-cyclohexyi]-amid | 34813 |
| 194 | 3,5-Dichlor-N-[4-(dimethylamino-thiophen-2-yl-methyl)-cyclohexyl]-benzamid | 410,10 |
| 195 | 5-Chlor-4-methoxy-thiophen-3-carbonsäure-[4-(dimethylamino-thiophen-2-yl-methyl)-cyclohexyl]-amid | 412,10 |
| 196 | N-[4-(Dimethylamino-phenyl-methyl)-cyclohexyl]-2,4,5-trifluor-benzamid | 390,19 |
| 197 | 3-Brom-N-[4-(dimethylamino-phenyl-methyl)-cyclohexyl]-benzamid | 414,13 |
| 198 | N-[4-(Dimethylamino-thiophen-2-yl-methyl)-cyclohexyl]-4-methyl-benzamid | 356,19 |
| 199 | N-[4-(Dimethylamino-thiophen-2-yl-methyl)-cyclohexyl]-3-methoxy-benzamid | 372,19 |
| 200 | N-[4-(Dimethylamino-thiophen-2-yl-methyl)-cyclohexyl]-3,3-dimethyl-butyramid | 336,22 |
| 201 | 2-tert-Butyl-5-methyl-2H-pyrazol-3-carbonsäure-[4-(dimethylamino-thiophen-2-yl-methyl)-cyclohexyl]-amid | 402,25 |
| 202 | N-[4-(Dimethylamino-thiophen-2-yl-methyl)-cyclohexyl]-2,4-dimethoxy-benzamid | 402,20 |
| 203 | N-[4-(Dimethylamino-phenyl-methyl)-cyclohexyl]-3-trifluormethyl-benzamid | 404,21 |
| 204 | N-{4-[Dimethylamino-(3-fluor-phenyl)-methyl]-cyclohexyl}-3,5-difluor-benzamid | 390,19 |
| 205 | N-[4-(Dimethylamino-phenyl-methyl)-cyclohexyl]-2-fluor-5-trifluormethyl-benzamid | 422,20 |
| 206 | 2-(4-Chlor-phenyl)-N-[4-(dimethylamino-phenyl-methyl)-cyclohexyl]-acetamid | 384,20 |
| 207 | N-[4-(Dimethylamino-thiophen-2-yl-methyl)-cyclohexyl]-2-methoxy-benzamid | 372,19 |
| 208 | N-[4-(Dimethylamino-thiophen-2-yl-methyl)-cyclohexyl]-2-methylsulfanyl-nicotinamid | 389,16 |
| 209 | 3,4-Dichlor-N-{4-[dimethylamino-(3-fluor-phenyl)-methyl]-cyclohexyl}-benzamid | 422,13 |

(fortgesetzt)

| Nr. | Name | Masse |
|---|---|---|
| 210 | N-[4-(1-Dimethylamino-3-phenyl-propyl)-cyclohexyl]-4-fluor-3-trifluormethyl-benzamid (polareres Diastereomer) | 450,23 |
| 211 | 5-Chlor-4-methoxy-thiophen-3-carbonsäure-{4-[dimethylamino-(4-fluor-phenyl)-methyl]-cyclohexyl}-amid | 424,14 |
| 212 | N-[4-(Dimethylamino-thiophen-2-yl-methyl)-cyclohexyl]-3,4 ,5-trimethoxy-benzamid | 432,21 |
| 213 | N-[4-(Dimethylamino-thiophen-2-yl-methyl)-cyclohexyl]-2-ethylsulfanyl-nicotinamid | 403,18 |
| 214 | 2-Methyl-5-phenyl-furan-3-carbonsäure-[4-(dimethylamino-phenyl-methyl)-cyclohexyl]-amid | 416,25 |
| 215 | N-{4-[Dimethylamino-(3-fluor-phenyl)-methyl]-cyclohexyl}-2-phenoxy-propionamid (unpolareres Diastereomer) | 398,24 |
| 216 | N-[4-(Dimethylamino-phenyl-methyl)-cyclohexyl]-2,4-dimethoxy-benzamid | 396,24 |
| 217 | 4-tert-Butyl-N-[4-(dimethylamino-phenyl-methyl)-cyclohexyl]-benzamid | 392,28 |
| 218 | 2-(4-Chlor-phenylsulfanyl)-N-[4-(dimethylamino-thiophen-2-yl-methyl)-cyclohexyl]-nicotinamid | 485,14 |
| 219 | 2-(4-Chlor-phenyl)-N-[4-(dimethylamino-thiophen-2-yl-methyl)-cyclohexyl]-acetamid | 390,15 |
| 220 | N-[4-(Dimethylamino-thiophen-2-yl-methyl)-cyclohexyl]-2-p-tolyloxy-nicotinamid | 449,21 |
| 221 | 3-Chlor-4-(propan-2-sulfonyl)-thiophen-2-carbonsäure-[4-(dimethylamino-thiophen-2-yl-methyl)-cyclohexyl]-amid | 488,10 |
| 222 | N-{4-[Dimethylamino-(3-fluor-phenyl)-methyl]-cyclohexyl}-2-phenoxy-propionamid (polareres Diastereomer) | 398,24 |
| 223 | 2-tert-Butyl-5-methyl-2H-pyrazol-3--carbonsäure-[4-(dimethylamino-phenyl-methyl)-cyclohexyl]-amid | 396,29 |
| 224 | 5-Methyl-isoxazol-3-carbonsäure-[4-(dimethylamino-thiophen-2-yl-methyl)-cyclohexyl]-amid | 347,17 |
| 225 | 5-Brom-N-[4-(dimethylamino-thiophen-2-yl-methyl)-cyclohexyl]-nicotinamid | 421,08 |
| 226 | Naphthyl-1-carbonsäure-[4-(dimethylamino-phenyl-methyl)-cyclohexyl]-amid | 386,24 |
| 227 | N-[4-(1-Dimethylamino-3-phenyl-propyl)-cyclohexyl]-4-fluor-3-trifluormethyl-benzamid (unpolareres Diastereomer) | 450,23 |
| 228 | N-[4-(Dimethylamino-phenyl-methyl)-cyclohexyl]-3,3-dimethyl-butyramid (polareres Diastereomer) | 330,27 |
| 229 | 5-(4-Chlor-phenyl)-2-methyl-furan-3-carbonsäure-[4-(dimethylamino-thiophen-2-yl-methyl)-cyclohexyl]-amid | 456,16 |
| 230 | N-[4-(Dimethylamino-phenyl-methyl)-cyclohexyl]-2-phenoxy-propionamid | 380,25 |
| 231 | Benzo[b]thiophen-2-carbonsäure-{4-[dimethylamino-(3-fluor-phenyl)-methyl]-cyclohexyl}-amid | 410,18 |
| 232 | 5-(4-Chlor-phenyl)-2-methyl-furan-3-carbonsäure-[4-(dimethylamino-phenyl-methyl)-cyclohexyl]-amid | 450,21 |
| 233 | 4-(4-Chlor-benzenesulfonyl)-3-methyl-thiophen-2-carbonsäure-[4-(dimethylamino-phenyl-methyl)-cyclohexyl]-amid | 530,15 |
| 234 | N-[4-(Dimethylamino-phenyl-methyl)-cyclohexyl]-2-phenyl-butyramid (unpolareres Diastereomer) | 378,27 |
| 235 | 5-Brom-N-[4-(dimethylamino-phenyl-methyl)-cyclohexyl]-nicotinamid | 415,13 |
| 236 | Adamantan-1-carbonsäure-[4-(dimethylamino-phenyl-methyl)-cyclohexyl]-amid | 394,30 |
| 237 | 2-Phenyl-thiazol-4-carbonsäure-[4-(dimethylamino-phenyl-methyl)-cyclohexyl]-amid | 419,20 |
| 238 | 4-Methyl-2-phenyl-thiazol-5-carbonsäure-[4-(dimethylamino-phenyl-methyl)-cyclohexyl]-amid | 433,22 |

(fortgesetzt)

| Nr. | Name | Masse |
|---|---|---|
| 239 | 2-(2,3-Dihydro-benzofuran-5-yl)-thiazol-4-carbonsäure-[4-(dimethylamino-thiophen-2-yl-methyl)-cyclohexyl]-amid | 467,17 |
| 240 | N-{4-[Dimethylamino-(3-fluor-phenyl)-methyl]-cyclohexyl}-2-phenyl-acetamid | 368,23 |
| 241 | 3-Chlor-N-[4-(1-dimethylamino-3-phenyl-propyl)-cyclohexyl]-benzamid | 398,21 |
| 242 | N-[4-(Dimethylamino-phenyl-methyl)-cyclohexyl]-4-methyl-benzamid | 350,24 |
| 243 | 3,5-DichlorN-{4-[dimethylamino-(3-fluor-phenyl)-methyl]-cyclohexyl}-benzamid | 422,13 |
| 244 | N-[4-(Dimethylamino-phenyl-methyl)-cyclohexyl]-2,3,6-trifluor-benzamid | 390,19 |
| 245 | Thiophen-2-carbonsäure-[4-(dimethylamino-phenyl-methyl)-cyclohexyl]-amid (unpolareres Diastereomer) | 342,18 |
| 246 | N-[4-(Dimethylamino-phe.nyl-methyl)-cyclohexyl]-3,3-dimethyl-butyramid (unpolareres Diastereomer) | 330,27 |
| 247 | 2-tert-Butyl-5-methyl-2H-pyrazol-3-carbonsäure-[4-(dimethylamino-phenyl-methyl)-cyclohexyl]-amid | 396,29 |
| 248 | N-[4-(1-Dimethylamino-3-phenyl-propyl)-cyclohexyl]-3-methyl-benzamid | 378,27 |
| 249 | Thiophen-2-carbonsäure-[4-(dimethylamino-phenyl-methyl)-cyclohexyl]-amid(polareres Diastereomer) | 342,18 |
| 250 | N-[4-(Dimethylamino-phenyl-methyl)-cyclohexyl]-2-phenyl-butyramid (polareres Diastereomer) | 378,27 |
| 251 | N-{4-[Dimethylamino-(3-fluor-phenyl)-methyl]-cyclohexyl}-3,3-dimethyl-butyramid | 348,26 |
| 252 | 3-Chlor-4-methanesulfonyl-thiophen-2-carbonsäure-[4-(dimethylamino-phenyl-methyl)-cyclohexyl]-amid | 454,12 |
| 253 | 4-(4-Chlor-benzenesulfonyl)-3-methyl-thiophen-2-carbonsäure-[4-(dimethylamino-phenyl-methyl)-cyclohexyl]-amid | 530,15 |
| 254 | 2-Benzyloxy-N-[4-(dimethylamino-phenyl-methyl)-cyclohexyl]-acetamid | 380,25 |
| 255 | N-[4-(Dimethylamino-phenyl-methyl)-cyclohexyl]-2-thiophen-2-yl-acetamid | 356,19 |
| 256 | 4-Methyl-2-phenyl-thiazol-5-carbonsäure-{4-[dimethylamino-(3-fluor-phenyl)-methyl]-cyclohexyl}-amid | 451,21 |
| 257 | 2-(4-Chlor-phenoxy)-N-[4-(dimethylamino-thiophen-2-yl-methyl)-cyclohexyl]-nicotinamid | 469,16 |
| 258 | N-[4-(1-Dimethylamino-3-phenyl-propyl)-cyclohexyl]-4-fluor-benzamid | 382,24 |
| 259 | 5-Brom-N-[4-(dimethylamino-phenyl-methyl)-cyclohexyl]-nicotinamid | 415,13 |
| 260 | 4-Brom-2-ethyl-5-methyl-2H-pyrazol-3-ccarbonsäure-[4-(dimethylamino-thiophen-2-yl-methyl)-cyclohexyl]-amid | 452,12 |
| 261 | 3-Cyano-N-[4-(dimethylamino-phenyl-methyl)-cyclohexyl]-benzamid | 361,22 |
| 262 | N-{4-[Dimethylamino-(4-fluor-phenyl)-methyl]-cyclohexyl}-4-fluor-benzamid | 372,20 |
| 263 | 3-Brom-N-{4-[dimethylamino-(4-fluor-phenyl)-methyl]-cyclohexyl}benzamid | 432,12 |
| 264 | 2-Phenyl-thiazol-4-carbonsäure-{4-[dimethylamino-(3-fluor-phenyl)-methyl]-cyclohexyl}-amid | 437,19 |
| 265 | 2,5-Dimethyl-furan-3-carbonsäure-{4-[dimethylamino-(3-fluor-phenyl)-methyl]-cyclohexyl}-amid | 372,22 |
| 266 | 2-Methyl-5-phenyl-furan-3-carbonsäure-{4-[dimethylamino-(3-fluor-phenyl)-methyl]-cyclohexyl}-amid | 434,24 |
| 267 | 5-Pyridin-2-yl-thipphen-2-carbonsäure-{4-[dimethylamino-(3-fluor-phenyl)-methyl]-cyclohexyl}-amid | 437,19 |

(fortgesetzt)

| Nr. | Name | Masse |
|---|---|---|
| 268 | 4-Brom-2-ethyl-5-methyl-2H-pyrazol-3-carbonsäure-[4-(dimethylamino-phenyl-methyl)-cyclohexyl]-amid | 446,17 |
| 269 | 3-Chlor-N-{4-[dimethylamino-(3-fluor-phenyl)-methyl]-cyclohexyl}-4-fluor-benzamid | 406,16 |
| 270 | 3,4-Dichlor-N-{4-[dimethylamino-(4-fluor-phenyl)-methyl]-cyclohexyl}-benzamid | 422,13 |
| 271 | N-{4-[Dimethylamino-(4-fluor-phenyl)-methyl]-cyclohexyl}-2,4,5-trifluor-benzamid | 408,18 |
| 272 | Cyclohexancarbonsäure-[4-(dimethylamino-phenyl-methyl)-cyclohexyl]-amid | 342,27 |
| 273 | N-{4-[Dimethylamino-(3-fluor-phenyl)-methyl]-cyclohexyl}-2-phenyl-butyramid | 396,26 |
| 274 | 2-(4-Chlor-phenyl)-N-{4-[dimethylamino-(3-fluor-phenyl)-methyl]-cyclohexyl}-acetamid | 402,19 |
| 275 | N-[4-(Dimethylamino-phenyl-methyl)-cyclohexyl]-3-nitro-benzamid | 381,21 |
| 276 | N-[4-(1-Dimethylamino-3-phenyl-propyl)-cyclohexyl]-2,5-difluor-benzamid | 400,23 |
| 277 | 3-Brom-N-[4-(1-dimethylamino-3-phenyl-propyl)-cyclohexyl]-benzamid | 442,16 |
| 278 | N-{4-[Dimethylamino-(3-fluor-phenyl)-methyl]-cyclohexyl}-2,6-difluor-benzamid | 390,19 |
| 279 | 2,5-Dimethyl-furan-3-carbonsäure-[4-(dimethylamino-phenyl-methyl)-cyclohexyl]-amid | 354,23 |
| 280 | 3-Chlor-N-{4-[dimethylamino-(4-fluor-phenyl)-methyl]-cyclohexyl}-4-fluor-benzamid | 406,16 |
| 281 | N-[4-(Dimethylamino-phenyl-methyl)-cyclohexyl]-5-fluor-2-trifluormethyl-benzamid | 422,20 |
| 282 | 5-Methyl-isoxazol-3-carbonsäure-[4-(dimethylamino-phenyl-methyl)-cyclohexyl]-amid | 341,21 |
| 283 | 2-(2,3-Dihydro-benzofuran-5-yl)4-methyl-thiazol-5-carbonsäure-[4-(dimethylamino-thiophen-2-yl-methyl)-cyclohexyl]-amid | 481,19 |
| 284 | 2-(4-Chlor-phenoxy)-N-[4-(dimethylamino-phenyl-methyl)-cyclohexyl]-acetamid | 400,19 |
| 285 | 5-(4-Chlor-phenyl)-2-methyl-furan-3-carbonsäure-{4-[dimethylamino-(3-fluor-phenyl)-methyl]-cyclohexyl}-amid | 468,20 |
| 286 | 2-Brom-N-[4-(dimethylamino-phepyl-methyl)-cyclohexyl]-benzamid | 414,13 |
| 287 | N-[4-(Dimethylamino-phenyl-methyl)-cyclohexyl]-2,6-dimethoxy-benzamid | 396,24 |
| 288 | Cyclopentancarbonsäure-[4-(dimethylamino-phenyl-methyl)-cyclohexyl]-amid | 328,25 |
| 289 | 2-(2,3-Dihydro-benzofuran-5-yl)-thiazol-4-carbonsäure-[4-(dimethylamino-phenyl-methyl)-cyclohexyl]-amid | 461,21 |
| 290 | Benzo[1,2,5]thiadiazol-5-carbonsäure-{4-[dimethylamino-(3-fluor-phenyl)-methyl]-cyclohexyl}-amid | 412,17 |
| 291 | N-[4-(Dimethylamino-thiophen-2-yl-methyl)-cyclohexylmethyl]-2-thiophen-2-yl-acetamid | 376,16 |
| 292 | Benzo[b]thiophen-3-carbonsäure-[4-(dimethylamino-phenyl-methyl)-cyclohexylmethyl]-amid | 406,21 |
| 293 | 5-Chlor-4-methoxy-thiophen-3-carbonsäure-[4-(dimethylamino-phenyl-methyl)-cyclohexylmethyl]-amid | 420,16 |
| 294 | N-[4-(Dimethylamino-phenyl-methyl)-cyclohexylmethyl]-3,4-difluor-benzamid (unpolareres Diastereomer) | 386,22 |
| 295 | N-{4-[Dimethylamino-(3-fluor-phenyl)-methyl]-cyclohexylmethyl}-3,3-dimethyl-butyramid | 362, 27 |
| 296 | 2-Brom-N-[4-(dimethylamino-thiophen-2-yl-methyl)-cyclohexylmethyl]-benzamid | 434,10 |
| 297 | N-[4-(Dimethylamino-thiophen-2-yl-methyl)-cyclohexylmethyl]-2,2-diphenyl-acetamid | 446,24 |
| 298 | N-[4-(Dimethylamino-phenyl-methyl)-cyclohexylmethyl]-3,3-dimethyl-butyramid | 344, 28 |
| 299 | N-[4-(Dimethylamino-phenyl-methyl)-cyclohexylmethyl]-2-methylsulfanyl-nicotinamid | 397,22 |
| 300 | N-[4-(Dimethylamino-thiophen-2-yl-methyl)-cyclohexylmethyl]-2,6-dimethoxy-benzamid | 416,21 |

(fortgesetzt)

| Nr. | Name | Masse |
|---|---|---|
| 301 | N-[4-(Dimethylamino-thiophen-2-yl-methyl)-cyclohexylmethyl]-3,3-dimethyl-butyramid | 350,24 |
| 302 | Benzo[b]thiophen-3-carbonsäure-[4-(dimethylamino-thiophen-2-yl-methyl)-cyclohexylmethyl]-amid | 412,16 |
| 303 | 5-Chlor-4-methoxy-thiophen-3-carbonsäure-{4-[dimethylamino-(3-fluor-phenyl)-methyl]-cyclohexylmethyl}-amino | 438,15 |
| 304 | N-[4-(Dimethylamino-thiophen-2-yl-methyl)-cyclohexylmethyl]-2-phenoxy-propionamid | 400,22 |
| 305 | N-[4-(Dimethylamino-thiophen-2-yl-methyl)-cyclohexylmethyl]-2-methoxy-benzamid | 386,20 |
| 306 | N-[4-(Dimethylamino-phenyl-methyl)-cyclohexylmethyl]-2-phenyl-acetamid | 364,25 |
| 307 | 3-Brom-N-[4-(dimethylamino-thiophen-2-yl-methyl)-cyclohexylmethyl]-benzamid | 434,10 |
| 308 | N-[4-(Dimethylamino-thiophen-2-yl-methyl)-cyclohexylmethyl]-3-fluor-5-trifluormethyl-benzamid | 442,17 |
| 309 | N-{4-[(4-Chlor-phenyl)-dimethylamino-methyl]-cyclohexylmethyl}-3,3-dimethyl-butyramid | 378,24 |
| 310 | N-[4-(Dimethylamino-phenyl-methyl)-cyclohexylmethyl]-2-ethylsulfanyl-nicotinamid | 411,23 |
| 311 | 2-(4-Chlor-phenyl)-N-[4-(dimethylamino-thiophen-2-yl-methyl)-cyclohexylmethyl]-acetamid | 404,17 |
| 312 | N-{4-[(4-Chlor-phenyl)-dimethylamino-methyl]-cyclohexylmethyl}-2,2-diphenyl-acetamid | 474,24 |
| 313 | N-[4-(Dimethylamino-thiophen-2-yl-methyl)-cyclohexylmethyl]-2,6-difluor-benzamid | 392,17 |
| 314 | Benzo[b]thiophen-3-carbonsäure-{4-[dimethylamino-(3-fluor-phenyl)-methyl]-cyclohexylmethyl}-amid | 424,20 |
| 315 | N-{4-[(4-Chlor-phenyl)-dimethylamino-methyl]-cyclohexylmethyl}-2-methylsulfanyl-nicotinamid | 431,18 |
| 316 | N-{4-[(4-Chlor-phenyl)-dimethylamino-methyl]-cyclohexylmethyl}-2-thiophen-2-yl-acetamid | 404,17 |
| 317 | N-[4-(Dimethylamino-phenyl-methyl)-cyclohexylmethyl]-2-methyl-benzamid (unpolareres Diastereomer) | 364,25 |
| 318 | 1-(4-Chlor-phenyl)-cyclopentan-carbonsäure-[4-(dimethylamino-phenyl-methyl)-cyclohexylmethyl]-amid | 452,26 |
| 319 | N-{4-[Dimethylamino-(3-fluor-phenyl)-methyl]-cyclohexylmethyl}-2-phenyl-acetamid (polareres Diastereomer) | 382,24 |
| 320 | N-{4-[(4-Chlor-phenyl)-dimethytamino-methyl]-cyclohexylmethyl}-2-(3-methoxy-phenyl)-acetamid | 428,22 |
| 321 | N-[4-(Dimethylamino-phenyl-methyl)-cyclohexylmethyl]-2-phenyl-butyramid | 392,28 |
| 322 | N-{4-[Dimethylamino-(4-fluor-phenyl)-methyl]-cyclohexylmethyl}-3,3-dimethyl-butyramid | 362,27 |
| 323 | N-[4-(Dimethylamino-phenyl-methyl)-cyclohexylmethyl]-2-phenoxy-propionamid | 394,26 |
| 324 | 2-(4-Chlor-phenyl)-N-[4-(dimethylamino-phenyl-methyl)-cyclohexylmethyl]-acetamid | 398,21 |
| 325 | N-[4-(Dimethylamino-phenyl-methyl)-cyclohexylmethyl]-2-methyl-benzamid (polareres Diastereomer) | 364,25 |
| 326 | N-[4-(Dimethylamino-phenyl-methyl)-cyclohexylmethyl]-3-trifluormethyl-benzamid (unpolareres Diastereomer) | 418,22 |
| 327 | 1-(4-Chlor-phenyl)-cyclopentan-carbonsäure-{4-[dimethylamino-(3-fluor-phenyl)-methyl]-cyclohexylmethyl}-amid | 470,25 |
| 328 | Thiophen-2-carbonsäure-[4-(dimethylamino-phenyl-methyl)-cyclohexylmethyl]-amid | 356,19 |
| 329 | 3,5-Dichlor-N-[4-(dimethylamino-phenyl-methyl)-cyclohexylmethyl]-benzamid | 418,16 |
| 330 | 2-Methyl-5-phenyl-furan-3-carbonsäure-{4-[(4-chlor-phenyl)-dimethylamino-methyl]-cyclohexylmethyl}-amid | 464,22 |
| 331 | 3-Chlor-N-[4-(dimethylamino-phenyl-methyl)-cyclohexylmethyl]-benzamid | 384,20 |

(fortgesetzt)

| Nr. | Name | Masse |
|---|---|---|
| 332 | N-{4-[Dimethylamino-(3-fluor-phenyl)-methyl]-cyclohexylmethyl}-2-phenoxy-propionamid (polareres Diastereomer) | 412,25 |
| 333 | N-[4-(Dimethylamino-phenyl-methyl)-cyclohexylmethyl]-3-trifluormethyl-benzamid (polareres Diastereomer) | 418,22 |
| 334 | Thiophen-2-carbonsäure-{4-[dimethylamino-(3-fluor-phenyl)-methyl]-cyclohexylmethyl}-amid (polareres Diastereomer) | 374,18 |
| 335 | 2-Phenyl-thiazol-4-carbonsäure-[4-(dimethylamino-phenyl-methyl)-cyclohexylmethyl]-amid | 433,22 |
| 336 | Benzo[b]thiophen-3-carbonsäure-{4-[(4-chlor-phenyl)-dimethylamino-methyl]-cyclohexylmethyl}-amid (unpolareres Diastereomer) | 440,17 |
| 337 | N-{4-[Dimethylamino-(3-fluor-phenyl)-methyl]-cyclohexylmethyl}-2-p-tolyloxy-nicotinamid | 475,26 |
| 338 | 2,4,6-Trichlor-N-[4-(dimethylamino-phenyl-methyl)-cyclohexylmethyl]-benzamid | 452,12 |
| 339 | 1-(4-Chlor-phenyl)-cyclopentan-carbonsäure-[4-(dimethylamino-phenyl-methyl)-cyclohexylmethyl]-amid | 452,26 |
| 340 | Thiophen-2-carbonsäure-{4-[dimethylamino-(3-fluor-phenyl)-methyl]-cyclohexylmethyl}-amid (unpolareres Diastereomer) | 374,18 |
| 341 | N-{4-[Dimethylamino-(3-fluor-phenyl)-methyl]-cyclohexylmethyl}-2-phenoxy-propionamid (unpolareres Diastereomer) | 412,25 |
| 342 | N-{4-[Dimethylamino-(3-fluor-phenyl)-methyl]-cyclohexylmethyl}-2-methyl-butyramid (polareres Diastereomer) | 348,26 |
| 343 | N-{4-[Dimethylamino-(3-fluor-phenyl)-methyl]-cyclohexylmethyl}-2-thiophen-2-yl-acetamid | 388,20 |
| 344 | Benzo[b]thiophen-3-carbonsäure-{4-[dimethylamino-(4-fluor-phenyl)-methyl]-cyclohexylmethyl}-amid | 424,20 |
| 345 | 2-Methyl-5-phenyl-furan-3-carbonsäure-[4-(dimethylamino-phenyl-methyl)-cyclohexylmethyl]-amid | 430,26 |
| 346 | N-{4-[Dimethylamino-(3-fluor-phenyl)-methyl]-cyclohexylmethyl}-2-phenoxy-propionamid | 412,25 |
| 347 | 3-Cyano-N-[4-(dimethylamino-thiophen-2-yl-methyl)-cyclohexylmethyl]-benzamid | 381,19 |
| 348 | N-{4-[Dimethylamino-(3-fluor-phenyl)-methyl]-cyclohexylmethyl}-2-phenyl-acetamid (unpolareres Diastereomer) | 382,24 |
| 349 | N-[4-(Dimethylamino-phenyl-methyl)-cyclohexylmethyl]-2-(3-methoxy-phenyl)-acetamid | 394,26 |
| 350 | N-{4-[Dimethylamino-(3-fluor-phenyl)-methyl]-cyclohexylmethyl}-4-fluor-3-trifluormethyl-benzamid | 454,20 |
| 351 | N-{4-[(4-Chlor-phenyl)-dimethylamino-methyl]-cyclohexylmethyl}-2-ethylsulfanyl-nicotinamid | 445,20 |
| 352 | N-[4-(Dimethylamino-thiophen-2-yl-methyl)-cyclohexylmethyl]-2-p-tolyloxy-nicotinamid (polareres Diastereomer) | 463,23 |
| 353 | 2-Methyl-5-phenyl-furan-3-carbonsäure-{4-[dimethylamino-(3-fluor-phenyl)-methyl]-cyclohexylmethyl}-amid | 448,25 |
| 354 | 2-Chlor-N-{4-[(4-chlor-phenyl)-dimethylamino-methyl]-cyclohexylmethyl}-benzamid | 418,16 |
| 355 | 2-Chlor-N-[4-(dimethylamino-phenyl-methyl)-cyclohexylmethyl]-nicotinamid | 385,19 |
| 356 | N-[4-(Dimethylamino-thiophen-2-yl-methyl)-cyclohexylmethyl]-4-propyl-benzamid | 398,24 |
| 357 | N-[4-(Dimethylamino-phenyl-methyl)-cyclohexylmethyl]-3,4-difluor-benzamid (polareres Diastereomer) | 386,22 |
| 358 | 3-Brom-N-[4-(dimethylamino-phenyl-methyl)-cyclohexylmethyl]-benzamid | 428,15 |
| 359 | N-{4-[Dimethylamino-(4-fluor-phenyl)-methyl]-cyclohexylmethyl}-2-thiophen-2-yl-acetamid | 388,20 |

(fortgesetzt)

| Nr. | Name | Masse |
|---|---|---|
| 360 | 2-(4-Chlor-phenoxy)-N-[4-(dimethylamino-phenyl-methyl)-cyclohexylmethyl]-nicotinamid (unpolareres Diastereomer) | 477,22 |
| 361 | 2,4-Dichlor-N-[4-(dimethylamino-phenyl-methyl)-cyclohexylmethyl]-benzamid | 418,16 |
| 362 | N-[4-(Dimethylamino-phenyl-methyl)-cyclohexylmethyl]-3-methyl-benzamid | 364,25 |
| 363 | 2-Brom-N-{4-[dimethylamino-(3-fluor-phenyl)-methyl]-cyclohexylmethyl}-benzamid | 446,14 |
| 364 | N-{4-[Dimethylamino-(3-fluor-phenyl)-methyl]-cyclohexylmethyl}-3-trifluormethyl-benzamid | 436,21 |
| 365 | 2-Phenyl-thiazol-4-carbonsäure-{4-[dimethylamino-(3-fluor-phenyl)-methyl]-cyclohexylmethyl}-amid | 451,21 |
| 366 | 2-tert-Butyl-5-methyl-2H-pyrazol-3-carbonsäure-[4-(dimethylamino-thiophen-2-yl-methyl)-cyclohexylmethyl]-amid | 416,26 |
| 367 | 3-Chlor-4-(propan-2-sulfonyl)-thiophen-2-carbonsäure-[4-(dimethylamino-thiophen-2-yl-methyl)-cyclohexylmethyl]-amid | 502,12 |
| 368 | N-{4-[Dimethylamino-(3-fluor-phenyl)-methyl]-cyclohexylmethyl}-2-methoxy-benzamid | 398,24 |
| 369 | N-{4-[(4-Chlor-phenyl)-dimethylamino-methyl]-cyclohexylmethyl}-3-trifluormethyl-benzamid | 452,18 |
| 370 | 1-(4-Chlor-phenyl)-cyclopentan-carbonsäure-{4-[dimethylamino-(4-fluor-phenyl)-methyl]-cyclohexylmethyl}-amid | 470,25 |
| 371 | 3,5-Dichlor-N-{4-[dimethylamino-(3-fluor-phenyl)-methyl]-cyclohexylmethyl}-benzamid | 436,15 |
| 372 | Benzo[b]thiophen-3-carbonsäure-{4-[(4-chlor-phenyl)-dimethylamino-methyl]-cyclohexylmethyl}-amid (polareres Diastereomer) | 440,17 |
| 373 | 2-Methyl-5-phenyl-furan-3-carbonsäure {4-[dimethylamino-(3-fluor-phenyl)-methyl]-cyclohexylmethyl}-amid | 448,25 |
| 374 | 2-(4-Chlor-phenylsulfanyl)-N-[4-(dimethylamino-phenyl-methyl)-cyclohexylmethyl]-nicotinamid | 493,20 |
| 375 | 4-Brom-2-ethyl-5-methyl-2H-pyrazol-3-carbonsäure-[4-(dimethylamino-thiophen-2-yl-methyl)-cyclohexylmethyl]-amid | 466,14 |
| 376 | 5-Chlor-4-methoxy-thiophen-3-carbonsäure-{4-[dimethylamino-(4-fluor-phenyl)-methyl]-cyclohexylmethyl}-amid | 438,15 |
| 378 | N-{4-[Dimethylamino-(3-fluor-phenyl)-methyl]-cyclohexylmethyl}-2-methyl-butyramid (unpolareres Diastereomer) | 348,26 |
| 379 | 5-Methyl-isoxazol-3--carbonsäure-[4-(dimethylamino-phenyl-methyl)-cyclohexylmethyl]-amid | 355,23 |
| 380 | Benzo[b]thiophen-3-carbonsäure-[4-(1-dimethylamino-3-phenyl-propyl)-cyclohexylmethyl]-amid | 434,24 |
| 381 | N-{4-[Dimethylamino-(4-fluor-phenyl)-methyl]-cyclohexylmethyl}-2-methylsulfanyl-nicotinamid | 415,21 |
| 382 | N-{4-[(4-Chlor-pheny)-dimethylamino-methyl]-cyclohexylmethyl}-2-p-tolyloxy-nicotinamid | 491,23 |
| 383 | 2-(4-Chlor-phenoxy)-N-[4-(dimethylamino-phenyl-methyl)-cyclohexylmethyl]-nicotinamid (polareres Diastereomer) | 477,22 |
| 384 | N-{4-[Dimethylamino-(3-fluor-phenyl)-methyl]-cyclohexylmethyl}-2-methyl-benzamid | 382,24 |
| 385 | 5-Methyl-isoxazol-3-carbonsäure-{4-[(4-chlor-phenyl)-dimethylamino-methyl]-cyclohexylmethyl}-amid | 389,19 |
| 386 | 5-Brom-N-[4-(dimethylamino-phenyl-methyl)-cyclohexylmethyl]-nicotinamid | 429,14 |
| 387 | N-[4-(Dimethylamino-phenyl-methyl)-cyclohexylmethyl]-2-methyl-butyramid | 330,27 |
| 388 | N-{4-[Dimethylamino-(3-fluor-phenyl)-methyl]-cyclohexylmethyl}-2-ethylsulfanyl-nicotinamid | 429,22 |
| 389 | N-[4-(Dimethylamino-thiophen-2-yl-methyl)-cydohexylmethyl]-2-p-tolyloxy-nicotinamid (unpolareres Diastereomer) | 463,23 |

(fortgesetzt)

| Nr. | Name | Masse |
|---|---|---|
| 390 | 4-Brom-2-ethyl-5-methyl-2H-pyrazol-3-carbonsäure-[4-(dimethylamino-phenyl-methyl)-cyclohexylmethyl]-amid | 460,18 |
| 391 | N-{4-[(4-Chlor-phenyl)-dimethylamino-methyl]-cyclohexylmethyl}-2-phenoxy-propionamid | 428,22 |
| 392 | N-[4-(Dimethylamino-phenyl-methyl)-cyclohexylmethyl]-3,5-dinitro-benzamid | 440,21 |
| 393 | N-{4-[(4-Chlor-phenyl)-dimethylamino-methyl]-cyclohexylmethyl}-3-methoxy-benzamid | 414,21 |
| 394 | 2-Brom-N-{4-[(4-chlor-phenyl)-dimethylamino-methyl]-cyclohexylmethyl}-benzamid | 462,11 |
| 395 | 2-Brom-N-(2-{4-[(4-chlor-phenyl)-dimethylamino-methyl]-cyclohexyl}ethyl)-benzamid | 476,12 |
| 396 | 2-Brom-N-(2-{4-[dimethylamino-(3-fluor-phenyl)-methyl]-cyclohexyl}-ethyl)-benzamid | 460,15 |
| 397 | 3-Chlor-N-{2-[4-(dimethylamino-phenyl-methyl-cyclohexyl]-ethyl}-benzamid (polareres Diastereomer) | 398,21 |
| 398 | 3-Chlor-N-{2-[4-(dimethylamino-phenyl-methyl)-cyclohexyl]-ethyl}-benzamid (unpolareres Diastereomer) | 398,21 |
| 399 | 3-Chlor-N-(2-{4-[(4-chlor-phenyl)-dimethylamino-methyl]-cyclohexyl}-ethyl)-benzamid | 432,17 |
| 400 | 3-Chlor-N-(2-{4-[dimethylamino-(3-fluor-phenyl)-methyl]-cyclohexyl}-ethyl)-benzamid (unpolareres Diastereomer) | 416,20 |
| 401 | 3-Chlor-N-(2-{4-[dimethylamino-(3-fluor-phenyl)-methyl]-cyclohexyl}-ethyl)-benzamid (polareres Diastereomer) | 416,20 |
| 402 | 2-Chlor-N-{2-[4-(dimethylamino-phenyl-methyl)-cyclohexyl]-ethyl}-benzamid | 398,21 |
| 403 | 2-Chlor-N-(2-{4-[(4-chlor-phenyl)-dimethylamino-methyl]-cyclohexyl}-ethyl)-benzamid | 432,17 |
| 404 | 2-Chlor-N-(2-{4-[dimethylamino-(3-fluor-phenyl)-methyl]-cyclohexyl}-ethyl)-benzamid | 416,20 |
| 405 | 4-Chlor-N-{2-[4-(dimethylamino-phenyl-methyl)-cyclohexyl]-ethyl}-benzamid | 398,21 |
| 406 | 4-Chlor-N-(2-{4-[dimethylamino-(3-fluor-phenyl)-methyl]-cyclohexyl}-ethyl)-benzamid | 416,20 |
| 407 | N-{2-[4-(Dimethylamino-phenyl-methyl)-cyclohexyl]-ethyl}-4-fluor-benzamid | 382,24 |
| 408 | N-(2-{4-[(4-Chlor-phenyl)-dimethylamino-methyl]-cyclohexyl}-ethyl)-4-fluor-benzamid | 416,20 |
| 409 | N-(2-{4-[Dimethylamino-(3-fluor-phenyl)-methyl]-cyclohexyl}-ethyl)-4-fluor-benzamid | 400,23 |
| 410 | N-{2-[4-(Dimethylamino-phenyl-methyl)-cyclohexyl]-ethyl}-2-fluor-benzamid | 382,24 |
| 411 | N-(2-{4-[Dimethylamino-(3-fluor-phenyl)-methyl]-cyclohexyl}-ethyl)-2-fluor-benzamid | 400,23 |
| 412 | N-(2-{4-[Dimethylamino-(3-fluor-phenyl)-methyl]-cyclohexyl}-ethyl)-3-methyl-benzamid | 396,26 |
| 413 | 2,6-Dichlor-N-{2-[4-(dimethylamino-phenyl-methyl)-cyclohexyl]-ethyl}-benzamid | 432,17 |
| 414 | N-{2-[4-(Dimethylamino-phenyl-methyl)-cyclohexyl]-ethyl}-2-methoxy-benzamid | 394,26 |
| 415 | N-(2-(4-[Dimethylamino-(3-fluor-phenyl)-methyl]-cyclohexyl)-ethyl)-2-methoxy-benzamid | 412,25 |
| 416 | 3,4-Dichlor-N-{2-[4-(dimethylamino-phenyl-methyl)-cyclohexyl]-ethyl}-benzamid | 432,17 |
| 417 | N-{2-[4-(Dimethylamino-phenyl-methyl)-cyclohexyl]-ethyl}-2-methyl-benzamid (polareres Diastereomer) | 378,27 |
| 418 | N-{2-[4-(Dimethylamino-phenyl-methyl)-cyclohexyl]-ethyl}-2-methyl-benzamid (unpolareres Diastereomer) | 378,27 |
| 419 | N-(2-{4-[(4-Chlor-phenyl)-dimethylamino-methyl]-cyclohexyl}-ethyl)-2-methyl-benzamid | 412,23 |
| 420 | N-(2-(4-[Dimethylamino-(3-fluor-phenyl)-methyl]-cyclohexyl)-ethyl)-2-methyl-benzamid | 396,26 |
| 421 | 4-Cyano-N-{2-[4-(dimethylamino-phenyl-methyl)-cyclohexyl)-ethyl}-benzamid | 389,25 |
| 422 | 3-Chlor-N-(2-{4-[dimethylamino-(4-fluor-phenyl)-methyl]-cyclohexyl}-ethyl)-benzamid | 416,20 |

(fortgesetzt)

| Nr. | Name | Masse |
|---|---|---|
| 423 | 3-Chlor-N-(2-{4-[dimethylamino-(4-fluor-phenyl)-methyl]-cyclohexyl}-ethyl)-benzamid (unpolareres Diastereomer) | 416,20 |
| 424 | 3-Chlor-N-{2-[4-(dimethylamino-thiophen-2-yl-methyl)-cyclohexyl]-ethyl}-benzamid | 404,17 |
| 425 | 2-Chlor-N-{2-[4-(dimethylamino-thiophen-2-yl-methyl)-cyclohexyl]-ethyl}-benzamid | 404,17 |
| 426 | N-{2-[4-(Dimethylamino-thiophen-2-yl-methyl)-cyclohexyl]-ethyl}-4-fluor-benzamid | 388,20 |
| 427 | N-(2-{4-[Dimethylamino-(4-fluor-phenyl)-methyl]-cyclohexyl}-ethyl)-2-fluor-benzamid | 400,23 |
| 428 | N-{2-[4-(Dimethylamino-thiophen-2-yl-methyl)-cyclohexyl]-ethyl}-2-fluor-benzamid | 388,20 |
| 429 | N-(2-{4-[Dimethylamino-(4-fluor-phenyl)-methyl]-cyclohexyl}-ethyl)-3-methyl-benzamid | 396,26 |
| 430 | N-{2-[4-(Dimethylamino-thiophen-2-yl-methyl)-cyclohexyl]-ethyl}-3-methyl-benzamid | 384,22 |
| 431 | 2,6-Dichlor-N-{2-[4-(dimethylamino-thiophen-2-yl-methyl)-cyclohexyl]-ethyl}-benzamid | 438,13 |
| 432 | N-(2-{4-[Dimethylamino-(4-fluor-phenyl)-methyl]-cyclohexyl}-ethyl)-2-methoxy-benzamid | 412,25 |
| 433 | N-{2-[4-(Dimethylamino-phenyl-methyl)-cyclohexyl]-ethyl}-3,5-difluor-benzamid | 400,23 |
| 434 | N-(2-{4-[(4-Chlor-phenyl)-dimethylamino-methyl]-cyclohexyl}-ethyl)-3,5-difluor-benzamid | 434,19 |
| 435 | N-(2-{4-[Dimethylamino-(3-fluor-phenyl)-methyl]-cyclohexyl}-ethyl)-3,5-difluor-benzamid | 418,22 |
| 436 | N-(2-{4-[(4-Chlor-phenyl)-dimethylamino-methyl]-cyclohexyl}-ethyl)-2,4-difluor-benzamid | 434,19 |
| 437 | 2,4-Dichlor-N-(2-{4-[(4-chlor-phenyl)-dimethylamino-methyl]-cyclohexyl}-ethyl)-5-fluor-benzamid | 484,13 |
| 438 | 2,4-Dichlor-N-(2-{4-[dimethylamino-(3-fluor-phenyl)-methyl]-cyclohexyl}-ethyl)-5-fluor-benzamid (polareres Diastereomer) | 468,15 |
| 439 | 2,4-Dichlor-N-(2-{4-[dimethylamino-(3-fluor-phenyl)-methyl]-cyclohexyl}-ethyl)-5-fluor-benzamid (unpolareres Diastereomer) | 468,15 |
| 440 | 2,4-Dichlor-N-{2-[4-(dimethylamino-thiophen-2-yl-methyl)-cyclohexyl]-ethyl}-5-fluor-benzamid | 456,12 |
| 441 | 2-Chlor-N-(2-{4-[(4-chlor-phenyl)-dimethylamino-methyl]-cyctohexyl}-ethyl)-nicotinamid | 433,17 |
| 442 | Naphthalen-2-carbonsäure (2-{4-[dimethylamino-(4-fluor-phenyl)-methyl]-cyclohexyl}-ethyl)-amid | 432,26 |
| 443 | N-{2-[4-(Dimethylamino-thiophen-2-yl-methyl)-cyclohexyl]-ethyl}-4-propyl-benzamid | 412,25 |
| 444 | N-{2-[4-(Dimethylamino-phenyl-methyl)-cyclohexyl]-ethyl}-3,4-difluor-benzamid | 400,23 |
| 445 | N-(2-{4-[(4-Chlor-phenyl)-dimethylamino-methyl]-cyclohexyl}-ethyl)-3,4-difluor-benzamid | 434,19 |
| 446 | N-{2-[4-(Dimethylamino-thiophen-2-yl-methyl)-cyclohexyl]-ethyl}-3,4-difluor-benzamid | 406,19 |
| 447 | N-(2-{4-[Dimethylamino-(4-fluor-phenyl)-methyl]-cyclohexyl}-ethyl)-3-methoxy-benzamid | 412,25 |
| 448 | N-{2-[4-(Dimethylamino-phenyl-methyl)-cyclohexyl]-ethyl}-2,2-diphenyl-acetamid | 454,30 |
| 449 | 1-(4-Chlor-phenyl)-cyclopentan-carbonsäure-{2-[4-(dimethylamino-phenyl-methyl)-cyclohexyl]-ethyl}-amid | 466,28 |
| 450 | 2-Benzyloxy-N-{2-[4-(dimethylamino-phenyl-methyl)-cyclohexyl]-ethyl}-acetamid | 408,28 |
| 451 | N-{2-[4-(Dimethylamino-phenyl-methyl)-cyclohexyl]-ethyl}-2-phenyl-acetamid | 378,27 |
| 452 | Thiophen-2-carbonsäure-{2-[4-(dimethylamino-phenyl-methyl)-cyclohexyl]-ethyl}-amid | 370,21 |
| 453 | N-(2-{4-[Dimethylamino-(3-fluor-phenyl)-methyl]-cyclohexyl}-ethyl)-2-(3-methoxy-phenyl)-acetamid | 426,27 |
| 454 | N-{2-[4-(Dimethylamino-thiophen-2-yl-methyl)-cyclohexyl]-ethyl}-2-(3-methoxy-phenyl)-acetamid | 414,23 |
| 455 | N-(2-{4-[(4-Chlor-phenyl)-dimethylamino-methyl]-cyclohexyl}-ethyl)-2-phenyl-butyramid | 440,26 |
| 456 | N-{2-[4-(Dimethylamino-thiophen-2-yl-methyl)-cyclohexyl]-ethyl}-2-phenyl-butyramid | 412,25 |

(fortgesetzt)

| Nr. | Name | Masse |
|---|---|---|
| 457 | Benzo[b]thiophen-2-carbonsäure-{2-[4-(dimethylamino-phenyl-methyl)-cyclohexyl]-ethyl}-amid | 420,22 |
| 458 | N-{2-[4-(Dimethylamino-phenyl-methyl)-cyclohexyl]-ethyl}-4-nitro-benzamid | 409,24 |
| 459 | 3-Brom-N-(2-{4-[dimethylamino-(4-fluor-phenyl)-methyl]-cyclohexyl}-ethyl)-benzamid | 460,15 |
| 460 | N-{2-[4-(Dimethylamino-phenyl-methyl)-cyclohexyl]-ethyl}-2,3,4,5,6-pentafluor-benzamid | 454,20 |
| 461 | N-{2-[4-(Dimethylamino-phenyl-methyl)-cyclohexyl]-ethyl}-2,6-difluor-benzamid | 400,23 |
| 462 | N-(2-{4-[Dimethylamino-(3-fluor-phenyl)-methyl]-cyclohexyl}-ethyl-2,6-difluor-benzamid | 418,22 |
| 463 | 2-Phenyl-thiazol-4-carbonsäure-{2-[4-(dimethylamino-phenyl-methyl)-cyclohexyl]-ethyl}-amid | 447,23 |
| 464 | 2-Phenyl-thiazol-4-carbonsäure-(2-{4-[dimethylamino-(4-fluor-phenyl)-methyl]-cyclohexyl}-ethyl)-amid | 465,22 |
| 465 | Benzo[b]thiophen-3-carbonsäure-{2-[4-(dimethylamino-thiophen-2-yl-methyl)-cyclohexyl]-ethyl}-amid | 426,18 |
| 466 | N-{2-[4-(Dimethylamino-phenyl-methyl)-cyclohexyl]-ethyl}-2-methylsulfanyl-nicotinamid | 411,23 |
| 467 | 2-Methyl-5-phenyl-furan-3-carbonsäure-{2-[4-(dimethylamino-phenyl-methyl)-cyclohexyl]-ethyl}-amid | 444,28 |
| 468 | 2-(2,3-Dihydro-benzofuran-5-yl)-thiazol-4-carbonsäure-{2-[4-(dimethylamino-phenyl-methyl)-cyctohexyl]-ethyl}-amid | 489,24 |
| 469 | 3-(2,6-Dichlor-phenyl)-5-methyl-isoxazol-4-carbonsäure-{2-[4-(dimethylamino-thiophen-2-yl-methyl)-cyclohexyl]-ethyl}-amid | 519,15 |
| 470 | 2-(4-Chlor-phenylsulfanyl)-N-{2-[4-(dimethylamino-phenyl-methyl)-cyclohexyl]-ethyl}-nicotinamid (polareres Diastereomer) | 507,21 |
| 471 | 2-(4-Chlor-phenylsulfanyl)-N-{2-[4-(dimethylamino-phenyl-methyl)-cyclohexyl]-ethyl}-nicotinamid (unpolareres Diastereomer) | 507,21 |
| 472 | Benzo[1,2;3]thiadiazol-5-carbonsäure-{2-[4-(dimethylamino-phenyl-methyl)-cyclohexyl]-ethyl}-amid | 422,21 |
| 473 | 5-Brom-N-{2-[4-(dimethylamino-phenyl-methyl)-cyclohexyl]-ethyl}-nicotinamid | 443,16 |
| 474 | 5-Chlor-4-methoxy-thiophen-3-carbonsäure-{2-[4-(dimethylamino-phenyl-methyl)-cyclohexyl]-ethyl}-amid | 434,18 |
| 475 | 5-Chlor-4-methoxy-thiophen-3-carbonsäure-(2-{4-[dimethylamino-(4-fluor-phenyl)-methyl]-cyclohexyl}-ethyl)-amid | 452,17 |
| 476 | 5-Chlor-4-methoxy-thiophen-3-carbonsäure-{2-[4-(1-dimethylamino-3-phenyl-propyl)-cyclohexyl]-ethyl}-amid | 462,21 |
| 477 | 3-Cyano-N-{2-[4-(dimethylamino-phenyl-methyl)-cyclohexyl]-ethyl}-benzamid | 389,25 |
| 478 | N-{2-[4-(Dimethylamino-phenyl-methyl)-cyclohexyl]-ethyl}-2,4-dimethoxy-benzamid | 424,27 |
| 479 | 2-Chlor-N-((4-((dimethylamino)(phenyl)methyl)cyclohexyl)methyl)benzamid | 384,20 |
| 480 | N-((4-((Dimethylamino)(phenyl)methyl)cyclohexyl)methyl)-4-fluorbenzamid | 368,23 |
| 481 | N-(2-(4-((Dimethylamino)(phenyl)methyl)cyclohexyl)ethyl)-4-fluorbenzamid | 382,24 |
| 482 | N-((4-((Dimethylamino)(phenyl)methyl)cyclohexyl)methyl)-2-fluorbenzamid | 368,23 |
| 483 | N-((4-((Dimethylamino)(phenyl)methyl)cyclohexyl)methyl)-3-methylbenzamid | 364,25 |
| 484 | N-((4-((Dimethylamino)(phenyl)methyl)cyclohexyl)methyl)-2-methoxybenzamid | 380,25 |
| 485 | N-(2-(4-((dimethylamino)(phenyl)methyl)cyclohexyl)ethyl)-3,5-dimethoxybenzamid | 424,27 |
| 486 | N-((4-((Dimethylamino)(3-fluorphenyl)methyl)cyclohexyl)methyl)-2,6-dimethoxybenzamid | 428,25 |

(fortgesetzt)

| Nr. | Name | Masse |
|---|---|---|
| 487 | N-((4-((Dimethylamino)(phenyl)methyl)cyclohexyl)methyl)-2,4-difluorbenzamid | 386,22 |
| 488 | N-((4-((Dimethylamino)(thiophen-2-yl)methyl)cyclohexyl)methyl)-3-methoxybenzamid | 386,20 |
| 489 | N-((4-((Dimethylamino)(thiophen-2-yl)methyl)cyclohexyl)methyl)-3,4,5-trimethoxybenzamid | 446,22 |
| 497 | 3-Thiophen-2-yl-[1,2,4]oxadiazol-5-carbonsäure[4-(dimethylamino-phenyl-methyl)-cyclohexyl]-amid | 410,18 |
| 498 | 3-Methyl-[1,2,4]oxadiazol-5-carbonsäure{2-[4-(dimethylamino-phenyl-methyl)-cyclohexyl]-ethyl}-amid | 370,24 |
| 499 | 3-Phenyl-[1,2,4]oxadiazol-5-carbonsäure{4-[dimethylamino-(3-fluoro-phenyl)-methyl]-cyclohexyl}-amid | 422,21 |
| 500 | 3-Cyclopropylmethyl-[1,2,4]oxadiazol-5-carbonsäure {4-[dimethylamino-(3-fluoro-phenyl)-methyl]-cyclohexyl}-amid | 400,23 |
| 501 | 3-Methoxymethyl-[1,2,4]oxadiazol-5-carbonsäure {2-[4-(1-dimethylamino-3-phenyl-propyl)-cyclohexyl]-ethyl}-amid | 428,28 |

**Amidierung der sekundären Amine ($R^1 = (CH_2)_nNR^6R^7$, $R^7 = COR^{13}$)**

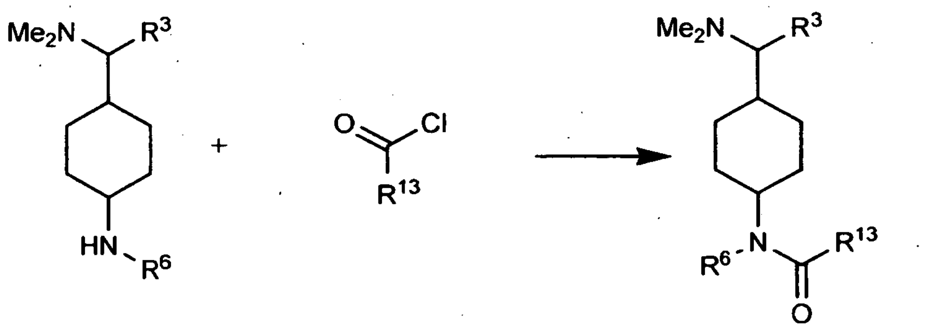

**N-(4-((Dimethylamino)(phenyl)methyl)cyclohexyl)-N-(3-phenylpropyl)acetamid Hydrochlorid 95 ($R^3$ = Phenyl)**

Das Amin **85** (1,1g, 3,1 mmol) wurde in wasserfreiem Pyridin (20 ml) gelöst und bei Raumtemperatur mit Acetanhydrid (3,2g, 31,4 mmol) versetzt. Der Reaktionsansatz wurde über Nacht bei RT gerührt. Zum Entfernen des überschüssigen Acetanhydrids wurde der Reaktionsansatz mit Toluol (20 ml) versetzt und am Rotationsverdampfer eingeengt. Dieser Vorgang wurde noch zweimal wiederholt. Der Rückstand wurde mit 1 N NaOH versetzt und das Produkt mit Essigester extrahiert. Die organische Phase wurde nach dem Trocknen über $Na_2SO_4$ am Rotationsverdampfer eingeengt. Die Aufreinigung des Rohprodukts erfolgte durch Flash-Chromatographie (Laufmittel: Diethylether) .

Das Produkt wurde in Methylethylketon (3 ml) gelöst und dann mit Wasser (0,01 ml/ 1 mmol) und Trimethylchlorsilan (1,3 ml/ lmmol) versetzt. Nach Absaugen und waschen mit Ether erhielt man weiße Kristalle, die im Vakuum getrocknet wurden.

**N-(4-((Dimethylamino)(phenyl)methyl)cyclohexyl)-N-phenylacetamid Hydrochlorid 96 ($R^3$ = Phenyl)**

Das Amin **92** (0,9g, 2,9 mmol) wurde in wasserfreiem Pyridin (20 ml) gelöst und bei Raumtemperatur mit Acetanhydrid (2,98 g, 29,2 mmol) versetzt. Der Reaktionsansatz wurde über Nacht bei RT gerührt. Zum Entfernen des überschüssigen Acetanhydrids wurde der Reaktionsansatz mit Toluol (20 ml) versetzt und am Rotationsverdampfer eingeengt. Dieser Vorgang wurde noch zweimal wiederholt. Der Rückstand wurde mit 1 N NaOH versetzt und das Produkt mit Essigester extrahiert. Die organische Phase wurde nach dem Trocknen über $Na_2SO_4$ am Rotationsverdampfer eingeengt. Die Aufreinigung des Rohprodukts erfolgte säulenchromatographisch (Laufmittel: Essigester/Methanol: 20 : 1)

Das Produkt wurde in Methylethylketon (5 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden. $^{13}C$ NMR (75 MHz, DMSO) 23,20; 25,16; 25,89; 25,58; 25,64; 31,45; 37,09; 42,93; 52,97; 68,87; 127,93; 128,73; 129,17 ; 129,81; 130,08; 130,92; 140,02; 168,62.

**N-(4-((Dimethylamino)(phenyl)methyl)cyclohexyl)-N-(4-phenylbutyl)propionamid Hydrochlorid 97 ($R^3$ = Phenyl)**

Das Amin **87** (0,49g, 1,3 mmol) wurde in DCM (1 ml) gelöst, mit Triethylamin (0,27g, 2,7 mmol) und einer Spatelspitze DMAP versetzt und auf -10°C abgekühlt. Das in DCM (2 ml) gelöste Propionylchlorid (0,19g, 2,7 mmol) wurde zugegeben und der Reaktionsansatz 5 h bei RT gerührt. Zur Aufarbeitung wurde KOH (5 N, 2 ml) zugegeben, die Phasen getrennt und die wässrige Phase noch zweimal mit je 5 ml Dichlormethan extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether/Methanol: 40 : 1) aufgereinigt.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(4-((Dimethylamino)(phenyl)methyl)cyclohexyl)-N-(4-phenylbutyl)acetamid Hydrochlorid 98 ($R^3$ = Phenyl)**

Das Amin **87** (0,50g, 1,4 mmol) wurde in DCM (1 ml) gelöst, mit Triethylamin (0,28g, 2,8 mmol) und einer-Spatelspitze DMAP versetzt und auf -10°C abgekühlt. Das in DCM (2 ml) gelöste Acetylchlorid (0,16g, 2,1 mmol) wurde zugegeben und der Reaktionsansatz 5 h bei RT gerührt. Zur Aufarbeitung wurde KOH (5 N, 2 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether/Methanol: 20 : 1) aufgereinigt.

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(4-((Dimethylamino)(phenyl)methyl)cyclohexyl)-N-(4-methoxyphenyl)acetamid Hydrochlorid 99 ($R^3$ = Phenyl)**

Das Amin **89** (0,68g, 2,0 mmol) wurde in DCM (2 ml) gelöst, mit Triethylamin (0,41g, 4,0 mmol) und einer Spatelspitze DMAP versetzt und auf -10°C abgekühlt. Das in DCM (2 ml) gelöste Acetylchlorid (0,24g, 3,0 mmol) wurde zugegeben und der Reaktionsansatz 5 h bei RT gerührt. Zur Aufarbeitung wurde KOH (5 N, 2 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether/Methanol: 20: 1) aufgereinigt.

Das Produkt wurde in Methylethylketon (4 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden. $^{13}C$ NMR (75 MHz, DMSO) 21,21; 23,16; 23,64; 23,77; 24,01; 29,65; 35,24; 41,02; 50,71; 53,27; 67,02; 112,32; 126,83; 127,53; 128,95; 130,65; 156,49; 167,10.

**N-Benzyl-N-(4-((dimethylamino)(phenyl)methyl)cyclohexyl)acetamid Hydrochlorid 100 ($R^3$ = Phenyl)**

Das Amin **86** (0,9g, 2,8 mmol) wurde in wasserfreiem Pyridin (18 ml) gelöst und bei Raumtemperatur mit Acetanhydrid (2,85 g, 27,9 mmol) versetzt. Der Reaktionsansatz wurde über Nacht bei RT gerührt. Zum Entfernen des überschüssigen Acetanhydrids wurde der Reaktionsansatz mit Toluol (20 ml) versetzt und am Rotationsverdampfer eingeengt. Dieser Vorgang wurde noch zweimal wiederholt. Der Rückstand wurde mit 1 N NaOH versetzt und das Produkt mit Essigester extrahiert. Die organische Phase wurde nach dem Trocknen über $Na_2SO_4$ am Rotationsverdampfer eingeengt. Die Aufreinigung des Rohprodukts erfolgte säulenchromatographisch (Laufmittel: Essigester/Methanol: 20 : 1)

Das Produkt wurde in Methylethylketon (5 ml) gelöst und dann mit Wasser (0,01 ml/ 1 mmol) und Trimethylchlorsilan (1,3 ml/ lmmol) versetzt. Nach Absaugen und waschen mit Ether erhielt man weiße Kristalle, die im Vakuum getrocknet wurden.

**N-Benzyl-N-(4-((dimethylamino)(phenyl)methyl)cyclohexyl)-2-ethylbutanamid Hydrochlorid 101($R^3$ = Phenyl)**

Das Amin **86** (0,5g, 1,6 mmol) wurde in DCM (1,6 ml) gelöst, mit Triethylamin (0,31g, 3,1 mmol) und einer Spatelspitze DMAP versetzt und auf -10°C abgekühlt. Das in DCM (2,8 ml) gelöste 2-Ethylbutyrchlorid (0,31g, 2,3 mmol) wurde zugegeben und der Reaktionsansatz 16 h bei RT gerührt. Zur Aufarbeitung wurde KOH (5 N, 10 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether) aufgereinigt.

Das Produkt wurde in Methylethylketon (3 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.

**N-Benzyl-N-(4-((dimethylamino)(phenyl)methyl)cyclohexyl)butyramid Hydrochlorid 102 ($R^3$ = Pheny)**

Das Amin **86** (0,5g, 1,6 mmol) wurde in DCM (1,6 ml) gelöst, mit Triethylamin (0,31g, 3,1 mmol) und einer Spatelspitze DMAP versetzt und auf -10°C abgekühlt. Das in DCM (2,8 ml) gelöste Buttersäurechlorid (0,25g, 2,3 mmol) wurde zugegeben und der Reaktionsansatz 16 h bei RT gerührt. Zur Aufarbeitung wurde KOH (5 N, 10 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether) aufgereinigt.

Das Produkt wurde in Methylethylketon (3 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-Benzyl-N-(4-((dimethylamino)(phenyl)methyl)cyclohexyl)-4-fluorbenzamid Hydrochlorid 103 ($R^3$ = Phenyl)**

Das Amin **86** (0,5g, 1,6 mmol) wurde in DCM (1,6 ml) gelöst, mit Triethylamin (0,31g, 3,1 mmol) und einer Spatelspitze DMAP versetzt und auf -10°C abgekühlt. Das in DCM (2,8 ml) gelöste 4-Fluorbenzoylchlorid (0,37g, 2,3 mmol) wurde zugegeben und der Reaktionsansatz 16 h bei RT gerührt. Zur Aufarbeitung wurde KOH (5 N, 10 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether) aufgereinigt.

Das Produkt wurde in Methylethylketon (4 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-Benzyl-N-(4-((dimethylamino)(phenyl)methyl)cyclohexyl)benzamid Hydrochlorid 104 ($R^3$ = Phenyl)**

Das Amin **86** (0,5g, 1,6 mmol) wurde in DCM (1,6 ml) gelöst, mit Triethylamin (0,31g, 3,1 mmol) und einer Spatelspitze DMAP versetzt und auf -10°C abgekühlt. Das in DCM (2,8 ml) gelöste Benzoylchlorid (0,33g, 2,3 mmol) wurde zugegeben und der Reaktionsansatz 16 h bei RT gerührt. Zur Aufarbeitung wurde KOH (5 N, 10 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Rohprodukt wurde säulenchromatographisch (Diethylether) aufgereinigt.

Das Produkt wurde in Methylethylketon (5 ml) gelöst und dann mit Wasser (0,01 ml/ 1 mmol) und Trimethylchlorsilan (1,3 ml/ 1mmo)) versetzt. Nach Absaugen und waschen mit Ether erhielt man weiße Kristalle, die im Vakuum getrocknet wurden.

**N-(4-((Dimethylamino)(phenyl)methyl)cyclohexyl)-2-ethyl-N-phenylbutanamid Hydrochlorid 105 ($R^3$ = Phenyl)**

Das Amin **92** (0,55g, 1,8 mmol) wurde in DCM (1,8 ml) gelöst, mit Triethylamin (0,36g, 3,6 mmol) und einer Spatelspitze DMAP versetzt und auf -10°C abgekühlt.

Das in DCM (3,2 ml) gelöste 2-Ethylbutylchlorid (0,36g, 2,7 mmol) wurde zugegeben und der Reaktionsansatz 16 h bei RT gerührt. Zur Aufarbeitung wurde KOH (5 N, 10 ml) zugegeben, die Phasen getrennt und die wässrige Phase noch zweimal mit je 5 ml DCM extrahiert. Die vereinigten organischen Phasen wurden mit gesättigter NaCl-Lösung gewaschen, über $Na_2SO_4$ getrocknet und eingeengt. Das Produkt wurde über präperative HPLC aufgereinigt.

**Sulfonylierung der primären Amine ($R^1 = (CH_2)_nNHSO_2R^{12}$)**

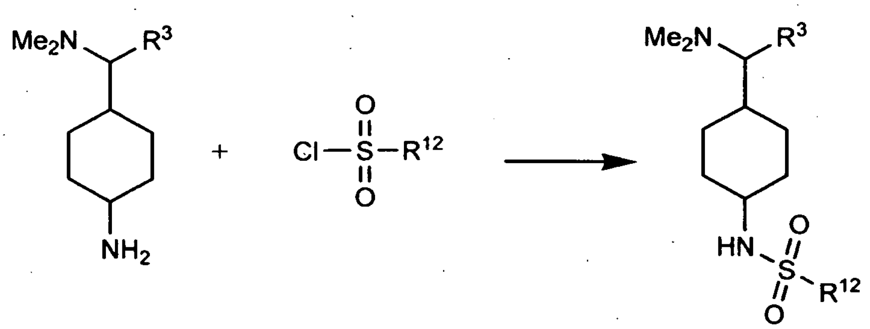

**4-Chlor-N-(4-((dimethylamino)(phenyl)methyl)cyclohexyl)benzolsulfonamid Hydrochlorid 106 ($R^3$ = Phenyl)**

Das Amin **17** (0,24g, 1 mmol) wurde in DCM (6,9 ml) gelöst und zunächst mit Triethylamin (0,13g, 1,2 mmol) und dann mit 4-Chlorbenzolsulfonsäurechlorid (0,44g, 2,1 mmol) versetzt und 22 h bei Raumtemperatur gerührt. Nach beendeter Reaktion wurde zunächst mit Wasser hydrolysiert und anschließend mit $Na_2CO_3$-Lösung alkalisch gestellt. Das Produkt wurde mit DCM extrahiert, getrocknet über $Na_2SO_4$ und eingeengt. Die Aufreinigung erfolgte säulenchromatographisch (Essigester/Methanol 20: 1)

Das Produkt wurde in Methylethylketon (5 ml) gelöst und dann mit Wasser (0,01 ml/ 1 mmol) und Trimethylchlorsilan (1,3 ml/ 1mmol) versetzt. Nach Absaugen und waschen mit Ether erhielt man ein rotbraunes Harz, das im Vakuum getrocknet wurden.

**N-(4-((Dimethylamino)(phenyl)methyl)cyclohexyl)-4-methoxybenzolsulfonamid Hydrochlorid 107 ($R^3$ = Phenyl)**

Das Amin **17** (0,24g, 1 mmol) wurde in DCM (6,9 ml) gelöst und zunächst mit Triethylamin (0,13g, 1,2 mmol) und dann mit 4-Methoxybenzolsulfonsäurechlorid (0,43g, 2,1 mmol) versetzt und 22 h bei Raumtemperatur gerührt. Nach beendeter Reaktion wurde zunächst mit Wasser hydrolysiert und anschließend mit $Na_2CO_3$-Lösung alkalisch gestellt. Das Produkt wurde mit DCM extrahiert, getrocknet über $Na_2SO_4$ und eingeengt. Die Aufreinigung erfolgte säulenchromatographisch (Essigester/Methanol 20:1)

Das Produkt wurde in Methylethylketon (5 ml) gelöst und dann mit Wasser (0,01 ml/ 1 mmol) und Trimethylchlorsilan (1,3 ml/ 1mmol) versetzt. Nach Absaugen und waschen mit Ether erhielt man ein rotbraunes Harz, das im Vakuum getrocknet wurden.

**4-tert-Butyl-N-(4-((dimethylamino)(phenyl)methyl)cyclohexyl)benzolsulfonamid Hydrochlorid 108 ($R^3$ = Phenyl)**

Das Amin **17** (0,24g, 1 mmol) wurde in DCM (6,9 ml) gelöst und zunächst mit Triethylamin (0,13g, 1,2 mmol) und dann mit4-tert-Butylbenzolsulfonsäurechlorid (0,48g, 2,1 mmol) versetzt und 22 h bei Raumtemperatur gerührt. Nach beendeter Reaktion wurde zunächst mit Wasser hydrolysiert und anschließend mit $Na_2CO_3$-Lösung alkalisch gestellt. Das Produkt wurde mit DCM extrahiert, getrocknet über $Na_2SO_4$ und eingeengt. Die Aufreinigung erfolgte säulenchromatographisch (Essigester/Methanol 20:1)

Das Produkt wurde in Methylethylketon (5 ml) gelöst und dann mit Wasser (0,01 ml/ 1 mmol) und Trimethylchlorsilan (1,3 ml/ 1mmol) versetzt. Nach Absaugen und waschen mit Ether-erhielt man weiße Kristalle, die im Vakuum getrocknet wurden.

**N-(4-((Dimethylamino)(phenyl)methyl)cyclohexyl)-2-nitrobenzolsulfonamid Hydrochlorid 109 ($R^3$ = Phenyl)**

Das Amin **17** (0,24g, 1 mmol) wurde in DCM. (6,9 ml) gelöst und zunächst mit Triethylamin (0,13g, 1,2 mmol) und dann mit 2-Nitrobenzolsulfonsäurechlorid (0,46g, 2,1 mmol) versetzt und 22 h bei Raumtemperatur gerührt. Nach beendeter Reaktion wurde zunächst mit Wasser hydrolysiert und anschließend mit $Na_2CO_3$-Lösung alkalisch gestellt. Das Produkt wurde mit DCM extrahiert, getrocknet über $Na_2SO_4$ und eingeengt. Die Aufreinigung erfolgte säulenchromatographisch (Essigester/Methanol 20:1)

Das Produkt wurde in Methylethylketon (5 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**N-(4-((Dimethylamino)(phenyl)methyl)cyclohexyl)benzolsulfonamid Hydrochlorid 110 ($R^3$ = Phenyl)**

Das Amin **17** (0,24g, 1 mmol) wurde in DCM (6,9 ml) gelöst und zunächst mit Triethylamin (0,13g, 1,2 mol) und dann mit Benzolsulfonsäurechlorid (0,36g, 2,1 mmol) versetzt und 22 h bei Raumtemperatur gerührt. Nach beendeter Reaktion wurde zunächst mit Wasser hydrolysiert und anschließend mit $Na_2CO_3$-Lösung alkalisch gestellt. Das Produkt wurde mit DCM extrahiert, getrocknet über $Na_2SO_4$ und eingeengt. Die Aufreinigung erfolgte säulenchromatographisch (Essigester/Methanol 20:1)

Das Produkt wurde in Methylethylketon (3 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**Synthese der Cyclohexanole, Hydroxymethyl-, Hydroxyethyl-, und Hydroxypropylcyclohexane**

Aus den entsprechenden Cyclohexanonen, Cyclohexylaldehyden und Cyclohexylacetaldehyden erhält man durch Reduktion die entsprechenden Alkohole.

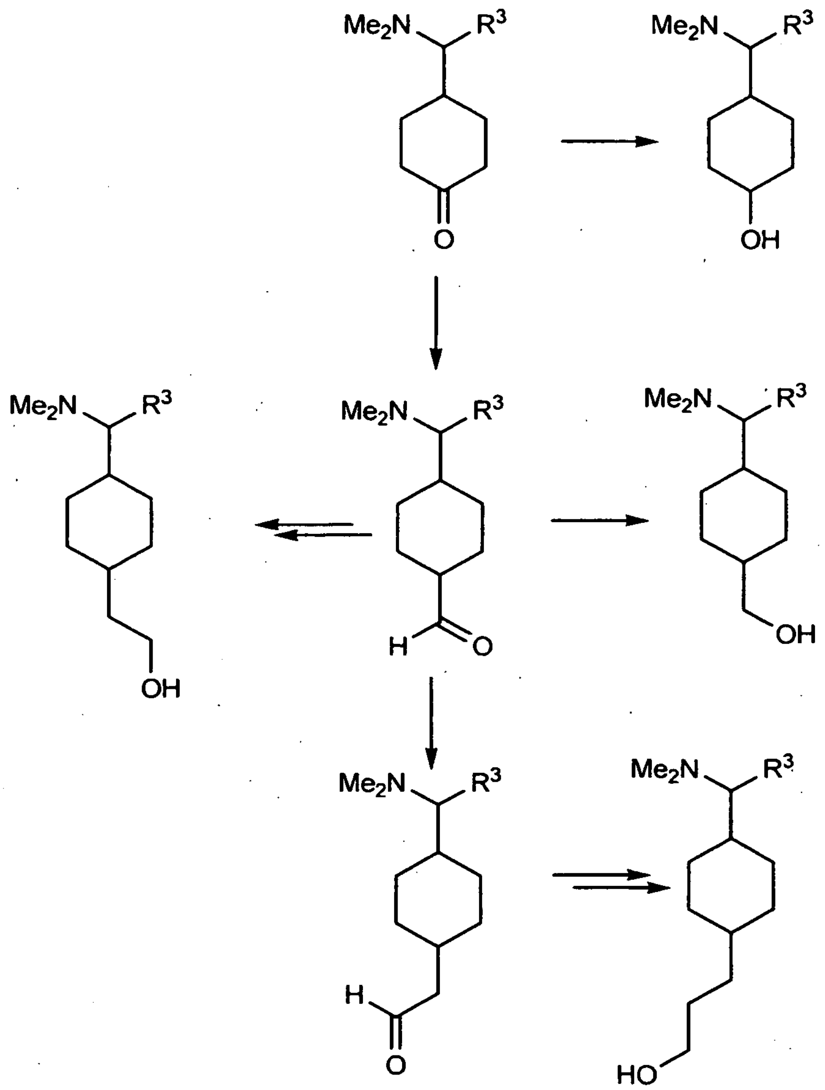

**Synthese der Cyclohexanole ($R^1 = (CH_2)_nOH$, n = 0)**

Die Cyclohexanole wurden durch Reduktion der entsprechend substituierten Cyclohexanone mit Natriumborhydrid dargestellt

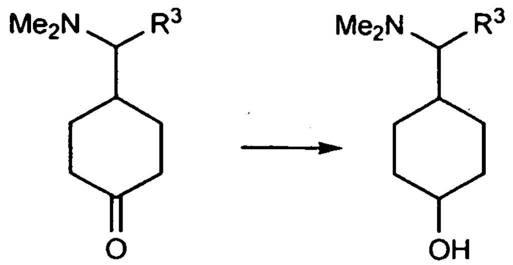

**4-[Dimethylamino-phenyl-methyl]-cyclohexanol 111 ($R^3$ = Phenyl)**

Zu einer Lösung des Ketons **10** (1,5g, 6,5 mmol) in THF (6,5 ml) tropft man $LiAlH_4$ (2,8 ml, 6,5 mmol, 2,3 M in THF) so zu, daß das THF gelinde siedet. Nach vollständiger Zugabe wird 15h bei RT gerührt.

Unter Eisbadkühlung wird der Ansatz mit Wasser (10ml) vorsichtig gequencht. Danach versetzt man den Ansatz mit NaOH-Lsg. (10ml, 5N). Nach 1h Rühren wird über Filtererde abfiltriert und mit Ether nachgewaschen. Es wird 3 x mit 40ml Ether extrahiert, über $Na_2SO_4$ getrocknet und eingeengt.

Ausbeute: 1,42 g (93%) Öl $^{13}$ C-NMR ($CDCl_3$): 27,50; 24,73; 35,40; 35,60; 37,71; 41,59 ($N(CH_3)_2$); 71,13; 75,11; 126,90; 127,65; 129,32, 137,14.

**4=[Dimethylamino-(4-fluorphenyl)-methyl]-cyclohexanol 112 ($R^3$=4-Fluorphenyl)**

Das Keton **11** (6,22 g, 25 mmol) wurde in Ethanol (250 ml) gelöst, mit Natriumborhydrid (1,89 g, 50 mmol) versetzt und 3 h bei RT gerührt. Das Reaktionsgemisch wurde i. Vak. eingeengt, der Rückstand mit Wasser versetzt und mit Essigester (3 x 70 ml) extrahiert. Die vereinigten Extrakte wurden mit Wasser und gesättigter Natriumchlorid-Lösung gewaschen, getrocknet ($Na_2SO_4$) und i. Vak. eingeengt.
Ausbeute: 5,92 g (94 %), Öl
$^{13}$C-NMR ($CDCl_3$): 23,47; 24,73; 27,36; 29,27; 32,28; 35,42; 37,39; 37,97; 41,68; 41,99 ($N(CH_3)_2$); 60,39; 66,91 (CH); 71,04; 74,34; 114,23; 114,44; 130,33; 130,40; 130,48; 132,79; 160,41; 162,83.

**4-[Dimethylamino-(3-fluorphenyl)-methyl]-cyclohexanol 113 ($R^3$ = 3-Fluorphenyl)**

Das Keton **12** (6,22 g, 25 mmol) wurde in Ethanol (250 ml) gelöst, mit Natriumborhydrid (1,89 g, 50 mmol) versetzt und 3 h bei RT gerührt. Das Reaktionsgemisch wurde i. Vak. eingeengt, der Rückstand mit Wasser versetzt und mit Essigester (3 x 70 ml) extrahiert. Die vereinigten Extrakte wurden mit Wasser und gesättigter Natriumchlorid-Lösung gewaschen, getrocknet ($Na_2SO_4$) und i. Vak. eingeengt.
Ausbeute: 6,00 g (96 %), Öl
$^{13}$C-NMR ($CDCl_3$): 23,46; 24,55; 27,32; 29,12; 32,15; 35,25; 37,77; 41,55; 41,63; 41,89 ($N(CH_3)_2$); 64,06; 66,66; (CH); 70,76;.74,62; 113,42; 113,64; 115,47;.115,68; 124,75; 124,89; 128,70; 128,78; 139,47; 139,52; 161,02; 163,45.

**4-[(4-Chlorphenyl)-dimethytamino-methyl]-cyclohexanol 114 ($R^3$ = 4-Chlorphenyl)**

Das Keton **13** (5,84 g, 22 mmol) wurde in Ethanol (200 ml) gelöst, mit Natriumborhydrid (1,66 g, 44 mmol) versetzt und 3 h bei RT gerührt. Das Reaktionsgemisch wurde i. Vak. eingeengt, der Rückstand mit Wasser versetzt und mit Essigester (3 x 70 ml) extrahiert. Die vereinigten Extrakte wurden mit Wasser und gesättigter Natriumchlorid-Lösung gewaschen, über Natriumsulfat getrocknet und i. Vak. eingeengt.
Ausbeute: 5,89 g (100 %), Öl
$^{13}$C-NMR ($CDCl_3$): 23,39; 24,67; 27,26; 29,21; 32,26; 32,42; 35,38; 35,59; 37,29; 37,85; 41,71; 42,03 ($N(CH_3)_2$); 66,86; 71,01; 73,21; 74,45 (CH); 127,69; 130,31; 132,43; 135,26; 135,66.

**4-(Dimethylamino-thiophen-2-yl-methyl)-cyclohexanol 115 ($R^3$ = 2-Thiophen)**

Das Keton **14** (5,93 g, 25 mmol) wurde in Ethanol (200 ml) gelöst, mit Natriumborhydrid (1,89 g, 50 mmol) versetzt und 20 h bei RT gerührt. Das Reaktionsgemisch wurde i. Vak. eingeengt, der Rückstand mit Wasser versetzt und mit Essigester (3 x 70 ml) extrahiert. Die vereinigten Extrakte wurden mit Wasser und gesättigter Natriumchlorid-Lösung gewaschen, getrocknet ($Na_2SO_4$) und i. Vak. eingeengt.
Ausbeute: 5,71 g (95 %), rötliches Öl $^{13}$ C-NMR ($CDCl_3$): 24,40; 24,48; 28,50; 28,98; 32,07; 35,26; 35,34; 39,11; 39,76; 41,05; 41,27; 67,12; 68,09; 69,69; 71,06; 123,83; 126,06; 126,35; 126,49; 139,89.

**4-(1-Dimethylamino-3-phenyl-propyl)-cyclohexanol 116 ($R^3$= Phenethyl)**

Das Keton **15** (5,7 g, 22 mmol) wurde in Ethanol (200 ml) gelöst, mit
Natriumborhydrid (1,66 g, 44 mmol) versetzt und 3 h bei RT gerührt. Das Reaktionsgemisch wurde i. Vak. eingeengt, der Rückstand mit Wasser versetzt und
mit Essigester (3 x 70 ml) extrahiert. Die vereinigten Extrakte wurden mit Wasser und gesättigter Natriumchlorid-Lösung gewaschen, über Natriumsulfat getrocknet und i.
Vak. eingeengt.
Ausbeute: 5,56 g (97 %), Öl

$^{13}C$-NMR ($CDCl_3$): 24,21; 25,16; 28,48; 29,28; 29,64; 32,68; 35,58; 39,20; 41,23; 41,29; ($N(CH_3)_2$): 66,68; 67,24; 67,82 (CH); 71,04; 125,57; 128,17; 142,68.

**Synthese der Hydroxymethylcyclohexane ($R^1$= $(CH_2)_nOH$, n = 1)**

Die Hydroxymethylcyclohexane wurden durch Reduktion der entsprechenden Cyclohexylaldehyde mit Natriumborhydrid erhalten.

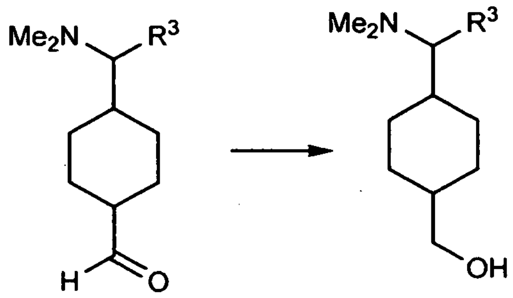

**[4-(Dimethylamino-phenyl-methyl)-cyclohexyl]-methanol 117 ($R^3$ = Phenyl)**

Der Aldehyd **28** (6,13 g, 25 mmol) wurde unter Argon in Ethanol (100 ml), Wasser (50 ml) und 1 N NaOH (25 ml, 25 mmol) gelöst und 30 min bei RT gerührt. Dann wurde eine Lösung aus $NaBH_4$ (1,82 g, 50 mmol) in Wasser (160 ml) langsam zugetropft und der Ansatz über Nacht gerührt. Das Ethanol wurde i. Vak. entfernt, der wässrige Rückstand dreimal mit Essigester (je 100 ml) extrahiert, die organische Phase mit Wasser (100 ml) und gesättigter NaCl-Lösung (100 ml) gewaschen, getrocknet ($Na_2SO_4$) und i. Vak. eingeengt.
Ausbeute: 5,86 g (95 %) $^{13}C$-NMR ($CDCl_3$): 28,84; 29,35; 29,50; 30,53; 38,78; 40,69; 41,95 ($N(CH_3)_2$); 68,39; 75,11; 126,56; 127,33; 129.14; 137,08.

**(4-[Dimethylamino-(4-fluorphenyl)-methyl]-cyclohexyl)-methanol 118 ($R^3$= 4-Fluorphenyl)**

Der Aldehyd **31** (6,2 g, 24 mmol) wurde unter Argon in Ethanol (105 ml), Wasser (53 ml) und 1 N NaOH (24 ml, 24 mmol) gelöst und 30 min bei RT gerührt. Dann wurde eine Lösung aus $NaBH_4$ (1,82 g, 48 mmol) in Wasser (158 ml) langsam zugetropft und der Ansatz über Nacht gerührt. Das Ethanol wurde i. Vak. entfernt, der wässrige Rückstand dreimal mit Essigester (je 100 ml) extrahiert, die organische Phase mit
Wasser (100 ml) und gesättigter NaCl-Lösung (100 ml) gewaschen, getrocknet
($Na_2SO_4$) und i. Vak. eingeengt.
Ausbeute: 5,99 g (94 %)
$^{13}C$-NMR ($CDCl_3$): 21,04; 25,56; 25,64; 26,02; 28,63; 29,47; 30,54; 38,95; 40,70; 41,40; 41,97 ($N(CH_3)_2$); 60,34; 68,39 (CH); 74,80; 114,10; 114,30; 130,33; 130,41; 132,91; 160,31; 162,73.

**{4-[Dimethylamino-(3-fluorphenyl)-methyl]-cyclohexyl}-methanol 119 ($R^3$ = 3-Fluorphenyl)**

Der Aldehyd **34** (7,11 g, 27 mmol) wurde unter Argon in Ethanol (120 ml), Wasser (60 ml) und 1 N NaOH (27 ml, 27 mmol) gelöst und 30 min bei RT gerührt. Dann wurde eine Lösung aus $NaBH_4$ (2,04 g, 54 mmol) in Wasser (200 ml) langsam zugetropft und der Ansatz über Nacht gerührt. Ethanol wurde i. Vak. entfernt, der wässrige Rückstand dreimal mit Essigester (je 100 ml) extrahiert, die organische Phase mit Wasser (100 ml) und gesättigter NaCl-Lösung (100 ml) gewaschen,
getrocknet ($Na_2SO_4$) und i. Vak. eingeengt.
Ausbeute: 7,1 g (99 %)
$^{13}C$-NMR ($CDCl_3$): 25,32; 25,57; 25,61; 28,71; 29,28, 29,45; 30,46; 37,86; 38,83; 40,70; 41,41; 41,96 ($N(CH_3)_2$)-, 65,72; 68,43; 71,20; 75,15; 113,38; 113,59; 115,56; 115,77; 124,89; 128.67: 128,75; 140,09; 140,15; 161,06; 163,50.

**{4-[(4-Chlorphenyl)-dimethylamino-methyl]-cyclohexyl}-methanol 120 ($R^3$ = 4-Chlorphenyl)**

Der Aldehyd **37** (5,59 g, 20 mmol) wurde unter Argon in Ethanol (100 ml), Wasser (50 ml) und 1 N NaOH (20 ml, 20 mmol) gelöst und 30 min bei RT gerührt. Dann wurde eine Lösung aus $NaBH_4$(1,51 g, 40 mmol) in Wasser (160 ml) langsam zugetropft und der Ansatz über Nacht gerührt. Ethanol wurde i. Vak. entfernt, der wässrige Rückstand dreimal mit Essigester (je 100 ml) extrahiert, die organische Phase mit Wasser (100 ml) und gesättigter NaCl-LÖsung (100 ml) gewaschen, getrocknet ($Na_2SO_4$) und i. Vak. eingeengt.
Ausbeute: 5,5 g (97%)
$^{13}$C-NMR ($CDCl_3$): 25,24; 25,55; 26,00; 28,61; 29,29; 29,46; 30,51; 37,86; 38,85 40,71; 41,45; 42,03 ($N(CH_3)_2$); 68,47; 74,92; 127,59; 130,64; 132,28; 135,82.

**[4-(Dimethylamino-thiophen-2-yl-methyl)-cytlohexyl]-methanol 121 ($R^3$ = 2-Thiophen)**

Der Aldehyd **40** (6,66 g, 26,4 mmol) wurde unter Argon in Ethanol (120 ml), Wasser (60 ml) und 1 N NaOH (26,4 ml, 27 mmol) gelöst und 30 min bei RT gerührt. Dann wurde eine Lösung aus $NaBH_4$ (1,89 g, 50 mmol) in Wasser (200 ml) langsam zugetropft und der Ansatz über Nacht gerührt. Ethanol wurde i. Vak. entfernt, der wässrige Rückstand dreimal mit Essigester (je 100 ml) extrahiert, die organische Phase mit Wasser (100 ml) und gesättigter NaCl-LÖsung (100 ml) gewaschen, getrocknet ($Na_2SO_4$) und i. Vak. eingeengt.
Ausbeute: 6,56 g (98 %)
$^{13}$C-NMR ($CDCl_3$): 25,19; 25,26; 25,92; 26,06; 29,16, 29,78; 30,23; 38,16; 40,56; 40,79;.40,91; 41,21 ($N(CH_3)_2$); 68,41; 70,07; 123,67; 123,71; 125,95; 126,00; 126,35; 140,03.

**[4-(1-Dimethylamino-3-phenyl-propyl)-cyclohexyl]-methanol 122 ($R^3$ = Phenethyl)**

Der Aldehyd **43** (7,20 g, 26 mmol) wurde unter Argon in Ethanol (121 ml), Wasser (61 ml) und 1 N NaOH (26 ml, 26 mmol) gelöst und 30 min bei RT gerührt. Dann wurde eine Lösung aus $NaBH_4$ (1,97 g, 52 mmol) in Wasser (209 ml) langsam zugetropft und der Ansatz über Nacht gerührt. Das Ethanol wurde i. Vak. entfernt, der wässrige Rückstand dreimal mit Essigester (je 100 ml) extrahiert, die organische Phase mit Wasser (100 ml) und gesättigter NaCl-Lösung (100 ml) gewaschen, getrocknet ($Na_2SO_4$) und i. Vak. eingeengt.
Ausbeute: 6,99 g (98 %)
$^{13}$C-NMR ($CDCl_3$): 21,03; 25,92; 26,12; 26,63; 29,00; 29,32; 29,60; 29,67; 30,93; 35,45; 38,77; 40,02; 40,56; 41,25 ($N(CH_3)_2$); 60,32; 68,30; 68,43 (CH); 125,44; 128,05; 128,09; 142,67.

**Synthese der Hydroxymethylcyclohexane ($R^1$ = $(CH_2)_nOH$, n = 2)**

Hydroxyethylcyclohexane wurden aus den entsprechenden Cyclohexylessigestem durch Reduktion mit Lithiumaluminiumhydrid dargestellt. Die Cyclohexylessigester erhält man durch Hydrierung aus den entsprechenden Cyclohexylidenessigestern, die aus den Cyclohexanonen gewonnen werden, in Anwesenheit von Pd/C.

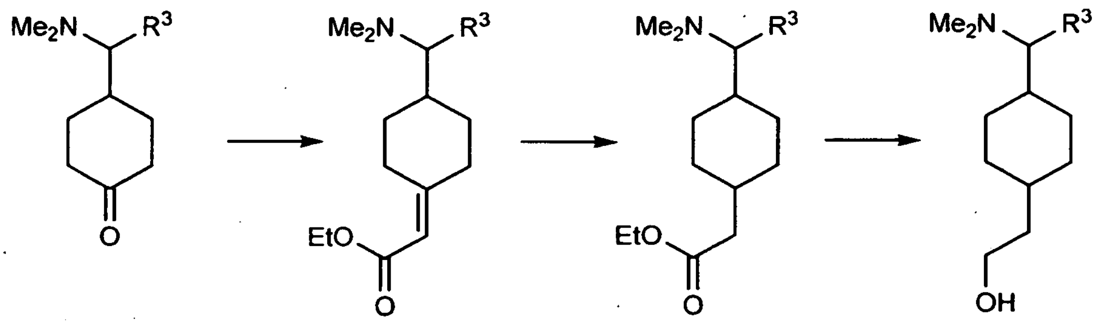

**[4-(Dimethylamino-phenyl-methyl)-cyclohexyliden]-essigsäureethylester 123 ($R^3$ = Phenyl)**

Zu einer Lösung von Phosphonoessigsäure-triethylester (30,26 g, 0,135 mol) in absol. DMF (200 ml) wurde unter Argon Kalium-tert-butylat (15,15 g, 0,135 mol) gegeben und 10 min gerührt. Anschließend wurde das Keton **10** (20,82 g, 0,09 mol), gelöst in DMF (200 ml), zugetropft. Nach ca. 20 min fällt ein Feststoff aus. Zur besseren Durchmischung wurde der Ansatz durch Zugabe von DMF (200 ml) verdünnt, 3 h bei RT gerührt und danach auf Eis gegossen.

Die Reaktionsmischung wurde mit Diethylether (3 x 100 ml) extrahiert, die organische Phase mit Wasser und gesättigter Natriumchlorid-Lösung gewaschen, getrocknet und im Vakuum eingeengt. Das Rohprodukt wurde durch Flashchromatographie mit Essigester/Cyclohexan (1:2) gereinigt.
Ausbeute: 21,83 g (80 %), Öl
$^{13}$C-NMR ($CDCl_3$): 25,93; 26,58; 27,09; 29,21; 29,90; 30,32; 30,73; 30,77; 35,38; 35,66; 38,73; ($C_4$); 40,06; 40,90; 41,19 ($N(CH_3)_2$); 48,78; 65,15; 68,22 (CH); 125,36; 127,99; 128,05; 142,69.

**[4-(Dimethylamino-phenyl-methyl)-cyclohexyl]-essigsäure-ethylester 124 ($R^3$ = Phenyl)**

Der Cyclohexylidenessigester **123** (16,4g, 0,0544 mol) wurde in Methanol (200 ml) gelöst, mit 10%-iger Palladium/Kohle (1,64 g) versetzt und bei 3 bar (RT) 24 h hydriert. Die Pd/C wurde über Kieselgur abgesaugt und das Lösungsmittel i. Vak. entfernt. Der Rückstand wurde in 1 N NaOH (100 ml) und Essigester (100 ml) gelöst, die organische Phase abgetrennt, mit Wasser gewaschen, getrocknet und eingeengt. Ausbeute: 15,73 g (95 %), farbloses Öl
$^{13}$C-NMR ($CDCl_3$): 14,22; 25;41; 25,77; 28,71; 28,88; 30,69; 32,17; 32,84; 35,08; 35,75; 38,26; 38,94; 41,20; 41,98; 42,04 ($N(CH_3)_2$); 60,01; 71,53; 75,48; 126,73; 126,78; 127,49; 127,57; 129,08; 129,31; 136,23; 137,31; 172,79; 173,30.

**2-[4-(Dimethylamino-phenyl-methyl)-cyclohexyl]-ethanol 125 ($R^3$ = Phenyl)**

Der Cyclohexylessigester **124** (9,86 g, 32,4 mmol) und $LiAlH_4$ (1,25 g, 33 mmol) wurden in absol. THF (200 ml) 7 h unter Rückfluss gekocht. Unter Eisbadkühlung (10 °C) wurde Wasser (50 ml) und 5N NaOH (40 ml) vorsichtig zugetropft, 1 h bei RT gerührt und anschließend über Kieselgur abgesaugt. Der Filterrückstand wurde mit Ether gewaschen, die wässrige Phase mit Ether extrahiert (2 x 50 ml) und die vereinigten organischen Lösungen getrocknet und i. Vak. eingeengt.
Ausbeute: 8,33 g (98 %)
$^{13}$C-NMR ($CDCl_3$): 25,33; 25,89; 29,00; 29,06; 30,89; 31,19; 33,00; 33,16; 34,37; 36,14; 36,57; 38,60; 40,18; 41,32; 41,99 ($N(CH_3)_2$); 60,66; 61,12; 75,60 (CH); 126,69; 126,73; 127,46; 127,53; 127,81; 136,49; 137,41.

**{4-[Dimethylamino-(4-fluorphenyl)-methyl]-cyclohexyliden)-essigsäure-ethylester 126 ($R^3$ = 4-Fluorphenyl)**

Zu einer Lösung von Phosphonoessigsäure-triethylester (26,9 g, 0,12 mol) in absol. DMF (250 ml) wurde unter Argon Kalium-tert-butylat (13,46 g, 0,12 mol) gegeben und 10 min gerührt. Anschließend wurde das Keton **11** (19,95 g, 0,08 mol), gelöst in DMF (200 ml), zugetropft. Nach ca. 20 min fällt ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethylether (3 x 100 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 19,7 g (77 %), Öl
$^{13}$C-NMR ($CDCl_3$): 14,18; 28,56; 28,76; 29,69; 30,17; 31,51; 32,24; 37,03; 38,07; 38,11; 41,80; 41,93; 59,34; 73,80; 73,84; 113,12; 114,24; 114,53; 130,35; 130,45; 132,48; 132,65; 160,11; 162,53; 162,59; 163,35; 166,54.

**{4-[Dimethylamino-(4-fluorphenyl)-methyl]-cyclohexyl}-essigsäure-ethylester 127 ($R^3$ = 4-Fluorphenyl)**

Der Cyclohexylidenessigester **126** (14,0 g, 0,044 mol) wurde in Methanol (200 ml) gelöst, mit 10%-iger Palladium/Kohle (1,4 g) versetzt und bei 3 bar (RT) 24 h hydriert. Die Pd/C wurde über Kieselgur abgesaugt und das Lösungsmittel i. Vak. entfernt. Der Rückstand wurde in 1N NaOH (100 ml) und EE (100 ml) gelöst, die organische Phase abgetrennt, mit Wasser gewaschen, getrocknet und eingeengt. Ausbeute: 136 g (96 %), farbloses Öl
$^{13}$C-NMR ($CDCl_3$): 14,19; 25,17; 25,72; 28,64; 28,76; 30,65; 32,06; 32,58; 32,77; 35,02; 35,99; 38,39; 38,83; 41,14; 41,93; 59,98; 70,82; 74,70; 114,15; 114,24; 114,43; 130,44; 130,54; 132,00; 133,05; 133,09; 160,10; 163,64; 172,90; 173,19.

**2-{4-[Dimethylamino-(4-fluorphenyl)-methyl]-cyclohexyl}-ethanol 128 ($R^3$ = 4-Fluorphenyl)**

Der Cyclohexylessigester **127** (8,26 g, 25,7 mmol) und $LiAlH_4$ (0,986 g, 26 mmol) wurden in absol. THF (150 ml) 7 h unter Rückfluss gekocht. Unter Eisbadkühlung (10 °C) wurde Wasser (50 ml) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (3 x 50 ml) und die vereinigten organischen Lösungen getrocknet und i.

Vak. eingeengt.
Ausbeute: 87,2 g (100 %)
$^{13}C$-NMR ($CDCl_3$): 25,11; 25,87; 28,83; 28,97; 30,89; 31,12; 32,94; 33,12; 34,38; 36,43; 36,48; 38,76; 40,15; 41,31; 42,00; 60,63; 61,09; 71,22; 74,87; 114,15; 114,23; 114,42; 114,50; 130,48; 130,58; 132,28; 133,20; 133,24; 160,10; 163,34.

**{4-[Dimethylamino-(3-fluorphenyl)-methyl]-cyclohexyliden}-essigsäure-ethylester 129 ($R^3$ = 3-Fluorphenyl)**

Zu einer Lösung von Phosphonoessigsäure-triethylester (30,26 g, 0,135 mol) in absol. DMF (200 ml) wurde unter Argon Kalium-*tert*-butylat (15,15 g, 0,135 mol) gegeben und 10 min gerührt. Anschließend wurde das Keton **12** (22,43 g, 0,09 mol), gelöst in DMF (200 ml), zugetropft. Nach ca. 20 min fällt ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethyl-ether (3 x 100 ml) extrahiert, die organische Phase mit Wasser und gesättigter NaCl-LÖsung gewaschen, getrocknet und i. Vak. eingeengt. Das Rohprodukt wurde durch Flashchromatographie mit Essigester/Cyclohexan (1:2) gereinigt.
Ausbeute: 24,78 g (86 %), Öl
$^{13}C$-NMR ($CDCl_3$): 14,36; 28,72; 28,92; 29,90; 30,39; 31,76; 30,06; 32,31; 36,96; 37,13; 38,12; 38,17; 41,91; 42,04 (N$(CH_3)_2$); 59,40; 74,21; 74,25; 113,15; 113,17; 113,53; 113,56; 113,74; 113,77; 115,47; 115,68; 124,78; 128,79; 128,86; 139,59; 139,66; 139,78; 139,83; 161,09; 162,18; 162,22; 163,52; 166,34; 171,55.

**{4-[Dimethylamino-(3-fluorphenyl)-methyl]-cyclohexyl}-essigsäure-ethylester. 130 ($R^3$ = 3-Fluorphenyl)**

Der Cyclohexylidenessigester **129** (17,5 g, 0,054 mol) wurde in Methanol (200 ml) gelöst, mit 10%-iger Palladium/Kohle (1,75 g) versetzt und bei 3 bar (RT) 24 h hydriert. Die Pd/C wurde über Kieselgur abgesaugt und das Lösungsmittel i. Vak. entfernt. Der Rückstand wurde in 1 N NaOH (100 ml) und EE (100 ml) gelöst, die organische Phase abgetrennt, mit Wasser gewaschen, getrocknet und eingeengt. Ausbeute: 15,5 g (90 %), farbloses Öl
$^{13}C$-NMR ($CDCl_3$): 14,37; 25,39; 25,82; 28,85; 30,74; 32,23; 32,721; 32,91; 35,17; 38,45; 39,00; 41,27; 41,68; 42,04 (N$(CH_3)_2$); 60,04; 71,24; 75,11; 113,37; 113,42; 113,58; 113,63; 115,55; 115,76; 124,89; 128,65; 128,74; 128,82; 139,12; 139,18; 140,18; 140,24; 161,09; 163,51; 172,64; 172,93.

**2-{4-[Dimethylamino-(3-fluorphenyl)-methyl]-cyclohexyl}-ethanol 131 ($R^3$ = 3-Fluorphenyl)**

Der Cyclohexylessigester **130** (9,46 g, 29 mmol) und $LiAlH_4$ (1,13 g, 30 mmol) wurden in absol. THF (150 ml) 7 h unter Rückfluss gekocht. Unter Eisbadkühlung (10 °C) wurde Wasser (50 ml) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (3 x 50 ml) und die vereinigten organischen Lösungen getrocknet und i. Vak. eingeengt.
Ausbeute: 8,0 g (99 %)
$^{13}C$-NMR ($CDCl_3$): 25,37; 25,98; 29,06; 29,14; 30,98; 31,35; 33,08; 33,26; 34,40; 34,55; 36,37; 38,81; 40,28; 40,69; 41,72 (N$(CH_3)_2$); 60,29; 60,76; 61,21; 71,57; 75,27 (CH); 113,36; 113,40; 113,57; 115,60; 115,81; 124,93; 128,65; 128,81; 139,41; 139,47; 140,33; 140,39; 161,09; 163,52.

**Synthese der Hydroxypropylcyclohexane ($R^1$= $(CH_2)_nOH$, n = 3)**

Die Hydroxypropylcyclohexane wurden aus den entsprechenden Cyclohexylpropionsäureestern durch Reduktion mit Lithiumaluminiumhydrid dargestellt. Die beschriebenen Cyclohexylpropionsäureester wurden durch Hydrierung aus den entsprechenden Cyclohexylacrylsäureester in Anwesenheit von Pd/C synthetisiert.

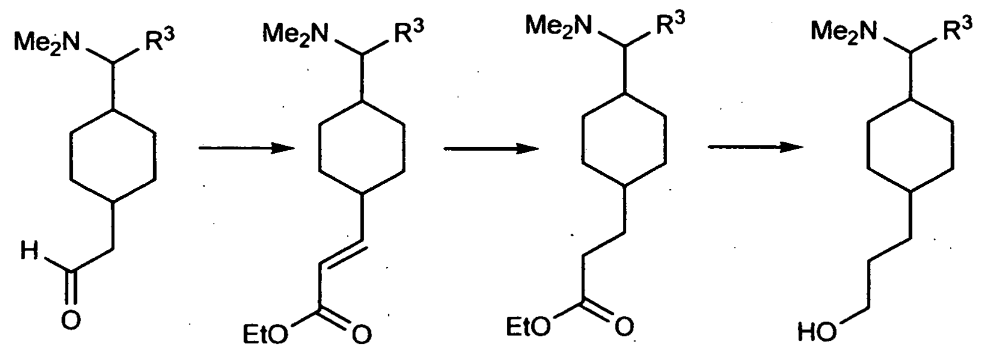

**3-[4-(Dimethylamino-phenyl-methyl)-cyclohexyl]-acrylsäure-ethylester 132 ($R^3$ = Phenyl)**

Zu einer Lösung von Phosphonoessigsäure-triethylester (33,62 g, 0,15 mol) in absol. DMF (250 ml) wurde unter Argon Kalium-*tert*-butylat (16,83 g, 0,15 mol) gegeben und 10 min gerührt. Anschließend wurde der Aldehyd **28** (24,27 g, 0,099 mol), gelöst in DMF (250 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 27,2 g (87 %), Öl
$^{13}$C-NMR ($CDCl_3$): 14,22; 25,94; 27,92; 28,23; 28,33; 28,65; 30,18; 30,45; 30,60; 31,45; 31,63; 32,15; 33,03; 37,74; 38,10; 38,55; 40,71; 41,04; 41,30;41,97; 59,67; 60,05; 71,34; 74,89; 75,61; 117,96; 118,97; 120,02; 126,81; 127,55; 137,20; 153,31; 153,90; 155,25; 166,28; 166,99.

**3-[4-(Dimethylamino-phenyl-methyl)-cyclohexyl]-propionsäure-ethylester 133 ($R^3$ = Phenyl)**

Der Cyclohexylacrylsäureester **132** (20,9 g, 0,066 mol) wurde in Methanol (150 ml) gelöst, mit 10%-iger Palladium/Kohle (2,0 g) versetzt und bei 3 bar (RT) 24 h hydriert. Die Pd/C wurde über Kieselgur abgesaugt und das Lösungsmittel im Vakuum entfernt. Der Rückstand wurde in 1 N NaOH (100 ml) und Essigester (100 ml) gelöst, die organische Phase abgetrennt, mit Wasser gewaschen, getrocknet und eingeengt.
Ausbeute:18,6 g (89 %), farbloses Öl
$^{13}$C-NMR ($CDCl_3$): 14,15; 25,49; 25,79; 28,54; 29,00; 30,84; 31,62; 31,91; 32,15; 32,35; 32,59; 32,76; 34,62; 35,80; 37,18; 37,37; 38,57; 41,14; 41,96; 60,05; 71,33; 75,55; 126,65; 127,43; 127,50; 127,95; 129,06; 129,27; 136,25; 137,40; 173,97.

**3-[4-(Dimethylamino-phenyl-methyl)-cyclohexyl]-propan-1-ol 134 ($R^3$ = Phenyl)**

Der Cyclohexylpropionsäureester **133** (9,7 g, 30,5 mmol) und $LiAlH_4$ (1,18 g, 31 mmol) wurden in absol. THF (150 ml) 7 h unter Rückfluss gekocht. Unter Eisbadkühlung (10 °C) wurde Wasser (50 mal) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (2 x 50 ml) und die vereinigten organischen Lösungen getrocknet und i. Vak. eingeengt.
Ausbeute: 8,4 g (100 %)
$^{13}$C-NMR ($CDCl_3$): 25,53; 28,91; 29,14; 29,78; 30,14; 31,00; 32,99; 33,16; 33,27; 34,80; 36,02; 37,63; 38,75; 41,20; 42,01; 63,16; 71,55; 75,69; 126,67; 127,46; 129,32; 137,53.

**3-{4-[Dimethylamino-(4-fluorphenyl)-methyl]-cyclohexyl}-acrylsäure-ethylester 135 ($R^3$ = 4-Fluorphenyl)**

Zu einer Lösung von Phosphonoessigsäure-triethylester (25,1 g, 0,112 mol) in absol. DMF (150 ml) wurde unter Argon Kalium-*tert*-butylat (12,56 g, 0,112 mol) gegeben und 10 min gerührt. Anschließend wurde der Aldehyd **31** (19,9 g, 0,075 mol), gelöst in DMF (225 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 23,7 g (95 %), Öl

$^{13}C$-NMR ($CDCl_3$): 14,30;.25,78; 26,06; 28,15; 28,32; 28,48; 30,23; 30,48; 31,45; 31,64; 32,32; 37,57; 37,63; 38,28 ($C_4$); 41,03; 41,80; 41,96 *($N(CH_3)_2$); 59,62; 60,01; 74,64; 74,80; 114,12; 114,29; 117,86; 118,87; 119,94; 130,28; 132,78; 152,84; 153,46; 154,85; 160,31; 162,73; 165,91; 166,61.

**3-{4-[Dimethylamino-(4-fluorphenyl)-methyl]-cyclohexyl}-propionsäure-ethylester 136 ($R^3$ = 4-Fluorphenyl)**

Der Cyclohexylacrylsäureester **135** (12,3g, 0,050 mol) wurde in Methanol (100 ml) gelöst, mit 10%-iger Palladium/Kohle (1,63 g) versetzt und bei 3 bar (RT) 24 h hydriert. Die Pd/C wurde über Kieselgur abgesaugt und das Lösungsmittel im Vakuum entfernt. Der Rückstand wurde in 1 N NaOH (100 ml) und EE (100 ml) gelöst, die organische Phase abgetrennt, mit Wasser gewaschen, getrocknet und eingeengt.
Ausbeute:16,7 g (100 %), farbloses Öl
$^{13}C$-NMR ($CDCl_3$): 14,32; 25,43; 25,93; 28,67; 28,72; 28,93; 31,00; 32,05; 32,50; 32,70; 32,88; 34,69; 36,26; 38,90 ($C_4$); 41,24; 42,08 ($N(CH_3)_2$); 60,11; 70,79 74,87; 114,08; 114,16; 114,27; 130,35; 130,43; 132,03; 133,17; 160,32; 162,74; 173,71.

**3-{4-[Dimethylamino-(4-fluorphenyl)-methyl]-cyclohexyl}-propan-1-ol 137 ($R^3$ = 4-Fluorphenyl)**

Der Cyclohexylpropionsäureester **136** (8,08 g, 24,1 mol) und $LiAlH_4$ (0,952 g, 25 mmol) wurden in absol. THF (150 ml) 8 h unter Rückfluss gekocht. Unter

Eisbadkühlung (10 °C) wurde Wasser (50 ml) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (2 x 50 ml) und die vereinigten organischen Lösungen getrocknet und i. Vak. eingeengt.
Ausbeute: 5,62 g (79 %)
$^{13}C$-NMR ($CDCl_3$): 25,26; 25,80; 28,83; 30,06; 30,56; 30,92; 32,87; 33,06; 33,21; 34,65; 36,23; 37,56; 38,82 ($C_4$); 41,20; 41,97 ($N(CH_3)_2$); 63,00; 70,84; 114,13; 114,20; 130,45; 130,52; 132,11; 133,22; 133,24; 160,45; 162,88.

**3-{4-[Dimethylamino-(3-fluorphenyl)-methyl]-cyclohexyl}-acrylsäure-ethylester 138 ($R^3$ = 3-Fluorphenyl)**

Zu einer Lösung von Phosphonoessigsäure-triethylester (24,66 g, 0,11 mol) in absol. DMF (200 ml) wurde unter Argon Kalium-*tert*-butylat (12,34 g, 0,11 mol) gegeben und 10 min gerührt. Anschließend wurde der Aldehyd **34** (19,3 g, 0,073 mol), gelöst in DMF (200 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 21,9 g (90 %), Öl
$^{13}C$-NMR ($CDCl_3$): 14,26; 25,82; 28,28; 28,49; 30,09; 30,35; 31,38; 31,56; 32,07; 35,80; 37,54; 38,12; 40,68; 41,05; 41, 31; 41, 98; 59, 75; 60,14; 75,07; 113, 57; 113, 84; 115,67; 115,94; 118,02; 119,04; 120,12; 125,03; 128,88; 140,13; 153,18; 153,80; 155,20; 160,92; 164,17; 167,03.

**3-{4-[Dimethylamino-(3-fluorphenyl)-methyl]-cyclohexyl}_propionsäure-ethylester 139 ($R^3$ = 3-Fluorphenyl)**

Der Cyclohexylacrylsäureester **138** (14,98 g, 0,045 mol) wurde in Methanol (100 ml) gelöst, mit 10%-iger Palladium/Kohle (1,5 g) versetzt und bei 3 bar (RT) 24 h hydriert. Die Pd/C wurde über Kieselgur abgesaugt und das Lösungsmittel im Vakuum entfernt. Der Rückstand wurde in 1N NaOH (100 ml) und EE (100 ml) gelöst, die organische Phase abgetrennt, mit Wasser gewaschen, getrocknet und eingeengt.
Ausbeute:14,3 g (95 %), farbloses Öl
$^{13}C$-NMR ($CDCl_3$): 14,18; 25,36; 25,72; 28,55; 28,86; 30,77; 31,94; 32,14; 32,38: 32,54; 32,73; 34,58; 35,94; 37,38; 38,64; 41,16; 41,98; 60,12; 71,08; 75,19; 113,41; 113,68; 115,64; 115,91; 125,03; 128,75; 128,86; 140,40; 160,86; 164,11; 174,02.

**3-{4-[Dimethylamino-(3-fluorphenyl)-methyl]-cyclohexyl}-propan-1-ol 140 ($R^3$ = 3-Fluorphenyl)**

Der Cyclohexylpropionsäureester **139** (8,51 g, 25 mmol) und $LiAlH_4$ (0,986 g, 26 mmol) wurden in absol. THF (150 ml) 7 h unter Rückfluss gekocht. Unter Eisbadkühlung (10 °C) wurde Wasser (50 ml) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (2 x 50 ml) und die vereinigten organischen Lösungen getrocknet und i. Vak. eingeengt.
Ausbeute: 7,33 g (100 %)

$^{13}C$-NMR ($CDCl_3$): 25,43; 25,57; 28,85; 29,00; 29,78; 30,15; 30,92; 32,93; 33,11; 33,24; 34,77; 36,05; 37,63; 38,76; 41,20; 42,01; 63,27; 67,93; 71,18; 75,27; 113,41; 113,68; 115,66; 115,94; 125,07; 128,76; 128,86; 140,48; 140,56; 160,87; 164,12.

**3-[4-(1-Dimethylamino-3-phenyl-propyl)-cyclohexyl]-acrylsäure-ethylester 141 ($R^3$ = Phenethyl)**

Zu einer Lösung von Phosphonoessigsäure-triethylester (26,9 g, 0,120 mol) in absol DMF (150 ml) wurde unter Argon Kalium-*tert*-butylat (13,46 g, 0,120 mol) gegeben und 10 min gerührt. Anschließend wurde der Aldehyd **43** (21,34 g, 0,080 mol), gelöst in DMF (225 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 19,1 g (71 %), Öl
$^{13}C$-NMR ($CDCl_3$): 14,14; 28,99; 29,53; 30,56; 31,53; 31,59; 35,26; 39,26; 40,46; 40,72; 41,09; 41,03 ($N(CH_3)_2$); 59,95; 68,01; 118,84; 125,54; 128,14; 128,17;.142,70; 153,83; 166,86.

**3-[4-(1-Dimethylamino-3-phenyl-propyl)-cyclohexyl]-propionsäure-ethylester 142 ($R^3$ = Phenethyl)**

Der Cyclohexylacrylsäureester **141** (14,04 g, 0,041 mol) wurde in Methanol (100 ml) gelöst, mit 10%-iger Palladium/Kohle (1,4 g) versetzt und bei 3 bar (RT) 48 h hydriert. Die Pd/C wurde über Kieselgur abgesaugt und das Lösungsmittel im Vakuum entfernt. Der Rückstand wurde in 1N NaOH (100 ml) und EE (100 ml) gelöst, die organische Phase abgetrennt, mit Wasser gewaschen, getrocknet und eingeengt.
Ausbeute: 11.7 g (82 %), farbloses Öl
$^{13}C$-NMR ($CDCl_3$): 14,18; 25,68; 26,37; 28,36; 29,11; 30,01; 31,23; 31,65; 32,18; 32,50; 32,85; 32,90; 34,12; 35,37; 37,25; 38,73; 39,78; 40,84; 41,17; 60,07; 65,41; 68,25; 125,56; 128,24; 142,93; 174,01.

**3-[4-(1-Dimethylamino-3-phenyl-propyl)-cyclohexyl]-propan-1-ol 143 ($R^3$ = Phenethyl)**

Der Cyclohexylpropionsäureester **142** (6,58 g, 19 mmol) und $LiAlH_4$ (0,76 g, 20 mmol) wurden in absol. THF (100 ml) 7 h unter Rückfluss gekocht. Unter Eisbadkühlung (10°C) wurde Wasser (50 ml) und 5N NaOH (25 ml) vorsichtig zugetropft, 1 h bei RT gerührt und anschließend über Kieselgur abgesaugt. Der Filterrückstand wurde mit Ether gewaschen, die wässrige Phase mit Ether extrahiert (2 x 50 ml) und die vereinigten organischen Lösungen getrocknet und i. Vak. eingeengt.
Ausbeute: 5,56 g (96 %)
$^{13}C$-NMR ($CDCl_3$): 29,15; 29,25; 30,09; 30,16; 31,42; 33,23; 33,32; 33,24; 35,40; 37,54; 39,92; 40,88; 41,20; 63,13; 68,39; 125,55; 128,19; 128,24; 142,93.

**Synthese der Ether ($R^1$= $(CH_2)_nOR^8$)**

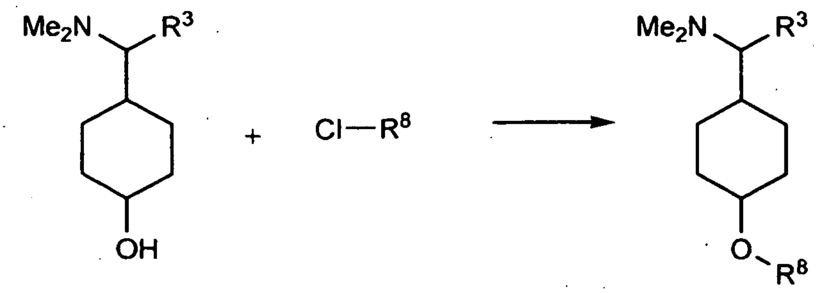

**4-(Benzyloxy)cyclohexyl)-N,N-dimethyl(phenyl)methanamin Hydrochlorid 144 ($R^3$ = Phenyl)**

Zu einer Suspension aus NaH (0,15g, 6,4 mmol) in THF (10 ml) wurde der Alkohol **111** (1,5g; 6,4 mmol), gelöst in THF (10 ml) und danach Benzylchlorid (0,9 g, 7,1 mmol) bei RT langsam zugegeben und anschließend 21 h unter Rückfluss erhitzt. Unter Eisbadkühlung wurde der Ansatz mit Wasser (10ml) vorsichtig gequencht und mit mit NaOH-Lsg. (10ml, 5N) versetzt. Nach 1 h Rühren wurde über Filtererde abfiltriert und mit Diethylether nachgewaschen. Es wurde mit Diethylether (3 x 40ml) extrahiert, über $Na_2SO_4$ getrocknet und eingeengt.
Das Rohprodukt wurde säulenchromatographisch mit Diethylether aufgereinigt.
Ausbeute: 451 mg (27,6%) gelber Feststoff
Das Produkt wurde in Methylethylketon (4 ml) gelöst und dann mit Wasser (0,014 ml) und Trimethylchlorsilan (0,197 ml) versetzt.
Nach einiger Zeit fiel ein Feststoff aus. Nach Absaugen und waschen mit Ether erhielt man weiße Kristalle, die im Vakuum getrocknet wurden.
$^1$H NMR (600 MHz, DMSO) 0,83 - 0,88 (m, 2 H); 1,17 - 1,24 (m, 1 H); 1,25 - 1,32 (m, 1 H); 1,56 - 1,63 (m, 1 H); 1,90 - 1,98 (m, 2 H); 2,03 - 2,10 (m, 1 H); 2,21 - 2,28 (m, 1 H); 2,52 - 2,59 (m, 3 H); 2,64 - 2,70 (m, 3 H); 3,10 - 3,17 (m, 1 H); 4,20 - 4,25 (m, 1 H); 4,46 (s, 2 H); 7,24 - 7,29 (m, 2 H); 7,30 - 7,33 (m, 2 H); 7,45 - 7,52 (m, 5 H); 10,35 (s, 1 H).

**4-(4-Fluorbenzyloxy)cyclohexyl)-N,N-dimethyl(phenyl)methanamin Hydrochlorid 145 ($R^3$ = Phenyl)**

Zu einer Suspension aus dem NaH (0,15g, 6,4 mmol) in THF (10 ml) wurde der Alkohol **111** (1,5g; 6,4 mmol), gelöst in THF (10 ml) und danach p-Fluorbenzylchlorid (1,02g, 7,1 mmol) bei RT langsam zugegeben und anschließend 21h unter Rückfluss erhitzt.

Unter Eisbadkühlung wurde der Ansatz mit Wasser (10ml) vorsichtig gequencht und mit mit NaOH-Lsg. (10ml, 5N) versetzt. Nach 1h Rühren wurd über Filtererde abfiltriert und mit Diethylether nachgewaschen. Es wurde mit Diethylether (3 x 40ml) extrahiert, über $Na_2SO_4$ getrocknet und eingeengt.

Das Rohprodukt wurde säulenchromatographisch mit Diethylether/Hexan (1 : 1) aufgereinigt. Das cis-Diastereomer konnte einheitlich soliert werden.

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden. 1 H NMR (600 MHz, DMSO) 0,80 - 0,88 (m, 2 H); 1,16 - 1,23 (m, 1 H); 1,25 - 1,32 (m, 1 H); 1,53 - 1,61 (m, 1 H); 1,89 - 1,97 (m, 2 H); 2,03 - 2,09 (m, 1 H); 2,21 - 2,28 (m, 1 H); 2,53- 2,59 (m, 3 H); 2,64 - 2,70 (m, 3 H); 3,10 - 3,18 (m, 1 H); 4,20 - 4,26 (m, 1 H); 4,44 (s, 2 H); 7,11 - 7,17 (m, 2 H); 7,30 - 7,36 (m, 2 H); 7,45 - 7,52 (m, 5 H); 10,25 (s, 1 H).

**Synthese der Grignard-Verbindungen ($R^2$ = OH)**

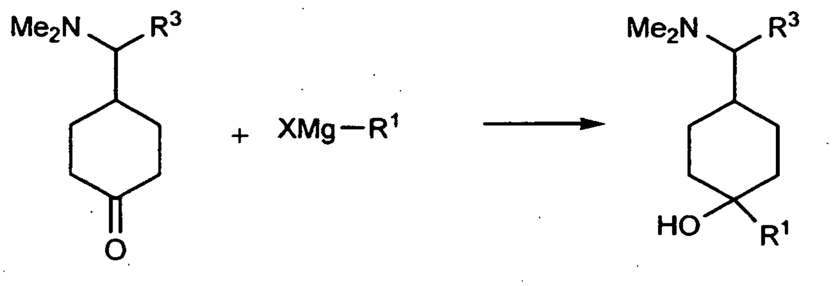

**N,N-Dimethyl(4-phenethylcyclohexyl)(phenyl)methanamin Hydrochlorid 146 ($R^3$ = Phenyl)**

Unter Stickstoffatmosphäre wurde die Phenethylmagnesiumchlorid-Lösung (9,1 ml, 9,1 mmol, 1,0 M in THF) vorgelegt und mit einem Eisbad auf ca. 10°C gekühlt. Das Keton **10** wurde in THF (9 ml) gelöst und zugetropft. Der Reaktionsansatz wurde über Nacht bei RT gerührt. Zur Aufarbeitung wurde unter Eiskühlung mit $NH_4Cl$-Lsg. (20 %, 9 ml) hydrolysiert und mit 3 x 40 ml (3 x 40 ml) extrahiert. Die vereinigten organischen Phasen wurden getrocknet ($Na_2SO_4$) und eingeengt.

Die Reinigung erfolgte säulenchromatographisch (Ether).

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden. $^{13}$C NMR (75 MHz, DMSO) 22,58 ; 24,58; 25,28; 26,00; 28,66; 29,08; 35,38; 35,51; 35,62; 35,70 ; 36,16; 36,70; 38,19; 38,50; 39,12; 39,40; 39,4.5; 42,20; 42,58; 46,04; 72,26; 74,00; 125,29; 125,30; 128,02; 128,05;

128,08; 128,12; 128,60; 128,56; 129,06; 129,24; 129,95;142,93.

**1-Benzyl-4-((dimethylamino)(phenyl)methyl)cyclohexanol Hydrochlorid 147 ($R^3$ = Phenyl)**

Unter Stickstoffatmosphäre wurde die Benzylmagnesiumchlorid-Lösung (4,5 ml, 9,1 mmol, 2,0 M in THF) vorgelegt und mit einem Eisbad auf ca. 10°C gekühlt. Das Keton **10** wurde in THF (9 ml) gelöst und zugetropft. Der Reaktionsansatz wurde über Nacht bei RT gerührt. Zur Aufarbeitung wurde unter Eiskühlung mit $NH_4Cl$-Lsg. (20 %, 9 ml) hydrolysiert und mit 3 x 40 ml (3 x 40 ml) extrahiert. Die vereinigten organischen Phasen wurden getrocknet ($Na_2SO_4$) und eingeengt.

Die Reinigung erfolgte durch Flashchromatographie (Ether). Das Produkt wurde in Methylethylketon (2 ml) gelöst und dann mit Wasser (0,01 ml/ 1mmol) und Trimethylchlorsilan (1,3 ml/ 1mmol) versetzt. Nach Absaugen und waschen mit Ether erhielt man weiße Kristalle, die im Vakuum getrocknet wurden.

**4-((Dimethylamino)(phenyl)methyl)-1-(4-fluorbenzyl)cyclohexanol Hydrochlorid 148 ($R^3$ = Phenyl)**

Magnesiumspäne (0, 1 9g, 7,8 mmol) wurden im Kolben vorgelegt und mit wenig THF (3 ml) versetzt. 1/20 des 4-Fluorbenzylchlorids (1,12g, 7,8 mmol) wurde zunächst pur zum Magnesium getropft, so daß die Reaktion einsetzte. Nach Reaktionsbeginn wurde das Halogenid mit THF (14 ml) verdünnt und so zugetropft, daß das Lösungsmittel gelinde siedet. Nach Beendigung des Zutropfens wurde noch ca. 1h bei Siedetemperatur nachgerührt. Anschließend wurde das Keton 10 (1,5g, 6,5 mmol) bei RT zugetropft und über Nacht bei RT nachrühren gelassen.

Bei Eiskühlung wurde anschließend mit $NH_4Cl$-Lsg. (20 %, 10 ml) hydrolysiert und mit Ether (3 x 40 ml) extrahiert. Die vereinigten organischen Phasen wurden getrocknet ($Na_2SO_4$) und eingeengt.

Die Aufreinigung erfolgte durch Flash-Chromatographie (Diethylether).

Das Produkt wurde in Methylethylketon (2 ml) gelöst und dann mit Wasser (0,01 ml/ 1 mmol) und Trimethylchlorsilan (1,3 ml/ 1 mmol) versetzt. Nach Absaugen und waschen mit Ether erhielt man weiße Kristalle, die im Vakuum getrocknet wurden.

**1-(2,5-Dimethoxyphenyl)-4-((dimethylamino)(phenyl)methyl)cyclohexanol hydrochlorid 149 ($R^3$ = Phenyl)**

Magnesiumspäne (0,15g, 6,2 mmol) wurden im Kolben vorgelegt und mit wenig THF (3 ml) versetzt. 1/20 des 1-Brom-2,5-Dimethoxybenzols (1,35g, 6,2 mmol) wurde zunächst pur zum Magnesium getropft, so daß die Reaktion einsetzte. Nach Reaktionsbeginn wurde das Halogenid mit THF (10 ml) verdünnt und so zugetropft, daß das Lösungsmittel gelinde siedet. Nach Beendigung des Zutropfens wurde noch ca. 1h bei Siedetemperatur nachgerührt. Anschließend wurde das Keton **10** (1,2g 5,2 mmol) bei RT zugetropft und über Nacht bei RT nachrühren gelassen.

Bei Eiskühlung wurde anschließend mit $NH_4Cl$-Lsg. (20 %, 10 ml) hydrolysiert und mit Ether (3 x 40 ml) extrahiert. Die vereinigten organischen Phasen wurden getrocknet ($Na_2SO_4$) und eingeengt.

Die Aufreinigung erfolgte durch Flash-Chromatographie (Diethylether).

Das Produkt wurde in Methylethylketon (2 ml) gelöst und dann mit Wasser (0,01 ml/ 1 mmol) und Trimethylchlorsilan (1,3 ml/ 1mmol) versetzt. Nach Absaugen und waschen mit Ether erhielt man weiße Kristalle, die im Vakuum getrocknet wurden.

**Synthesevorschrift für die automatisierte Synthese**

a) Verwendung von Grignard-Reagenz-Lösungen

In einem ausgeheizten und mit $N_2$ gefluteten Reaktorblock [ACT Vantage] wurde bei 0°C das Cyclohexanon-Derivat (200 $\mu$mol, 400 $\mu$l, 0,5 mol/l in THF) vorgelegt und mit dem entsprechenden Grignard-Reagenz (400 $\mu$mol, 800 $\mu$l, 0,5 mol/l in THF oder Diethylether) versetzt. Das Reaktionsgemisch wurde 2,5 h bei Raumtemperatur geschüttelt und anschließend durch die Zugabe von 2 ml einer halbgesättigten $NH_4Cl$-Lösung bei 0°C gequencht. Die Lösung wurde ca. 30 min. bei Raumtemperatur nachgeschüttelt und mit 1 ml Essigester versetzt.

Zur Aufarbeitung wurde die organische Phase abgenommen [MYRIAD Allex] und in ein tariertes Gefäß überführt. Anschließend wurde die wässrige Phase noch einmal mit 2,5 ml Essigester extrahiert und die organischen Phasen gesammelt. Die vereinigten, organischen Phasen werden bis zur Trockene eingeengt und zur Ausbeutebestimmung zurückgewogen.

Die Aufreinigung erfolgte durch HPLC.

b) Verwendung von Grignard-Reagenzien aus Iodaromaten

In einem ausgeheizten und mit $N_2$ gefluteten Reaktorblock [ACT Vantage] wurden bei 0°C die Lösung des Iodaromaten (325 µmol, 650 µl, 0,5 mol/l in THF) vorgelegt und mit Isopropylmagnesiumchlorid (275 µmol, 550 µl, 0,5 mol/l in THF) versetzt. Zu dieser Reaktionslösung wurde nach ca. 30 min. schütteln bei 0°C das Cyclohexanon-Derivat (200 µmol, 400 µl, 0,5 mol/l in THF) zupipettiert. Das Reaktionsgemisch wurde 5 h bei Raumtemperatur geschüttelt und anschließend durch die Zugabe von 2 ml einer halbgesättigten $NH_4Cl$-Lösung bei 0°C gequencht. Die Lösung wurde ca. 30 min. bei Raumtemperatur nachgeschüttelt und mit 1 ml Essigester versetzt.

Zur Aufarbeitung wurde die organische Phase abgenommen [MYRIAD Allex]. Anschließend wurde die wässrige Phase noch einmal mit 3 ml Essigester extrahiert. Die vereinigten, organischen Phasen wurden bis zur Trockene eingeengt. Die Aufreinigung erfolgte durch HPLC.

Auf diese Weise wurden die folgenden Beispiele synthetisiert. Die Analytik erfolgte über HPLC-MS (ESI). In allen hier aufgeführten Fällen wurde die Masse als M +1 gefunden:

| Nr. | Name | Masse |
|---|---|---|
| 150 | 4-((Dimethylamino)(phenyl)methyl)-1-(4-fluor-3-methylphenyl)cyclohexanol | 337,24 |
| 151 | 4-(Dimethylamino-phenyl-methyl)-1-(4-fluor-3-methyl-phenyl)-cyclohexanol | 341,22 |
| 152 | 1-Benzyl-4-[dimethylamino-(3-fluor-phenyl)-methyl]-cyclohexanol | 341,22 |
| 153 | 4-[Dimethylamino-(3-fluor-phenyl)-methyl]-1-phenethyl-cyclohexanol | 355,23 |
| 154 | 4-[Dimethylamino-(3-fluor-phenyl)-methyl]-1-pentyl-cyclohexanol | 321,25 |
| 155 | 1-(3,5-Dichlor-pheny)-4-[dimethylamino-(3-fluor-phenyl)methyl]-cyclohexanol | 395,12 |
| 156 | 4-[Dimethylamino-(3-fluor-phenyl)-methyl]-1-(3-methoxy-benzyl)-cyclohexanol | 371,23 |
| 157 | 1-(4-Chlor-3-trifluormethyl-phenyl)-4-[dimethylamino-(3-fluor-phenyl)-methyl]-cyclohexanol | 429,15 |
| 158 | 4-[(4-Chlor-phenyl)-dimethylamino-methyl]-1-phenyl-cyclohexanol | 343,17 |
| 159 | 1-Benzyl-4-[(4-chlor-phenyl)-dimethylamino-methyl]-cyclohexanol | 357,19 |
| 160 | 4-[(4-Chlor-phenyl)-dimethylamino-methy-1-(4-fluor-3-methyl-phenyl)-cyclohexanol | 375,18 |
| 161 | 4-[(4-Chlor-phenyl)-dimethylamino-methyl]-1-o-tolyl-cyclohexanol | 357,19 |
| 162 | 4-[(4-Chlo-phenyl)-dimethylamino-methyl]-1-(4-fluor-phenyl)-cyclohexanol | 361,16 |
| 163 | 4-[(4-Chlor-phenyl)-dimethylamino-methyl]-1-phenethyl-cyclohexanol | 371,20 |
| 164 | 4-[(4-Chlor-phenyl)-dimethylamino-methyl]-1-(3-methoxy-phenyl)-cyclohexanol | 373,18 |
| 65 | 4-[(4-Chlor-phenyl)-dimethylamino-methyl]-1-p-tolyl-cyclohexanol | 357,19 |
| 166 | 4-[(4-Chlor-phenyl)-dimethylamino-methyl]-1-(3,5-difluor-phenyl)-cyclohexanol | 379,15 |
| 167 | 1-Butyl-4-[(4-chlor-phenyl)-dimethylamino-methyl]-cyclohexanol | 323,20 |
| 168 | 4-[(4-Chlor-phenyl)-dimethylamino-methyl]-1-hexyl-cyclohexanol | 351,23 |
| 169 | 4-[(4-Chlor-phenyl)-dimethylamino-methyl]-1-pentyl-cyclohexanol (polareres Diastereomer) | 337,22 |
| 170 | 4-[(4-Chlor-phenyl)-dimethylamino-methyl]-1-pentyl-cyclohexanol (unpolareres Diastereomer) | 337,22 |
| 171 | 4-[(4-Chlor-phenyl)-dimethylamino-methyl]-1-(3-fluor-phenyl)-cyclohexanol | 361,16 |
| 172 | 4-[(4-Chlor-phenyl)-dimethylamino-methyl]-1-(4-fluor-benzyl)-cyclohexanol | 375,18 |
| 173 | 4-[(4-Chlor-phenyl)-dimethylamino-methyl]-1-(3-methoxy-benzyl)- cyclohexanol | 387,20 |

**Synthese von Amiden aus Estern ($R^1 = (CH_2)_nCONR^{10}R^{11}$ bzw. $R^1 = XCONR^{10}R^{11}$)**

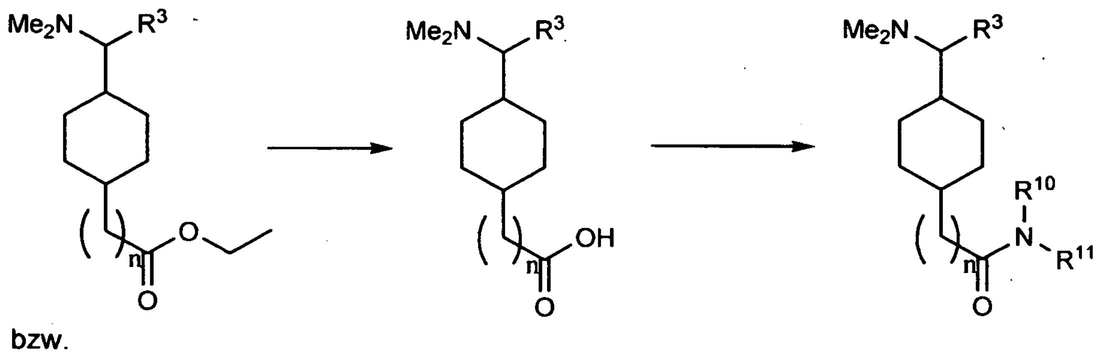

bzw.

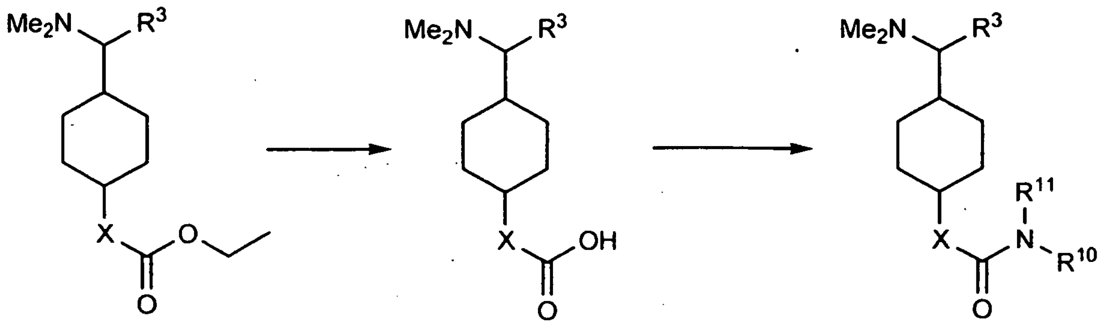

X = =CH, -CH=CH

**Allgemeine Methode zur Hydrolyse der Ester**

Zu einer Lösung des Cyclohexylessigsäure-, Cyclohexylidenessigsäure-, Cyclohexylacrylsäure- oder Cyclohexylpropionsäureesters (20 mmol) in THF (130 ml) und Wasser (80 ml) wurde Natronlauge (6 M, 40 ml) zugegeben und bei Raumtemperatur für 4 - 16 Stunden gerührt. Anschließend wurde das Lösungsmittel weitgehend abdestilliert und konz. Salzsäure solange langsam zugegeben bis ein pH-Wert 7 erreicht wurde. Das Lösungsmittel wurde vollständig abdestilliert und der Rückstand mit 2-Propanol (3 x 200 ml) gewaschen.

**Automatisierte Synthese zur Bildung der Amide**

In ein trockenes Gewindeglas wurden bei RT Cyclohexylessigsäure, Cyclohexylidenessigsäure, Cyclohexylacrylsäure oder Cyclohexylpropionsäure (100$\mu$mol, 0,05 M Lösung in $CH_2Cl_2$) vorgelegt und mit Carbonyldiimidazollösung (105$\mu$mol, 0,1 M Lösung in $CH_2Cl_2$) versetzt. Nach 1 Stunde Rührzeit bei RT wurden zu der Reaktionslösung das Amin (100$\mu$mol, 0,1 M Lösung in $CH_2Cl_2$) zugegeben und für 16h bei RT gerührt. Nach der Zugabe von Wasser (3 ml) und Extraktion wurde die organische Phase separiert und mit gesättigter NaCl-Lösung (3 ml) gewaschen. Die abgetrennte organische Phase wurde über $MgSO_4$ getrocknet und das Lösungsmittel abdestilliert.

**Alternatives Syntheseverfahren**

*Allgemeines Syntheseschema*

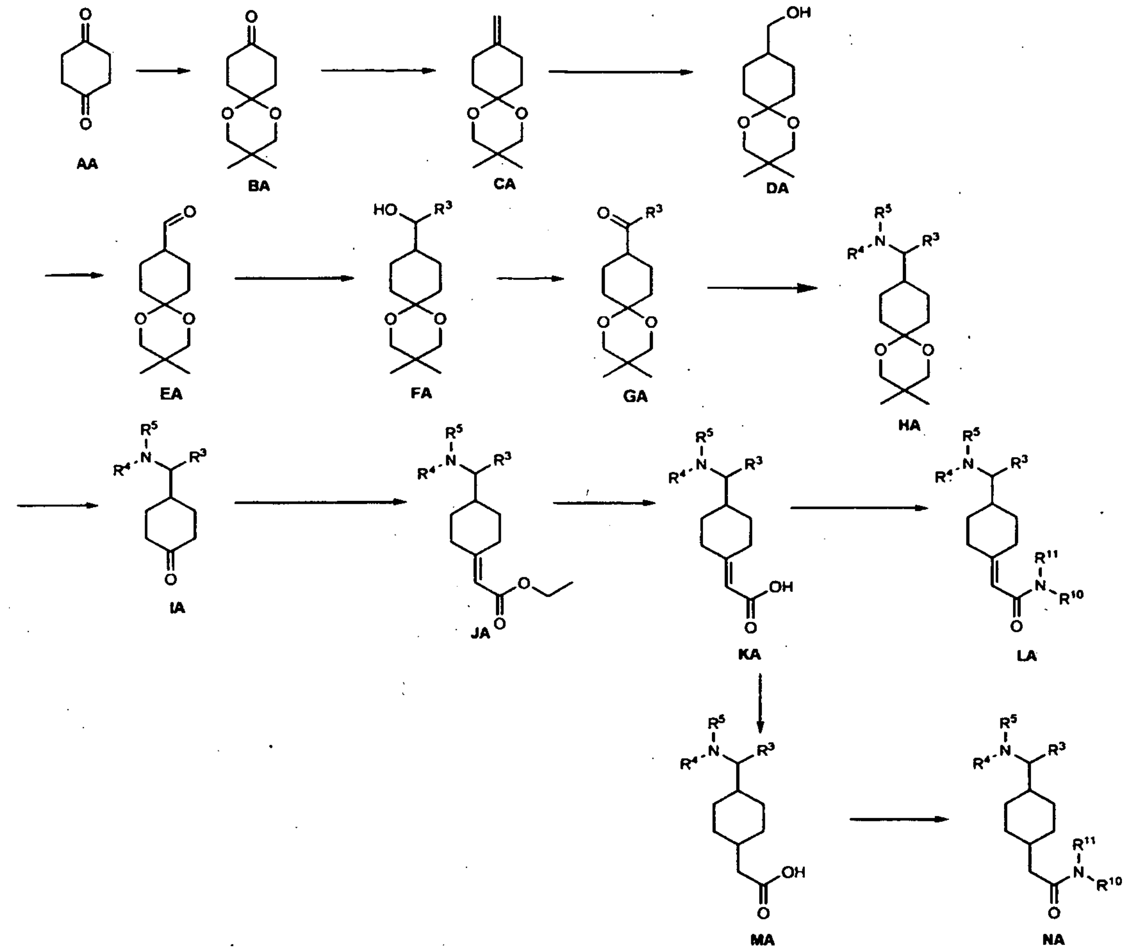

AA BA CA DA EA FA GA HA IA JA KA LA MA NA

## Allgemeines Verfahren

1,4-Cyclohexandion **AA** wird unter den Fachmann bekannten Bedingungen in einer Acetalbildungsreaktion mit einem Glykolderivat in einem organischen Lösungsmittel wie Dichlormethan, Cyclohexan, Toluol, Benzol, Ethanol, Methanol oder Xylol möglicherweise auch in Gegenwart eines wasserentziehenden Reagenzes, wie Schwefelsäure, Natrium- oder Magnesiumsulfat, Molsieb oder Phosphoroxiden, gegebenenfalls auch unter Zusatz katalytischer Mengen p-Toluolsulfonsäure, bei einer Temperatur von RT bis Rückflußtemperatur des jeweiligen organischen Lösungsmittels zu dem Acteal **BA** umgesetzt.

Acetalketone **BA** werden unter den Fachmann bekannten Methoden in einer Wittigreaktion unter Verwendung von Phösphoryliden in organischen Lösungsmitteln, wie THF, DME oder Diethylether, in Gegenwart metallorganischer Basen, wie n-BuLi, tert.-BuLi, LDA, Metallhydride wie NaH, KH, bei einer Temperatur von -10°C bis Rückflußtemperatur des jeweiligen organischen Lösungsmittels, zu den Produkten **CA** umgesetzt.

Die Verbindung **CA** wird in einer Hydroxylierungsreaktion in Gegenwart von Bortrifluoridetherat und Metallhydriden wie Natriumborhydrid oder Lithiumaluminiumhydrid in einem organischen Lösungsmittel wie THF oder Diethylether, auch unter Zusatz von Diglyme, bei einer Temperatur von -10°C bis RT zu den Alkoholen **DA** umgesetzt.

Die Alkohole **DA** lassen sich unter dem Fachmann bekannten Bedingungen durch Verwendung von Reagenzien, wie PCC, Periodinan, IBX, TPAP, NMO, $MnO_2$ oder Oxalylchlorid, gegebenenfalls auch in Gegenwart von Molekularsieb oder einer Base, wie Triethylamin, in einem organischen Lösungsmittel wie Dichlormethan, DMSO, Methanol, Ethanol Diethylether, THF, DMF, DME, bei einer Temperatur von -78°C bis zur Rückflußtemperatur des jeweiligen organischen Lösungsmittels, zum Aldehyd **EA** umsetzen.

Die Alkohole **FA** erhält man unter dem Fachmann bekannten Bedingungen durch die Addition von Metallorganylen, wie Magnesium-, Kupfer-, Zink oder Lithiumorganyle in organischen Lösungsmitteln, wie Ether, THF Methanol, Ethanol oder Dichlormethan, bei einer Temperatur von -78°C bis RT.

Die Alkohole **FA** lassen sich unter dem Fachmann bekannten Bedingungen durch Verwendung von Reagenzien, wie Chromtrioxid, PCC, Periodinan, PDC, IBX, TPAP, NMO, $MnO_2$ oder Oxalylchlorid, gegebenenfalls auch in

Gegenwart von Molekularsieb oder einer Base, wie Triethylamin, oder einer Säure, wie wässriger Schwefelsäure, in einem organischen Lösungsmittel wie Dichlormethan, DMSO, Aceton, Methanol, Ethanol Diethylether, THF, DMF, DME, bei einer Temperatur von - 78°C bis zur Rückflußtemperatur des jeweiligen organischen Lösungsmittels, zu den ketoner **GA** umsetzen.

Die Ketone **GA** werden mit Aminen in einer reduktiven Aminierung unter Verwendung von Reduktionsmitteln, wie Natriumcyanoborhydrid oder Natriumtriacetoxyborhydrid oder Boran-Pyridin Komplex, in einem organischen Lösungsmittel, wie Dichlormethan, Diethylether, 1,2-Dichlorethan, DME, DMF, Methanol, Ethanol oder THF, bei einer Temperatur von 0°C bis Rückflußtemperatur, zu den Verbindungen **HA** umgesetzt.

Die Aminketone **IA** erhält man unter dem Fachmann bekannten Bedingungen in einer Acetalspaltungsreaktion in einem organischen Lösungsmittel wir THF, Methanol, Ethanol, Dichlormethan oder Diethylether unter Zusatz von anorganischen Säuren, wie Schwefelsäure, Salzsäure, Ammoniumchlorid oder Hydrogensulfat oder in Gegenwart organischer Säuren, wie p-Toluolsulfonsäure oder Trifluoressigsäure, bei einer Temperatur von -10°C bis RT.

Die Verbindungen **IA** werden unter dem Fachmann bekannten Bedingungen mit Triethylphosphonacetat, in einem organischen Lösungsmittel, wie DME, THF, Diethylether oder Dichlormethan, In Gegenwart von Basen wie n-BuLi, tert.-BuLi, LDA, Metallhydride wie NaH, KH, bei einer Temperatur von -10°C bis Rückflußtemperatur des jeweiligen organischen Lösungsmittels zu den Produkten **JA** umgesetzt.

Die Verbindungen **JA** werden in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Ethanol, Dioxan, Dichlormethan, THF, Diethylether oder diese Lösungsmittel als Gemische, zu den Säuren **KA,** bei einer Temperatur von -10°C bis RT, umgesetzt.

Die Verbindungen **KA** können unter dem Fachmann bekannten Methoden in einer Hydrierungsreaktion in Gegenwart eines Katalysators, wie Raney/Nickel oder Palladium, jeweils unter Verwendung von Wasserstoff, Natriumborhydrid, Magnesium oder Palladium, in Gegewart von Ammoniumformat, in organischen Lösungsmittelen, wie Ethanol oder Methanol, bei einer Temperatur von 0°C bis RT, zu den Verbindungen **MA** umgesetzt werden.

Die Säuren **KA** oder **MA** können unter dem Fachmann bekannten Bedingungen in einer Amdibildung unter Verwendung primärer oder sekundärer Amine in Gegenwart wasserentziehender Mittel wie Natrium- oder Magnesiumsulfat, Phosphoroxid oder Reagenzien wie beispielsweise CDI, DCC (ggf. polymergebunden), TBTU, EDCI, PyBOP oder PFPTFA auch in Gegenwart von HOAt oder HOBt und einer organischen Base beispielsweise DIPEA oder Pyridin in einem organischen Lösungsmittel wie THF, Dichlormethan, Diethylether, Dioxan, DMF oder Acetonirtril zu den finalen Produkten der allgemeinen Formeln **LA** oder **NA** umgesetzt werden.

**Herstellung der Beispielverbindungen 491-496**

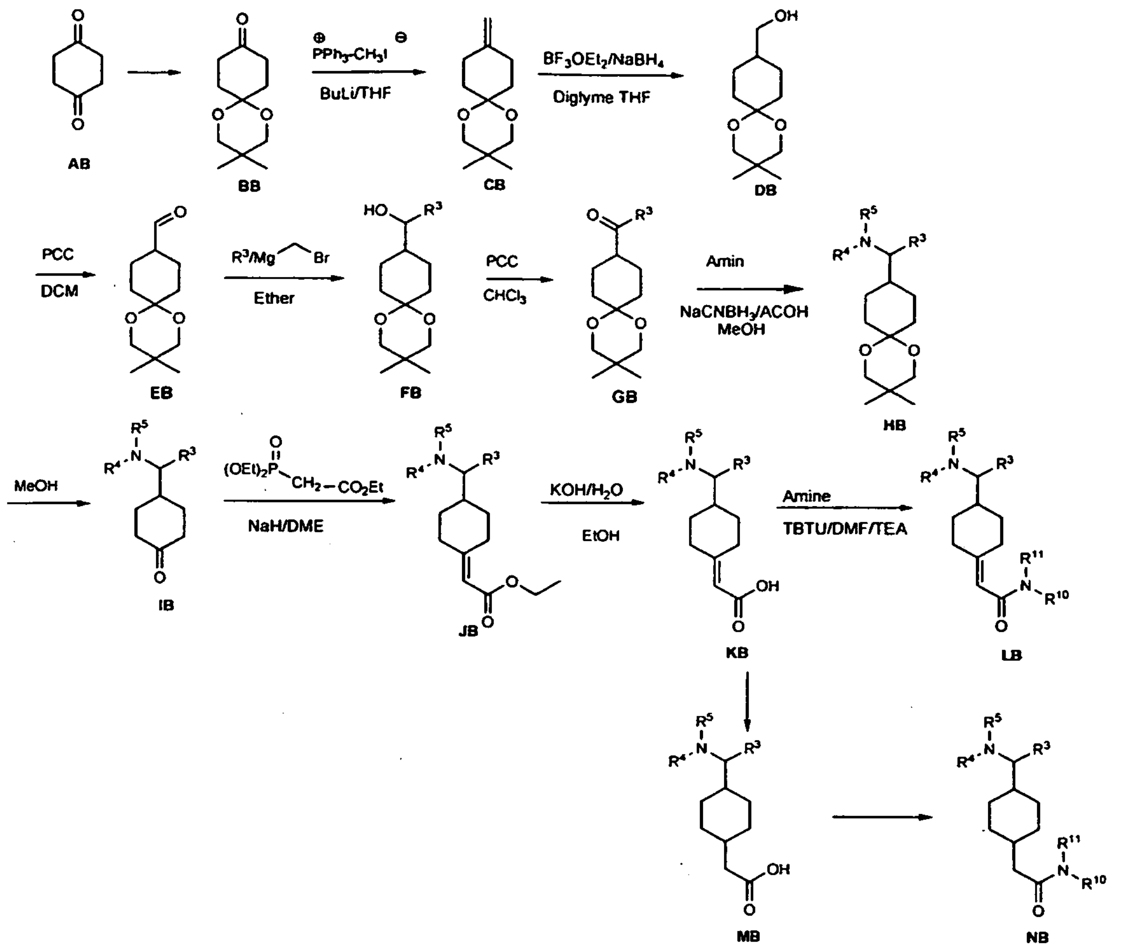


## Herstellung von BB

Zu einer Lösung von 1,4-Cyclohexandion **AB** (50 g, 1 Äquivalent) in DCM (400 ml) gab man Neopentylglycol (47 g, 1 Äquivalent) und $H_2SO_4$ (8 g, 0.2 Äquivalente) und rührte die Reaktionslösung über Nacht bei RT. Die Reaktionslösung wurde unter Eiskühlung in eine wässrige gesättigte $Na_2CO_3$-Lösung gegeben und die organische Phase abgetrennt. Nach Trocknung der organischen Phase mit $Na_2SO_4$ und Filtration wurde das Lösungsmittel unter Vakuum entfernt. Der Rückstand wurde mit Heptan (200 ml) versetzt und abfiltriert. Man erhielt das Produkt **BB** mit einer Ausbeute von 71% (51 g).

## Herstellung von CB:

Zum Wittigreagenz (122 g, 1.2 Äquivalente) in absolutem THF (600 ml) gab man bei 0°C n-BuLi (236 ml, 1.5 Äquivalente) tropfenweise hinzu und rührte für 1 h bei 0°C und für weitere 2 h bei -5°C bis 0°C. Nach tropfenweiser Zugabe einer Lösung von **BB** (50 g, 1 Äquvalent) in THF (150 ml) wurde die Reaktionsmischung für 1 h bei - 5°C bis 0°C gerührt. Nach Erwärmen bis auf RT ließ man die Reaktionslösung für weitere 4 h bei RT rühren.

Die Reaktionslösung wurde mit wässriger gesättigter $NH_4Cl$-Lösung (250 ml) versetzt und mit Ethylacetat (3 x 200 ml) extrahiert. Die organische Phase wurde abgetrennt, über $Na_2SO_4$ getrocknet, filtriert und das Lösungsmittel unter Vakuum entfernt. Der Rückstand wurde über Säulenchromatographie (5% EtOAc/Heptan) aufgereinigt. Man erhielt das Produkt **CB** mit einer Ausbeute von 75% (40g).

## Herstellung von DB:

In einem Dreihalskolben wurde $NaBH_4$ (13 g, 1.5 Äquivalente) und Diglyme (135 ml) für 10 min gerührt und gab anschließend $BF_3OEt_2$ (65 g, 2 Äquivalente) tropfenweise über eine Zeit von 30 min hinzu. Das dabei entstehende

$BH_3$ Gas wurde in eine auf 0°C abgekühlte Lösung von **CB** (45 g, 1 Äquivalent) in THF (450 ml) eingeleitet.

Die Reaktionsmischung wurde mit Natronlauge versetzt und mit Ethylacetat (3 x 150 ml) extrahiert. Die organische Phase wurde abgetrennt und das Lösungsmittel unter Vakuum entfernt. Man erhielt das Produkt **DB** in eine Menge von 45g.

**Herstellung von EB**

Eine Reaktionsmischung von PCC (105.4 g, 2 Äquivalente), DCM (550 ml) und Celite wurde bei für 10 min bei 0°C gerührt. Anschließend gab man eine Lösung von **DB** (45 g, 1 Äquivalent) in DCM (125 ml) tropfenweise über 15 min hinzu. Die Reaktionsmischung wurde für 1 h auf 60°C erhitzt.

Nach Filtration der Reaktionsmischung über Celite wurde mit DCM (125 ml) gewaschen. Das Lösungsmittel wurde unter Vakuum entfernt und das Rohprodukt über Säulenchromatographie (10% EtOAc/Heptan) aufgereinigt. Man erhielt das Produkt **EB** mit einer Ausbeute von 40% (18 g).

**Herstellung von FB:**

Zur Herstellung der Lösung 1 wurde absoluter Diethylether (100 ml), Mg (4.52 g, 4 Äquivalente) und Alkylhalogenid (2 Äquivalente) nacheinander zusammengegeben und für 10 min bei RT gerührt. Zu einer Lösung des Aldehyds **EB** (47 mmol, 1

Äquvalent) in absolutem THF (100 ml) gab die Lösung 1 tropfenweise unter Inertgasatmosphäre hinzu und rührte für 4 h bei RT.

Die Reaktionslösung wurde mit wässriger gesättigter $NH_4Cl$-Lösung (100 ml) versetzt und mit Ethylacetat (3 x 100 ml) extrahiert. Das Lösungsmittel wurde unter Vakuum entfernt und das Produkt **FB** über Säulenchromatographie (5% EtOAc/Heptan) aufgereinigt.

**Herstellung von GB:**

Zu einer Lösung von **FB** (23 mmol, 1 Äquivalent) in $CHCl_3$ (140 ml) gab man Celite und PCC (2 Äquvalente) und rührte die Reaktionsmischung für 2 h bei RT. Die Reaktionsmischung wurde über Celite abfiltriert und mit $CHCl_3$ gewaschen. Nach Entfernung des Lösungsmittels unter Vakuum wurde das Rohprodukt **GB** über Säulenchromatographie (7% EtOAc/Heptan) aufgereinigt.

**Herstellung von HB**

Zu einer Lösung von **GB** (18 mmol, 1 Äquivalent) in Methanol (5 ml) wurde ein Amin (1.5 Äquivalente), $NaCNBH_4$ (2 Äquivalente) und ACOH (16 ml) hinzugegeben und für 12 h bei RT gerührt.

Die Reaktionslösung wurde mit gesättigter wässriger $Na_2CO_3$-Lösung (50 ml) versetzt und mit Ethylacetat (3 x 100 ml) extrahiert. Das Lösungsmittel wurde unter Vakuum entfernt und der Rückstand **HB** über Säulenchromatographie (10% EtOAc/Heptan) aufgereinigt.

**Herstellung von IB**

Zu einer Lösung von **HB** (12 mmol, 1 Äquivalent) in Methanol (45 ml) wurde bei 0°C 10%-ige HCl (80 ml) hinzugegeben und für 10 min gerührt. Die Reaktionsmischung wurde mit Natronlauge (20 ml) versetzt und mit Ethylacetat extrahiert (3 x 50 ml).

Das Lösungsmittel wurde unter Vakuum entfernt und das Produkt ohne weitere Aufreinigung in die nächste Stufe eingesetzt.

**Herstellung von JB**

Zu einer Lösung von Triethylphosohonacetat (1.2 Äquivalente) in DME (35 ml) wurde NaH (1.4 Äquivalente) zugegeben und für 2 h unter Inertgasatmosphäre bei RT gerührt. Anschließend gab man eine Lösung von **IB** (12 mmol) in DME (33 ml) tropfenweise hinzu und rührte für weitere 3 h bei RT.

Die Reaktionsmischung wurde langsam mit Eiswasser (100 ml) versetzt und die Reaktionsmischung mit Ethylacetat (3 x 50 ml) extrahiert. Das Ethylacetat wurde unter Vakuum entfernt und das Rohprodukt über Säulenchromatographie (15% EtOAc/Heptan) aufgereinigt.

**Herstellung von KB**

Zu einer Lösung von **JB** (2 mmol) in Ethanol (14 ml) wurde KOH (2 Äquivalente) und Wasser (3 ml) zugegeben. Anschließend ließ man die Reaktionsmischung für 3 h bei RT rühren.

Die Raktionsmischung wurde mit HCl neutralisiert und mit Ethylacetat (3 x 50 ml) extrahiert. Nach Entfernung des Ethylacetat unter Vakuum erhielt man das Produkt KB, welches ohne weitere Aufarbeitung in die nächste Stufe eingesetzt wurde.

**Herstellung von MB**

In eine Lösung von **KB** (0.5 g) in Ethanol (15 ml) gab man eine katalytische Menge Raney/Ni in eine Wasserstoffatmosphäre und rührte die Reaktionslösung für 30 min bei RT. Nach Filtration über Celite wurde das Lösungsmittel unter Vakuum entfernt.

**Herstellung der Beispielverbindungen**

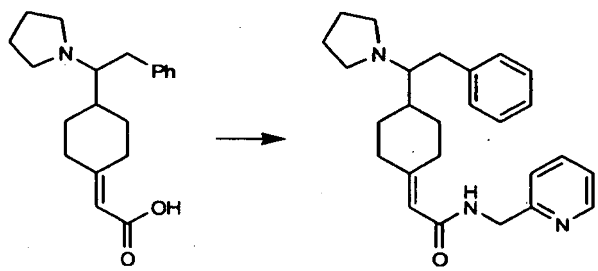

**Herstellung von 2-(4-(2-Phenyl-1-(pyrrolidin-1-yl)ethyl)cyclohexyliden)-N-(pyridin-2-ylmethyl)acetamid (Beispiel 495)**

Zu einer Lösung von **KB** (0.3 mmol, 100 mg) in DMF (1 ml) gab man TBTU (0.1 g, 1 Äquivalent) und Triethylamin (64 mg, 2 Äquivalente) hinzu und rührte für 10 min bei

RT. Nach der Zugabe von 2-(Aminomethyl)-pyridin (34 mg, 1 Äquivalent) wurde für 2 h bei RT gerührt.

Die Reaktionsmischung wurde in Eiswasser gegeben und mit Ethylacetat (3x10 ml) extrahiert. Die organische Phase wurde separiert, über $Na_2SO_2$ getrocknet und filtriert. Das Ethylacetat wurde unter Vakuum entfernt und der Rückstand über Säulenchromatographie (50% EtOAc/Heptan) aufgereinigt. Man erhielt das Produkt mit einer Ausbeute von 18% (22 mg).

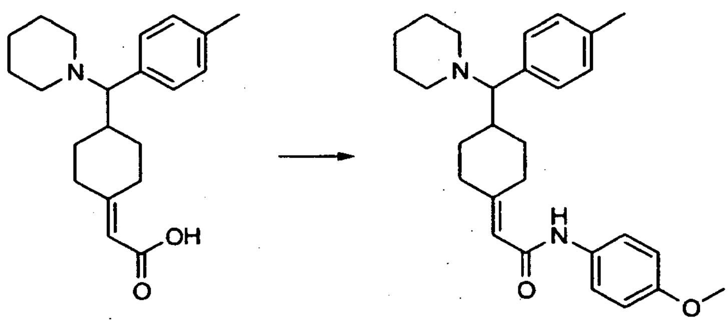

**Herstellung von N-(4-Methoxyphenyl)-2-(4-(piperidin-1-yl(p-tolyl)methyl)cyclohexyliden)acetamid (Beispiel 493)**

Zu einer Lösung von **KB** (0.06 mmol, 20 mg) in DMF (0.5 ml) gab man TBTU (20 mg, 1 Äquivalent) und

Triethylamin (6 mg, 2 Äquivalente) hinzu und rührte für 10 min bei RT. Nach der Zugabe von p-Methoxyanilin (30 mg, 1 Äquivalent) wurde für 45 min bei RT gerührt.

Die Reaktionsmischung wurde in Eiswasser gegeben und das Produkt abfiltriert.

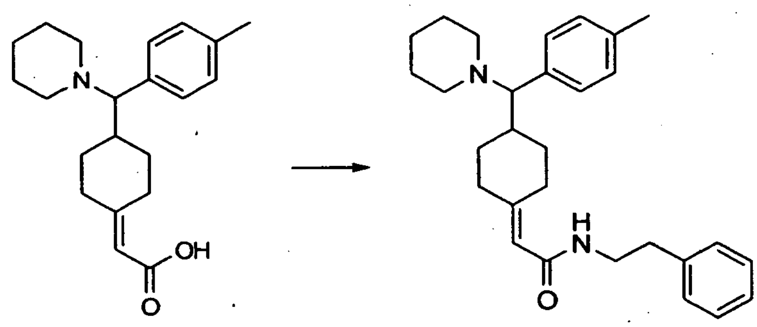

**Herstellung von N-Phenethyl-2-(4-(piperidin-1-yl(p-tolyl)methyl)cyclohexyliden)acetamid (Beispiel 494)**

Zu einer Lösung von **KB** (20 mg) in DMF (3 ml) gab man TBTU (20 mg, 1 Äquivalent) und Triethylamin (6 mg, 2 Äquivalente) hinzu und rührte für 10 min bei RT. Nach der Zugabe von Phenylethylamin (7 mg, 1 Äquivalent) wurde für 3 h bei RT gerührt.

Die Reaktionsmischung wurde mit Ethylacetat (2x100 ml) extrahiert. Die organische Phase wurde separiert, über $Na_2SO_2$ getrocknet und filtriert. Das Ethylacetat wurde unter Vakuum entfernt und der Rückstand über Säulenchromatographie (10% EtOAc/Heptan) aufgereinigt.

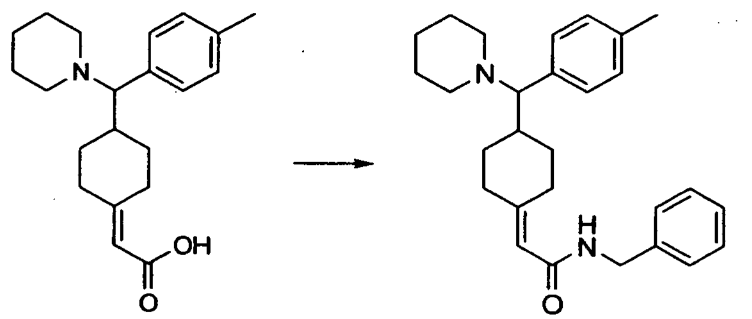

**Herstellung von N-Benzyl-N-methyl-2-(4-(piperidin-1-yl(p-tolyl)methyl)cyclohexyliden)acetamid Beispiel (496)**

Zu einer Lösung von **MB** (20 mg) in DMF (3 ml) gab man TBTU (20 mg, 1 Äquivalent) und Triethylamin (6 mg, 2 Äquivalente) hinzu und rührte für 10 min bei RT. Nach der Zugabe von N-Methylbenzylamin (7 mg, 1 Äquivalent) wurde für 3 h bei RT gerührt.

Die Reaktionsmischung wurde in Eiswasser (100 ml) gegeben und mit Ethylacetat (2 x 100 ml) extrahiert. Die organische Phase wurde separiert, über $Na_2SO_2$ getrocknet und filtriert. Das Ethylacetat wurde unter Vakuum entfernt und der Rückstand über Säulenchromatographie (10% EtOAc/Heptan) aufgereinigt. Man erhielt das Produkt mit einer Ausbeute von 22% (13 mg).

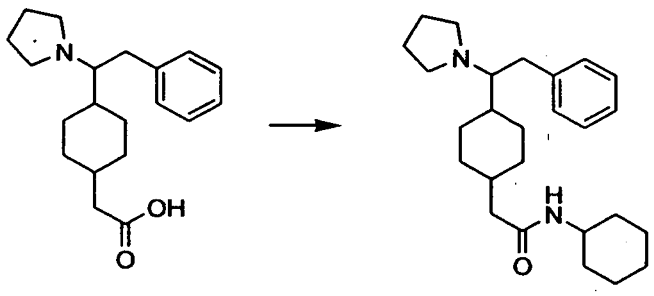

**Herstellung von N-Cyclohexyl-2-(4-(2-phenyl-1-(pyrrolidin-1-yl)ethyl)cyclohexyl)acetamid (Beispiel 491)**

Zu einer Lösung von **MB** (0.3 mmol, 100 mg) in DMF (1 ml) gab man TBTU (0.1 g, 1 Äquivalent) und Triethylamin (30 mg, 2 Äquivalente) hinzu und rührte für 10 min bei RT. Nach der Zugabe von Cyclohexylamin (30 mg, 1 Äquivalent) wurde für 30 min bei RT gerührt.

Die Reaktionsmischung wurde in Eiswasser (20 ml) gegeben und das Produkt abfiltriert. Man erhielt das Produkt mit einer Ausbeute von 96% (32 mg).

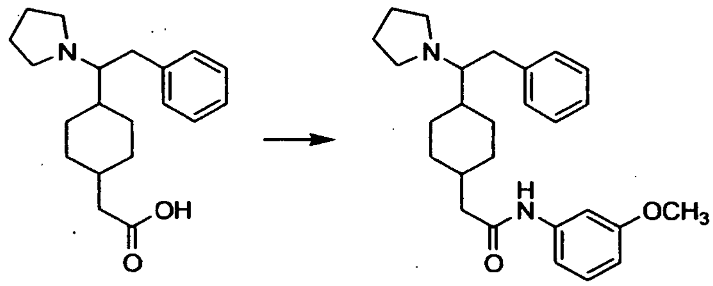

**Herstellung von N-(3-Methoxyphenyl)-2-(4-(2-phenyl-1-(pyrrolidin-1-yl)ethyl)cyclohexyl)acetamid (Beispiel 492)**

Zu einer Lösung von **KB** (0.3 mmol, 100 mg) in DMF (1 ml) gab man TBTU (0.1 g, 1 Äquivalent) und Triethylamin (30 mg, 2 Äquivalente) hinzu und rührte für 10 min bei RT. Nach der Zugabe von m-Methoxyanilin (30 mg, 1 Äquivalent) wurde für 30 min bei RT gerührt.

Die Reaktionsmischung wurde in Eiswasser (20 ml) gegeben und das Produkt abfiltriert. Man erhielt das Produkt mit einer Ausbeute von 96% (32 mg).

**Trennung der Diastereomeren**

In den Fällen, in denen Diastereomere getrennt wurden, wurde dies nach der folgenden Methode durchgeführt:

An einer HPLC-Säule VP 100/21 Nucleodur C 18 (5μm), 100 mm, 21 mm Innendurchmesser von Macherey-Nagel wurde mit Hilfe einer Waters 600 HPLC-Pumpe bei einem Starteluenten von 60% Wasser und 40% Methanol bei 25°C und einem Fluss von 20 ml/min das Rohprodukt aufgetragen. Innerhalb von 14 min wurde der Methanol-Anteil des Eluenten kontinuierlich auf 100% erhöht. Es wurden weitere 5,5 min mit 100% Methanol eluiert. Detektiert wurde mit einem Waters 2487 UV Detektor bei 220 und 254 nm und ES-MS. Die getrennten Fraktionen wurden gesammelt, eingeengt und mit Hilfe von ES Massenspektroskopie analysiert. In der vorliegenden Erfindung wurden die Beispielverbindungen, die in der ersten Fraktion eluiert wurden, als "polareres Diastereomer" und in der zweiten Fraktion als ,, unpolareres Diastereomer" bezeichnet.

**Untersuchungen zur Wirksamkeit der erfindungsgemäßen Verbindungen**

**Methode zur Bestimmung der Affinität zum humanen μ-Opiatrezeptor**

Die Rezeptoraffinität zum humanen μ-Opiatrezeptor wird in einem homgenen Ansatz in Mikrotiterplatten bestimmt. Hierzu werden Verdünnungsreihen der zu prüfenden Substanzen mit einer Rezeptormembranpräparation (15-40

μg Protein /250 μl Inkubationsansatz) von CHO-K1-Zellen, welche den humanen μ-Opiatrezeptor exprimieren (RB-HOM-Rezeptormembran-Präparation von Fa PerkinElmer Life Sciences, Zaventem, Belgien) in Gegenwart von 1 nmol/l des radioaktiven Liganden [$^3$H]-Naloxon (NET719, Fa. PerkinElmer Life Sciences, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 μl für 90 Minuten bei Raumtemperatur inkubiert. Als Inkubationspuffer wird 50 mmol/l Tris-HCl supplementiert mit 0,06 % bovinem Serumalbumin verwendet. Zur Bestimmung der unspezifischen Bindung wird zusätzlich 100 μmol/l Naloxon zugegeben. Nach Beendigung der neunzigminütigen Inkubationszeit werden die Mikrotiterplatten für 20 Minuten bei 1000 g abzentrifugiert und die Radioaktivität in einem ß-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen μ-Opiatrezeptor bei einer Konzentration der Prüfsubstanzen von 1 μmol/l bestimmt und als Prozent Hemmung der spezifischen Bindung angegeben.

**Noradrenalin (NA)- und Serotonin (5HT)-Wiederaufnahme-Inhibierung**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den NA-Uptake, werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Tabellen.**

Tabelle 1 : Monoamin-Wiederaufnahme-Inhibierung der Aldehyde

| Verb. | NA-Wiederaufnahme, %Hemmung [10 μM] | Serotonin-Wiederaufnahme, %Hemmung [10μM] |
|---|---|---|
| 28 | 84 | 85 |
| 34 | 87 | 87 |
| 37 | 97 | 75 |
| 40 | 77 | 78 |
| 43 | 98 | 80 |
| 46 | 95 | 92 |
| 49 | 97 | 93 |
| 52 | 96 | 94 |
| 55 | 95 | 88 |
| 67 | 86 | 95 |
| 70 | 93 | 70 |

Tabelle 2: μ-Affinität der Aldehyde

| Verb. | μ-Opioid-Rezeptor, %Hemmung [1 μM] | μ-Opioid-Rezeptor, $K_i$ [μM] |
|---|---|---|
| 28 | 69 | n.b. |
| 34 | 34 | n.b. |
| 37 | 45 | n.b. |
| 40 | 69 | n.b. |
| 46 | 75 | n.b. |
| 49 | 34 | n.b: |

(fortgesetzt)

| Verb. | μ-Opioid-Rezeptor, %Hemmung [1 μM] | μ-Opioid-Rezeptor, $K_i$ [μM] |
|---|---|---|
| 52 | 67 | n.b. |
| 55 | 50 | n.b. |
| 67 | 79 | 0,2 |

Tabelle 3: NA-Wiederaufnahme-Inhibierung der Ester

| Verb. | NA-Wiederaufnahme, %Hemmung [10 μM] | NA-Wederaufnahme, $K_i$ [μM] |
|---|---|---|
| 123 | 66 | n.b. |
| 124 | 76 | n.b. |
| 126 | 81 | n.b. |
| 127 | 89 | n.b. |
| 129 | 81 | n.b. |
| 130 | 82 | n.b. |
| 132 | 84 | 0,59 |
| 133 | 87 | n.b. |
| 135 | 90 | 0,49 |
| 136 | 89 | 0,58 |
| 138 | 96 | 0,62 |
| 139 | 94 | 0,79 |
| 141 | 98 | n.b. |
| 142 | 99 | n.b. |

Tabelle 4: Serotonin-Wiederaufnahme-Inhibierung der Ester

| Verb. | Serotonin-Wederaufnahme, %Hemmung [10 μM] | Serotonin-Wiederaufnahme, $K_i$ [μM] |
|---|---|---|
| 123 | 92 | |
| 124 | 85 | 0,097000 |
| 126 | 86 | n.b. |
| 127 | 90 | n.b. |
| 129 | 91 | 0,086 |
| 130 | 93 | 0,016 |
| 132 | 86 | 0,42 |
| 133 | 89 | 0,099 |
| 135 | 78 | 0,22 |
| 136 | 86 | 0,083 |
| 138 | 84 | 0,31 |
| 139 | 89 | 0,058 |
| 141 | 83 | n.b. |
| 142 | 84 | n.b. |

Tabelle 5: μ-Affinität der Ester

| Verb. | μ-Opioid-Rezeptor, %Hemmung [1 μM] | μ-Opioid-Rezeptor, $K_i$ [μM] |
|---|---|---|
| 123 | 75 | 0,19 |
| 124 | 79 | 0,079 |
| 126 | 35 | n.b. |
| 127 | 34 | n.b. |
| 129 | 47 | 0,54 |
| 130 | 82 | 0,23 |
| 132 | 91 | 0,12 |
| 133 | 81 | 0,096 |
| 135 | 70 | 0,38 |
| 136 | 73 | 0,23 |
| 138 | 73 | 0,092 |
| 139 | 90 | 0,044 |

Tabelle 6: Alkohole

| Verb. | NA-Wiederaufnahme, %Hemmung [10 μM] | Serotonin-Wiederaufnahme, %Hemmung [10 μM] | μ-Opioid-Rezeptor, %Hemmung [1μM] |
|---|---|---|---|
| 112 | 74 | 82 | 66 |
| 113 | 82 | 83 | 46 |
| 114 | 93 | 64 | 43 |
| 115 | 47 | 63 | 61 |
| 116 | 95 | 74 | 37 |
| 117 | 82 | 85 | 57 |
| 118 | 91 | 90 | 33 |
| 119 | 91 | 88 | 43 |
| 120 | 93 | 79 | 51 |
| 121 | 66 | 74 | 61 |
| 122 | 97 | 77 | 32 |
| 125 | 86 | 82 | 62 |
| 128 | 88 | 84 | 18 |
| 131 | 89 | 91 | 68 |
| 134 | 90 | 86 | 45 |
| 137 | 93 | 89 | 13 |
| 140 | 94 | 85 | 31 |
| 143 | 100 | 90 | 24 |

Tabelle 7: Etherderivate

| Verb. | NA-Wiederaufnahme , %Hemmung [10 μM] | Serotonin-Wiederaufnahme %Hemmung [10 μM] | Serotonin-Wiederaufnahme, $K_i$ [μM] | μ-Opioid-Rezeptor, %Hemmung [1μM] | μ-Opioid-Rezeptor, $K_i$ [μM] |
|---|---|---|---|---|---|
| 144 | 75 | 80 | n.b. | 81 | 0,12 |
| 145 | 84 | 83 | 0,29 | 73 | 0,31 |

Tabelle 8: NA-Wiederaufnahme-Inhibierung der Grignard-Verbindungen

| Verb. | NA-Wiederaufnahme, %Hemmung [10 μM] | $K_i$ [μM] |
|---|---|---|
| 146 | 94 | 0,6 |
| 147 | 12 | n.b. |
| 148 | 97 | n.b. |
| 149 | 75 | n.b. |

Tabelle 9: 5HT-Uptake-Inhibierung der Grignard-Derivate

| Verb. | Serotonin-Wiederaufnahme, %Hemmung [10 μM] | Serotonin-Wiederaufnahme, $K_i$ [μM] |
|---|---|---|
| 146 | 92 | 0,15 |
| 148 | 90 | 0,37 |
| 149 | 85 | n.b. |

Tabelle 10: μ-Affinität der Grignard-Derivate

| Verb. | μ-Opioid-Rezeptor, %Hemmung [1 μM] |
|---|---|
| 146 | 97 |
| 148 | 91 |
| 149 | 82 |
| 150 | 74 |
| 151 | 71 |
| 152 | 63 |
| 153 | 87 |
| 154 | 50 |
| 155 | 77 |
| 156 | 73 |
| 157 | 72 |
| 158 | 58 |
| 159 | 83 |
| 160 | 67 |
| 161 | 60 |
| 162 | 59 |

(fortgesetzt)

| Tabelle 10: μ-Affinität der Grignard-Derivate | |
|---|---|
| Verb. | μ-Opioid-Rezeptor, %Hemmung [1 μM] |
| 163 | 81 |
| 164 | 58 |
| 165 | 52 |
| 166 | 62 |
| 167 | 58 |
| 168 | 58 |
| Verb. | μ-Opioid-Rezeptor, %Hemmung [1μM] |
| 169 | 71 |
| 170 | 63 |
| 171 | 67 |
| 172 | 77 |
| 173 | 84 |

Tabelle 11: primäre Amine

| Verb. | Serotonin-Wiederaufnahme, %Hemmung [10 μM] | Serotonin-Wiederaufnahme, $K_i$ [μM] | NA-Wiederaufnahme, %Hemmung [10 μM] | NA-Wiederaufnahme, $K_i$ [μM] |
|---|---|---|---|---|
| 17 | 86 | 0,86 | 92 | 0,87 |
| 19 | 87 | n.b. | 93 | n.b. |
| 21 | 80 | n.b. | 90 | n.b. |
| 23 | 64 | n.b. | 97 | n.b. |
| 25 | 71 | n.b. | 84 | n.b. |
| 27 | 78 | n.b. | 95 | n.b. |
| 30 | 89 | n.b. | 89 | n.b. |
| 33 | 95 | n.b. | 94 | n.b. |
| 36 | 89 | 0,096 | 93 | 0,11 |
| 39 | 87 | n.b. | 97 | n.b. |
| 42 | 86 | n.b. | 83 | n.b. |
| 45 | 90 | n.b. | 96 | n.b. |
| 48 | 95 | n.b. | 100 | n.b. |
| 51 | 97 | n.b. | 98 | n.b. |
| 54 | 94 | n.b. | 102 | n.b. |
| 66 | 96 | n.b. | 96 | n.b. |
| 69 | 83 | n.b. | 98 | n.b. |
| 72 | 79 | n.b. | 91 | n.b. |

Tabelle 12: μ-Affinität der primären Amine

| | μ-Opioid-Rezeptor, %Hemmung [1μM] | μ-Opioid-Rezeptor, $K_i$ [μM] |
|---|---|---|
| 17 | 83 | 0,44 |
| 21 | 48 | n.b. |
| 23 | 58 | n.b. |
| 25 | 74 | 0,26 |
| 30 | 94 | 0,21 |
| 36 | 68 | n.b. |
| 39 | 56 | n.b. |
| 42 | 80 | 0,24 |
| 48 | 67 | n.b. |
| 54 | 62 | n.b. |
| 66 | 62 | n.b. |
| 69 | 61 | n.b. |

Tabelle 13: sek. Amine

| | Serotonin-Wiederaufnahme, %Hemmung [10 μM] | NA-Wiederaufnahme, %Hemmung [10 μM] |
|---|---|---|
| 83 | 93 | 100 |
| 84 | 76 | 93 |
| 85 | 77 | 103 |
| 86 | 88 | 95 |
| 87 | 95 | 106 |
| 88 | 88 | 84 |
| 89 | 73 | 85 |
| 90 | 87 | 98 |
| 91 | 76 | 93 |
| 92 | 69 | 75 |

Tabelle 14: μ-Affinität der sek. Amine

| | μ-Opioid-Rezeptor, %Hemmung [1μM] | μ-Opioid-Rezeptor, $K_i$ [μM] |
|---|---|---|
| 83 | 95 | 0,007 |
| 84 | 95 | 0,012 |
| 85 | 98 | 0,0028 |
| 86 | 98 | 0,0038 |
| 87 | 85 | 0,0037 |
| 88 | 99 | 0,0024 |
| 89 | 95 | 0,059 |
| 90 | 94 | 0,045 |
| 91 | 90 | 0,0081 |

(fortgesetzt)

| | μ-Opioid-Rezeptor, %Hemmung [1μM] | μ-Opioid-Rezeptor, $K_i$ [μM] |
|---|---|---|
| 92 | 91 | 0,017 |
| 174 | 95 | 0,004900 |
| 175 | 86 | 0,011000 |

Tabelle 15: Harnstoffe

| | Serotonin-Wiederaufnahme, %Hemmung [10 μM] | Serotonin-Wiederaufnahme, $K_i$ [μM] | NA-Wiederaufnahme, %Hemmung [10 μM] | NA-Wiederaufnahme, $K_i$ [μM] |
|---|---|---|---|---|
| 73 | 90 | 0,061 | 94 | 0,18 |
| 75 | 80 | 0,013 | 88 | 0,55 |
| 74 | 86 | 0,12 | 93 | 0,22 |
| 76 | 84 | n.b. | 100 | n.b. |
| 77 | 84 | n.b. | 93 | n.b. |
| 78 | 87 | 0,16 | 98 | 0, 29 |
| 79 | 97 | 0,091 | 96 | 0,12 |
| 80 | 97 | 0,25 | 97 | 0,49 |
| 81 | 97 | n.b. | 97 | n.b. |
| 82 | 98 | 0,11 | 98 | 0,12 |

Tabelle 16: μ-Affinität der Harnstoffe

| | μ-Opioid-Rezeptor, %Hemmung [1 μM] | μ-Opioid-Rezeptor, $K_i$ [μM] |
|---|---|---|
| 73 | 96 | 0,046 |
| 75 | 88 | 0,098 |
| 74 | 88 | 0,16 |
| 76 | 61 | n.b. |
| 77 | 88 | 0,078 |
| 78 | 91 | 0,054 |
| 79 | 103 | 0,0083 |
| 80 | 95 | 0,02 |
| 81 | 95 | 0,033 |
| 82 | 92 | 0,19 |

Tabelle 17: Sulfonamide

| | Serotonin-Wiederaufnahme, %Hemmung [10 μM] | NA-Wederaufnahme, %Hemmung [10 μM] | μ-Opioid-Rezeptor, %Hemmung [1μM] | μ-Opioid-Rezeptor, $K_i$ [μM] |
|---|---|---|---|---|
| 106 | 83 | 68 | 96 | 0,0037 |
| 107 | 90 | 78 | 99 | 0,015 |

(fortgesetzt)

| | Serotonin-Wiederaufnahme, %Hemmung [10 μM] | NA-Wederaufnahme, %Hemmung [10 μM] | μ-Opioid-Rezeptor, %Hemmung [1μM] | μ-Opioid-Rezeptor, $K_i$ [μM] |
|---|---|---|---|---|
| 108 | 75 | 75 | 92 | 0,022 |
| 109 | 93 | 76 | 92 | 0,077 |
| 110 | 86 | 62 | 93 | 0,025 |

Tabelle 18: Acylierte Amine

| Verb. | Serotonin-Wiederaufnahme, %Hemmung [10 μM] | Serotonin-Wiederaufnahme, $K_i$ [μM] | NA-Wiederaufnah me, %Hemmung [10 μM] | NA-Wiederaufnah me, $K_i$ [μM] |
|---|---|---|---|---|
| 95 | 66 | n.b. | 80 | n.b. |
| 96 | 83 | 0,66 | 88 | 0,8 |
| 97 | 76 | 0,99 | 94 | 0,7 |
| 98 | 74 | 0,63 | 95 | 0,7 |
| 99 | 83 | 0,54 | 75 | |
| 93 | 75 | 0,66 | 48 | |
| 100 | 86 | 0,52 | 91 | 0,32 |
| 101 | 81 | 0,88 | 86 | 0,67 |
| 102 | 85 | 0,45 | 92 | 0,13 |
| 103 | 93 | 0,56 | 87 | 0,4 |
| 104 | 90 | 0,44 | 92 | 0,32 |
| 105 | 85 | | 88 | |

Tabelle 19: μ-Affinität acylierter Amine

| Verb. | μ-Opioid-Rezeptor, %Hemmung [1 μM] | μ-Opioid-Rezeptor, $K_i$ [μM] |
|---|---|---|
| 95 | 95 | 0,0025 |
| 96 | 99 | 0,086 |
| 97 | 97 | 0,0037 |
| 98 | 104 | |
| 99 | 77 | 0,47 |
| 93 | 94 | 0,094 |
| 100 | 100 | 0,0035 |
| 101 | 101 | 0,0023 |
| 102 | 94 | 0,0015 |
| 103 | 99 | 0,0088 |
| 104 | 92 | 0,014 |
| 105 | 100 | 0,02 |
| 176 | 86 | 0,019 |

(fortgesetzt)

| Verb. | μ-Opioid-Rezeptor, %Hemmung [1 μM] | μ-Opioid-Rezeptor, $K_i$ [μM] |
|---|---|---|
| 177 | 86 | 0,0072 |
| 178 | 96 | 0,0012 |
| 179 | 99 | 0,003 |
| 180 | 90 | 0,02 |
| 181 | 95 | 0,0039 |
| 182 | 95 | 0,0021 |

Tabelle 20: μ-Affinität der acylierten Amine

| Verb. | μ-Opioid-Rezeptor, %Hemmung [1μM] | μ-Opioid-Rezeptor, [μM] |
|---|---|---|
| 183 | 103 | 0,0039 |
| 184 | 101 | 0,0052 |
| 185 | 100 | 0,037 |
| 186 | 100 | 0,018 |
| 187 | 100 | 0,0085 |
| 188 | 100 | 0,0052 |
| 189 | 99 | 0,011 |
| 190 | 99 | 0,025 |
| 191 | 99 | |
| 192 | 98 | 0,018 |
| 193 | 96 | 0,023 |
| 194 | 96 | 0,0053 |
| 195 | 96 | 0,015 |
| 196 | 96 | 0,019 |
| 197 | 96 | 0,016 |
| 198 | 95 | 0,015 |
| 199 | 94 | 0,021 |
| 200 | 94 | 0,029 |
| 201 | 94 | 0,016 |
| 202 | 94 | 0,031 |
| 203 | 94 | 0,051 |
| 204 | 94 | 0,018 |
| 205 | 94 | 0,022 |
| 206 | 93 | 0,056 |
| 207 | 92 | 0,028 |
| 208 | 92 | 0,13 |
| 209 | 92 | 0,047 |
| 210 | 92 | 0,024 |
| 211 | 92 | |

(fortgesetzt)

| Verb. | μ-Opioid-Rezeptor, %Hemmung [1μM] | μ-Opioid-Rezeptor, [μM] |
|---|---|---|
| 212 | 91 | 0,019 |
| 213 | 91 | 0,096 |
| 214 | 91 | 0,028 |
| 215 | 90 | 0,034 |
| 216 | 90 | 0,056 |
| 217 | 90 | 0,062 |
| 218 | 89 | |
| 219 | 89 | 0,056 |
| 220 | 88 | 0,15 |
| 221 | 88 | 0,029 |
| 222 | 88 | 0,02 |
| 223 | 88 | 0,02 |
| 224 | 87 | |
| 225 | 87 | 0,02 |
| 226 | 87 | 0,058 |
| 227 | 87 | 0,058 |
| 228 | 87 | 0,019 |
| 229 | 86 | 0,039 |
| 230 | 86 | 0,021 |
| 231 | 86 | 0,045 |
| 232 | 84 | 0,074 |
| 233 | 84 | 0,071 |
| 234 | 84 | 0,046 |
| 235 | 84 | 0,061 |
| 236 | 83 | 0,063 |
| 237 | 83 | 0,048 |
| 238 | 83 | 0,038 |
| 239 | 82 | 0,08 |
| 240 | 82 | 0,051 |
| 241 | 82 | 0,068 |
| 242 | 82 | 0,046 |
| 243 | 82 | 0,025 |
| 244 | 82 | 0,03 |
| 245 | 82 | 0,066 |
| 246 | 82 | 0,039 |
| 247 | 82 | 0,036 |
| 248 | 81 | 0,088 |
| 249 | 81 | 0,064 |

(fortgesetzt)

| Verb. | μ-Opioid-Rezeptor, %Hemmung [1μM] | μ-Opioid-Rezeptor, [μM] |
|---|---|---|
| 250 | 81 | 0,036 |
| 251 | 81 | 0,091 |
| 252 | 81 | 0,02 |
| 253 | 80 | 0,079 |
| 254 | 80 | 0,056 |
| 255 | 80 | 0,046 |
| 256 | 80 | 0,08 |
| 257 | 79 | 0,081 |
| 258 | 79 | 0,068 |
| 259 | 79 | 0,062 |
| 260 | 78 | |
| 261 | 78 | 0,13 |
| 262 | 78 | 0,096 |
| 263 | 78 | 0,028 |
| 264 | 78 | 0,11 |
| 265 | 78 | 0,14 |
| 266 | 78 | 0,036 |
| 267 | 77 | 0,15 |
| 268 | 76 | 0,086 |
| 269 | 75 | 0,047 |
| 270 | 75 | 0,19 |
| 271 | 75 | 0,019 |
| 272 | 75 | 0,09 |
| 273 | 75 | 0,081 |
| 274 | 74 | 0,099 |
| 275 | 74 | 0,1 |
| 276 | 74 | 0,065 |
| 277 | 74 | 0,074 |
| 278 | 74 | 0,086 |
| 279 | 74 | 0,14 |
| 280 | 73 | 0,31 |
| 281 | 73 | 0,046 |
| 282 | 73 | 0,11 |
| 283 | 72 | 0,15 |
| 284 | 72 | 0,063 |
| 285 | 71 | |
| 286 | 71 | 0,054 |
| 287 | 71 | 0,15 |

(fortgesetzt)

| Verb. | μ-Opioid-Rezeptor, %Hemmung [1μM] | μ-Opioid-Rezeptor, [μM] |
|---|---|---|
| 288 | 70 | 0,16 |
| 289 | 70 | 0,078 |
| 290 | 70 | |
| 291 | 102 | |
| 292 | 102 | 0,0013 |
| 293 | 102 | 0,0046 |
| 294 | 101 | 0,0083 |
| 295 | 101 | 0,0073 |
| 296 | 100 | 0,0003 |
| 297 | 100 | 0,004 |
| 298 | 100 | 0,004 |
| 299 | 100 | 0,001 |
| 300 | 99 | 0,0014 |
| 301 | 99 | 0,0027 |
| 302 | 99 | 0,0016 |
| 303 | 99 | 0,019 |
| 304 | 99 | 0,015 |
| 305 | 98 | 0,012 |
| 306 | 98 | 0,0099 |
| 307 | 98 | 0,0062 |
| 308 | 98 | 0,0074 |
| 309 | 98 | 0,0056 |
| 310 | 98 | 0,016 |
| 311 | 98 | 0,011 |
| 312 | 97 | 0,053 |
| 313 | 97 | 0,035 |
| 314 | 97 | 0,0042 |
| 315 | 97 | 0,16 |
| 316 | 96 | |
| 317 | 95 | 0,029 |
| 318 | 95 | 0,023 |
| 319 | 95 | 0,015 |
| 320 | 95 | 0,028 |
| 321 | 95 | 0,022 |
| 322 | 95 | 0,031 |
| 323 | 95 | 0,015 |
| 324 | 95 | 0,022 |
| 325 | 94 | 0,034 |

(fortgesetzt)

| Verb. | μ-Opioid-Rezeptor, %Hemmung [1μM] | μ-Opioid-Rezeptor, [μM] |
|---|---|---|
| 326 | 94 | 0,011 |
| 327 | 94 | 0,063 |
| 328 | 94 | 0,016 |
| 329 | 94 | 0,035 |
| 330 | 94 | 0,039 |
| 331 | 93 | 0,011 |
| 332 | 93 | 0,02 |
| 333 | 92 | 0,017 |
| 334 | 92 | 0,062 |
| 335 | 92 | 0,033 |
| 336 | 92 | 0,039 |
| 337 | 92 | |
| 338 | 91 | 0,028 |
| 339 | 91 | 0,025 |
| 340 | 91 | 0,055 |
| 341 | 91 | 0,013 |
| 342 | 90 | 0,028 |
| 343 | 90 | |
| 344 | 90 | 0,013 |
| 345 | 90 | 0,013 |
| 346 | 90 | 0,065 |
| 347 | 90 | 0,026 |
| 348 | 89 | 0,03 |
| 349 | 89 | 0,05 |
| 350 | 89 | 0,037 |
| 351 | 89 | 0,058 |
| 352 | 87 | |
| 353 | 87 | 0,096 |
| 354 | 86 | 0,053 |
| 355 | 86 | 0,12 |
| 356 | 86 | 0,12 |
| 357 | 86 | 0,06 |
| 358 | 86 | 0,011 |
| 359 | 86 | |
| 360 | 86 | 0,035 |
| 361 | 85 | 0,042 |
| 362 | 85 | 0,06 |
| 363 | 85 | 0,038 |

(fortgesetzt)

| Verb. | μ-Opioid-Rezeptor, %Hemmung [1μM] | μ-Opioid-Rezeptor, [μM] |
|---|---|---|
| 364 | 85 | 0,035 |
| 365 | 85 | 0,074 |
| 366 | 85 | 0,056 |
| 367 | 85 | 0,11 |
| 368 | 84 | 0,034 |
| 369 | 84 | 0,068 |
| 370 | 84 | 0,081 |
| 371 | 84 | 0,13 |
| 372 | 84 | 0,033 |
| 373 | 84 | 0,061 |
| 374 | 84 | |
| 375 | 84 | |
| 376 | 84 | 0,04 |
| 378 | 83 | 0,055 |
| 379 | 83 | |
| 380 | 83 | 0,095 |
| 381 | 83 | 0,099 |
| 382 | 83 | |
| 383 | 83 | 0,086 |
| 384 | 82 | 0,038 |
| 385 | 82 | 0,068 |
| 386 | 82 | 0,1 |
| 387 | 81 | 0,07 |
| 388 | 81 | 0,036 |
| 389 | 81 | 0,058 |
| 390 | 81 | |
| 391 | 81 | 0,026 |
| 392 | 80 | 0,18 |
| 393 | 80 | 0,044 |
| 394 | 79 | 0,048 |
| 395 | 81 | 0,046 |
| 396 | 97 | 0,012 |
| 397 | 97 | 0,0072 |
| 398 | 101 | 0,0074 |
| 399 | 80 | 0,11 |
| 400 | 94 | 0,023 |
| 401 | 97 | 0,011 |
| 402 | 96 | 0,0079 |

(fortgesetzt)

| Verb. | μ-Opioid-Rezeptor, %Hemmung [1μM] | μ-Opioid-Rezeptor, [μM] |
|---|---|---|
| 403 | 81 | 0,074 |
| 404 | 97 | 0,019 |
| 405 | 88 | 0,025 |
| 406 | 81 | 0,04 |
| 407 | 87 | 0,048 |
| 408 | 82 | 0,012 |
| 409 | 84 | 0,028 |
| 410 | 88 | 0,0058 |
| 411 | 99 | 0,011 |
| 412 | 93 | 0,032 |
| 413 | 81 | 0,031 |
| 414 | 99 | 0,0046 |
| 415 | 99 | 0,0051 |
| 416 | 86 | 0,019 |
| 417 | 91 | 0,031 |
| 418 | 92 | 0,043 |
| 419 | 86 | 0,032 |
| 420 | 94 | 0,016 |
| 421 | 80 | 0,091 |
| 422 | 83 | 0,035 |
| 423 | 83 | 0,015 |
| 424 | 99 | 0,0027 |
| 425 | 94 | 0,006 |
| 426 | 99 | 0,0058 |
| 427 | 84 | 0,04 |
| 428 | 97 | 0,0054 |
| 429 | 88 | 0,055 |
| 430 | 101 | 0,0044 |
| 431 | 93 | 0,026 |
| 432 | 95 | 0,018 |
| 433 | 89 | 0,0038 |
| 434 | 85 | 0,024 |
| 435 | 99 | 0,014 |
| 436 | 91 | 0,029 |
| 437 | 80 | 0,1 |
| 438 | 87 | 0,1 |
| 439 | 85 | 0,052 |
| 440 | 95 | 0,015 |

(fortgesetzt)

| Verb. | μ-Opioid-Rezeptor, %Hemmung [1μM] | μ-Opioid-Rezeptor, [μM] |
|---|---|---|
| 441 | 83 | 0,2 |
| 442 | 86 | 0,11 |
| 443 | 96 | 0,04 |
| 444 | 101 | 0,011 |
| 445 | 86 | 0,069 |
| 446 | 99 | 0,0064 |
| 447 | 83 | 0,13 |
| 448 | 95 | 0,033 |
| 449 | 88 | 0,036 |
| 450 | 89 | 0.081 |
| 451 | 92 | 0,024 |
| 452 | 99 | 0,012 |
| 453 | 83 | 0,043 |
| 454 | 87 | 0,051 |
| 455 | 81 | 0,069 |
| 456 | 96 | 0,023 |
| 457 | 91 | 0,04 |
| 458 | 93 | 0,049 |
| 459 | 90 | 0,031 |
| 460 | 97 | 0,0095 |
| 461 | 100 | 0,017 |
| 462 | 91 | 0,055 |
| 463 | 104 | 0,0086 |
| 464 | 92 | 0,075 |
| 465 | 99 | 0,0031 |
| 466 | 86 | 0,041 |
| 467 | 82 | 0,062 |
| 468 | 83 | 0,16 |
| 469 | 87 | 0,057 |
| 470 | 91 | 0,071 |
| 471 | 82 | 0,087 |
| 472 | 85 | |
| 473 | 91 | 0,018 |
| 474 | 100 | 0,0027 |
| 475 | 97 | 0,0069 |
| 476 | 87 | 0,084 |
| 477 | 96 | 0,013 |
| 478 | 95 | 0,027 |

Tabelle 21: Beispiele 492-501

| Nr. | Serotonin-Wiederaufnahme %Hemmung [10μM] | NA-Wiederaufnahme, %Hemmung [10μM] |
|---|---|---|
| **492** | 3 | 54 |
| **493** | 38 | 27 |
| **494** | 40 | 81 |
| **495** | 7 | 16 |
| **496** | 18 | 26 |
| **497** | | 101 |
| **498** | | 99 |
| **499** | | 103 |
| **500** | | 101 |
| **501** | | 106 |

**In-vivo-Untersuchungen zur Analgesie: Tail-flick Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Die vertiefte Untersuchung auf analgetische Wirksamkeit wurde im Tail-Flick-Test an der Maus durchgeführt, wie obenstehend beschrieben.

Die untersuchten erfindungsgemäßen Verbindungen zeigten eine analgetische Wirkung. Die Ergebnisse ausgewählter Untersuchungen sind in der nachfolgenden Tabelle zusammengefaßt.

| Verbindung Nr. | Dosierung mg/kg (i.v.) | Wirkung % MPE | $ED_{50}$ i.v. (4,64-21,5) |
|---|---|---|---|
| 78 | | | 8,94 mg/kg |
| 79 | 10 | 76 | |
| 80 | 10 | 59 | |
| 86 | 21,5 | 100 | |
| 87 | 10 | 39 | |
| 88 | 21,5 | 90 | |
| 91 | 21,5 | 90 | |
| 95 | 1 | 100 | |

(fortgesetzt)

| Verbindung Nr. | Dosierung mg/kg (i.v.) | Wirkung % MPE | $ED_{50}$ i.v. (4,64-21,5) |
|---|---|---|---|
| 98 | 10 | 100 | |
| 102 | 10 | 82 | |
| 146 | 10 | 21 | |
| 148 | 10 | 40 | |

**Patentansprüche**

**1.** Substituierte Cyclohexylmethyl-Derivate der allgemeinen Formel I,

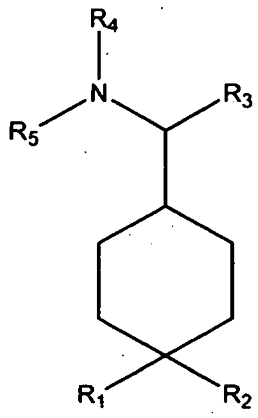

I

worin
$R^1$ $C_{1-8}$-Alkyl, jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert; $(CH_2)_mCHN$-OH, $(CH_2)_nNR^6R^7$ oder $(CH_2)_nOR^8$ bedeutet, wobei n für 0, 1, 2 oder 3 und m für 0, 1 oder 2 steht; oder über eine $C_{1-3}$-Alkylgruppe, die gesättigt oder ungesättigt sein kann, verknüpftes $C(O)OR^9$; $CONR^{10}R^{11}$ bedeutet;
$R^2$ H oder OH bedeutet;
oder $R^1$ und $R^2$ gemeinsam für , oder =N-OH stehen;
$R^3$ Aryl oder Heteroaryl, unsubstituiert oder einfach oder mehrfach substituiert; oder eine über eine $C_{1-3}$-Alkylgruppe verknüpften Arylrest, der unsubstituiert oder einfach oder mehrfach substituiert sein kann, bedeutet;
$R^4$ und $R^5$ unabhängig voneinander H; $C_{1-3}$-Alkyl, unsubstituiert, bedeutet, wobei $R^4$ und $R^5$ nicht gleichzeitig H bedeuten,
oder die Reste $R^4$ und $R^5$ zusammen $CH_2CH_2OCH_2CH_2$, oder $(CH_2)_{3-6}$ bedeuten,
$R^6$ H; $C_{1-8}$-Alkyl, gesättigt oder ungesättigt, verzweigt oder unverzweigt, unsubstituiert oder einfach oder mehrfach substituiert; Aryl, Heteroaryl oder $C_{3-10}$-Cycloalkyl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, bedeutet;
$R^7$ H; $C_{1-8}$-Alkyl, gesättigt oder ungesättigt, verzweigt oder unverzweigt, unsubstituiert oder einfach oder mehrfach substituiert; Aryl, Heteroaryl oder $C_{3-10}$-Cycloalkyl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert; $C(O)NR^{10}R^{11}$, $C(S)NR^{10}R^{11}$, $SO_2R^{12}$ oder $C(O)R^{13}$ bedeutet;
$R^8$ H; $C_{1-8}$-Alkyl, jeweils verzweigt oder unverzweigt, gesättigt oder ungesättigt, unsubstituiert oder einfach oder mehrfach substituiert; $C_{3-10}$-Cycloalkyl, gesättigt oder ungesättigt, unsubstituiert oder einfach oder mehrfach sub-

stituiert; über eine $C_{1-4}$-Alkylgruppe verknüpften Aryl- oder Heteroarylrest, unsubstituiert oder einfach oder mehrfach substituiert, bedeutet;

$R^9$ H; $C_{1-4}$-Alkyl gesättigt oder ungesättigt, verzweigt oder unverzweigt, unsubstiuiert oder einfach oder mehrfach substituiert, bedeutet;

$R^{10}$ und $R^{11}$ unabhängig voneinander H; $C_{3-10}$-Cycloalkyl, gesättigt oder ungesättigt, unsubstituiert oder einfach oder mehrfach substituiert; Aryl oder Heteroaryl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, bedeutet;

$R^{12}$ Aryl oder Heteroaryl, jeweils unsubstituiert oder einfach oder mehrfach substituiert; $C_{1-8}$-Alkyl, jeweils gesättigt oder ungesättigt, verzweigt oder unverzweigt, unsubstituiert oder einfach oder mehrfach substituiert; $C_{3-10}$-Cycloalkyl, gesättigt oder ungesättigt, unsubstituiert oder einfach oder mehrfach substituiert; oder einen über eine $C_{1-3}$-Alkylkette verknüpften Aryl- oder Heteroarylrest, jeweils unsubstituiert oder einfach oder mehrfach substituiert; bedeutet;

$R^{13}$ $C_{1-8}$-Alkyl, gesättigt oder ungesättigt, verzweigt oder unverzweigt, unsubstituiert oder einfach oder mehrfach substituiert; $C_{3-10}$-Cycloalkyl, Aryl oder Heteroaryl, jeweils unsubstituiert oder einfach oder mehrfach substituiert; oder einen über eine $C_{1-4}$-Alkylkette verknüpften Aryl- oder Heteroarylrest, jeweils unsubstituiert oder einfach oder mehrfach substituiert, wobei die Alkylkette verzweigt oder unverzweigt, unsubstituiert oder einfach oder mehrfach substituiert sein kann; bedeutet;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**2.** Substituierte Cyclohexylmethyl-Derivate gemäß Anspruch 1, worin $R^1$ $C_{1-8}$-Alkyl, jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert und $R^2$ OH bedeutet.

**3.** Substituierte Cyclohexylmethyl-Derivate gemäß Anspruch 1, worin $R^1$ $(CH_2)_mCHN\text{-}OH$, $(CH_2)_nNR^6R^7$ oder $(CH_2)_nOR^8$ bedeutet, wobei n für 0, 1, 2 oder 3 und m für 0, 1 oder 2 steht; oder über eine $C_{1-3}$-Alkylgruppe, die gesättigt oder ungesättigt sein kann, verknüpftes $C(O)OR^9$ oder $CONR^{10}R^{11}$ bedeutet, und $R^2$ H bedeutet.

**4.** Substituierte Cyclohexylmethyl-Derivate gemäß Anspruch 1 oder 2, worin $R^1$ $C_{1-8}$-Alkyl, jeweils verzweigt oder unverzweigt, gesättigt oder ungesättigt, unsubstituiert oder einfach oder mehrfach substituiert mit Methyl, =O, Phenyl oder $CO_2CH_3$; Phenyl, Naphthyl, Benzyl, Phenethyl, 2-Pyridyl oder 2-Thienyl, unsubstituiert oder einfach oder mehrfach substituiert mit F, $CH_3$, Cl, tert.-Butyl, Methoxy oder $CF_3$; Cyclohexyl oder Cyclopentyl bedeutet.

**5.** Substituierte Cyclohexylmethyl-Derivate gemäß Anspruch 1 oder 2, worin $R^1$ für 2,4-Difluorphenyl, 4-Fluor-3-methylphenyl, Phenyl, 3-Methoxybenzyl, 4-Chlorphenyl, Benzyl, 2-Methylphenyl, 4-tert.-Butylphenyl, Cyclopentyl, 4-Fluorphenyl, Phenethyl, 2-Thienyl, 2,4-Dichlorphenyl, 3-Methoxyphenyl, 4-Methylphenyl, 4-Methoxyphenyl, 3,5-Difluorphenyl, Isopropyl, Butyl, Ethyl, Hexyl, sec-Butyl, 2,4,6-Trimethylphenyl, Pentyl, Propyl, 3-Fluorphenyl, 3,5-Dichlorphenyl, 4-Fluorbenzyl, 4-Chlor-3-trifluormethylphenyl, Cyclohexyl, Isobutyl oder 2,5-Dimethoxyphenyl steht.

**6.** Substituierte Cyclohexylmethyl-Derivate gemäß einem der Ansprüche 1-3, worin $R^3$ Phenyl oder Thienyl, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Phenylrest, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl, bedeutet;

**7.** Substituierte Cyclohexylmethyl-Derivate gemäß Anspruch 6, worin $R^3$ Phenyl, unsubstituiert oder einfach substituiert mit Cl oder F; Phenethyl oder Thienyl bedeutet.

**8.** Substituierte Cyclohexylmethyl-Derivate gemäß einem der Ansprüche 1-3, worin $R^4$ und $R^5$ für H oder $CH_3$ stehen, wobei $R^4$ und $R^5$ nicht gleichzeitig H bedeuten.

**9.** Substituierte Cyclohexylmethyl-Derivate gemäß einem der Ansprüche 1-3, worin $R^4$ und $R^5$ zusammen $(CH_2)_{3-6}$ bedeuten.

**10.** Substituierte Cyclohexylmethyl-Derivate gemäß einem der Ansprüche 1 oder 3 worin $R^6$ H; $C_{1-8}$-Alkyl, gesättigt

oder ungesättigt, verzweigt oder unverzweigt, unsubstituiert oder einfach oder mehrfach substituiert mit F, Cl, -CN, SH, $SCH_3$, $OCH_3$, OH, =O, $CO_2C_2H_5$ oder $CO_2CH_3$; Aryl, unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl-, oder einen über eine $C_{1-4}$-Alkylkette verknüpften Aryl- oder Heteroarylrest, jeweils unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl, wobei die Alkylkette verzweigt oder unverzweigt, unsubstituiert oder einfach oder mehrfach substituiert mit $CO_2C_2H_5$ oder $CO_2CH_3$ sein kann, bedeutet;

**11.** Substituierte Cyclohexylmethyl-Derivate gemäß Anspruch 10, worin $R^6$ 2-Indolylethyl, Phenethyl, 3-Phenylpropyl, Benzyl, Phenyl, 4-Phenylbutyl, 1-(1H-Indol-3-yl)propan-2-yl, 4 2-(3-Indolyl)propionsäuremethylester, jeweils unsubstituiert oder einfach oder mehrfach substituiert mit F oder $OCH_3$, bedeutet.

**12.** Substituierte Cyclohexylmethyl-Derivate gemäß gemäß Anspruch 10, worin $R^6$ H bedeutet.

**13.** Substituierte Cyclohexylmethyl-Derivate gemäß einem der Ansprüche1 oder 3 worin $R^7$ C(O)$R^{13}$ bedeutet.

**14.** Substituierte Cyclohexylmethyl-Derivate gemäß einem der Ansprüche 1 oder 3 worin $R^8$ H; einen über eine $C_{1-4}$-Alkylgruppe verknüpften Phenylrest, unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl, bedeutet.

**15.** Substituierte Cyclohexylmethyl-Derivate gemäß Anspruch 14, worin $R^8$ Benzyl, unsubstituiert oder einfach oder mehrfach substituiert mit F bedeutet.

**16.** Substituierte Cyclohexylmethyl-Derivate gemäß einem der Ansprüche 1 oder 3, worin $R^9$ $C_{1-8}$-Alkyl, verzweigt oder unverzweigt.

**17.** Substituierte Cyclohexylmethyl-Derivate gemäß einem der Ansprüche 1 oder 3, worin $R^{10}$ und $R^{11}$ unabhängig voneinander H; Phenyl, Naphthyl oder einen über eine $C_{1-4}$-Alkylkette verknüpften Phenyl- oder Indolylrest, jeweils unsubstituiert oder substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl.

**18.** Cyclohexylmethyl-Derivate gemäß Anspruch 17, worin $R^{10}$ und $R^{11}$ unabhängig voneinander H; Naphthyl, Phenyl oder Benzyl, jeweils unsubstituiert oder einfach oder mehrfach substituiert mit $CF_3$, F, $NO_2$ oder Br; oder Cyclohexyl, wobei $R^{10}$ und $R^{11}$ nicht gleichzeitig H bedeuten.

**19.** Cyclohexylmethyl-Derivate gemäß einem der Ansprüche 1 oder 3, worin $R^{12}$ Naphthyl, Phenyl oder Benzyl, jeweils unsubstituiert oder einfach oder mehrfach substituiert mit F, Cl, Br, CN, $NH_2$, $NO_2$, SH, $SCH_3$, OH, $OCH_3$, $CF_3$, Methyl, Ethyl, Propyl, Butyl oder tert.-Butyl bedeutet.

**20.** Substituierte Cyclohexylmethyl-Derivate gemäß einem der Ansprüche 1 oder 3, worin $R^{13}$ H; $C_{1-8}$-Alkyl, gesättigt oder ungesättigt, verzweigt oder unverzweigt, unsubstituiert oder einfach oder mehrfach substituiert mit F, Cl, -CN, NH-$C_{1-6}$-Alkyl, $C_{1-6}$-Alkyl, N($C_{1-6}$-Alkyl)$_2$, SH, S-$C_{1-6}$-Alkyl, S-Benzyl, O-$C_{1-6}$-Alkyl, OH, =O, O-Benzyl, C(=O)$C_{1-6}$-Alkyl, $CO_2H$, $CO_2$-$C_{1-6}$-Alkyl, Phenyl oder Benzyl; $C_{3-10}$-Cycloalkyl, unsubstituiert oder einfach oder mehrfach substituiert mit F, Cl, -CN, NH-$C_{1-6}$-Alkyl, $C_{1-6}$-Alkyl, N($C_{1-6}$-Alkyl)$_2$, SH, S-$C_{1-6}$-Alkyl, $C_{1-6}$-Alkyl, S-Benzyl; O-$C_{1-6}$-Alkyl, OH, =O, O-Benzyl, C(=O)$C_{1-6}$-Alkyl, $CO_2H$, $CO_2$-$C_{1-6}$-Alkyl, Phenyl oder Benzyl; Aryl oder Heteroaryl, jeweils unsubstituiert oder einfach oder mehrfach substituiert mit F, Cl, Br, CN, $NH_2$, NH-$C_{1-6}$-Alkyl, N($C_{1-6}$Alkyl)$_2$, $NO_2$, SH, Pyridyl, S-$C_{1-6}$-Alkyl, S-Phenyl, OH, $(CH_2)_{0-30}$O-$C_{1-6}$-Alkyl, $C_{1-3}$-Alkyl-$C_{3-6}$-Cycloalkyl, O-$C_{1-6}$Alkyl-OH, O-Phenyl, Phenyl, Benzyl, C(=O)$C_{1-6}$-Alky), $CO_2H$, $CO_2$-$C_{1-6}$-Alkyl. $CF_3$, $C_{1-6}$-Alkyl, Dihydrobenzofuran, $SO_2$Phenyl oder $SO_2C_{1-6}$-Alkyl; oder einen über eine $C_{1-4}$-Alkylkette verknüpften Aryl- oder Heteroarylrest, jeweils unsubstituiert oder einfach oder mehrfach substituiert mit F, Cl, Br, CN, $NH_2$, NH-$C_{1-6}$-Alkyl, N($C_{1-6}$Alkyl)$_2$, $NO_2$, SH, Pyridyl, S-$C_{1-6}$-Alkyl, S-Phenyl, OH, O-$C_{1-6}$-Alkyl, O-$C_{1-6}$Alkyl-OH, O-Phenyl, Phenyl, Benzyl, C(=O)$C_{1-6}$-Alkyl, $CO_2H$, $CO_2$-$C_{1-6}$-Alkyl, $CF_3$, $C_{1-6}$-Alkyl, $SO_2$Phenyl oder $SO_2C_{1-6}$-Alkyl, wobei die Alkylkette verzweigt oder unverzweigt, unsubstituiert oder einfach oder mehrfach substituiert mit F, Cl, -CN, SH, S-$C_{1-6}$-Alkyl, S-Benzyl, O-$C_{1-6}$-Alkyl, OH, O-$C_{1-6}$-Alkyl-OH, O-Benzyl, $CO_2$-$C_{1-6}$-Alkyl, Phenyl oder Benzyl sein kann; bedeutet;

**21.** Substituierte Cyclohexylmethyl-Derivate gemäß Anspruch 20, worin $R^{13}$ Methyl, Ethyl, Phenyl, Benzyl, 3-Pentyl, n-Propyl, Benzothienyl, 1-(4-Chlorphenyl)-cyclopentyl, 4-Propylphenyl, 3-Cyanophenyl, 3-Chlorphenyl, 5-Chlor-4-methoxy-thiophen-3-yl, 3-Fluor-5-trifluormethylphenyl, 4-Fluor-5-trifluormethylphenyl, 2-Thienyl, 3,5-Dichlorphenyl,

2,4,5-Trifluorphenyl, 3-Bromphenyl, 4-Methylphenyl, 3-Methoxyphenyl, 2,2-Dimethylpropyl, 2-tert-Butyl-5-methyl-pyrazol-3-yl, 2,4-Dimethoxyphenyl, 3-Trifluormethylphenyl, 3,5-Difluorphenyl, 2-Fluor-5-trifluormethylphenyl, 4-Chlorbenzyl, 2-Methoxyphenyl, 2-Methylsulfanyl-3-pyridyl, 3,4,5-Trimethoxyphenyl, 2-Ethylsulfanyl-3-pyridyl, 2-Methyl-5-phenyl-furan-3-yl, 1-Phenoxyethyl, tert.-Butylphenyl, 2-(4-Chlor-phenylsulfanyl)-3-pyridyl, 2-p-Tolyloxy-3-pyridyl, 3-Chlor-4-(sulfonyl-2-propyl)-thiophen-2-yl, 5-Methylisoxazol-3-yl, 5-Brom-3-pyridyl, Naphthyl, 2-Methyl-5-(4-chlor-phenyl)-furan-3-yl, 4-(4-Chlor-phenylsulfonyl)-3-methyl-thiophen-2-yl, 1-Phenylpropyl, Adamantyl, 2-Phenyl-thiazol-4-yl, 4-Methyl-2-phenyl-thiazol-5-yl, 2-(2,3-Dihydro-benzofuran-5-yl)-thiazol-4-yl, 3-Methylphenyl, 3-Chlor-4-methylsulfonyl-thiophen-2-yl, Benzyloxymethyl, Methylthienyl, 4-Brom-2-ethyl-5-methyl-pyrazol-3-yl, 2,5-Dimethylfuryl, 5-Pyridin-2-yl-thiophen, 3-Chlor-4-fluorphenyl, Cyclohexyl, 3-Nitrophenyl, 2,5-Difluorphenyl, 2,6-Difluorphenyl, 2-Trifluormethyl-5-fluor-phenyl, 4-Chlorphenoxy-methyl, 2-Bromphenyl, Cyclopentyl, Benzothiadiazolyl, Diphenylmethyl, 2-Methylphenyl, 3-Methoxybenzyl, 2,4,6-Trichlorphenyl, 2-Butyl, 2-Chlorphenyl, 3,5-Dinitrophenyl, 4-Cyanophenyl, 2,4-Dichlor-5-fluorphenyl, 2-Chlor-3-pyridyl, 4-Nitrophenyl, 2,3,4,5,6-Pentafluorphenyl oder 3-(2,6-Dichlor-phenyl)-5-methyl-isoxazol-4-yl, 5-Chlor-4-methylthiophen-3-yl, 4-Fluorphenyl, 4-Chlorphenyl, 2-Fluorphenyl, 3-Methylphenyl, 3-Bromphenyl, 2,6-Dichlorphenyl, 3,4-Dichlorphenyl, 4-Cyanophenyl, 2,4-Difluorphenyl, 2,4-Dichlorphenyl, 2,4-Dichlor-5-fluorphenyl, 2-Chlorpyridin-3-yl, 3,5-Dimethoxyphenyl, 2,6-Dimethoxyphenyl, 2,3,6-Trifluorphenyl, 2-(4-Chlorphenoxy)-3-pyridyl, 3,4-Difluorphenyl, 2-(2,3-Dihydro-benzofuran-5-yl)-4-methyl-thiazol-5-yl, 3-Methyl-oxadiazolyl, 3-Phenyl-oxadiazolyl, 3-Cyclopropylmethyl-oxadiazolyl, 3-Methoxymethyl-oxadiazolyl oder 2,4-Dimethoxyphenyl bedeutet.

**22.** Substituierte Cyclohexylmethylderivate gemäß Anspruch 1 aus der Gruppe

(16) 4-(Dimethylamino-phenyl-methyl)-cyclohexanon-oxim
(17) 4-(Dimethylamino-phenyl-methyl)-cyclohexylamin
(18) 4-[Dimethylamino-(4-fluorphenyl)-methyl]-cyclohexanon-oxim
(19) 4-[Dimethylamino-(4-fluorphenyl)-methyl]-cyclohexylamin
(20) 4-[Dimethylamino-(3-fluorphenyl)-methyl]-cyclohexanon-oxim
(21) 4-[Dimethylamino-(3-fluorphenyl)-methyl]-cyclohexylamin
(22) 4-[(4-Chlorphenyl)-dimethylamino-methyl]-cyclohexanonoxim
(23) 4-[(4-Chlorphenyl)-dimethylamino-methyl]-cyclohexylamin
(24) 4-(Dimethylamino-thiophen-2-yl-methyl)-cyclohexanonoxim
(25) 4-(Dimethylamino-thiophen-2-yl-methyl)-cyclohexylamin
(26) 4-(1-Dimethylamino-3-phenyl-propyl)-cyclohexanon oxim
(27) 4-(1-Dimethylamino-3-phenyl-propyl)-cyclohexylamin
(29) 4-(Dimethylamino-phenyl-methyl)-cyclohexan-carbaldehyd-oxim
(30) [(4-Aminomethyl-cyclohexyl)-phenyl-methyl]-dimethylamin
(32) 4-[Dimethylamino-(4-fluorphenyl)methyl]-cyclohexanecarbaldehydoxim
(33) [(4-Aminomethyl-cyclohexyl)-(4-fluorphenyl)-methyl]-dimethylamin
(35) 4-[Dimethylamino-(3-fluorphenyl)-methyl]-cyclohexanecarbaldehydoxim
(36) [(4-Aminomethyl-cyclohexyl)-(3-fluorphenyl)-methyl]-dimethylamin
(38) 4-[(4-Chlorphenyl)-dimethylamino-methyl]-cyclohexanecarbaldehydoxim
(39) [(4-Aminomethyl-cyclohexyl)-(4-chlorphenyl)-methyl]-dimethylamin
(41) 4-(Dimethylamino-thiophen-2-yl-methyl)-cyclohexanecarbaldehydoxim
(42) [(4-Aminomethyl-cyclohexyl)-thiophen-2-yl-methyl]-dimethylamin
(44) 4-(1-Dimethylamino-3-phenyl-propyl)-cyclohexanecarbaldehydoxim
(45) [1-(4-Aminomethyl-cyclohexyl)-3-phenyl-propyl]-dimethylamin
(47) [4-(Dimethylamino-phenyl-methyl)-cyclohexyl]-acetaldehyd-oxim
(48) 2-[4-Dimethylamino-phenyl-methyl)-cyclohexyl]-ethylamin
(50) {4-[Dimethylamino-(4-fluorphenyl)-methyl]-cyclohexyl}-acetaldehydoxim
(51) 2-{4-[Dimethylamino-(4-fluorphenyl)-methyl]-cyclohexyt}-ethylamin
(53) {4-[Dimethylamino-(3-fluorphenyl)-methyl]-cyclohexyl}-acetaldehydoxim
(54) 2-{4-[Dimethylamino-(3-fluorphenyl)-methyl]-cyclohexyl}-ethylamin
(56) {4-[Dimethylamino-(4-chlorphenyl)-methyl]-cyclohexyl}-acetaldehydoxim
(66) 2-{4-[Dimethylamino-(4-chlorphenyl)-methyl]-cyclohexyl}-ethylamin
(68) 2-(4-((dimethylamino)(thiophen-2-yl)methyl)cyclohexyl)acetaldehydoxim
(69) 2-[4-(Dimethylamino-thiophen-2-yl-methyl)-cyclohexyl]-ethylamin
(71) [4-(1-Dimethylamino-3-phenyl-propyl)-cyclohexyl]-acetaldehydeoxim
(72) {1-[4-(2-Amino-ethyl)-cyclohexyl]-3-phenyl-propyl}-dimethylamin
(111) 4-[Dimethylamino-phenyl-methyl]-cyclohexanol

(112) 4-[Dimethylamino-(4-fluorphenyl)-methyl]-cyclohexanol
(113) 4-[Dimethylamino-(3-fluorphenyl)-methyl]-cyclohexanol
(114) 4-[(4-Chlorphenyl)-dimethylamino-methyl]-cyclohexanol
(115) 4-(Dimethylamino-thiophen-2-yl-methyl)-cyclohexanol
(116) 4-(1-Dimethylamino-3-phenyl-propyl)-cyclohexanol
(117) [4-(Dimethylamino-phenyl-methyl)-cyclohexyl]-methanol
(118) {4-[Dimethylamino-(4-fluorphenyl)-methyl]-cyclohexyl}-methanol
(119) {4-[Dimethylamino-(3-fluorphenyl)-methyl]-cyclohexyl}-methanol
(120) {4-[(4-Chlorphenyl)-dimethylamino-methyl]-cyclohexyl}-methanol
(121) [4-(Dimethylamino-thiophen-2-yl-methyl)-cyclohexyl]-methanol
(122) [4-(1-Dimethylamino-3-phenyl-propyl)-cyclohexyl]-methanol
(123) [4-(Dimethylamino-phenyl-methyl)-cyclohexyliden]-essigsäure-ethylester
(124) [4-(Dimethylamino-phenyl-methyl)cyclohexyl]-essigsäure-ethylester
(125) 2-[4-(Dimethylamino-phenyl-methyl)-cyclohexyl]-ethanol
(126) {4-[Dimethylamino-(4-fluorphenyl)-methyl]-cyclohexyliden}-essigsäure-ethylester
(127) {4-[Dimethylamino-(4-fluorphenyl)-methyl]-cyclohexyl}-essigsäure-ethylester
(128) 2-{4-(Dimethylamino-(4-fluorphenyl)-methyl]-cyclohexyl}-ethanol
(129) {4-[Dimethylamino-(3-fluorphenyl)-methyl]-cyclohexyliden}-essigsäure-ethylester
(130) {4-[Dimethylamino-(3-fluorphenyl)-methyl]-cyclohexyl}-essigsäure-ethylester
(131) 2-{4-[Dimethylamino-(3-fluorphenyl)-methyl]-cyclohexyl}-ethanol
(132) 3-[4-(Dimethylamino-phenyl-methyl)-cyclohexyl]-acrylsäure-ethylester
(133) 3-[4-(Dimethylamino-phenyl-methyl)-cyclohexyl]-propionsäureethylester
(134) 3-[4-(Dimethylamino-phenyl-methyl)-cyclohexyl]-propan-1-ol
(135) 3-{4-[Dimethylamino-(4-fluorphenyl)-methyl]-cyclohexyl}-acrylsäureethylester
(136) 3-{4-[Dimethylamino-(4-fluorphenyl)-methyl]-cyclohexyl}-propionsäureethyl-ester
(137) 3-{4-[Dimethylamino-(4-fluorphenyl)-methyl]-cyclohexyl}-propan-1-ol
(138) 3-{4-[Dimethylamino-(3-fluorphenyl)-methyl]-cyclohexyl}-acrylsäure-ethylester
(139) 3-{4-[Dimethylamino-(3-fluorphenyl)-methyl]-cyclohexyl}-propionsäureethyl-ester
(140) 3-{4-[Dimethylamino-(3-fluorphenyl)-methyl]-cyclohexyl}-propan-1-ol
(141) 3-[4-(1-Dimethylamino-3-phenyl-propyl)-cyclohexyl]-acrylsäureethylester
(142) 3-[4-(1-Dimethylamino-3-phenyl-propyl)-cyclohexyl]-propionsäureethylester
(143) 3-[4-(1-Dimethylamino-3-phenyl-propyl)-cyclohexyl]-propan-1-ol
(73) 1-(4-((Dimethylamino)(phenyl)methyl)cyclohexyl)-3-(naphthalen-1-yl)harnstoff
(74) 1-(2,4-Difluorphenyl)3-(4-((dimethylamino)(phenyl)methyl)cyclohexyl)harnstoff Hydrochlorid
(75) 1-(4-((Dimethylamino)(phenyl)methyl)cyclohexyl)-3-(3-(trifluormethyl)phenyl)harnstoff Hydrochlorid
(76) 1-(4-((Dimethylamino)(phenyl)methyl)cyclohexyl)-3-(2-nitrophenyl)harnstoff Hydrochlorid
(77) 1-(3-Bromphenyl)-3-(4-((dimethylamino)(phenyl)methyl)cyclohexyl)harnstoff Hydrochlorid
(78) 1-(4-((Dimethylamino)(phenyl)methyl)cyclohexyl)-3-phenylharnstoff Hydrochlorid
(79) 1-Benzyl-3-(4-((dimethylamino)(phenyl)methyl)cyclohexyl)harnstoff
(80) 1-Cyclohexyl-3-(4-((dimethylamino)(phenyl)methyl)cyclohexyl)harnstoff
(81) 1-(4-Bromphenyl)-3-(4-((dimethylamino)(phenyl)methyl)cyclohexyl)harnstoff
(82) 1-(4-((Dimethylamino)(phenyl)methyl)cyclohexyl)-3-(4-methoxyphenyl)harnstoff
(83) N-(2-(1H-Indol-3-yl)ethyl)-4-((dimethylamino)(phenyl)methyl)cyclohexanamin hydrochlorid
(84) 4-((Dimethylamino)(phenyl)methyl)-N-phenethylcyclohexanamin Hydrochlorid
(85) 4-((Dimethylamino)(phenyl)methyl)-N-(3-phenylpropyl)cyclohexanamin Dihydrochlorid
(86) N-Benzyl-4-((dimethylamino)(phenyl)methyl)cyclohexanamin Hydrochlorid
(87) 4-((Dimethylamino)(phenyl)methyl)-N-(4-phenylbutyl)cyclohexanamin Hydrochlorid
(88) N-(1-(1H-Indol-3-yl)propan-2-yl)-4-((dimethylamino)(phenyl)methyl)-cyclohexanamin Hydrochlorid .
(89) N-(4-((Dimethylamino)(phenyl)methyl)cyclohexyl)-4-methoxybenzenamin Hydrochlorid
(90) 4-((Dimethylamino)(phenyl)methyl)-N-(4-methoxybenzyl)cyclohexanamin Dihydrochlorid
(91) 4-((Dimethylamino)(phenyl)methyl)N-(4-fluorbenzyl)cyclohexanamin Hydrochlorid
(92) N-(4-((Dimethylamino)(phenyl)methyl)cyclohexyl)benzenamin Hydrochlorid
(93) N-(4-((Dimethylamino)(phenyl)methyl)cyclohexyl)-2-ethylbutanamid Hydrochlorid
(94) N-(4-((Dimethylamino)(phenyl)methyl)cyclohexyl)benzamid Hydrochlorid
(95) N-(4-((Dimethylamino)(phenyl)methyl)cyclohexyl)-N-(3-phenylpropyl)acetamid Hydrochlorid
(96) N-(4-((Dimethylamino)(phenyl)methyl)cyclohexyl)-N-phenylacetamid Hydrochlorid
(97) N-(4-((Dimethylamino)(phenyl)methyl)cyclohexyl)-N-(4-phenylbutyl)propionamid Hydrochlorid
(98) N-(4-((Dimethylamino)(phenyl)methyl)cyclohexyl)-N-(4-phenylbutyl)acetamid Hydrochlorid

(99) N-(4-((Dimethylamino)(phenyl)methyl)cyclohexyl)-N-(4-methoxyphenyl)acetamid Hydrochlorid
(100) N-Benzyl-N-(4-((dimethylamino)(phenyl)methyl)cyclohexyl)acetamid Hydrochlorid
(101) N-Benzyl-N-(4-((dimethylamino)(phenyl)methyl)cyclohexyl)-2-ethylbutanamid Hydrochlorid
(102) N-Benzyl-N-(4-((dimethylamino)(phenyl)methyl)cyclohexyl)butyramid Hydrochlorid
(103) N-Benzyl-N-(4-((dimethylamino)(phenyl)methyl)cyclohexyl)-4-fluorbenzamid Hydrochlorid
(104) N-Benzyl-N-(4-((dimethylamino)(phenyl)methyl)cyclohexyl)benzamid Hydrochlorid
(105) N-(4-((Dimethylamino)(phenyl)methyl)cyclohexyl)-2-ethyl-N-phenylbutanamid Hydrochlorid
(106) -Chlor-N-(4-((dimethylamino)(phenyl)methyl)cyclohexyl)benzolsulfonamid Hydrochlorid
(107) N-(4-((Dimethylamino)(phenyl)methyl)cyclohexyl)-4-methoxybenzolsulfonamid Hydrochlorid
(108) 4-tert-Butyl-N-(4-((dimethylamino)(phenyl)methyl)cyclohexyl)benzolsulfonamid Hydrochlorid
(109) N-(4-((Dimethylamino)(phenyl)methyl)cyclohexyl)-2-nitrobenzolsulfonamid Hydrochlorid
(110) N-(4-((Dimethylamino)(phenyl)methyl)cyclohexyl)benzolsulfonamid Hydrochlorid
(144) 4-(benzyloxy)cyclohexyl)-N,N-dimethyl(phenyl)methanamin Hydrochlorid
(145) 4-(4-Fluorbenzyloxy)cyclohexyl)-N,N-dimethyl(phenyl)methanamin Hydrochlorid
(146) trans-N,N-dimethyl(4-phenethylcyclohexyl)(phenyl)methanamin Hydrochlorid
(147) 1-Benzyl-4-((dimethylamino)(phenyl)methyl)cyclohexanol Hydrochlorid
(148) 4-((dimethylamino)(phenyl)methyl)-1-(4-fluorbenzyl)cyclohexanol Hydrochlorid
(149) 1-(2,5-Dimethoxyphenyl)-4-((dimethylamino)(phenyl)methyl)cyclohexanol
(150) 4-((Dimethylamino)(phenyl)methyl)-1-(4-fluor-3-methylphenyl)cyclohexanol
(151 ) -(Dimethylamino-phenyl-methyl)-1-(4-fluor-3-methyl-phenyl)-cyclohexanol
(152) 1-Benzyl-4-[dimethylamino-(3-fluor-phenyl)-methyl]-cyclohexanol
(153) 4-[Dimethylamino-(3-fluor-phenyl)-methyl]-1-phenethyl-cyclohexanol
(154) 4-[Dimethylamino-(3-fluor-phenyl)-methyl]-1-pentyl-cyclohexanol
(155) 1-(3,5-Dichlor-phenyl)-4-[dimethylamino-(3-fluor-phenyl)-methyl]-cyclohexanol
(156) 4-[Dimethylamino-(3-fluor-phenyl)-methyl]-1-(3-methoxy-benzyl)-cyclohexanol
(157) 1-(4-Chlor-3-trifluormethyl-phenyl)-4-[dimethylamino-(3-fluor-phenyl)-methyl]-cyclohexanol
(158) 4-[(4-Chlor-phenyl)-dimethylamino-methyl]-1-phenyl-cyclohexanol
(159) 1-Benzyl-4-[(4-chlor-phenyl)-dimethylamino-methyl]-cyclohexanol
(160) 4-[(4-Chlor-phenyl)-dimethylamino-methyl]-1-(4-fluor-3-methyl-phenyl)-cyclohexanol
(161) 4-[(4-Chlor-phenyl)-dimethylamino-methyl]-1-o-tolyl-cyclohexanol
(162) 4-[(4-Chlor-phenyl)-dimethylamino-methyl]-1-(4-fluor-phenyl)-cyclohexanol
(163) 4-[(4-Chlor-phenyl)-dimethylamino-methyl]-1-phenethyl-cyclohexanol
(164) 4-[(4-Chlor-phenyl)-dimethylamino-methyl]-1-(3-methoxy-phenyl)-cyclohexanol
(165) 4-[(4-Chlor-phenyl)-dimethylamino-methyl]-1-p-tolyl-cyclohexanol
(166) 4-[(4-Chlor-phenyl)-dimethylamino-methyl]-1-(3,5-difluor-phenyl)-cyclohexanol
(167) 1-Butyl-4-[(4-chlor-phenyl)-dimethylamino-methyl]-cyclohexanol
(168) 4-[(4-Chlor-phenyl)-dimethylamino-methyl]-1-hexyl-cyclohexanol
(169) 4-[(4-Chlor-phenyl)-dimethylamino-methyl]-1-pentyl-cyclohexanol (polareres Diastereomer)
(170) 4-[(4-Chlor-phenyl)-dimethylamino-methyl]-1-pentyl-cyclohexanol (unpolareres Diastereomer)
(171) 4-[(4-Chlor-phenyl)-dimethylamino-methyl]-1-(3-fluor-phenyl)-cyclohexanol
(172) 4-[(4-Chlor-phenyl)-dimethylamino-methyl]-1-(4-fluor-benzyl)-cyclohexanol
(173) 4-[(4-Chlor-phenyl)dimethylamino-methyl]1-(3-methoxy-benzyl)-cyclohexanol
(174) Methyl 2-(4-((dimethylamino)(phenyl)methyl)cyclohexylamino)-3-(1 H-indol-3-yl)propanoat (polareres Diastereomer)
(175) Methyl2-(4-((dimethylamino)(phenyl)methyl)cyclohexylamino)-3-(1H-indol-3-yl)propanoat (unpolareres Diastereomer)
(176) N-(4-((Dimethylamino)(phenyl)methyl)cyclohexyl)-N-(4-methoxybenzyl)acetamid
(177) N-(1-(1H-indol-3-yl)propan-2-yl)-N-(4-(dimethylamino)(phenyl)methyl)cyclohexyl)acetamid (polareres Diastereomer)
178) N-(1-(1 H-indol-3-yl)propan-2-yl)-N-(4-((dimethylamino)(phenyl)methyl)cyclohexyl)acetamid (unpolareres Diastereomer)
(179) N-(4-((Dimethylamino)(phenyl)methyl)cyclohexyl)-N-(4-fluorbenzyl)acetamid
(180) N-(4-((Dimethylamino)(phenyl)methyl)cyclohexyl)-N-phenylbutyramid
(181) N-(2-(1H-indol-3-yl)ethyl)-N-(4-((dimethylamino)(phenyl)methyl)-cyclohexyl)butyramid
(182) N-(2-(1H-indol-3-yl)ethyl)-N-(4-((dimethylamino)(phenyl)methyl)cyclohexyl)acetamid
(183) Benzo[b]thiophen-3-carbonsäure-[4-(dimethylamino-phenyl-methyl)-cyclohexyl]-amid
(184) 1-(4-Chlor-phenyl)-cyclopentancarbonsäure-[4-(dimethylamino-phenyl-methyl)-cyclohexyl]-amid
(185) N-[4-(Dimethylamino-thiophen-2-yl-methyl)-cyclohexyl]-4-propyl-benzamid

(186) 3-Cyano-N-[4-(dimethylamino-thiophen-2-yl-methyl)-cyclohexyl]-benzamid
(187) 3-Chlor-N-[4-(dimethylamino-phenyl-methyl)-cyclohexyl]-benzamid
(188) 5-Chlor-4-methoxy-thiophen-3-carbonsäure-[4-(dimethylamino-phenyl-methyl)-cyclohexyl]-amid
(189) 3,4-Dichlor-N-[4-(dimethylamino-phenyl-methyl)-cyclohexyl]-benzamid
(190) N-[4-(Dimethylamino-phenyl-methyl)cyclohexyl]-3-fluor-5-trifluormethyl-benzamid
(191) 5-Chlor-4-methoxy-thiophen-3-carbonsäure-{4-[dimethylamino-(3-fluor-phenyl)-methyl]-cyclohexyl}-amid
(192) N-[4-(Dimethylamino-thiophen-2-yl-methyl)-cyclohexyl]-4-fluor-3-trifluormethyl-benzamid
(193) Thiophen-2-carbonsäure-[4-(dimethylamino-thiophen-2-yl-methyl)-cyclohexyl]-amid
(194) 3,5-Dichlor-N-[4-(dimethylamino-thiophen-2-yl-methyl)-cyclohexyl]-benzamid
(195) 5-Chlor-4-methoxy-thiophen-3-carbonsäure-[4-(dimethylamino-thiophen-2-yl-methyl)-cyclohexyl]-amid
(196) N-[4-(Dimethylamino-phenyl-methyl)-cyclohexyl]-2,4,5-trifluor-benzamid
(197) 3-Brom-N-[4-(dimethylamino-phenyl-methyl)-cyclohexyl]-benzamid
(198) N-[4-(Dimethylamino-thiophen-2-yl-methyl)-cyclohexyl]-4-methyl-benzamid
(199) N-[4-(Dimethylamino-thiophen-2-yl-methyl)-cyclohexyl]-3-methoxy-benzamid
(200) N-[4-(Dimethylamino-thiophen-2-yl-methyl)-cyclohexyl]-3,3-dimethyl-butyramid
(201) 2-tert-Butyl-5-methyl-2H-pyrazol-3-carbonsäure-[4-(dimethylamino-thiophen-2-yl-methyl)cyclohexyl]-amid
(202) N-[4-(Dimethylamino-thiophen-2-yl-methyl)-cyclohexyl]-2 ,4-dimethoxy-benzamid
(203) N-[4-(Dimethylamino-phenyl-methyl)cyclohexyl]-3-trifluormethyl-benzamid
(204) N-{4-[Dimethylamino-(3-fluor-phenyl)-methyl]-cyclohexyl}-3,5-difluor-benzamid
(205) N-[4-(Dimethylamino-phenyl-methyl)-cyclohexyl]-2-fluor-5-trifluormethyl-benzamid
(206) 2-(4-Chlor-phenyl)N-[4-(dimethylamino-phenyl-methyl)cyclohexyl]-acetamid
(207) N-[4-(Dimethylamino-thiophen-2-yl-methyl)-cyclohexyl]-2-methoxy-benzamid
(208) N-[4-(Dimethylamino-thiophen-2-yl-methyl)-cyclohexyl]-2-methylsulfanyl-nicotinamid
(209) 3,4-Dichlor-N-{4-[dimethylamino-(3-fluor-phenyl)-methyl]-cyclohexyl}-benzamid
(210) N-[4-(1-Dimethylamino-3-phenyl-propyl)-cyclohexyl]-4-fluor-3-trifluormethyl-benzamid (polareres Diastereomer)
(211) 5-Chlor-4-methoxy-thiophen-3-carbonsäure-{4-[dimethylamino-(4-fluor-phenyl)-methyl]-cyclohexyl}-amid
(212) N-[4-(Dimethylamino-thiophen-2-yl-methyl)-cyclohexyl]-3,4,5-trimethoxy-benzamid
(213) N-[4-(Dimethylamino-thiophen-2-yl-methyl)-cyclohexyl]-2-ethylsulfanyl-nicotinamid
(214) 2-Methyl-5-phenyl-furan-3-carbonsäure-[4-(dimethylamino-phenyl-methyl)-cyclohexyl]-amid
(215) N-{4-[Dimethylamino-(3-fluor-phenyl)-methyl]-cyclohexyl}-2-phenoxy-propionamid unpolareres Diastereomer)
(216) N-[4-(Dimethylamino-phenyl-methyl)-cyclohexyl]-2,4-dimethoxy-benzamid
(217) 4-tert-Butyl-N-[4-(dimethylamino-phenyl-methyl)-cyclohexyl]-benzamid
(218) 2-(4-Chlor-phenylsulfanyl)-N-[4-(dimethylamino-thiophen-2-yl-methyl)-cyclohexyl]-nicotinamid
(219) 2-(4-Chlor-phenyl)-N-[4-(dimethylamino-thiophen-2-yl-methyl)-cyclohexyl]-acetamid
(220) N-[4-(Dimethylamino-thiophen-2-yl-methyl)-cyclohexyl]-2-p-totyloxy-nicotinamid
(221) 3-Chlor-4-(propan-2-sulfonyl)-thiophen-2-carbonsäure-[4-(dimethylamino-thiophen-2-yl-methyl)-cyclohexyl]-amid
(222) N-{4-[Dimethylamino-(3-fluor-phenyl)-methyl]-cyclohexyl}-2-phenoxy-propionamid (polareres Diastereomer)
(223) 2-tert-Butyl-5-methyl-2H-pyrazol-3--carbonsäure-[4-(dimethylamino-phenyl-methyl)-cyclohexyl]-amid
(224) 5-Methyl-isoxazol-3-carbonsäure-[4-(dimethylamino-thiophen-2-yl-methyl)-cyclohexyl]-amid
(225) 5-Brom-N-[4-(dimethylamino-thiophen-2-yl-methyl)-cyclohexyl]-nicotinamid
(226) Naphthyl-1-carbonsäure-[4-(dimethylamino-phenyl-methyl)-cyclohexyl]-amid
(227) N-[4-(1-Dimethylamino-3-phenyl-propyl)-cyclohexyl]-4-fluor-3-trifluormethyl-benzamid (unpolareres Diastereomer)
(228) N-[4-(Dimethylamino-phenyl-methyl)-cyclohexyl]-3,3-dimethyl-butyramid (polareres Diastereomer)
(229) 5-(4-Chlor-phenyl)-2-methyl-furan-3-carbonsäure-[4-(dimethylamino-thiophen-2-yl-methyl)-cyclohexyl]-amid
(230) N-[4-(Dimethylamino-phenyl-methyl)-cyclohexyl]-2-phenoxy-propionamid
(231) Benzo[b]thiophen-2-carbonsäure-{4-[dimethylamino-(3-fluor-phenyl)-methyl]-cyclohexyl}-amid
(232) 5-(4-Chlor-phenyl)-2-methyl-furan-3-carbonsäure-[4-(dimethylamino-phenyl-methyl)-cyclohexyl]-amid
(233) 4-(4-Chlor-benzenesulfonyl)-3-methyl-thiophen-2-carbonsäure-[4-(dimethylamino-phenyl-methyl)-cyclohexyl]-amid

(234) N-[4-(Dimethylamino-phenyl-methyl)-cyclohexyl]-2-phenyl-butyramid (unpolareres Diastereomer)
(235) 5-Brom-N-[4-(dimethylamino-phenyl-methyl)-cyclohexyl]-nicotinamid
(236) Adamantan-1-carbonsäure-[4-(dimethylamino-phenyl-methyl)-cyclohexyl]-amid
(237) 2-Phenyl-thiazol-4-carbonsäure-[4-(dimethylamino-phenyl-methyl)-cyclohexyl]-amid
(238) 4-Methyl-2-phenyl-thiazol-5-carbonsäure-[4-(dimethylamino-phenyl-methyl)-cyclohexyl]-amid
(239) 2-(2,3-Dihydro-benzofuran-5-yl)-thiazol-4-carbonsäure-[4-(dimethylamino-thiophen-2-yl-methyl)cyclohexyl]-amid
(240) N-{4-[Dimethylamino-(3-fluor-phenyl)-methyl]-cyclohexyl}-2-phenyl-acetamid
(241) 3-Chlor-N-[4-(1-dimethylamino-3-phenyl-propyl)cyclohexyl]-benzamid
(242) N-[4-(Dimethylamino-phenyl-methyl)-cyclohexyl]-4-methyl-benzamid
(243) 3,5-Dichlor-N-{4-[dimethylamino-(3-fluor-phenyl)-methyl]-cyclohexyl}-benzamid
(244) N-[4-(Dimethylamino-phenyl-methyl)-cyclohexyl]-2,3,6-trifluor-benzamid
(245) Thiophen-2-carbonsäure-[4-(dimethylamino-phenyl-methyl)cyclohexyl]-amid (unpolareres Diastereomer)
(246) N-[4-(Dimethylamino-phenyl-methyl)-cyclohexyl]-3,3-dimethyl-butyramid (unpolareres Diastereomer)
(247) 2-tert-Butyl-5-methyl-2H-pyrazol-3-carbonsäure-[4-(dimethylamino-phenyl-methyl)-cyclohexyl]-amid
(248) N-[4-(1-Dimethylamino-3-phenyl-propyl)-cyclohexyl]-3-methyl-benzamid
(249) Thiophen-2-carbonsäure-[4-(dimethylamino-phenyl-methyl)-cyclohexyl]-amid. (polareres Diastereomer)
(250) N-[4-(Dimethylamino-phenyl-methyl)-cyclohexyl]-2-phenyl-butyramid (polareres Diastereomer)
(251) N-{4-[Dimethylamiro-(3-fluor-phenyl)-methyl]-cyclohexyl}-3,3-dimethyl-butyramid
(252) 3-Chlor-4-methanesulfonyl-thiophen-2-carbonsäure-[4-(dimethylamino-phenyl-methyl)-cyclohexyl]-amid
(253) 4-(4-Chlor-benzenesulfonyl)-3-methyl-thiophen-2-carbonsäure-[4-(dimethylamino-phenyl-methyl)-cyclohexyl]-amid
(254) 2-Benzyloxy-N-[4-(dimethylamino-phenyl-methyl)-cyclohexyl]-acetamid
(255) N-[4-(Dimethylamino-phenyl-methyl)-cyclohexyl]-2-thiophen-2-yl-acetamid
(256) 4-Methyl-2-phenyl-thiazol-5-carbonsäure-{4-[dimethylamino-(3-fluor-phenyl)-methyl]-cyclohexyl}-amid
(257) 2-(4-Chlor-phenoxy)-N-[4-(dimethylamino-thiophen-2-yl-methyl)-cyclohexyl]-nicotinamid
(258) N-[4-(1-Dimethylamino-3-phenyl-propyl)-cyclohexyl]-4-fluor-benzamid
(259) 5-Brom-N-[4-(dimethylamino-phenyl-methyl)-cyclohexyl]-nicotinamid
(260) 4-Brom-2-ethyl-5-methyl-2H-pyrazol-3-ccarbonsäure-[4-(dimethylamino-thiophen-2-yl-methyl)-cyclohexyl]-amid
(261) 3-Cyano-N-[4-(dimethylamino-phenyl-methyl)-cyclohexyl]-benzamid
(262) N-{4-[Dimethylamino-(4-fluor-phenyl)methyl]-cyclohexyl}-4-fluor-benzamid
(263) 3-Brom-N-{4-[dimethylamino-(4-fluor-phenyl)-methyl]-cyclohexyl}-benzamid
(264) 2-Phenyl-thiazol-4-carbonsäure-{4-[dimethylamino-(3-fluor-phenyl)-methyl]-cyclohexyl}-amid
(265) 2,5-Dimethyl-furan-3-carbonsäure-{4-[dimethylamino-(3-fluor-phenyl)-methyl]-cyclohexyl}-amid
(266) 2-Methyl-5-phenyl-furan-3-carbonsäure-{4-[dimethylamino-(3-fluor-phenyl)-methyl]-cyclohexyl}-amid
(267) 5-Pyridin-2-yl-thiophen-2-carbonsäure-{4-[dimethylamino-(3-fluor-phenyl)-methyl]-cyclohexyl}-amid
(268) 4-Brom-2-ethyl-5-methyl-2H-pyrazol-3-carbonsäure-[4-(dimethylamino-phenyl-methyl)-cyclohexyl]-amid
(269) 3-Chlor-N-{4-[dimethylamino-(3-fluor-phenyl)-methyl]-cyclohexyl}-4-fluor-benzamid
(270) 3,4-Dichlor-N-{4-[dimethylamino-(4-fluor-phenyl)-methyl]-cyclohexyl}-benzamid
(271) N-{4-[Dimethylamino-(4-fluor-phenyl)-methyl]-cyclohexyl}-2,4,5-trifluor-benzamid
(272) Cyclohexancarbonsäure-[4-(dimethylamino-phenyl-methyl)-cyclohexyl]-amid
(273) N-{4-[Dimethylamino-(3-fluor-phenyl)-methyl]-cyclohexyl}-2-phenyl-butyramid
(274) 2-(4-Chlor-phenyl)-N-{4-[dimethylamino-(3-fluor-phenyl)-methyl]-cyclohexyl}-acetamid
(275) N-[4-(Dimethylamino-phenyl-methyl)-cyclohexyl]-3-nitro-benzamid
(276) N-[4-(1-Dimethylamino-3-phenyl-propyl)-cyclohexyl]-2,5-difluor-benzamid
(277) 3-Brom-N-[4-(1-dimethylamino-3-phenyl-propyl)-cyclohexyl]-benzamid
(278) N-{4-[Dimethylamino-(3-fluor-phenyl)-methyl]-cyclohexyl}2,6-difluor-benzamid
(279) 2,5-Dimethyl-furan-3-carbonsäure-[4-(dimethylamino-phenyl-methyl)-cyclohexyl]-amid
(280) 3-Chlor-N-{4-[dimethylamino-(4-fluor-phenyl)-methyl]-cyclohexyl}-4-fluor-benzamid
(281) N-[4-(Dimethylamino-phenyl-methyl)-cyclohexyl]-5-fluor-2-trifluormethyl-benzamid
(282) 5-Methyl-isoxazol-3-carbonsäure-[4-(dimethylamino-phenyl-methyl)-cyclohexyl]-amid
(283) 2-(2,3-Dihydro-benzofuran-5-yl)-4-methyl-thiazol-5-carbonsäure-[4-(dimethylamino-thiophen-2-yl-methyl)-cyclohexyl]-amid
(284) 2-(4-Chlor-phenoxy)-N-[4-(dimethylamino-phenyl-methyl)-cyclohexyl]-acetamid
(285) 5-(4-Chlor-phenyl)-2-methyl-furan-3-carbonsäure-{4-[dimethylamino-(3-fluorphenyl)-methyl]-cyclohexyl}-amid
(286) 2-Brom-N-[4-(dimethylamino-phenyl-methyl)-cyclohexyl]-benzamid

(287) N-[4-(Dimethylamino-phenyl-methyl)-cyclohexyl]-2,6-dimethoxy-benzamid
(288) Cyclopentancarbonsäure-[4-(dimethylamino-phenyl-methyl)cyclohexyl]-amid
(289) 2-(2,3-Dihydro-benzofuran-5-yl)-thiazol-4-carbonsäure-[4-(dimethylaminophenyl-methyl)-cyclohexyl]-amid
(290) Benzo[1,2,5]thiadiazol-5-carbonsäure-{4-[dimethylamino-(3-fluor-phenyl)-methyl]-cyclohexyl}-amid
(291) N-[4-(Dimethylamino-thiophen-2-yl-methyl)-cyclohexylmethyl]-2-thiophen-2-yl-acetamid
(292) Benzo[b]thiophen-3-carbonsäure-[4-(dimethylamino-phenyl-methyl)-cyclohexylmethyl]-amid
(293) 5-Chlor-4-methoxy-thiophen-3-carbonsäure-[4-(dimethylamino-phenyl-methyl)-cyclohexylmethyl]-amid
(294) N-[4-(Dimethylamino-phenyl-methyl)-cyclohexylmethyl]-3,4-difluor-benzamid (unpolareres Diastereomer)
(295) N-{4-[Dimethylamino-(3-fluor-phenyl)-methyl]-cyclohexylmethyl}-3,3-dimethyl-butyramid
(296) 2-Brom-N-[4-(dimethylamino-thiophen-2-yl-methyl)-cyclohexylmethyl]-benzamid
(297) N-[4-(Dimethylamino-thiophen-2-yl-methyl)-cyclohexylmethyl]-2,2-diphenyl-acetamid
(298) N-[4-(Dimethylamino-phenyl-methyl)cyclohexylmethyl]-3,3-dimethyl-butyramid
(299) N-[4-(Dimethylamino-phenyl-methyl)-cyclohexylmethyl]-2-methylsulfanyl-nicotinamid
(300) N-[4-(Dimethylamino-thiophen-2-yl-methyl)-cyclohexylmethyl]-2,6-dimethoxy-benzamid
(301) N-[4-(Dimethylamino-thiophen-2-yl-methyl)-cyclohexylmethyl]-3,3-dimethyl-butyramid
(302) Benzo[b]thiophen-3-carbonsäure-[4-(dimethylamino-thiophen-2-yl-methyl)-cyclohexylmethyl]-amid
(303) 5-Chlor-4-methoxy-thiophen-3-carbonsäure-{4-[dimethylamino-(3-fluor-phenyl)-methyl]-cyclohexylmethyl}-amid
(304) N-[4-(Dimethylamino-thiophen-2-yl-methyl)-cyclohexylmethyl]-2-phenoxy-propionamid
(305) N-[4-(Dimethylamino-thiophen-2-yl-methyl)-cyclohexylmethyl]-2-methoxy-benzamid
(306) N-[4-(Dimethylamino-phenyl-methyl)-cyclohexylmethyl]-2-phenyl-acetamid
(307) 3-Brom-N-[4-(dimethylamino-thiophen-2-yl-methyl)-cyclohexylmethyl]-benzamid
(308) N-[4-(Dimethylamino-thiophen-2-yl-methyl)-cyclohexylmethyl]-3-fluor-5-trifluormethyl-benzamid
(309) N-{4-[(4-Chlor-phenyl)-dimethylamino-methyl]-cyclohexylmethyl}-3,3-dimethyl-butyramid
(310) N-[4-(Dimethylamino-phenyl-methyl)cyclohexylmethyl]-2-ethylsulfanyl-nicotinamid
(311) 2-(4-Chlor-phenyl)-N-[4-(dimethylamino-thiophen-2-yl-methyl)-cyclohexylmethyl]-acetamid
(312) N-{4-[(4-Chlor-phenyl)-dimethylamino-methyl]-cyclohexylmethyl}-2,2-diphenyl-acetamid
(313) N-[4-(Dimethylamino-thiophen-2-yl-methyl)-cyclohexylmethyl]-2,6-difluor-benzamid
(314) Benzo[b]thiophen-3-carbonsäure-{4-[dimethylamino-(3-fluor-phenyl)-methyl]-cyclohexylmethyl}-amid
(315) N-{4-[(4-Chlor-phenyl)-dimethylamino-methyl]-cyclohexylmethyl}-2-methylsulfanyl-nicotinamid
(316) N-{4-[(4-Chlor-phenyl)-dimethylamino-methyl]-cyclohexylmethyl}-2-thiophen-2-yl-acetamid
(317) N-[4-(Dimethylamino-phenyl-methyl)-cyclohexylmethyl]-2-methyl-benzamid (unpolareres Diastereomer)
(318) 1-(4-Chlor-phenyl)-cyclopentan-carbonsäure-[4-(dimethylamino-phenyl-methyl)-cyclohexylmethyl]-amid
(319) N-{4-[Dimethylamino-(3-fluor-phenyl)-methyl]-cyclohexylmethyl}-2-phenyl-acetamid (polareres Diastereomer)
(320) N-{4-[(4-Chlor-phenyl)-dimethylamino-methyl]-cyclohexylmethyl}-2-(3-methoxyphenyl)-acetamid
(321) N-[4-(Dimethylamino-phenyl-methyl)-cyclohexylmethyl]-2-phenyl-butyramid
(322) N-{4-[Dimethylamino-(4-fluor-phenyl)-methyl]-cyclohexylmethyl}-3,3-dimethyl-butyramid
(323) N-[4-(Dimethylamino-phenyl-methyl)-cyclohexylmethyl]-2-phenoxy-propionamid
(324) 2-(4-Chlor-phenyl)-N-[4-(dimethylamino-phenyl-methyl)-cyclohexylmethyl]-acetamid
(325) N-[4-(Dimethylamino-phenyl-methyl)-cyclohexylmethyl]-2-methyl-benzamid (polareres Diastereomer)
(326) N-[4-(Dimethylamino-phenyl-methyl)-cyclohexylmethyl]-3-trifluormethyl-benzamid (unpolareres Diastereomer)
(327) 1-(4-Chlor-phenyl)-cyclopentan-carbonsäure-{4-[dimethylamino-(3-fluor-phenyl)-methyl]-cyclohexylmethyl}-amid
(328) Thiophen-2-carbonsäure-[4-(dimethylamino-phenyl-methyl)-cyclohexylmethyl]-amid
(329) 3,5-Dichlor-N-[4-(dimethylamino-phenyl-methyl)-cyclohexylmethyl]-benzamid
(330) 2-Methyl-5-phenyl-furan-3-carbonsäure-{4-[(4-chlor-phenyl)-dimethylaminomethyl]-cyclohexylmethyl}-amid
(331) 3-Chlor-N-[4-(dimethylamino-phenyl-methyl)-cyclohexylmethyl]-benzamid
(332) N-{4-[Dimethylamino-(3-fluor-phenyl)-methyl]-cyclohexylmethyl}-2-phenoxy-propionamid (polareres Diastereomer)
(333) N-[4-(Dimethylamino-phenyl-methyl)-cyclohexylmethyl]-3-trifluormethyl-b enzamid (polareres Diastereomer)
(334) Thiophen-2-carbonsäure-{4-[dimethylamino-(3-fluor-phenyl)-methyl]-cyclohexylmethyl}-amid (polareres Diastereomer)

(335) 2-Phenyl-thiazol-4-carbonsäure-[4-(dimethylamino-phenyl-methyl)-cyclohexylmethyl]-amid
(336) Benzo[b]thiophen-3-carbonsäure-{4-[(4-chlor-phenyl)-dimethylamino-methyl]-cyclohexylmethyl}-amid (unpolareres Diastereomer)
(337) N-{4-[Dimethylamino-(3-fluor-phenyl)-methyl]-cyclohexylmethyl}-2-p-tolyloxy-nicotinamid
(338) 2,4,6-Trichlor-N-[4-(dimethylamino-phenyl-methyl)-cyclohexylmethyl]-benzamid
(339) 1-(4-Chlor-phenyl)-cyclopentan-carbonsäure-[4-(dimethylamino-phenyl-methyl)-cyclohexylmethyl]-amid
(340) Thiophen-2-carbonsäure-{4-[dimethylamino-(3-fluor-phenyl)-methyl]-cyclohexylmethyl}-amid (unpolareres Diastereomer)
(341) N-{4-[Dimethylamino-(3-fluor-phenyl)-methyl]-cyclohexylmethyl}-2-phenoxy-propionamid (unpolareres Diastereomer)
(342) N-{4-[Dimethylamino-(3-fluor-phenyl)-methyl]-cyclohexylmethyl}-2-methyl-butyramid (polareres Diastereomer)
(343) N-{4-[Dimethylamino-(3-fluor-phenyl)-methyl]-cyclohexylmethyl}-2-thiophen-2-yl-acetamid
(344) Benzo[b]thiophen-3-carbonsäure-{4-[dimethylamino-(4-fluor-phenyl)-methyl]-cyclohexylmethyl}-amid
(345) 2-Methyl-5-phenyl-furan-3-carbonsäure-[4-(dimethylamino-phenyl-methyl)-cyclohexylmethyl]-amid
(346) N-{4-[Dimethylamino-(3-fluor-phenyl)-methyl]-cyclohexylmethyl}-2-phenoxy-propionamid
(347) 3-Cyano-N-[4-(dimethylamino-thiophen-2-yl-methyl)-cyclohexylmethyl]-benzamid
(348) N-{4-[Dimethylamino-(3-fluor-phenyl)-methyl]-cyclohexylmethyl}-2-phenyl-acetamid (unpolareres Diastereomer)
(349) N-[4-(Dimethylamino-phenyl-methyl)cyclohexylmethyl]-2-(3-methoxy-phenyl)-acetamid
(350) N-{4-[Dimethylamino-(3-fluor-phenyl)-methyl]-cyclohexylmethyl}-4-fluor-3-t rifluormethyl-benzamid
(351) N-{4-[(4-Chlor-phenyl)-dimethylamino-methyl]-cyclohexylmethyl}-2-ethylsulfanyl-nicotinamid
(352) N-[4-(Dimethylamino-thiophen-2-yl-methyl)-cyclohexylmethyl]-2-p-tolyloxy-nicotinamid (polareres Diastereomer)
(353) 2-Methyl-5-phenyl-furan-3-carbonsäure-{4-[dimethylamino-(3-fluor-phenyl)-methyl]-cyclohexylmethyl}-amid
(354) 2-Chlor-N-{4-[(4-chlor-phenyl)-dimethylamino-methyl]-cyclohexylmethyl}-benzamid
(355) 2-Chlor-N-[4-(dimethylamino-phenyl-methyl)-cyclohexylmethyl]-nicotinamid
(356) N-[4-(Dimethylamino-thiophen-2-yl-methyl)-cyclohexylmethyl]-4-propyl-benzamid
(357) N-[4-(Dimethylamino-phenyl-methyl)-cyclohexylmethyl]-3,4-difluor-benzamid (polareres Diastereomer)
(358) 3-Brom-N-[4-(dimethylamino-phenyl-methyl)-cyclohexylmethyl]-benzamid
(359) N-{4-[Dimethylamino-(4-fluor-phenyl)-methyl]-cyclohexylmethyl}-2-thiophen-2-yl-acetamid
(360) 2-(4-Chlor-phenoxy)-N-[4-(dimethylamino-phenyl-methyl)-cyclohexylmethyl]-nicotinamid (unpolareres Diastereomer)
(361) 2,4-Dichlor-N-[4-(dimethylamino-phenyl-methyl)-cyclohexylmethyl]-benzamid
(362) N-[4-(Dimethylamino-phenyl-methyl)-cyclohexylmethyl]-3-methyl-benzamid
(363) 2-Brom-N-{4-[dimethylamino-(3-fluor-phenyl)-methyl]-cyclohexylmethyl}-benzamid
(364) N-{4-[Dimethylamino-(3-fluor-phenyl)-methyl]-cyclohexylmethyl}-3-trifluormethyl-benzamid
(365) 2-Phenyl-thiazol-4-carbonsäure-{4-[dimethylamino-(3-fluor-phenyl)-methyl]-cyclohexylmethyl}-amid
(366) 2-tert-Butyl-5-methyl-2H-pyrazol-3-carbonsäure-[4-(dimethylamino-thiophen-2-yl-methyl)-cyclohexylmethyl]-amid
(367) 3-Chlor-4-(propan-2-sulfonyl)-thiophen-2-carbonsäure-[4-(dimethylamino-thiophen-2-yl-methyl)-cyclohexylmethyl]-amid
(368) N-{4-[Dimethylamino-(3-fluor-phenyl)-methyl]-cyclohexylmethyl}-2-methoxy-benzamid
(369) N-{4-[(4-Chlor-phenyl)-dimethylamino-methyl]-cyclohexylmethyl}-3-trifluormethyl-benzamid
(370) 1-(4-Chlor-phenyl)-cyclopentan-carbonsäure-{4-[dimethylamino-(4-fluor-phenyl)-methyl]-cyclohexylmethyl}-amid
(371) 3,5-Dichlor-N-{4-[dimethylamino-(3-fluor-phenyl)-methyl]-cyclohexylmethyl}-benzamid
(372) Benzo[b]thiophen-3-carbonsäure-{4-[(4-chlor-phenyl)-dimethylamino-methyl]-cyclohexylmethyl}-amid (polarere Diastereomer)
(373) 2-Methyl-5-phenyl-furan-3-carbonsäure {4-[dimethylamino-(3-fluor-phenyl)-methyl]-cyctohexylmethyl}-amid
(374) 2-(4-Chlor-phenylsulfanyl)-N-[4-(dimethylamino-phenyl-methyl)-cyclohexylmethyl]-nicotinamid
(375) 4-Brom-2-ethyl-5-methyl-2H-pyrazol-3-carbonsäure-[4-(dimethylamino-thiophen-2-yl-methyl)-cyclohexylmethyl]-amid
(376) 5-Chlor-4-methoxy-thiophen-3-carbonsäure-{4-[dimethylamino-(4-fluor-phenyl)-methyl]-cyclohexylmethyl}-amid
(378) N-{4-[Dimethylamino-(3-fluor-phenyl)-methyl]-cyclohexylmethyl}-2-methyl-butyramid (unpolareres Diastereomer)
(379) 5-Methyl-isoxazol-3-carbonsäure-[4-(dimethylamino-phenyl-methyl)-cyclohexylmethyl]-amid
(380) Benzo[b]thiophen-3-carbonsäure-[4-(1-dimethylamino-3-phenyl-propyl).-cyclohexylmethyl]-amid
(381) N-{4-[Dimethylamino-(4-fluor-phenyl)-methyl]-cyclohexylmethyl}-2-methylsulfanyl-nicotinamid
(382) N-{4-[(4-Chlor-phenyl)-dimethylamino-methyl]-cyclohexylmethyl}-2-p-tolyloxy-nicotinamid
(383) 2-(4-Chlor-phenoxy)-N-[4-(dimethylamino-phenyl-methyl)-cyclohexylmethyl]-nicotinamid (polareres Diastereomer)
(384) N-{4-[Dimethylamino-(3-fluor-phenyl)-methyl]-cyclohexylmethyl}-2-methyl-benzamid
(385) 5-Methyl-isoxazol-3-carbonsäure-{4-[(4-chlor-phenyl)-dimethylamino-methyl]-cyclohexylmethyl}-amid
(386) 5-Brom-N-[4-(dimethylamino-phenyl-methyl)-cyclohexylmethyl]-nicotinamid
(387) N-[4-(Dimethylamino-phenyl-methyl)-cyclohexylmethyl]-2-methyl-butyramid
(388) N-{4-[Dimethylamino-(3-fluor-phenyl)-methyl]-cyclohexylmethyl}-2-ethylsulfanyl-nicotinamid
(389) N-[4-(Dimethylamino-thiophen-2-yl-methyl)-cyclohexylmethyl]-2-p-tolyloxy-nicotinamid (unpolareres Diastereomer)
(390) 4-Brom-2-ethyl-5-methyl-2H-pyrazol-3-carbonsäure-[4-(dimethylamino-phenyl-methyl)-cyclohexylmethyl]-amid
(391) N-{4-[(4-Chlor-phenyl)-dimethylamino-methyl]-cyclohexylmethyl}-2-phenoxy-propionamid
(392) N-[4-(Dimethylamino-phenyl-methyl)-cyclohexylmethyl]-3,5-dinitro-benzamid
(393) N-{4-[(4-Chlor-phenyl)-dimethylamino-methyl]-cyclohexylmethyl}-3-methoxy-benzamid
(394) 2-Brom-N-{4-[(4-chlor-phenyl)-dimethylamino-methyl]-cyclohexylmethyl}-benzamid
(395) 2-Brom-N-(2-{4-[(4-chlor-phenyl)-dimethylamino-methyl]-cyclohexyl}-ethyl)-benzamid
(396) 2-Brom-N-(2-{4-[dimethylamino-(3-fluor-phenyl)-methyl]-cyclohexyl}-ethyl)-benzamid
(397) 3-Chlor-N-{2-[4-(dimethylamino-phenyl-methyl)-cyclohexyl]-ethyl}-benzamid (polareres Diastereomer)
(398) 3-Chlor-N-{2-[4-(dimethylamino-phenyl-methyl)-cyclohexyl]-ethyl}-benzamid (unpolareres Diastereomer)
(399) 3-Chlor-N-(2-{4-[(4-chlor-phenyl)-dimethylamino-methyl]-cyclohexyl}-ethyl)-benzamid
(400) 3-Chlor-N-(2-{4-[dimethylamino-(3-fluor-phenyl)-methyl]-cyclohexyl}-ethyl)-benzamid (unpolareres Diastereomer)
(401) 3-Chlor-N-(2-{4-[dimethylamino-(3-fluor-phenyl)-methyl]-cyclohexyl}-ethyl)-benzamid (polareres Diastereomer)
(402) 2-Chlor-N-{2-[4-(dimethylamino-phenyl-methyl)-cyclohexyl]-ethyl}-benzamid
(403) 2-Chlor-N-(2-{4-[(4-chlor-phenyl)-dimethylamino-methyl]-cyclohexyl}-ethyl)-benzamid
(404) 2-Chlor-N-(2-{4-[dimethylamino-(3-fluor-phenyl)-methyl]-cyclohexyl}-ethyl)-benzamid
(405) 4-Chlor-N-{2-[4-(dimethylamino-phenyl-methyl)-cyclohexyl]-ethyl}-benzamid
(406) 4-Chlor-N-(2-{4-[dimethylamino-(3-fluor-phenyl)methyl]-cyclohexyl}-ethyl)-benzamid
(407) N-{2-[4-(Dimethylamino-phenyl-methyl)-cyclohexyl]-ethyl}-4-fluor-benzamid
(408) N-(2-{4-[(4-Chlor-phenyl)-dimethylamino-methyl]-cyclohexyl}-ethyl)-4-fluor-benzamid
(409) N-(2-{4-[Dimethylamino-(3-fluor-phenyl)-methyl]-cyclohexyl}-ethyl)-4-fluor-benzamid
(410) N-{2-[4-(Dimethylamino-phenyl-methyl)-cyclohexyl]-ethyl}-2-fluor-benzamid
(411) N-(2-{4-[Dimethylamino-(3-fluor-phenyl)-methyl]-cyclohexyl}-ethyl)-2-fluor-benzamid
(412) N-(2-{4-[Dimethylamino-(3-fluor-phenyl)-methyl]-cyclohexyl}-ethyl)-3-methyl-benzamid
(413) 2,6-Dichlor-N-{2-[4-(dimethylamino-phenyl-methyl)-cyclohexyl]-ethyl}-benzamid
(414) N-{2-[4-(Dimethylamino-phenyl-methyl)-cyclohexyl]-ethyl}-2-methoxy-benzamid
(415) N-(2-{4-[Dimethylamino-(3-fluor-phenyl)-methyl]-cyclohexyl}-ethyl)-2-methoxy-benzamid
(416) 3,4-Dichlor-N-{2-[4-(dimethylamino-phenyl-methyl)-cyclohexyl]-ethyl}benzamid
(417) N-{2-[4-(Dimethylamino-phenyl-methyl)-cyclohexyl]-ethyl}-2-methyl-benzamid (polareres Diastereomer)
(418) N-{2-[4-(Dimethylamino-phenyl-methyl)-cyclohexyl]-ethyl}-2-methyl-benzamid (unpolareres Diastereomer)
(419) N-(2-{4-[(4-Chlor-phenyl)-dimethylamino-methyl]-cyclohexyl}-ethyl)-2-methyl-benzamid
(420) N-(2-{4-[Dimethylamino-(3-fluor-phenyl)-methyl]-cyclohexyl}-ethyl)-2-methyl-benzamid
(421) 4-Cyano-N-{2-[4-(dimethylamino-phenyl-methyl)-cyclohexyl]-ethyl}-benzamid
(422) 3-Chlor-N-(2-{4-[dimethylamino-(4-fluor-phenyl)-methyl]-cyclohexyl}-ethyl)-benzamid (polareres Diastzereomer)
(423) 3-Chlor-N-(2-{4-[dimethylamino-(4-fluor-phenyl)-methyl]-cyclohexyl}-ethyl)-benzamid (unpolareres Diastereomer)
(424) 3-Chlor-N-{2-[4-(dimethylamino-thiophen-2-yl-methyl)-cyclohexyl]-ethyl}-benzamid
(425) 2-Chlor-N-{2-[4-(dimethylamino-thiophen-2-yl-methyl)-cyclohexyl]-ethyl}-benzamid
(426) N-{2-(4-(Dimethylamino-thiophen-2-yl-methyl)-cyclohexyl]-ethyl}-4-fluor-benzamid
(427) N-(2-{4-[Dimethylamino-(4-fluor-phenyl)-methyl]-cyclohexyl}-ethyl)-2-fluor-benzamid

(428) N-{2-[4-(Dimethylamino-thiophen-2-yl-methyl)-cyclohexyl]-ethyl}-2-fluor-benzamid
(429) N-(2-{4-[Dimethylamino-(4-fluor-phenyl)-methyl]-cyclohexyl}-ethyl)-3-methyl-benzamid
(430) N-{2-[4-(Dimethylamino-thiophen-2-yl-methyl)_cyclohexyl]-ethyl}-3-methyl-benzamid
(431) 2,6-Dichlor-N-{2-[4-(dimethylamino-thiophen-2-yl-methyl)-cyclohexyl]-ethyl}-benzamid
(432) N-(2-{4-[Dimethylamino-(4-fluor-phenyl)-methyl]-cyclohexyl}-ethyl)-2-methoxy-benzamid
(433) N-{2-[4-(Dimethylamino-phenyl-methyl)-cyclohexyl]-ethyl}-3,5-difluor-benzamid
(434) N-(2-{4-[(4-Chlor-phenyl)-dimethylamino-methyl]-cyclohexyl}-ethyl)-3,5-difluor-benzamid
(435) N-(2-{4-[Dimethylamino-(3-fluor-phenyl)-methyl]-cyclohexyl}-ethyl)-3,5-difluor-benzamid
(436) N-(2-{4-[(4-Chlor-phenyl)-dimethylamino-methyl]-cyclohexyl}-ethyl)-2,4-difluor-benzamid
(437) 2,4-Dichlor-N-(2-{4-[(4-chlor-phenyl)-dimethylamino-methyl]-cyclohexyl}-ethyl)-5-fluor-benzamid
(438) 2,4-Dichlor-N-(2-{4-[dimethylamino-(3-fluor-phenyl)-methyl]-cyclohexyl}-ethyl)-5-fluor-benzamid (polareres Diastereomer)
(439) 2,4-Dichlor-N-(2-{4-[dimethylamino-(3-fluor-phenyl)-methyl]-cyclohexyl}-ethyl)-5-fluor-benzamid (unpolareres Diastereomer)
(440) 2,4-Dichlor-N-{2-[4-(dimethylamino-thiophen-2-yl-methyl)-cyclohexyl]-ethyl}-5-fluor-benzamid
(441) 2-Chlor-N-(2-{4-[(4-chlor-phenyl)-dimethylamino-methyl]-cyclohexyl}-ethyl)-nicotinamid
(442) Naphthalen-2-carbonsäure(2-{4-[dimethylamino-(4-fluor-phenyl)-methyl]-cyclohexyl}-ethyl)-amid
(443) N-{2-[4-(Dimethylamino-thiophen-2-yl-methyl)-cyclohexyl]-ethyl}-4-propyl-benzamid
(444) N-{2-[4-(Dimethylamino-phenyl-methyl)-cyclohexyl]-ethyl}-3,4-difluor-benzamid
(445) N-(2-{4-[(4-Chlor-phenyl)-dimethylamino-methyl]-cyclohexyl}-ethyl)-3,4-difluor-benzamid
(446) N-{2-[4-(Dimethylamino-thiophen-2-yl-methyl)cyclohexyl]-ethyl}-3,4-difluor-benzamid
(447) N-(2-{4-[Dimethylamino-(4-fluor-phenyl)-methyl]-cyclohexyl}-ethyl)-3-methoxy-benzamid
(448) N-{2-[4-(Dimethylamino-phenyl-methyl)-cyclohexyl]-ethyl}-2,2-diphenyl-acetamid
(449) 1-(4-Chlor-phenyl)-cyclopentan-carbonsäure-{2-[4-(dimethylamino-phenyl-methyl)-cyclohexyl]-ethyl}-amid
(450) 2-Benzyloxy-N-{2-[4-(dimethylamino-phenyl-methyl)-cyclohexyl]-ethyl}-acetamid
(451) N-{2-[4-(Dimethylamino-phenyl-methyl)-cyclohexyl]-ethyl}-2-phenyl-acetamid
(452) Thiophen-2-carbonsäure-{2-[4-(dimethylamino-phenyl-methyl)-cyclohexyl]-ethyl}-amid
(453) N-(2-{4-[Dimethylamino-(3-fluor-phenyl)-methyl]-cyclohexyl}-ethyl)-2-(3-methoxy-phenyl)-acetamid
(454) N-{2-[4-(Dimethylamino-thiophen-2-yl-methyl)-cyclohexyl]-ethyl}-2-(3-methoxyphenyl)-acetamid
(455) N-(2-{4-[(4-Chlor-phenyl)-dimethylamino-methyl]-cyclohexyl}-ethyl)-2-phenyl-butyramid
(456) N-{2-[4-(Dimethylamino-thiophen-2-yl-methyl)-cyclohexyl]-ethyl}-2-phenyl-butyramid
(457) Benzo[b]thiophen-2-carbonsäure-{2-[4-(dimethylamino-phenyl-methyl)-cyclohexyl]-ethyl}-amid
(458) N-{2-[4-(Dimethylamino-phenyl-methyl)-cyclohexyl]-ethyl}-4-nitro-benzamid
(459) 3-Brom-N-(2-{4-[dimethylamino-(4-fluor-phenyl)-methyl]-cyclohexyl}-ethyl)-benzamid
(460) N-{2-[4-(Dimethylamino-phenyl-methyl)-cyclohexyl]-ethyl}-2,3,4,5,6-pentafluor-benzamid
(461) N-{2-[4-(Dimethylamino-phenyl-methyl)-cyclohexyl]-ethyl}-2,6-difluor-benzamid
(462) N-(2-{4-[Dimethylamino-(3-fluor-phenyl)-methyl]-cyclohexyl}-ethyl)-2,6-difluor-benzamid
(463) 2-Phenyl-thiazol-4-carbonsäure-{2-[4-(dimethylamino-phenyl-methyl)-cyclohexyl]-ethyl}-amid
(464) 2-Phenyl-thiazol-4-carbonsäure-(2-{4-[dimethylamino-(4-fluor-phenyl)-methyl]-cyclohexyl}-ethyl)-amid
(465) Benzo[b]thiophen-3-carbonsäure-{2-[4-(dimethylamino-thiophen-2-yl-methyl)-cyclohexyl]-ethyl}-amid
(466) N-{2-[4-(Dimethylamino-phenyl-methyl)-cyclohexyl]-ethyl}-2-methylsulfanyl-nicotinamid
(467) 2-Methyl-5-phenyl-furan-3-carbonsäure-{2-[4-(dimethylamino-phenyl-methyl)-cyclohexyl]-ethyl}-amid
(468) 2-(2,3-Dihydro-benzofuran-5-yl)-thiazol-4-carbonsäure-{2-[4-(dimethylaminophenyl-methyl)-cyclohexyl]-ethyl}-amid
(469) 3-(2,6-Dichlor-phenyl)-5-methyl-isoxazol-4-carbonsäure-{2-[4-(dimethylamino-thiophen-2-yl-methyl)-cyclohexyl]-ethyl}-amid
(470) 2-(4-Chlor-phenylsulfanyl)-N-{2-[4-(dimethylamino-phenyl-methyl)-cyclohexyl]-ethyl}-nicotinamid (polareres Diastereomer)
(471) 2-(4-Chlor-phenylsulfanyl)-N-{2-[4-(dimethylamino-phenyl-methyl)-cyclohexyl]-ethyl}-nicotinamid (unpolareres Diastereomer)
(472) Benzo[1,2,3]thiadiazol-5-carbonsäure-{2-[4-(dimethylamino-phenyl-methyl)-cyclohexyl]-ethyl}-amid
(473) 5-Brom-N-{2-[4-(dimethylamino-phenyl-methyl)cyclohexyl]-ethyl}-nicotinamid
(474) 5-Chlor-4-methoxy-thiophen-3-carbonsäure-{2-[4-(dimethylamino-phenyl-methyl)-cyclohexyl]-ethyl}-amid
(475) 5-Chlor-4-methoxy-thiophen-3-carbonsäure-(2-{4-[dimethylamino-(4-fluorphenyl)-methyl]-cyclohexyl}-ethyl)-amid
(476) 5-Chlor-4-methoxy-thiophen-3-carbonsäure-{2-[4-(1-dimethylamino-3-phenylpropyl)-cyclohexyl]-ethyl}-amid
(477) 3-Cyano-N-{2-[4-(dimethylamino-phenyl-methyl)-cyclohexyl]-ethyl}-benzamid
(478) N-{2-[4-(Dimethylamino-phenyl-methyl)-cyclohexyl]-ethyl}-2,4-dimethoxy-benzamid
(479) 2-Chlor-N-((4-((dimethylamino)(phenyl)methyl)cyclohexyl)methyl)benzamid
(480) N-((4-((Dimethylamino)(phenyl)methyl)cyclohexyl)methyl)-4-fluorbenzamid
(481) N-(2-(4-((Dimethylamino)(phenyl)methyl)cyclohexyl)ethyl)-4-fluorbenzamid
(482) N-((4-((Dimethylamino)(phenyl)methyl)cyclohexyl)methyl)-2-fluorbenzamid
(483) N-((4-((Dimethylamino)(phenyl)methyl)cyclohexyl)methyl)-3-methylbenzamid
(484) N-((4-((Dimethylamino)(phenyl)methyl)cyclohexyl)methyl)-2-methoxybenzamid
(485) N-(2-(4-((dimethylamino)(phenyl)methyl)cyclohexyl)ethyl)-3,5-dimethoxybenzamid
(486) N-((4-((Dimethylamino)(3-fluorphenyl)methyl)cyclohexyl)methyl)-2,6-dimethoxybenzamid
(487) N-((4-((Dimethylamino)(phenyl)methyl)cyclohexyl)methyl)-2,4-difluorbenzamid
(488) N-((4-((Dimethylamino)(thiophen-2-yl)methyl)cyclohexyl)methyl)-3-methoxybenzamid
(489) N-((4-((Dimethylamino)(thiophen-2-yl)methyl)cyclohexyl)methyl)-3,4,5-trimethoxybenzamid
(490) 4-((Dimethylamino)(phenyl)methyl)-1-(4-fluor-3-methylphenyl)cyclohexanol
(491) N-Cyclohexyl-2-(4-(2-phenyl-1-(pyrrolidin-1-yl)ethyl)cyclohexyl)acetamid
(492) N-(3-Methoxyphenyl)-2-(4-(2-phenyl-1-(pyrrolidin-1-yl)ethyl)cyclohexyl)acetamid
(493) N-(4-Methoxyphenyl)-2-(4-(piperidin-1-yl(p-tolyl)methyl)cyclohexyliden)acetamid
(494) N-Phenethyl-2-(4-(piperidin-1-yl(p-tolyl)methyl)cyclohexyliden)acetamid
(495) 2-(4-(2-Phenyl-1-(pyrrolidin-1-yl)ethyl)cyclohexyliden)-N-(pyridin-2-ylmethyl)acetamid
(496) N-Benzyl-N-methyl-2-(4-(piperidin-1-yl(p-tolyl)methyl)cyclohexyliden)acetamid
(497) 3-Thiophen-2-yl-[1,2,4]oxadiazol-5-carbonsäure [4-(dimethylamino-phenyl-methyl)-cyclohexyl]-amid
(498) 3-Methyl-[1,2,4]oxadiazol-5-carbonsäure {2-[4-(dimethylamino-phenyl-methyl)-cyclohexyl]-ethyl}-amid
(499) 3-Phenyl-[1,2,4]oxadiazol-5-carbonsäure {4-[dimethylamino-(3-fluoro-phenyl)-methyl]-cyclohexyl}-amid
(500) 3-Cyclopropylmethyl-[1,2,4]oxadiazol-5-carbonsäure {4-[dimethylamino-(3-fluoro-phenyl)-methyl]-cyclohexyl}-amid
(501) 3-Methoxymethyl-[1,2,4]oxadiazol-5-carbonsäure {2-[4-(1-dimethylamino-3-phenyl-propyl)-cyclohexyl]-ethyl}-amid

**23.** Verfahren zur Herstellung eines erfindungsgemäßen Cyclohexylmethyl-Derivates gemäß Anspruch 1, worin $R^1$ $(CH_2)_nC(O)H$ bedeutet, wobei Ketone der allgemeinen Formel G

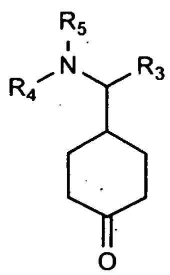

**G**

mit (Methoxymethyl)triphenylphosphoniumchlorid und einer starken Base, beispielsweise Kalium-tert-butylat, bei einer Temperatur zwischen -20°C und +30°C zu den entsprechenden Aldehyden H umgesetzt werden, wobei der Reaktionsschritt gegebenenfalls für die Synthese von Aldehyden mit n > 0 wiederholt wird.

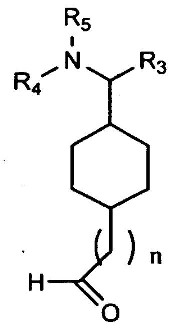

H

**24.** Verfahren zur Herstellung eines erfindungsgemäßen Cyclohexylmethyl-Derivates gemäß Anspruch 1, worin $R^1$ $(CH_2)_mCHN\text{-}OH$, $=N\text{-}OH$, $(CH_2)_nNH_2$ bedeutet, wobei das Keton G bzw. die Aldehyde H

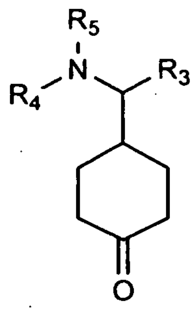

G

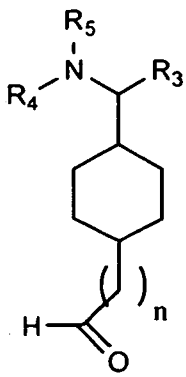

H

durch Umsetzung mit Hydroxylamin Hydrochlorid in einem organischen Lösungsmittel, beispielsweise Ethanol, unter Zugabe einer Base, beispielsweise einem basischen Ionenaustauscher Amberlyst zu Oximen der allgemeinen Formel K umgesetzt werden und die Amine der allgemeinen Formel L durch Umsetzung mit einem Reduktionsmittel, beispielsweise $LiAlH_4$ erhalten werden.

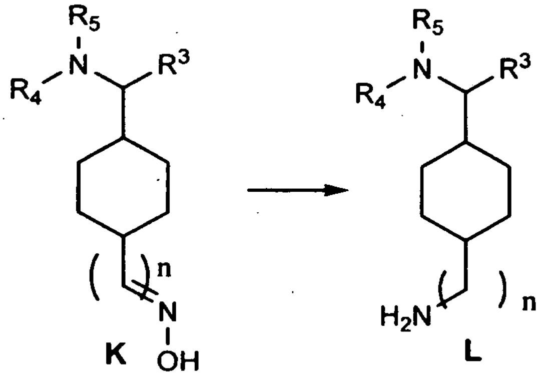

K L

**25.** Verfahren zur Herstellung eines erfindungsgemäßen Cyclohexylmethyl-Derivates gemäß Anspruch 1, worin $R^1$ $(CH_2)_nNHC(O)R^{13}$ oder $(CH_2)_nNHSO_2R^{12}$ bedeutet, wobei Amine der allgemeinen Formel L

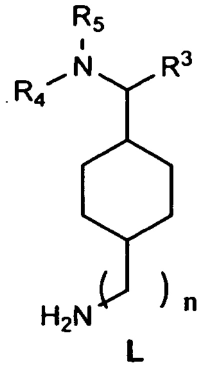

L

mit Carbonsäuren oder Sulfonsäuren unter Zugabe von Kupplungsreagenzien oder durch Aktivierung der Säurekomponente, insbesondere durch Herstellung des Säurechlorids, verknüpft werden.

**26.** Verfahren zur Herstellung eines erfindungsgemäßen Cyclohexylmethyl-Derivates gemäß Anspruch 1, worin $R^1$ $(CH_2)_nNHC(O)NR^{10}R^{11}$ bzw $(CH_2)_nNHC(S)NR^{10}R^{11}$ bedeutet, wobei Amine der allgemeinen Formel L

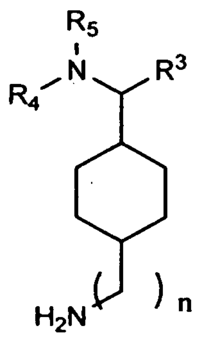

L

mit geeigneten Isocyanaten der allgemeinen Formel $R^{10}$-N=C=O bzw. Isothiocyanaten der allgemeinen Formel $R^{10}$-N=C=S, ggf. in Gegenwart wenigstens einer Base, zu einer Verbindung der allgemeinen Formel I umgesetzt wird, worin $R^1$ $(CH_2)_nNHC(O)NR^{10}R^{11}$ oder $(CH_2)_nNHC(S)NR^{10}R^{11}$ und $R^{11}$ H bedeutet und diese Verbindung gegebenenfalls in Gegenwart wenigstens einer Base mit einer Verbindung der allgemeinen Formel LG-$R^{11}$, worin LG für eine Abgangsgruppe steht, und $R^{11}$ die vorstehend genannte Bedeutung mit Ausnahme von Wasserstoff hat, zu einer Verbindung der allgemeinen Formel I umgesetzt wird, worin $R^1$ $(CH_2)_nNHC(O)NR^{10}R^{11}$ oder $(CH_2)_nNHC(S)NR^{10}R^{11}$ bedeutet, wobei $R^{11}$ nicht H bedeutet.

**27.** Verfahren zur Herstellung eines erfindungsgemäßen Cyclohexylmethyl-Derivates gemäß Anspruch 1, worin $R^1$ eine über eine $C_{1-3}$-Alkylgruppe, die gesättigt oder ungesättigt sein kann, verknüpftes $C(O)OR^9$ bedeutet;
oder $R^1$ und $R^2$ gemeinsam für stehen, wobei ein Phosphonoessigsäureester, vorzugsweise Phosphonoessigsäuretrimethylester oder Phosphonoessigsäure-triethylester, zunächst mit einer starken Base, vorzugsweise Kalium-tert.butylat, Natriumhydrid oder Butyllithium, dann mit einem Keton der allgemeinen Formel G oder einem Aldehyd H

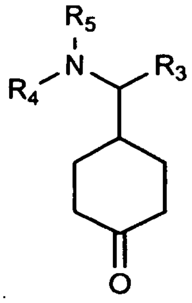

**G**

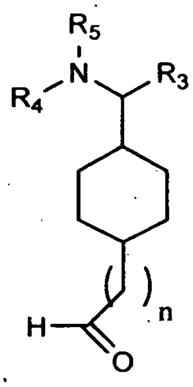

**H**

umgesetzt wird und gegebenenfalls die Ester mit einer geeigneten wässrigen, basischen Lösung, bevorzugt mit Kaliumhydroxid- oder Lithiumhydroxid-Lösung, bei RT oder leicht erhöhter Temperatur zu den korrespondierenden Carbonsäuren hydrolysiert werden oder die Doppelbindung reduziert, bevorzugt durch heterogene, katalytische Hydrierung an Palladium- oder Platin-Katalysatoren oder durch homogen katalysierte Hydrierung mit Rhodium-Katalysatoren, jeweils bei Temperaturen zwischen RT und 60°C und unter Wasserstoff-Drücken zwischen 1 bar und 6 bar, besonders bevorzugt bei RT unter einem Wasserstoffdruck zwischen 2 und 3 bar an Palladium auf Kohle. Anschließend wird wie oben beschrieben mit der Esterhydrolyse weiterverfahren. Die Ester können mit einem Reduktionsmittel, beispielsweise $LiAlH_4$, zu den entsprechenden Alkoholen reduziert werden.

**28.** Verfahren zur Herstellung eines erfindungsgemäßen Cyclohexylmethyl-Derivates gemäß Anspruch 1, worin $R^1$ für $(CH_2)_nC(O)NR^{10}R^{11}$ steht, wobei Carbonsäuren gemäß Anspruch 27 in Gegenwart wasserentziehender Mittel oder nach Überführung in ein Säurechlorid oder einen Aktivester mit einem primären oder sekundären Amin umgesetzt werden.

**29.** Verfahren zur Herstellung eines erfindungsgemäßen Cyclohexylmethyl-Derivates gemäß Anspruch 1, worin $R^1$ $(CH_2)_nOR^8$ bedeutet, wobei das Keton G bzw. die Aldehyde H

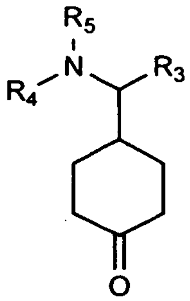

**G**

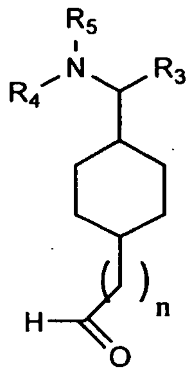

**H**

durch Umsetzung mit einem Reduktionsmittel, beispielsweise Natriumborhydrid, zu erfindungsgemäßen Verbindungen umgesetzt werden, bei denen $R^1(CH_2)_nOH$ bedeutet,
oder
die Ester gemäß Anspruch 27 mit einem Reduktionsmittel, beispielsweise $LiAlH_4$, zu den entsprechenden Alkoholen reduziert werden,
und diese Alkohole unter Zugabe einer Base, beispielsweise NaH, mit einer Verbindung der allgemeinen Formel $R^8Hal$, wobei Hal bevorzugt für Cl steht, zu Verbindungen umgesetzt werden, worin $R^1$ $(CH_2)_nOR^8$ bedeutet, wobei hier $R^8$ nicht H bedeutet.

**30.** Verfahren zur Herstellung eines erfindungsgemäßen Cyclohexylmethyl-Derivates gemäß Anspruch 1, worin $R^1$ $(CH_2)_nNHR^6$ bedeutet, wobei das Keton G bzw. die Aldehyde H

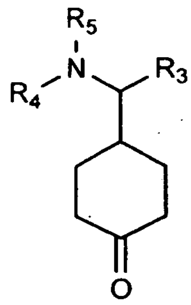

G

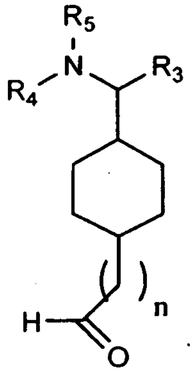

H

in polaren, aprotischen Lösungsmitteln, bespielsweise THF gelöst und mit dem entsprechenden Aminen der allgemeinen Formel $NH_2R^6$ unter Zugabe eines geeigneten Reduktionsmittels, beispielsweise Natriumborhydrid, umgesetzt wird.

**31.** Verfahren zur Herstellung eines erfindungsgemäßen Cyclohexylmethyl-Derivates gemäß Anspruch 1, worin $R^2$ OH und $R^1$ $C_{1-8}$-Alkyl , jeweils verzweigt oder unverzweigt, gesättigt oder ungesättigt, unsubstituiert oder einfach oder mehrfach substituiert; Aryl oder Heteroaryl, unsubstituiert oder einfach oder mehrfach substituiert; $C_{3-10}$-Cycloalkyl, gesättigt oder ungesättigt, unsubstituiert oder einfach oder mehrfach substituiert; einen über eine $C_{1-4}$-Alkylkette verknüpften Aryl- oder Heteroarylrest, jeweils unsubstituiert oder einfach oder mehrfach substituiert bedeutet, wobei Ketone der allgemeinen Formel G

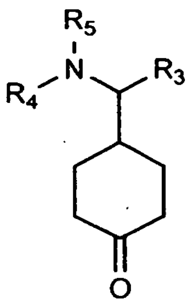

G

mit metallorganischen Verbindungen der allgemeinen Formel $R^{1a}MgHal$ mit Hal = Cl oder Br bzw. $R^{1a}Li$ unter Kühlung auf -30 bis +10°C in einem organischen Lösungsmittel, beispielsweise Diethylether oder THF, umgesetzt werden

oder

ein Aryliodid in einem organischen Lösungsmittel, beispielsweise THF, bei einer Temperatur zwischen -30°C und 0° mit Isopropylmagnesiumchlorid-Lsg. versetzt und nach einer Rührzeit von mindestens 10 min mit dem Keton der allgemeinen Formel G zu einer Verbindung der allgemeinen Formel I, worin $R^2$ OH und $R^1$ Aryl bedeutet, umgesetzt wird.

**32.** Arzneimittel enthaltend wenigstens ein substituiertes Cyclohexylmethyl-Derivat gemäß Anspruch 1 gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatz- und/oder Hilfsstoffe und/oder gegebenenfalls weiterer Wirkstoffe.

**33.** Verwendung eines substituierten Cyclohexylmethyl-Derivats gemäß Anspruch 1 gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, neuropathischem oder chronischem Schmerz.

**34.** Verwendung eines substituierten Cyclohexylmethyl-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Depressionen, Harninkontinenz, Diarrhöe, Pruritus, Alkohol- und Drogenmißbrauch, Medikamentenabhängigkeit, Antriebslosigkeit und/oder zur Anxiolyse.

**Claims**

**1.** A substituted cyclohexylmethyl derivative of the general formula I

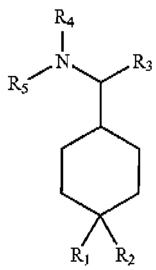

I

wherein
$R^1$ represents $C_{1-8}$-alkyl, in each case branched or unbranched, saturated or unsaturated, unsubstituted or mono- or poly-substituted; aryl or heteroaryl, which is unsubstituted or mono- or poly-substituted; $C_{3-10}$-cycloalkyl, which is saturated or unsaturated, unsubstituted or mono- or poly-substituted; an aryl or heteroaryl radical linked via a $C_{1-4}$-alkyl chain and in each case unsubstituted or mono- or poly-substituted; $(CH2)_mCHN\text{-}OH$, $(CH_2)_nNR^6R^7$ or $(CH_2)_nOR^8$, wherein n represents 0, 1, 2 or 3 and m represents 0, 1 or 2; or $C(O)OR^9$ linked via a $C_{1-3}$-alkyl group, which can be saturated or unsaturated; $CONR^{10}R^{11}$;
$R^2$ represents H or OH;
or $R^1$ and $R^2$ together represent or =N-OH;
$R^3$ represents aryl or heteroaryl, which is unsubstituted or mono- or poly-substituted; or an aryl radical which is linked via a $C_{1-3}$-alkyl group and may be unsubstituted or mono- or poly-substituted;
$R^4$ and $R^5$ independently of one another represent H; $C_{1-3}$-alkyl, which is unsubstituted, wherein $R^4$ and $R^5$ arc not simultaneously H;
or the radicals $R^4$ and $R^5$ together represent $CH_2CH_2OCH_2CH_2$ or $(CH_2)_{3-6}$;
$R^6$ represents H; $C_{1-8}$-alkyl, which is saturated or unsaturated, branched or unbranched, unsubstituted or mono- or poly-substituted; aryl, heteroaryl or $C_{3-10}$-cycloalkyl, in each case unsubstituted or mono- or poly-substituted; or an aryl or heteroaryl radical linked via a $C_{1-4}$-alkyl chain and in each case unsubstituted or mono- or poly-substituted;
$R^7$ represents H; $C_{1-8}$-alkyl, which is saturated or unsaturated, branched or unbranched, unsubstituted or mono- or poly-substituted; aryl, heteroaryl or $C_{3-10}$-cycloalkyl, in each case unsubstituted or mono- or poly-substituted; or an aryl or heteroaryl radical linked via a $C_{1-4}$-alkyl chain and in each case unsubstituted or mono- or poly-substituted; $C(O)NR^{10}R^{11}$, $C(S)NR^{10}R^{11}$, $SO_2R^{12}$ or $C(O)R^{13}$;
$R^8$ represents H; $C_{1-8}$-alkyl, in each case branched or unbranched, saturated or unsaturated, unsubstituted or mono- or poly-substituted; $C_{3-10}$-cycloalkyl, which is saturated or unsaturated, unsubstituted or mono- or poly-substituted; an aryl or heteroaryl radical linked via a $C_{1-4}$-alkyl group and unsubstituted or mono- or poly-substituted;
$R^9$ represents H; $C_{1-8}$-alkyl, which is saturated or unsaturated, branched or unbranched, unsubstituted or mono- or poly-substituted;
$R^{10}$ and $R^{11}$ independently of one another represent H; $C_{3-10}$-cycloalkyl, which is saturated or unsaturated, unsubstituted or mono- or poly-substituted; aryl or heteroaryl, in each case unsubstituted or mono- or poly-substituted or

an aryl or heteroaryl radical linked via a $C_{1-4}$-alkyl chain and in each case unsubstituted or mono- or poly-substituted; $R^{12}$ represents aryl or heteroaryl, in each case unsubstituted or mono- or poly-substituted; $C_{1-8}$-alkyl, in each case saturated or unsaturated, branched or unbranched, unsubstituted or mono- or poly-substituted; $C_{3-10}$-cycloalkyl, saturated or unsaturated, unsubstituted or mono- or poly-substituted; or an aryl or heteroaryl radical linked via a $C_{1-3}$-alkyl chain and in each case unsubstituted or mono- or poly-substituted;
$R^{13}$ represents $C_{1-8}$-alkyl, which is saturated or unsaturated, branched or unbranched, unsubstituted or mono- or poly-substituted; $C_{3-10}$-cyloalkyl, aryl or heteroaryl, in each case unsubstituted or mono- or poly-substituted; or an aryl or heteroaryl radical linked via a $C_{1-4}$-alkyl chain and in each case unsubstituted or mono- or poly-substituted, wherein the alkyl chain can be branched or unbranched, unsubstituted or mono- or poly-substituted;
in the form of the racemate; of the enantiomers, diastereomers, mixtures of the enantiomers or diastereomers or of a single enantiomer or diastereomer; the bases and/or salts of physiologically tolerated acids.

**2.** A substituted cyclohexylmethyl derivative according to claim 1, wherein $R^1$ represents $C_{1-8}$-alkyl, in each case branched or unbranched, saturated or unsaturated, unsubstituted or mono- or poly-substituted; aryl or heteroaryl, unsubstituted or mono- or poly-substituted; $C_{3-10}$-cycloalkyl, saturated or unsaturated, unsubstituted or mono- or poly-substituted; or an aryl or heteroaryl radical linked via a $C_{1-4}$-alkyl chain and in each case unsubstituted or mono- or poly-substituted; and $R^2$ denotes OH.

**3.** A substituted cyclohexylmethyl derivative according to claim 1, wherein $R^1$ represents $(CH_2)_mCHN\text{-}OH$, $(CH_2)_nNR^6R^7$ or $(CH_2)_nOR^8$, wherein n represents 0, 1, 2 or 3 and m represents 0, 1 or 2; or $C(O)OR^9$ linked via a $C_{1-3}$-alkyl group, which can be saturated or unsaturated, or $CONR^{10}R^{11}$; and $R^2$ denotes H.

**4.** A substituted cyclohexylmethyl derivative according to claim 1 or 2, wherein $R^1$ denotes $C_{1-8}$-alkyl, in each case branched or unbranched, saturated or unsaturated, unsubstituted or mono- or poly-substituted by methyl, =O, phenyl or $CO_2CH_3$; phenyl, naphthyl, benzyl, phenethyl, 2-pyridyl or 2-thienyl, unsubstituted or mono- or poly-substituted by F, $CH_3$, Cl, tert-butyl, methoxy or $CF_3$; cyclohexyl or cyclopentyl.

**5.** A substituted cyclohexylmethyl derivative according to claim 1 or 2, wherein $R^1$ represents 2,4-difluorophenyl, 4-fluoro-3-methylphenyl, phenyl, 3-methoxy-benzyl, 4-chlorophenyl, benzyl, 2-xnethylphenyl, 4-tert-butylphenyl, cyclopentyl, 4-fluorophenyl, phenethyl, 2-thienyl, 2,4-dichlorophenyl, 3-methoxyphenyl, 4-methylphenyl, 4-methoxyphenyl, 3,5-difluorophenyl, isopropyl, butyl, ethyl, hexyl, sec-butyl, 2,4,6-trimethylphenyl, pentyl, propyl, 3-fluorophenyl, 3,5-dichloro-phenyl, 4-fluorobenzyl, 4-chloro-3-trifluoromethylphenyl, cyclohexyl, isobutyl or 2,5-dimethoxyphenyl.

**6.** A substituted cyclohexylmethyl derivative according to any of claims 1 - 3, wherein $R^3$ represents phenyl or thienyl, in each case unsubstituted or mono-or poly-substituted by F, Cl, CN, $NO_2$, SH, S-$C_{1-6}$-alkyl, OH, O-$C_{1-6}$-alkyl, $CO_2H$, $CO_2$-$C_{1-6}$-alkyl, $CF_3$, $C_{1-6}$-alkyl; a phenyl radical bonded via a $C_{1-3}$-alkyl chain, in each case unsubstituted or mono- or poly-substituted by F, Cl, CN, $NO_2$, SH, S-$C_{1-6}$-alkyl, OH, O-$C_{1-6}$-alkyl, $CO_2H$, $CO_2C_{1-6}$-alkyl, $CF_3$, $C_{1-6}$-alkyl.

**7.** A substituted cyclohexylmethyl derivative according to claim 6, wherein $R^3$ represents phenyl, unsubstituted or monosubstituted by C1 or F; phenethyl or thienyl.

**8.** A substituted cyclohexylmethyl derivative according to any of claims 1-3, wherein $R^4$ and $R^5$ represent H or $CH_3$, wherein $R^4$ and $R^5$ arc not simultaneously H.

**9.** A substituted cyclohexylmethyl derivative according to any of claims 1-3, wherein $R^4$ and $R^5$ together represent $(CH_2)_{3-6}$.

**10.** A substituted cyclohexylmethyl derivative according to either or claims 1 and 3, wherein $R^6$ represents H; $C_{1-8}$-alkyl, saturated or unsaturated, branched or unbranched, unsubstituted or mono- or poly-substituted by F, Cl, -CN, SH, $SCH_3$, $OCH_3$, OH, =O, $CO_2C2H_5$ or $CO_2CH_3$; aryl, unsubstituted or mono- or poly-substituted by F, Cl, Br, CN, $NH_2$, $NO_2$, SH, $SCH_3$, OH, $OCH_3$, $CF_3$, methyl, ethyl, propyl, butyl or tert-butyl; or an aryl or heteroaryl radical linked via a $C_{1-4}$-alkyl chain and in each case unsubstituted or mono- or poly-substituted by F, Cl, Br, CN, $NH_2$, $NO_2$, SH, $SCH_3$, OH, $OCH_3$, $CF_3$, methyl, ethyl, propyl, butyl or tert-butyl, wherein the alkyl chain can be branched or unbranched, unsubstituted or mono- or poly-substituted by $CO_2C_2H_5$ or $CO_2CH_3$-

**11.** A substituted cyclohexylmethyl derivative according to claim 10, wherein $R^6$ represents 2-indolylethyl, phenethyl, 3-phenylpropyl, benzyl, phenyl, 4-phenylbutyl, 1-(1H-indol-3-yl)propan-2-yl, 4 2-(3-indolyl)propionic acid methyl ester, in each case unsubstituted or mono- or poly-substituted by F or $OCH_3$.

**12.** A substituted cyclohexylmethyl derivative according to claim 10, wherein $R^6$ is H.

**13.** A substituted cyclohexylmethyl derivative according to either of claims 1 and 3, wherein $R^7$ represents $C(O)R^{13}$.

**14.** A substituted cyclohexylmethyl derivative according to either of claims 1 and 3, wherein $R^8$ represents H; a phenyl radical linked via a $C_{1-4}$-alkyl group and unsubstituted or mono- or poly-substituted by F, Cl, Br, CN, $NH_2$, $NO_2$, SH, $SCH_3$, OH, $OCH_3$, $CF_3$, methyl, ethyl, propyl, butyl or tert-butyl.

**15.** A substituted cyclohexylmethyl derivative according to claim 14, wherein $R^8$ represents benzyl, unsubstituted or mono- or poly-substituted by F.

**16.** A substituted cyclohexylmethyl derivative according to either of claims 1 and 3, wherein $R^9$ represents $C_{1-8}$-alkyl, which is branched or unbranched.

**17.** A substituted cyclohexylmethyl derivative according to either of claims 1 and 3, wherein $R^{10}$ and $R^{11}$ independently of one another represent H; phenyl, naphthyl, or a phenyl or indolyl radical linked via a $C_{1-4}$-alkyl chain, in each case unsubstituted or substituted by F, Cl, Br, CN, $NH_2$, $NO_2$, SH, $SCH_3$, OH, $OCH_3$, $CF_3$, methyl, ethyl, propyl, butyl or tert-butyl.

**18.** A cyclohexylmethyl derivative according to claim 17, wherein $R^{10}$ and $R^{11}$ independently of one another represent H; naphthyl, phenyl or benzyl, in each case unsubstituted or mono- or poly-substituted by $CF_3$, F, $NO_2$ or Br; or cyclohexyl, wherein $R^{10}$ and $R^{11}$ are not simultaneously H.

**19.** A cyclohexylmethyl derivative according to either of claims 1 and 3, wherein $R^{12}$ represents naphthyl, phenyl or benzyl, in cach case unsubstituted or mono- or poly-substituted by F, Cl, Br, CN, $NH_2$, $NO_2$, SH, $SCH_3$, OH, $OCH_3$, $CF_3$, methyl, ethyl, propyl, butyl or tert-butyl.

**20.** A substituted cyclohexylmethyl derivative according to either of claims 1 and 3, wherein $R^{13}$ represents H; $C_{1-8}$-alkyl, saturated or unsaturated, branched or unbranched, unsubstituted or mono- or poly-substituted by F, Cl, -CN, NH-$C_{1-6}$-alkyl, $C_{1-6}$-alkyl, N($C_{1-6}$-alkyl)$_2$, SH, S-$C_{1-6}$-alkyl, S-benzyl, O-$C_{1-6}$-alkyl, OH, =O, O-benzyl, C(=O)$C_{1-6}$-alkyl, $CO_2H$, $CO_2$-$C_{1-6}$-alkyl, phenyl or benzyl; $C_{3-10}$-cycloalkyl, unsubstituted or mono- or poly-substituted by F, Cl, -CN, NH-$C_{1-6}$-alkyl, $C_{1-6}$-alkyl, N($C_{1-6}$-alkyl)$_2$, SH, S-$C_{1-6}$-alkyl, $C_{1-6}$-alkyl, S-benzyl, O-$C_{1-6}$-alkyl, OH, =O, O-benzyl, C (=O)$C_{1-6}$-alkyl, $CO_2H$, $CO_2$-$C_{1-6}$-alkyl, phenyl or benzyl; aryl or heteroaryl, in each case unsubstituted or mono- or poly-substituted by F, Cl, Br, CN, $NH_2$, NH-$C_{1-6}$-alkyl, N($C_{1-6}$-alkyl)$_2$, $NO_2$, SH, pyridyl, S-$C_{1-6}$-alkyl, S-phenyl, OH, $(CH_2)_{0-3}$O-$C_{1-6}$-alkyl, $C_{1-3}$-alkyl-$C_{3-6}$-cycloalkyl, O-$C_{1-6}$,-alkyl-OH, O-phenol, phenyl, benzyl, C(=O)$C_{1-6}$-alkyl, $CO_2H$, $CO_2$-$C_{1-6}$-alkyl, $CF_3$, $C_{1-6}$-alkyl, dihydrobenzofuran, $SO_2$phenyl or $SO_2C_{1-6}$-alkyl; or an aryl or heteroaryl radical linked via a $C_{1-4}$-alkyl chain and in each case unsubstituted or mono- or poly-substituted by F, Cl, Br, CN, $NH_2$, NH-$C_{1-6}$-alkyl, N($C_{1-6}$-alkyl)$_2$, $NO_2$, SH, pyridyl, S-$C_{1-6}$-alkyl, S-phenyl, OH, O-$C_{1-6}$-alkyl, O-$C_{1-6}$-alkyl-OH, O-phenyl, phenyl, benzyl, C(=O)$C_{1-6}$-alkyl, $CO_2H$, $CO_2$-$C_{1-6}$-alkyl, $CF_3$, ($C_{1-6}$-alkyl, $SO_2$-phenyl or $SO_2C_{1-6}$-alkyl, wherein the alkyl chain can be branched or unbranched, unsubstituted or mono- or poly-substituted by F, Cl, -CN, SH, S-$C_{1-6}$-alkyl, S-benzyl, O-$C_{1-6}$-alkyl, OH, O-$C_{1-6}$-alkyl-OH, O-benzyl, $CO_2$-$C_{1-6}$-alkyl, phenyl or benzyl.

**21.** A substituted cyclohexylmethyl derivative according to claim 20, wherein $R^{13}$ represents methyl, ethyl, phenyl, benzyl, 3-pentyl, n-propyl, benzothienyl, 1-(4-chlorophenyl)-cyclopentyl, 4-propylphenyl, 3-cyanophenyl, 3-chlorophenyl, 5-chloro-4-methoxy-thiophen-3-yl, 3-fluoro-5-trifluoromethylphenyl, 4-fluoro-5-trifluoromethylphenyl, 2-thienyl, 3,5-dichlorophenyl, 2,4,5-trifluorophenyl, 3-bromophenyl, 4-methylphenyl, 3-methoxyphenyl, 2,2-dimethylpropyl, 2-tert-butyl-5-methyl-pyrazol-3-yl, 2,4-dimethoxyphenyl, 3-trifluoromethylphenyl, 3,5-difluorophenyl, 2-fluoro-5-trifluoromethylphenyl, 4-chlorobenzyl, 2-methoxyphenyl, 2-methylsulfanyl-3-pyridyl, 3,4,5-trimethoxyphenyl, 2-ethylsulfanyl-3-pyridyl, 2-methyl-5-phenyl-furan-3-yl, 1-phenxoxyethyl, tert-butylphenyl, 2-(4-chloro-phenylsulfanyl)-3-pyridyl, 2-p-tolyloxy-3-pyridyl, 3-chloro-4-(sulfonyl-2-propyl)-thiophen-2-yl, 5-methylisoxazol-3-yl, 5-promo-3-pyridyl, naphthyl, 2-methyl-5-(4-chloro-phenyl)-furan-3-yl, 4-(4-chloro-phenylsulfonyl)-3-methyl-thiophen-2-yl, 1-phenylpropyl, adamantyl, 2-phenyl-thiazol-4-yl, 4-methyl-2-phenyl-thiazol-5-yl, 2-(2,3-dihydro-benzofuran-5-yl)-thiazol-4-yl, 3-methylphenyl, 3-chloro-4-methylsulfonyl-thiophen-2-yl, benzyloxymethyl, methylthienyl, 4-bromo-2-ethyl-5-methyl-pyrazol-3-yl, 2,5-dimethylfuryl, 5-pyridin-2-yl-thiophene, 3-chloro-4-fluorophenyl, cyclohexyl, 3-nitrophenyl, 2,5-difluorophenyl, 2,6-difluorophenyl, 2-trifluoromethyl-5-fluoro-phenyl, 4-chlorophenoxy-methyl, 2-bromophenyl, cyclopentyl, benzothiadiazolyl, diphenylmethyl, 2-methylphenyl, 3-methoxybenzyl, 2,4,6-trichlorophenyl, 2-butyl, 2-chlorophenyl, 3,5-dinitrophenyl, 4-cyanophenyl, 2,4-dichloro-5-fluorophenyl, 2-chloro-3-pyridyl, 4-nitrophenyl, 2,3,4,5,6-pentafluorophenyl or 3-(2,6-dichloro-phenyl)-5-methyl-isoxazol-4-yl, 5-chloro-4-methylthiophen-3-yl, 4-fluorophenyl, 4-chlorophenyl, 2-fluorophenyl, 3-methylphenyl, 3-bromophenyl, 2,6-dichlorophenyl, 3,4-dichlorophenyl, 4-cyanophenyl, 2,4-difluorophenyl, 2,4-dichlorophenyl, 2,4-dichloro-5-fluorophenyl, 2-chloropyridin-3-yl, 3,5-dimethoxyphenyl, 2,6-dimethoxyphenyl, 2,3,6-trifluorophenyl, 2-(4-chlorophenoxy)-3-pyridyl, 3,4-difluorophenyl, 2-(2,3-dihydro-benzofuran-5-yl)-4-methyl-thiazol-5-yl, 3-methyl-oxadiazolyl, 3-phenyl-oxadiazolyl, 3-cyclopropylmethyl-oxadiazolyl, 3-methoxymethyl-oxadiazolyl or 2,4-dimethoxyphenyl.

**22.** A substituted cyclohexylmethyl derivative according to claim 1, wherein said compound is selected from the group consisting of:

(16) 4-(dimethylamino-phenyl-methyl)-cyclohexanone oxime
(17) 4-(dimethylamino-phenyl-methyl)-cyclohexylamine
(18) 4-[dimethylamino-(4-fluorophenyl)-methyl]-cyclohexanone oxime
(19) 4-[dimethylamino-(4-fluorophenyl)-methyl]-cyclohexylamine
(20) 4-[dimethylamino-(3-fluorophenyl)-methyl]-cyclohexanone oxime
(21) 4-[dimethylamino-(3-fluorophenyl)-methyl]-cyclohexylamine
(22) 4-[(4-chlorophenyl)-dimethylamino-methyl]-cyclohexanone oxime
(23) 4-[(4-chlorophenyl)-dimethylamino-methyl]-cyclohexylamine
(24) 4-(dimethylamino-thiophen-2-yl-methyl)-cyclohexanone oxime
(25) 4-(dimethylamino-thiophen-2-yl-methyl)-cyclohexylamine
(26) 4-(1-dimethylamino-3-phenyl-propyl)-cyclohexanone oxime
(27) 4-(1-dimethylamino-3-phenyl-propyl)-cyclohexylamine
(29) 4-(dimethylamino-phenyl-methyl)-cyclohexane-carbaldehyde oxime
(30) [(4-aminomethyl-cyclohexyl)-phenyl-methyl]-dimethylamine
(32) 4-[dimethylamino-(4-fluorophenyl)-methyl]-cyclohexanecarbaldehyde oxime
(33) [(4-aminomethyl-cyclohexyl)-(4-fluorophenyl)-methyl]-dimethylamine
(35) 4-[dimethylamino-(3-fluorophenyl)-methyl]-cyclohexanecarbaldehyde oxime
(36) [(4-aminomethyl-cyclohexyl)-(3-fluorophenyl)-methyl]-dimethylamine
(38) 4-[(4-chlorophenyl)-dimethylamino-methyl]-cyclohexanecarbaldehyde oxime
(39) [(4-aminomethyl-cyclohexyl)-(4-chlorophenyl)-methyl]-dimethylamine
(41) 4-(dimethylamino-thiophen-2-yl-methyl)-cyclohexanecarbaldehyde oxime
(42) [(4-aminomethyl-cyclohexyl)-thiophen-2-yl-methyl]-dimethylamine
(44) 4-(1-dimethylamino-3-phenyl-propyl)-cyclohexanecarbaldehyde oxime
(45) [1-(4-aminomethyl-cyclohexyl)-3-phenyl-propyl]-dimethylamine
(47) [4-(dimethylamino-phenyl-methyl)-cyclohexyl]-acetaldehyde oxime
(48) 2-[4-dimethylamino-phenyl-methyl)-cyclohexyl]-ethylamine
(50) {4-[dimethylamino-(4-fluorophenyl)-methyl]-cyclohexyl}-acetaldehyde oxime
(51) 2-(4-[dimethylamino-(4-fluorophenyl)-methyl]-cyclohexyl)-ethylamine
(53) {4-[dimethylamino-(3-fluorophenyl)-methyl]-cyclohexyl}-acetaldehyde oxime
(54) 2-{4-[dimethylamino-(3-fluorophenyl)-methyl]-cyclohexyl}-ethylamine
(56) {4-[dimethylamino-(4-chlorophenyl)-methyl]-cyclohexyl}-acetaldehyde oxime
(66) 2-{4-[dimethylamino-(4-chlorophenyl)-methyl]-cyclohexyl}-ethylamine
(68) 2-(4-((dimethylamino)(thiophen-2-yl)methyl)cyclohexyl)acetaldehyde oxime
(69) 2-[4-(dimethylamino-thiophen-2-yl-methyl)-cyclohexyl]-ethylamine
(71) [4-(1-dimethylamino-3-phenyl-propyl)-cyclohexyl]-acetaldehyde oxime
(72) {1-[4-(2-amino-ethyl)-cyclohexyl]-3-phenyl-propyl}-dimethylamine
(111) 4-[dimethylamino-phenyl-methyl]-cyclohexanol
(112) 4-[dimethylamino-(4-fluorophenyl)-methyl]-cyclohexanol
(113) 4-[dimethylamino-(3-fluorophenyl)-methyl]-cyclohexanol
(114) 4-[(4-chlorophenyl)-dimethylamino-methyl]-cyclohexanol
(115) 4-(dimethylamino-thiophen-2-yl-methyl)-cyclohexanol
(116) 4-(1-dimethylamino-3-phenyl-propyl)-cyclohexanol
(117) [4-(dimethylamino-phenyl-methyl)-cyclohexyl]-methanol
(118) {4-[dimethylamino-(4-fluorophenyl)-methyl]-cyclohexyl}-methanol
(119) {4-[dimethylamino-(3-fluorophenyl)-methyl]-cyclohexyl}-methanol
(120) {4-[(4-chlorophenyl)-dimethylamino-methyl]-cyclohexyl}-methanol
(121) [4-(dimethylamino-thiophen-2-yl-methyl)-cyclohexyl]-methanol

(122) [4-(1-dimethylamino-3-phenyl-propyl)-cyclohexyl]-methanol
(123) [4-(dimethylamino-phenyl-methyl)-cyclohexylidene]-acetic acid ethyl ester
(124) [4-(dimethylamino-phenyl-methyl)-cyclohexyl]-acetic acid ethyl ester
(125) 2-[4-(dimethylamino-phenyl-methyl)-cyclohexyl]-ethanol
(126) {4-[dimethylamino-(4-fluorophenyl)-methyl]-cyclohexylidene}-acetic acid ethyl ester
(127) {4-[dimethylamino-(4-fluorophenyl)-methyl]-cyclohexyl}-acetic acid ethyl ester
(128) 2-{4-[dimethylamino-(4-fluorophenyl)-methyl]-cyclohexyl}-ethanol
(129) {4-[dimethylamino-(3-fluorophenyl)-methyl]-cyclohexylidene}-acetic acid ethyl ester
(130) {4-[dimethylamino-(3-fluorophenyl)-methyl]-cyclohexyl}-acetic acid ethyl ester
(131) 2-{4-[dimethylamino-(3-fluorophenyl)-methyl]-cyclohexyl}-ethanol
(132) 3-[4-(dimethylamino-phenyl-methyl)-cyclohexyl]-acrylic acid ethyl ester
(133) 3-[4-(dimethylamino-phenyl-methyl)-cyclohexyl]-propionic acid ethyl ester
(134) 3-[4-(dimethylamino-phenyl-methyl)-cyclohexyl]-propan-1-ol
(135) 3-{4-[dimethylamino-(4-fluorophenyl)-methyl]-cyclohexyl}-acrylic acid ethyl ester
(136) 3-{4-[dimethylamino-(4-fluorophenyl)-methyl]-cyclohexyl}-propionic acid ethyl ester
(137) 3-{4-[dimethylamino-(4-fluorophenyl)-methyl]-cyclohexyl}-propan-1-ol
(138) 3-{4-[dimethylamino-(3-fluorophenyl)-methyl]-cyclohexyl}-acrylic acid ethyl ester
(139) 3-{4-[dimethylamino-(3-fluorophenyl)-methyl]-cyclohexyl}-propionic acid ethyl ester
(140) 3-{4-[dimethylamino-(3-fluorophenyl)-methyl]-cyclohexyl}-propan-1-ol
(141) 3-[4-(1-dimethylamino-3-phenyl-propyl)-cyclohexyl]-acrylic acid ethyl ester
(142) 3-[4-(1-dimethylamino-3-phenyl-propyl)-cyclohexyl]-propionic acid ethyl ester
(143) 3-[4-(1-dimethylamino-3-phenyl-propyl)-cyclohexyl]-propan-1-ol
(73) 1-(4-((dimethylamino)(phenyl)methyl)cyclohexyl)-3-(naphthalen-1-yl)urca
(74) 1-(2,4-difluorophenyl)-3-(4-((dimethylamino)(phenyl)methyl)cyclohexyl)urea hydrochloride
(75) 1-(4-((dimethylamino)(phenyl)methyl)cyclohexyl)-3-(3-(trifluoromethyl)-phenyl)urea hydrochloride
(76) 1-(4-((dimethylamino)(phenyl)methyl)cyclohexyl)-3-(2-nitrophenyl)urea hydro chloride
(77) 1-(3-bromophenyl)-3-(4-((dimethylamino)(phenyl)methyl)cyclohexyl)urea hydrochloride
(78) 1-(4-((dimethylamino)(phenyl)methyl)cyclohexyl)-3-phenylurca hydrochloride
(79) 1-benzyl-3-(4-((dimethylamino)(phenyl)methyl)cyclohexyl)urea
(80) 1-cyclohexyl-3-(4-((dimethylamino)(phenyl)methyl)cyclohexyl)urea
(81) 1-(4-bromophenyl)-3-(4-((dimethylamino)(phenyl)methyl)cyclohexyl)urca
(82) 1-(4-((dimethylamino)(phenyl)methyl)cyclohexyl)-3-(4-methoxyphenyl)urea
(83) N-(2-(1H-indol-3-yl)ethyl)-4-((dimethylamino)(phenyl)methyl)-cyclohexanamine hydrochloride
(84) 4-((dimethylamino)(phenyl)methyl)-N-phenethylcyclohexanamine hydrochloride
(85) 4-((dimethylamino)(phenyl)methyl)-N-(3-phenylpropyl)cyclohexanamine dihydrochloride
(86) N-benzyl-4-((dimethylamino)(phenyl)methyl)cyclohexanamine hydrochloride
(87) 4-((dimethylamino)(phenyl)methyl)-N-(4-phenylbutyl)cyclohexanamine hydrochloride
(88) N-(1-(1H-indol-3-yl)propan-2-yl)-4-((dimethylamino)(phenyl)methyl)-cyclohexanamine hydrochloride
(89) N-(4-((dimethylamino)(phenyl)methyl)cyclohexyl)-4-methoxy-benzenamine hydrochloride
(90) 4-((dimethylamino)(phenyl)methyl)-N-(4-methoxybenzyl)cyclo-hexanamine dihydrochloride
(91) 4-((dimethylamino)(phenyl)methyl)-N-(4-fluorobenzyl)cyclohexanamine hydrochloride
(92) N-(4-((dimethylamino)(phenyl)methyl)cyclohexyl)benzenamine hydrochloride
(93) N-(4-((dimethylamino)(phenyl)methyl)cyclohexyl)-2-ethylbutanamide hydrochloride
(94) N-(4-((dimethylamino)(phenyl)methyl)cyclohexyl)benzamide hydrochloride
(95) N-(4-((dimethylamino)(phenyl)methyl)cyclohexyl)-N-(3-phenylpropyl)acetamide hydrochloride
(96) N-(4-((dimethylamino)(phenyl)methyl)cyclohexyl)-N-phenylacetamide hydrochloride
(97) N-(4-((dimethylamino)(phenyl)methyl)cyclohexyl)-N-(4-phenylbutyl)propionamide hydrochloride
(98) N-(4-((dimethylamino)(phenyl)methyl)cyclohexyl)-N-(4-phenylbutyl)-acetamide hydrochloride
(99) N-(4-((dimethylamino)(phenyl)methyl)cyclohexyl)-N-(4-methoxyphenyl)-acetamide hydrochloride
(100) N-benzyl-N-(4-((dimethylamino)(phenyl)methyl)cyclohexyl)acctamide hydrochloride
(101) N-benzyl-N-(4-((dimethylamino)(phenyl)methyl)cyclohexyl)-2-cthylbutanamide hydrochloride
(102) N-benzyl-N-(4-((dimethylamino)(phenyl)methyl)cyclohexyl)butyramide hydrochloride
(103) N-benzyl-N-(4-((dimethylamino)(phenyl)methyl)cyclohexyl)-4-fluoro-benzamide hydrochloride
(104) N-benzyl-N-(4-((dimethylamino)(phenyl)methyl)cyclohexyl)benzamide hydrochloride
(105) N-(4-((dimethylamino)(phenyl)methyl)cylohexyl)-2-ethyl-N-phenylbutanamide hydrochloride
(106) 4-chloro-N-(4-((dimethylamino)(phenyl)methyl)cyclohexyl)benzenesulfonamide hydrochloride
(107) N-(4-((dimethylamino)(phenyl)methyl)cyclohexyl)-4-methoxybenzene-sulfonamide hydrochloride
(108) 4-tert-butyl-N-(4-((dimethylamino)(phenyl)methyl)cyclohexyl)benzenesulfonamide hydrochloride

(109) N-(4-((dimethylamino)(phenyl)methyl)cyclohexyl)-2-nitrobenzenesulfonamide hydrochloride
(110) N-(4-((dimethylamino)(phenyl)methyl)cyclohexyl)benzenesulfonamide hydrochloride
(144) 4-(benzyloxy)cyclohexyl)-N,N-dimethyl(phenyl)methanamine hydrochloride
(145) 4-(4-fluorobenzyloxy)cyclohexyl)-N,N-dimethyl(phenyl)methanamine hydrochloride
(146) trans-N,N-dimethyl(4-phenethylcyclohexyl)(phenyl)methanamine hydrochloride
(147) 1-benzyl-4-((dimethylamino)(phenyl)methyl)cyclohexanol hydrochloride
(148) 4-((dimethylamino)(phenyl)methyl)-1-(4-fluorobenzyl)cyclohexanol hydrochloride
(149) 1-(2,5-dimethoxyphenyl)-4-((dimethylamino)(phenyl)methyl)cyclohexanol
(150) 4-((dimethylamino)(phenyl)methyl)-1-(4-fluoro-3-methylphenyl)cyclohexanol
(151) 4-(dimethylamino-phenyl-methyl)-1-(4-fluoro-3-methyl-phenyl)-cyclohexanol
(152) 1-benzyl-4-[dimethylamino-(3-fluoro-phenyl)-imethyl]-cyclohexanol
(153) 4-[dimethylamino-(3-fluoro-phenyl)-methyl]-1-phenethyl-cyclohexanol
(154) 4-[dimethylamino-(3-fluoro-phenyl)-methyl]-1-pentyl-cyclohexanol
(155) 1-(3,5-dichloro-phenyl)-4-[dimethylamino-(3-fluoro-phenyl)-methyl]-cyclohexanol
(156) 4-[dimethylamino-(3-fluoro-phenyl)-methyl]-1-(3-methoxy-benzyl)-cyclohexanol
(157) 1-(4-chloro-3-trifluromethyl-phenyl)-4-[dimethylamino-(3-fluorophenyl)-methyl]-cyclohexanol
(158) 4-[(4-chloro-phenyl)-dimethylamino-methyl]-1-phenyl-cylohexanol
(159) 1-benzyl-4-[(4-chloro-phenyl)-dimethylamino-methyl]-cyclohexanol
(160) 4-[(4-chloro-phenyl)-dimethylamino-methyl]-1-(4-fluoro-3-methylphenyl)-cyclohexanol
(161) 4-[(4-chloro-phenyl)-dimethylamino-methyl]-1-o-tolyl-cyclohexanol
(162) 4-[(4-chloro-phenyl)-dimethylamino-methyl]-1-(4-fluoro-phenyl)-cyclohexanol
(163) 4-[(4-chloro-phenyl)-dimethylamino-methyl]-1-phenethyl-cyclohexanol
(164) 4-[(4-chloro-phenyl)-dimethylamino-methyl]-1-(3-methoxy-phenyl)-cyclohexanol
(165) 4-[(4-chloro-phenyl)-dimethylamino-methyl]-1-p-tolyl-cyclohexanol
(166) 4-[(4-chloro-phenyl)-dimethylamino-methyl]-1-(3,5-difluoro-phenyl)-cyclohexanol
(167) 1-butyl-4-[(4-chloro-phenyl)-dimethylamino-methyl]-cyclohexanol
(168) 4-[(4-chloro-phenyl)-dimethylamino-methyl]-1-hexyl-cyclohexanol
(169) 4-[(4-chloro-phenyl)-dimethylamino-methyl]-1-pentyl-cyclohexanol (more polar diastereomer)
(170) 4-[(4-chloro-phenyl)-dimethylamino-methyl]-1-pentyl-cyclohexanol (less polar diastereomer)
(171) 4-[(4-chloro-phenyl)-dimethylamino-methyl]-1-(3-fluoro-phenyl)-cyclohexanol
(172) 4-[(4-chloro-phenyl)-dimethylamino-methyl]-1-(4-fluoro-benzyl)-cyclohexanol
(173) 4-[(4-chloro-phenyl)-dimethylamino-methyl]-1-(3-methoxy-benzyl)-cyclohexanol
(174) methyl 2-(4-((dimethylamino)(phenyl)methyl)cyclohexylamino)-3-(1H-indol-3-yl)propanoate (more polar diastereomer)
(175) methyl 2-(4-((dimethylamino)(phenyl)methyl)cyclohexylamino)-3-(1H-indol-3-yl)propanoate (less polar diastercomer)
(176) N-(4-((dimethylamino)(phenyl)methyl)cyclohexyl)-N-(4-methoxybenzyl)-acetamide
(177) N-(1-(1H-indol-3-yl)propan-2-yl)-N-(4-(dimethylamino)(phenyl)methyl)-cyclohexyl)acetamide (more polar diastereomer)
(178) N-(1-(1H-indol-3-yl)propan-2-yl)-N-(4-((dimethylamino)(phenyl)methyl)-cydohexyl)acetamide (less polar diastereomer)
(179) N-(4-((dimethylamino)(phenyl)methyl)cyclohexyl)-N-(4-fluorobenzyl)acetamide
(180) N-(4-((dimethylamino)(phenyl)methyl)cyclohexyl)-N-phenylbutyramide
(181) N-(2-(1H--indol-3-yl)ethyl)-N-(4-((dimethylamino)(phenyl)methyl)-cyclohexyl)butyramide
(182) N-(2-(1H-indol-3-yl)ethyl)-N-(4-((dimethylamino)(phenyl)methyl)cyclohexyl)-acetamide
(183) benzo[b]thiophene-3-carboxylic acid [4-(dimethylamino-phenyl-methyl)-cyclohexyl]-amide
(184) 1-(4-chloro-phenyl)-cyclopentanecarboxylic acid [4-(dimethylaminophenyl-methyl)-cyclohexyl]-amide
(185) N-[4-(dimethylamino-thiophen-2-yl-methyl)-cyclohexyl]-4-propyl-benzamide
(186) 3-cyano-N-[4-(dimethylamino-thiophen-2-yl-methyl)-cyclohexyl]-benzamide
(187) 3-chloro-N-[4-(dimethylamino-phenyl-methyl)-cyclohexyl]-benzamide
(188) 5-chloro-4-methoxy-thiophene-3-carboxylic acid [4-(dimethylaminophenyl-methyl)-cyclohexyl]-amide
(189) 3,4-dichloro-N-[4-(dimethylamino-phenyl-methyl)-cyclohexyl]-benzamide
(190) N-[4-(dimethylamino-phenyl-methyl)-cyclohexyl]-3-fluoro-5-trifluoromethyl-benzamide
(191) 5-chloro-4-methoxy-thiophene-3-carboxylic acid {4-[dimethylamino-(3-fluoro-phenyl)-methyl]-cyclohexyl}-amide
(192) N-[4-(dimethylamino-thiophen-2-yl-methyl)-cyclohexyl]-4-fluoro-3-trifluoromethyl-benzamide
(193) thiophene-2-carboxylic acid [4-(dimethylamino-thiophen-2-yl-methyl)-cyclohexyl]-amide
(194) 3,5-dichloro-N-[4-(dimethylamino-thiophen-2-yl-methyl)-cyclohexyl]-benzamide

(195) 5-chloro-4-methoxy-thiophene-3-carboxylic acid [4-(dimethylamino-thiophen-2-yl-methyl)-cyclohexyl]-amide
(196) N-[4-(dimethylamino-phenyl-methyl)-cyclohexyl]-2,4,5-trifluoro-benzamide
(197) 3-bromo-N-[4-(dimethylamino-phenyl-methyl)-cyclohexyl]-benzamide
(198) N-[4-(dimethylamino-thiophen-2-yl-methl)-cyclohexyl]-4-methyl-benzamide
(199) N-[4-(dimethylamino-thiophen-2-yl-methyl)-cyclohexyl]-3-methoxybenzamide
(200) N-[4-(dimethylamino-thiophen-2-yl-methyl)-cyclohexyl]-3,3-dimethyl-butyramide.
(201) 2-tert-butyl-5-methyl-2H-pyrazole-3-carboxylic acid [4-(dimethylamino-thiophen-2-yl-methyl)-cyclohexyl]-amide
(202) N-[4-(dimethylamino-thiophen-2-yl-methyl)-cyclohexyl]-2,4-dimethoxybenzamide
(203) N-[4-(dimethylamino-phenyl-methyl)-cyclohexyl]-3-trifluoromethyl-benzamide
(204) N-{4-[dimethylamino-(3-fluoro-phenyl)-methyl]-cyclohexyl}-3,5-difluoro-benzamide
(205) N-[4-(dimethylamino-phenyl-methyl)-cyclohexyl]-2-fluoro-5-trifluoromethyl-benzamide
(206) 2-(4-chloro-phenyl)-N-[4-(dimethylamino-phenyl-methyl)-cyclohexyl]-acetamide
(207) N-[4-(dimethylamino-thiophen-2-yl-methyl)-cyclohexyl]-2-methoxybenzamide
(208) N-[4-(dimethylamino-thiophen-2-yl-methyl)-cyclohexyl]-2-methylsulfanyl-nicotinamide
(209) 3,4-dichloro-N-{4-[dimethylamino-(3-fluoro-phenyl)-methyl]-cyclohexyl}-benzamide
(210) N-[4-(1-dimethylamino-3-phenyl-propyl)-cyclohexyl]-4-fluoro-3-trifluoromethyl-benzamide (more polar diastercomer)
(211) 5-chloro-4-methoxy-thiophene-3-carboxylic acid {4-[dimethylamino-(4-fluoro-phenyl)-methyl]-cyclohexyl}-amide
(212) N-[4-(dimethylamino-thiophen-2-yl-methyl)-cyclohexyl]-3,4,5-trimethoxy-benzamide
(213) N-[4-(dimethylamino-thiophen-2-yl-methyl)-cyclohexyl]-2-ethylsulfanyl-nicotinamide
(214) 2-methyl-5-phenyl-furan-3-carboxylic acid [4-(dimethylamino-phenylmethyl)-cyclohexyl]-amide
(215) N-{4-[dimethylamino-(3-fluoro-phenyl)-methyl]-cyclohexyl}-2-phenoxy-propionamide (less polar diastereomer)
(216) N-[4-(dimethylamino-phenyl-methyl)-cyclohexyl]-2,4-dimethoxy-benzamide
(217) 4-tert-butyl-N-[4-(dimethylamino-phenyl-methyl)-cyclohexyl]-benzamide
(218) 2-(4-chloro-phenylsulfanyl)-N-[4-(dimethylamino-thiophen-2-yl-methyl)cyclohexyl]-nicotinamide
(219) 2-(4-chloro-phenyl)-N-[4-(dimethylamino-thiophen-2-yl-methyl)-cyclohexyl]-acetamide
(220) N-[4-(dimethylamino-thiophen-2-yl-methyl)-cyclohexyl]-2-p-tolyloxy-nicotinamide
(221) 3-chloro-4-(propan-2-sulfonyl)-thiophene-2-carboxylic acid [4-(dimethylamino-thiophen-2-yl-methyl)-cyclohexyl]-amide
(222) N-{4-[dimethylamino-(3-fluoro-phenyl)-methyl]-cyclohexyl}-2-phenoxy-propionamide (more polar diastereomcr)
(223) 2-tert-butyl-5-methyl-2H-pyrazole-3-carboxylic acid [4-(dimethylaminophenyl-methyl)-cyclohexyl]-amide
(224) 5-methyl-isoxazole-3-carboxylic acid [4-(dimethylamino-thiophen-2-yl-methyl)-cyclohexyl]-amide
(225) 5-bromo-N-[4-(dimethylamino-thiophen-2-yl-methyl)-cyclohexyl]-nicotinamide
(226) naphthyl-1-carboxylic acid [4-(dimethylamino-phenyl-methyl)-cyclohexyl]-amide
(227) N-[4-(1-dimethylamino-3-phenyl-propyl)-cyclohexyl]-4-fluoro-3-trifluoromethyl-benzamide (less polar diastereomer)
(228) N-[4-(dimethylamino-phenyl-methyl)-cyclohexyl]-3,3-dimethyl-butyramide (more polar diastereomer)
(229) 5-(4-chloro-phenyl)-2-methyl-furan-3-carboxylic acid [4-(dimethylamino-thiophen-2-yl-methyl)-cyclohexyl]-amide
(230) N-[4-(dimethylamino-phenyl-methyl)-cyclohexyl]-2-phenoxy-propionamide
(231) benzo[b]thiophene-2-carboxylic acid {4-[dimethylamino-(3-fluorophenyl)-methyl]-cyclohexyl}-amide
(232) 5-(4-chloro-phenyl)-2-methyl-furan-3-carboxylic acid [4-(dimethylaminophenyl-methyl)-cyclohexyl]-amide
(233) 4-(4-chloro-benzenesulfonyl)-3-methyl-thiophene-2-carboxylic acid [4-(dimethylamino-phenyl-methyl)-cyclohexyl]-amide
(234) N-[4-(dimethylamino-phenyl-methyl)-cyclohexyl]-2-phenyl-butyramide (less polar diastereomer)
(235) 5-bromo-N-[4-(dimethylamino-phenyl-methyl)-cyclohexyl]-nicotinamide
(236) adamantane-1-carboxylic acid [4-(dimethylamino-phenyl-methyl)-cyclohexyl]-amide
(237) 2-phenyl-thiazole-4-carboxylic acid [4-(dimethylamino-phenyl-methyl)-cyclohexyl]-amide
(238) 4-methyl-2-phenyl-thiazole-5-carboxylic acid [4-(dimethylamino-phenylmethyl)-cyclohexyl]-amide
(239) 2-(2,3-dihydro-benzofuran-5-yl)-thiazole-4-carboxylic acid [4-(dimethylamino-thiophen-2-yl-methyl)-cyclohexyl]-amide
(240) N-{4-[dimethylamino-(3-fluoro-phenyl)-methyl]-cyclohexyl}-2-phenyl-acetamide

(241) 3-chloro-N-[4-(1-dimethylamino-3-phenyl-propyl)-cyclohexyl]-benzamide
(242) N-[4-(dimethylamino-phenyl-methyl)-cyclohexyl]-4-methyl-benzamide
(243) 3,5-dichloro-N-{4-[dimethylamino-(3-fluoro-phenyl)-methyl]-cyclohexyl}-benzamide
(244) N-[4-(dimethylamino-phenyl-methyl)-cyclohexyl]-2,3,6-trifluoro-benzamide
(245) thiophene-2-carboxylic acid [4-(dimethylamino-phenyl-methyl)-cyclohexyl]-amide (less polar diastereomer)
(246) N-[4-(dimethylamino-phenyl-methyl)-cyclohexyl]-3,3-dimethyl-butyramide (less polar diastereomer)
(247) 2-tert-butyl-5-methyl-2H-pyrazole-3-carboxylic acid [4-(dimethylaminophenyl-methyl)-cyclohexyl]-amide
(248) N-[4-(1-dimethylamino-3-phenyl-propyl)-cyclohexyl]-3-methyl-benzamide
(249) thiophene-2-carboxylic acid [4-(dimethylamino-phenyl-methyl)-cyclohexyl]-amide (more polar diastereomer)
(250) N-[4-(dimethylainino-phenyl-methyl)-cyclohexyl]-2-phenyl-butyramide (more polar diastereomer)
(251) N-{4-[dimethylamino-(3-fluoro-phenyl)-methyl]-cyclohexyl}-3,3-dimethyl-butyramide
(252) 3-chloro-4-methanesulfonyl-thiophene-2-carboxylic acid [4-(dimethylamino-phenyl-methyl)-cyclohexyl]-amide
(253) 4-(4-chloro-benzenesulfonyl)-3-methyl-thiophene-2-carboxylic acid [4-(dimethylamino-phenyl-methyl)-cyclohexyl]-amide
(254) 2-benzyloxy-N-[4-(dimethylamino-phenyl-methyl)-cyclohexyl]-acetamide
(255) N-[4-(dimethylamino-phenyl-methyl)-cyclohexyl]-2-thiophen-2-yl-acetamide
(256) 4-methyl-2-phenyl-thiazole-5-carboxylic acid {4-[dimethylamino-(3-fluoro-phenyl)-methyl]-cyclohexyl}-amide
(257) 2-(4-chloro-phenoxy)-N-[4-(dimethylamino-thiophen-2-yl-methyl)-cyclohexyl]-nicotinamide
(258) N-[4-(1-dimethylamino-3-phenyl-propyl)-cyclohexyl]-4-fluoro-benzamide
(259) 5-bromo-N-[4-(dimethylamino-phenyl-methyl)-cyclohexyl]-nicotinamide
(260) 4-bromo-2-ethyl-5-methyl-2H-pyrazole-3-carboxylic acid [4-(dimethylamino-thiophen-2-yl-methyl)-cyclohexyl]-amide
(261) 3-cyano-N-[4-(dimethylamino-phenyl-methyl)-cyclohexyl]-benzamide
(262) N-{4-[dimethylamino-(4-fluoro-phenyl)-methyl]-cyclohexyl}-4-fluoro-benzamide
(263) 3-bromo-N-{4-[dimethylamino-(4-fluoro-phenyl)-methyl]-cyclohexyl}-benzamide
(264) 2-phenyl-thiazole-4-carboxylic acid {4-[dimethylamino-(3-fluoro-phenyl)-methyl]-cyclohexyl}-amide
(265) 2,5-dimethyl-furan-3-carboxylic acid {4-[dimethylamino-(3-fluorophenyl)-methyl]-cyclohexyl}-amide
(266) 2-methyl-5-phenyl-furan-3-carboxylic acid {4-[dimethylamino-(3-fluorophenyl)-methyl]-cyclohexyl}-amide
(267) 5-pyridin-2-yl-thiophene-2-carboxylic acid {4-[dimethylamino-(3-fluorophenyl)-methyl]-cyclohexyl}-amide
(268) 4-bromo-2-ethyl-5-methyl-2H-pyrazole-3-carboxylic acid [4-(dimethylamino-phenyl-methyl)-cyclohexyl]-amide
(269) 3-chloro-N-{4-[dimethylamino-(3-fluoro-phenyl)-methyl]-cyclohexyl}-4-fluoro-benzamide
(270) 3,4-dichloro-N-{4-[dimethylamino-(4-fluoro-phenyl)-methyl]-cyclohexyl}-benzamide
(271) N-{4-[dimethylamino-(4-fluoro-phenyl)-methyl]-cyclohexyl}-2,4,5-trifluoro-benzamide
(272) cyclohexanecarboxylic acid [4-(dimethylamino-phenyl-methyl)-cyclohexyl]-amide
(273) N-{4-[dimethylamino-(3-fluoro-phenyl)-methyl]-cyclohcxyl}-2-phenyl-butyramide
(274) 2-(4-chloro-phenyl)-N-{4-[dimethylamino-(3-fluoro-phenyl)-methyl]-cyclohexyl}-acetamide
(275) N-[4-(dimethylamino-phenyl-methyl)-cyclohexyl]-3-nitro-benzamide
(276) N-[4-(1-dimethylamino-3-phenyl-propyl)-cyclohexyl]-2,5-difluoro-benzamide
(277) 3-bromo-N-[4-(1-dimethylamino-3-phenyl-propyl)-cyclohexyl]-benzamide
(278) N-{4-[dimethylamino-(3-fluoro-phenyl)-methyl]-cyclohexyl}-2,6-difluoro-benzamide
(279) 2,5-dimethyl-furan-3-carboxylic acid [4-(dimethylamino-phenyl-methyl)-cyclohexyl]-amide
(280) 3-chloro-N-{4-[dimethylamino-(4-fluoro-phenyl)-methyl]-cyclohexyl}-4-fluoro-benzamide
(281) N-[4-(dimethylamino-phenyl-methyl)-cyclohexyl]-5-fluoro-2-trifluoromethyl-benzamide
(282) 5-methyl-isoxazole-3-carboxylic acid [4-(dimethylamino-phenyl-methyl)-cyclohexyl]-amide
(283) 2-(2,3-dihydro-benzofuran-5-yl)-4-methyl-thiazole-5-carboxylic acid [4-(dimethylamino-thiopen-2-yl-methyl)-cyclohexyl]-amide
(284) 2-(4-chloro-phenoxy)-N-[4-(dimethylamino-phenyl-methyl)-cyclohcxyl] acetamide
(285) 5-(4-chloro-phenyl)-2-methyl-furan-3-carboxylic acid {4-[dimethylamino-(3-fluoro-phenyl)-methyl]-cyclohexyl}-amide
(286) 2-bromo-N-[4-(dimethylamino-phenyl-methyl)-cyclohexyl]-benzamide
(287) N-[4-(dimethylamino-phenyl-methyl)-cyclohexyl]-2,6-dimethoxybenzamide
(288) cyclopentanecarboxylic acid [4-(dimethylamino-phenyl-methyl)-cyclohexyl]-amide
(289) 2-(2,3-dihydro-benzofuran-5-yl)-thiazole-4-carboxylic acid [4-(dimethylamino-phenyl-methyl)-cyclohexyl]-amide
(290) benzo[1,2,5]thiadiazole-5-carboxylic acid {4-[dimethylamino-(3-fluorophenyl)-methyl]-cyclohexyl}-amide
(291) N-[4-(dimethylamino-thiophen-2-yl-methyl)-cyclohexylmethyl]-2-thiophen-2-yl-acetamide
(292) benzo[b]thiophene-3-carboxylic acid [4-(dimethylamino-phenyl-methyl)-cyclohexylmethyl]-amide
(293) 5-chloro-4-methoxy-thiophene-3-carboxylic acid [4-(dimethylaminophenyl-methyl)-cyclohexylmethyl]-amide
(294) N-[4-(dimethylamino-phenyl-methyl)-cyclohexylmethyl]-3,4-difluoro-benzamide (less polar diastereomer)
(295) N-{4-[dimethylamino-(3-fluoro-phenyl)-methyl]-cyclohexylmethyl}-3,3-dimethyl-butyramide
(296) 2-bromo-N-[4-(dimethylamino-thiophen-2-yl-methyl)-cyclohexylmethyl]-benzamide
(297) N-[4-(dimethylamino-thiophen-2-yl-methyl)-cyclohexylmethyl]-2,2-diphenyl-acetamide
(298) N-[4-(dimethylamino-phenyl-methyl)-cyclohexylmethyl]-3,3-dimethyl-butyramide
(299) N-[4-(dimethylamino-phenyl-methyl)-cyclohexylmethyl]-2-methyl-sulfanyl-nicotinamide
(300) N-[4-(dimethylamino-thiophen-2-yl-methyl)-cyclohexylmethyl]-2,6-dimethoxy-benzamide
(301) N-[4-(dimethylamino-thiophen-2-yl-methyl)-cyclohexylmethyl]-3,3-dimethylbutyramide
(302) benio[b]thiophene-3-carboxylic acid [4-(dimethylamino-thiophen-2-yl-methyl)-cyclohexylmethyl]-amide
(303) 5-chloro-4-methoxy-thiophene-3-carboxylic acid {4-[dimethylamino-(3-fluoro-phenyl)-methyl]-cyclohexylmethyl}-amide
(304) N-[4-(dimethylamino-thiophen-2-yl-methyl)-cyclohexylmethyl]-2-phenoxy-propionamide
(305) N-[4-(dimethylamino-thiophen-2-yl-methyl)-cyclohexylmethyl]-2-methoxy-benzamide
(306) N-[4-(dimethylamino-phenyl-methyl)-cyclohexylmethyl]-2-phenyl-acetamide
(307) 3-bromo-N-[4-(dimethylamino-thiophen-2-yl-methyl)-cyclohexylmethyl]-benzamide
(308) N-[4-(dimethylamino-thiophen-2-yl-methyl)-cyclohexylmethyl]-3-fluoro-5-trifluoromethyl-benzamide
(309) N-{4-[(4-chloro-phenyl)-dimethylamino-methyl]-cyclohexylmethyl}-3,3-dimethyl-butyramide
(310) N-[4-(dimethylamino-phenyl-methyl)-cyclohexylmethyl]-2-ethylsulfanyl-nicotinamide
(311) 2-(4-chloro-phenyl)-N-[4-(dimethylamino-thiophen-2-yl-methyl)-cyclohexylmethyl]-acetamide
(312) N-{4-[(4-chloro-phenyl)-dimethylamino-methyl]-cyclohexylmethyl}-2,2-diphenyl-acetamide
(313) N-[4-(dimethylamino-thiophen-2-yl-methyl)-cyclohexylmethyl]-2,6-difluorobenzamide
(314) benzo[b]thiophene-3-carboxylic acid {4-[dimethylamino-(3-fluorophenyl)-methyl]-cyclohexylmethyl}-amide
(315) N-{4-[(4-chloro-phenyl)-dimethylamino-methyl]-cyclohexylmethyl} -2-methylsulfanyl-nicotinamide
(316) N-{4-[(4-chloro-phenyl)-dimethylamino-methyl]-cyclohexylmethyl}-2-thiophen-2-yl-acetamide
(317) N-[4-(dimethylamino-phenyl-methyl)-cyclohexylmethyl]-2-methyl-benzamide (less polar diastereomer)
(318) 1-(4-chloro-phenyl)-cyclopentane-carboxylic acid [4-(dimethylaminophenyl-methyl)-cyclohexylmethyl]-amide
(319) N-{4-[dimethylamino-(3-fluoro-phenyl)-methyl]-cyclohexylmethyl}-2-phenylacetamide (more polar diastereomer)
(320) N-{4-[(4-chloro-phenyl)-dimethylamino-methyl]-cyclohexylmethyl}-2-(3-methoxy-phenyl)-acetamide
(321) N-[4-(dimethylamino-phenyl-methyl)-cyclohexylmethyl]-2-phenyl-butyramide
(322) N-{4-[dimethylamino-(4-fluoro-phenyl)-methyl]-cyclohexylmethyl}-3,3-dimethyl-butyramide
(323) N-[4-(dimethylamino-phenyl-methyl)-cyclohexylmethyl]-2-phenoxy-propionamide
(324) 2-(4-chloro-phenyl)-N-[4-(dimethylamino-phenyl-methyl)-cyclohexylmethyl]-acetamide
(325) N-[4-(dimethylamino-phenyl-methyl)-cyclohexylmethyl]-2-methyl-benzamide (more polar diastereomer)
(326) N-[4-(dimethylamino-phenyl-methyl)-cyclohexylmethyl]-3-trifluoromethyl-benzamide (less polar diastereomer)
(327) 1-(4-chloro-phenyl)-cyclopentane-carboxylic acid {4-[dimethylamino-(3-fluoro-phenyl)-methyl]-cyclohexylmethyl}-amide
(328) thiophene-2-carboxylic acid [4-(dimethylamino-phenyl-methyl)-cyclohexyl-methyl]-amide
(329) 3,5-dichloro-N-[4-(dimethylamino-phenyl-methyl)-cyclohexylmethyl]-benzamide
(330) 2-methyl-5-phenyl-furan-3-carboxylic acid {4-[(4-chloro-phenyl)-dimethylamino-methyl]-cyclohexylmethyl}-amide
(331) 3-chloro-N-[4-(dimethylamino-phenyl-methyl)-cyclohexylmethyl]-benzamide
(332) N-{4-[dimethylamino-(3-fluoro-phenyl)-methyl]-cyclohexylmethyl}-2-phenoxy-propionamide (more polar diastereomer)
(333) N-[4-(dimethylamino-phenyl-methyl)-cyclohexylmethyl]-3-trifluoromethyl-benzamide (more polar diastereomer)
(334) thiophene-2-carboxylic acid {4-[dimethylamino-(3-fluoro-phenyl)-methyl]-cyclohexylmethyl}-amide (more polar diastereomer)
(335) 2-phenyl-thiazole-4-carboxylic acid [4-(dimethylamino-phenyl-methyl)-cyclohexylmethyl]-amide

(336) benzo[b]thiophene-3-carboxylic acid {4-[(4-chloro-phenyl)-dimethylamino-methyl]-cyclohexylmethyl}-amide (less polar diastereomer)
(337) N-{4-[dimethylamino-(3-fluoro-phenyl)-methyl]-cyclohexylmethyl}-2-p-tolyloxy-nicotinamide
(338) 2,4,6-trichloro-N-[4-(dimethylamino-phenyl-methyl)-cyclohexylmethyl]-benzamide
(339) 1-(4-chloro-phenyl)-cyclopentane-carboxylic acid [4-(dimethylaminophenyl-methyl)-cyclohexylmethyl]-amide
(340) thiophene-2-carboxylic acid -{4-{dimethylamino-(3-fluoro-phenyl)-methyl]-cyclohexylmethyl}-amide (less polar diastereomer)
(341) N-{4-[dimethylamino-(3-fluoro-phenyl)-methyl]-cyclohexylmethyl}-2-phenoxy-propionamide (less polar diastereomer)
(342) N-{4-[dimethylamino-(3-fluoro-phenyl)-methyl]-cyclohexylmethyl}-2-methylbutyramide (more polar diastereomer)
(343) N-{4-[dimethylamino-(3-fluoro-phenyl)-methyl-cyclohexylmethyl}-2-thiophen-2-yl-acetamide
(344) benzo[b]thiophene-3-carboxylic acid {4-[dimethytamino-(4-fluorophenyl)-methyl]-cyclohexylmethyl}-amide
(345) 2-methyl-5-phenyl-furan-3-carboxylic acid [4-(dimethylamino-phenylmethyl)-cyclohexylmethyl]-amide
(346) N-{4-[dimethylamino-(3-fluoro-phenyl)-methyl]-cyclohexylmethyl}-2-phenoxy-propionamide
(347) 3-cyano-N-[4-(dimethylamino-thiophen-2-yl-methyl)-cyclohexylmethyl]-benzamide
(348) N-{4-[dimethylamino-(3-fluoro-phenyl)-methyl]-cyclohexylmethyl}-2-phenyl-acetamide (less polar diastereomer)
(349) N-[4-(dimethylamino-phenyl-methyl)-cyclohexylmethyl]-2-(3-methoxyphenyl)-acetamide
(350) N-{4-[dimethylamyno-(3-fluoro-phenyl)-methyl]-cyclohexylmethyl}-4-fluoro-3-trifluoromethyl-benzamide
(351) N-{4-[(4-chloro-phenyl)-dimethylamino-methyl]-cyclohexylmethyl}-2-ethyl-sulfanyl-nicotinamide
(352) N-[4-(dimethylamino-thiophen-2-yl-methyl)-cyclohexylmethyl]-2-p-tolyloxy-nicotinamide (more polar diastereomer)
(353) 2-methyl-5-phenyl-furan-3-carboxylic acid {4-[dimethylamino-(3-fluorophenyl)-methyl]-cyclohexylmethyl}-amide
(354) 2-chloro-N-{4-[(4-chloro-phenyl)-dimethylamino-methyl]-cyclohexylmethyl}-benzamide
(355) 2-chloro-N-[4-(dimethylamino-phenyl-methyl)-cyclohexylmethyl]-nicotinamide
(356) N-[4-(dimethylamino-thiophen-2-yl-methyl)-cyclohexylmethyl]-4-propyl-benzamide
(357) N-[4-(dimethylamino-phenyl-methyl)-cyclohexylmethyl]-3,4-difluoro-benzamide (more polar diastereomer)
(358) 3-bromo-N-[4-(dimethylamino-phenyl-methyl)-cyclohexylmethyl]-benzamide
(359) N-{4-[dimethylamino-(4-fluoro-phenyl)-methyl]-cyclohexylmethyl}-2-thiophen-2-yl-acetamide
(360) 2-(4-chloro-phenoxy)-N-[4-(dimethylamino-phenyl-methyl)-cyclohexylmethyl]-nicotinamide (less polar diastereomer)
(361) 2,4-dichloro-N-[4-(dimethylamino-phenyl-methyl)-cyclohexylmethyl]-benzamide
(362) N-[4-(dimethylamino-phenyl-methyl)-cyclohexylmethyl]-3-methyl-benzamide
(363) 2-bromo-N-{4-[dimethylamino-(3-fluoro-phenyl)-methyl]-cyclohexylmethyl}-benzamide
(364) N-{4-[dimethylamino-(3-fluoro-phenyl)-methyl]-cyclohexylmethyl}-3-trifluoro-methyl-benzamide
(365) 2-phenyl-thiazole-4-carboxylic acid {4-[dimethylamino-(3-fluoro-phenyl)-methyl]-cyclohexylmethyl}-amide
(366) 2-tert-butyl-5-methyl-2H-pyrazole-3-carboxylic acid [4-(dimethylamino-thiophen-2-yl-methyl)-cyclohexylmethyl]-amide
(367) 3-chloro-4-(propane-2-sulfonyl)-thiophcnc-2-carboxylic acid [4-(dimethylamino-thiophen-2-yl-methyl)-cyclohexylmethyl]-amide
(368) N-{4-[dimethylamino-(3-fluoro-phenyl)-methyl]-cyclohexylmethyl}-2-methoxy-benzamide
(369) N-{4-[(4-chloro-phenyl)-dimethylamino-methyl]-cyclohexylmethyl}-3-trifluoromethyl-benzamide
(370) 1-(4-chloro-phenyl)-cyclopentane-carboxylic acid {4-[dimethylamino-(4-fluoro-phenyl)-methyl]-cyclohexylmethyl}-amide
(371) 3,5-dichloro-N-{4-[dimethylamino-(3-fluoro-phenyl)-methyl]-cyclohexylmethyl}-benzamide
(372) benzo[b]thiophene-3-carboxylic acid {4-[(4-chloro-phenyl)-dimethylamino-methyl]-cyclohexylmethyl}-amide (more polar diastereomer)
(373) 2-methyl-5-phenyl-furan-3-carboxylic acid {4-[dimethylamino-(3-fluorophenyl)-methyl]-cyclohexylmethyl}-amide
(374) 2-(4-chloro-phenylsulfanyl)-N-[4-(dimethylamino-phenyl-methyl)-cyclohexyl-methyl]-nicotinamide
(375) 4-bromo-2-ethyl-5-methyl-2H-pyrazole-3-carboxylic acid [4-(dimethylamino-thiophen-2-yl-methyl)-cyclohexylmethyl]-amide

(376) 5-chloro-4-methoxy-thiophene-3-carboxylic acid {4-[dimethylamino-(4-fluoro-phenyl)-methyl]-cyclohexylmethyl}-amide
(378) N-{4-[dimethylamino-(3-fluoro-phenyl)-methyl]-cyclohexylmethyl}-2-methylbutyramide (less polar diastereomer)
(379) 5-methyl-isoxazole-3-carboxylic acid [4-(dimethylamino-phenyl-methyl)-cyclohexylmethyl]-amide
(380) benzo[b]thiophene-3-carboxylic acid [4-(1-dimethylamino-3-phenylpropyl)-cyclohexylmethyl]-amide
(381) N-{4-[dimethylamino-(4-fluoro-phenyl)-methyl]-cyclohexylmethyl}-2-methylsulfanyl-nicotinamide
(382) N-{4-[(4-chloro-phenyl)-dimethylamino-methyl]-cyclohexylmethyl}-2-p-tolyloxy-nicotinamide
(383) 2-(4-chloro-phenoxy)-N-[4-(dimethylamino-phenyl-methyl)-cyclohexylmethyl]-nicotinamide (more polar diastereomer)
(384) N-{4-[dimethylamino-(3-fluoro-phenyl)-methyl]-cyclohexylmethyl}-2-methylbenzamide
(385) 5-methyl-isoxazole-3-carboxylic acid {4-[(4-chloro-phenyl)-dimethylamino-methyl]-cyclohexylmethyl}-amide
(386) 5-bromo-N-[4-(dimethylamino-phenyl-methyl)-cyclohexylmethyl]-nicotinamide
(387) N-[4-(dimethylamino-phenyl-methyl)-cycloxelmethyl]-2-methyl-butyramidc
(388) N-{4-[dimetliylamino-(3-fluoro-phenyl)-methyl]-cyclohexylmethyl}-2-ethylsulfanyl-nicotinamide
(389) N-[4-(dimethylamino-thiophen-2-yl-methyl)-cyclohexylmethyl]-2-p-tolyloxy-nicotinamide (less polar diastereomer)
(390) 4-bromo-2-ethyl-5-methyl-2H-pyrazole-3-carboxylic acid [4-(dimethylamino-phenyl-methyl)-cyclohexylmethyl]-amide
(391) N-{4-[(4-chloro-phenyl)-dimethylamino-methyl]-cyclohexylmethyl}-2-phenoxy-propionamide
(392) N-[4-(dimethylamino-phenyl-methyl)-cyclohexylmethyl]-3,5-dinitro-benzamide
(393) N-{4-[(4-chloro-phenyl)-dimethylamino-methyl]-cyclohexylmethyl}-3-methoxy-benzamide
(394) 2-bromo-N-{4-[(4-chloro-phenyl)-dimethylamino-methyl]-cyclohexylmethyl}-benzamide
(395) 2-bromo-N-(2-{4-[(4-chloro-phenyl)-dimethylamino-methyl]-cyclohexyl}-ethyl)-benzamide
(396) 2-bromo-N-(2-{4-[dimethylamino-(3-fluoro-phenyl)-methyl]-cyclohexyl}-ethyl)-benzamide
(397) 3-chloro-N-{2-[4-(dimethylamino-phenyl-methyl)-cyclohexyl]-ethyl}-benzamide (more polar diastereomer)
(398) 3-chloro-N-{2-[4-(dimethylamino-phenyl-methyl)-cyclohexyl]-ethyl}-benzamide (less polar diastereomer)
(399) 3-chloro-N-(2-{4-[(4-chloro-phenyl)-dimethylamino-methyl]-cyclohexyl}-ethyl)-benzamide
(400) 3-chloro-N-(2-{4-[dimethylamino-(3-fluoro-phenyl)-methyl]-cyclohexyl}-ethyl)-benzamide (less polar diastereomer)
(401) 3-chloro-N-(2-{4-[dimethylamino-(3-fluoro-phenyl)-methyl]-cyclohexyl}-ethyl)-benzamide (more polar diastereomer)
(402) 2-chloro-N-{2-[4-(dimethylamino-phenyl-methyl)-cyclohexyl]-ethyl}-benzamide
(403) 2-chloro-N-(2-{4-[(4-chloro-phenyl)-dimethylamino-methyl]-cyclohexyl}-ethyl)-benzamide
(404) 2-chloro-N-(2-{4-[dimethylamino-(3-fluoro-phenyl)-methyl]-cyclohexyl}-ethyl)-benzamide
(405) 4-chloro-N-{2-[4-(dimethylamino-phenyl-methyl)-cyclohexyl]-ethyl}-benzamide
(406) 4-chloro-N-(2-{4-[dimethylamino-(3-fluoro-phenyl)-methyl]-cyclohexyl}-ethyl)-benzamide
(407) N-{2-[4-(dimethylamino-phenyl-methyl)-cyclohexyl]-ethyl}-4-fluoro-benzamide
(408) N-(2-{4-[(4-chloro-phenyl)-dimethylamino-methyl]-cyclohexyl}-ethyl)-4-fluoro-benzamide
(409) N-(2-{4-[dimethylamino-(3-fluoro-phenyl)-methyl]-cyclohexyl}-ethyl)-4-fluoro-benzamide
(410) N-{2-[4-(dimethylamino-phenyl-methyl)-cyclohexyl]-ethyl}-2-fluoro-benzamide
(411) N-(2-{4-[dimethylamino-(3-fluoro-phenyl)-methyl]-cyclohexyl}-ethyl)-2-fluoro-benzamide
(412) N-(2-{4-[dimethylamino-(3-fluoro-phenyl)-methyl]-cyclohexyl}-ethyl)-3-methyl-benzamide
(413) 2,6-dichloro-N-{2-[4-(dimethylamino-phenyl-methyl)-cyclohexyl]-ethyl}-benzamide
(414) N-{2-[4-(dimethylamino-phenyl-methyl)-cyclohexyl]-ethyl}-2-methoxybenzamide
(415) N-(2-{4-[dimethylamino-(3-fluoro-phenyl)-methyl]-cyclohexyl}-ethyl)-2-methoxy-benzamide
(416) 3,4-dichloro-N-{2-[4-(dimethylamino-phenyl-methyl)-cyclohexyl]-ethyl}-benzamide
(417) N-{2-[4-(dimethylamino-phenyl-methyl)-cyclohexyl]-ethyl}-2-methyl-benzamide (more polar diastereomer)
(418) N-{2-[4-(dimethylamino-phenyl-methyl)-cyclohexyl]-ethyl}-2-methyl-benzamide (less polar diastereomer)
(419) N-(2-{4-[(4-chloro-phenyl)-dimethylamino-methyl]-cyclohexyl}-ethyl)-2-methyl-benzamide
(420) N-(2-{4-[dimethylamino-(3-fluoro-phenyl)-methyl]-cyclohexyl}-ethyl)-2-methyl-benzamide
(421) 4-cyano-N-{2-[4-(dimethylamino-phenyl-methyl)-cyclohexyl]-ethyl}-benzamide
(422) 3-chloro-N-(2-{4-[dimethylamino-(4-fluoro-phenyl)-methyl]-cyclohexyl}-ethyl)-benzamide (more polar diastereomer)
(423) 3-chloro-N-(2-{4-[dimethylamino-(4-fluoro-phenyl)-methyl]-cyclohexyl}-ethyl)-benzamide (less polar diastereomer)

(424) 3-chloro-N-{2-[4-(dimethylamino-thiophen-2-yl-methyl)-cyclohexyl]-ethyl}-benzamide
(425) 2-chloro-N-{2-[4-(dimethylamino-thiophen-2-yl-methyl)-cyclohexyl]-ethyl}-benzamide
(426) N-{2-[4-(dimethylamino-thiophen-2-yl-methyl)-cyclohexyl]-ethyl}-4-fluoro-benzamide
(427) N-(2-{4-[dimethylamino-(4-fluoro-phenyl)-methyl]-cyclohexyl}-ethyl)-2-fluoro-benzamide
(428) N-{2-[4-(dimethylamino-thiophen-2-yl-methyl)-cyclohexyl]-ethyl}-2-fluoro-benzamide
(429) N-(2-{4-[dimethylamino-(4-fluoro-phenyl)-methyl]-cyclohexyl}-ethyl)-3-methyl-benzamide
(430) N-{2-[4-(dimethylamino-thiophen-2-yl-methyl)-cyclohexyl]-ethyl}-3-methyl-benzamide
(431) 2,6-dichloro-N-{2-[4-(dimethylamino-thiophen-2-yl-methyl)-cyclohexyl]-ethyl}-benzamide
(432) N-(2-{4-[dimethylamino-(4-fluoro-phenyl)-methyl]-cyclohexyl}-ethyl)-2-methoxy-benzamide
(433) N-{2-[4-(dimethylamino-phenyl-methyl)-cyclohexyl]-ethyl}-3,5-difluoro-benzamide
(434) N-(2-{4-[(4-chloro-phenyl)-dimethylamino-methyl]-cyclohexyl}-ethyl)-3,5-difluoro-benzamide
(435) N-(2-{4-[dimethylamino-(3-fluoro-phenyl)-methyl]-cyclohexyl}-ethyl)-3,5-difluoro-benzamide
(436) N-(2-{4-[(4-chloro-phenyl)-dimethylamino-methyl]-cyclohexyl}-ethyl)-2,4-difluoro-benzamide
(437) 2,4-dichloro-N-(2-{4-[(4-chloro-phenyl)-dimethylamino-methyl]-cyclohexyl}-ethyl)-5-fluoro-benzamide
(438) 2,4-dichloro-N-(2-{4-[dimethylamino-(3-fluoro-phenyl)-methyl]-cyclohexyl}-ethyl)-5-fluoro-benzamide (more polar diastereomer)
(439) 2,4-dichloro-N-(2-{4-[dimethylamino-(3-fluoro-phenyl)-methyl]-cyclohexyl}-ethyl)-5-fluoro-benzamide (less polar diastereomer)
(440) 2,4-dichloro-N-{2-[4-(dimethylamino-thiophen-2-yl-methyl)-cyclohexyl]-ethyl}-5-fluoro-benzamide
(441) 2-chloro-N-(2-{4-[(4-chloro-phenyl)-dimethylamino-methyl]-cyclohexyl}-ethyl)-nicotinamide
(442) naphthalene-2-carboxylic acid (2-{4-[dimethylamino-(4-fluoro-phenyl)-methyl]-cyclohexyl}-ethyl)-amide
(443) N-{2-[4-(dimethylamino-thiophen-2-yl-methyl)-cyclohexyl]-ethyl}-4-propyl-benzamide
(444) N-{2-[4-(dimethylamino-phenyl-methyl)-cyclohexyl]-ethyl}-3,4-difluoro-benzamide
(445) N-(2-{4-[(4-chloro-phenyl)-dimethylamino-methyl]-cyclohexyl}-ethyl)-3,4-difluoro-benzamide
(446) N-{2-[4-(dimethylamino-thiophen-2-yl-methyl)-cyclohexyl]-ethyl}-3,4-difluoro-benzamide
(447) N-(2-{4-[dimethylamino-(4-fluoro-phenyl)-methyl]-cyclohexyl}-ethyl)-3-methoxy-benzamide
(448) N-{2-[4-(dimethylamino-phenyl-methyl)-cyclohexyl]-ethyl}-2,2-diphenylacetamide
(449) 1-(4-chloro-phenyl)-cyclopentane-carboxylic acid {2-[4-(dimethylaminophenyl-methyl)-cyclohexyl]-ethyl}-amide
(450) 2-benzyloxy-N-{2-[4-(dimethylamino-phenyl-methyl)-cyclohexyl]-ethyl}-acetamide
(451) N-{2-[4-(dimethylamino-phenyl-methyl)-cyclohexyl]-ethyl}-2-phenyl-acetamide
(452) thiophene-2-carboxylic acid {2-[4-(dimethylamino-phenyl-methyl)-cyclohexyl]-ethyl}-amide
(453) N-(2-{4-[dimethylamino-(3-fluoro-phenyl)-methyl]-cyclohexyl}-ethyl)-2-(3-methoxy-phenyl)-acetamide
(454) N-{2-[4-(dimethylamino-thiophen-2-yl-methyl)-cyclohexyl]-ethyl}-2-(3-methoxy-phenyl)-acetamide
(455) N-(2-{4-[(4-chloro-phenyl)-dimethylamino-methyl]-cyclohexyl}-ethyl)-2-phenyl-butyramide
(456) N-{2-[4-(dimethylamino-thiophen-2-yl-methyl)-cyclohexyl]-ethyl}-2-phenyl-butyramide
(457) benzo[b]thiophene-2-carboxylic acid {2-[4-(dimethylamino-phenylmethyl)-cyclohexyl]-ethyl}-amide
(458) N-{2-[4-(dimethylamino-phenyl-methyl)-cyclohexyl]-ethyl}-4-nitrobenzamide
(459) 3-bromo-N-(2-{4-[dimethylamino-(4-fluoro-phenyl)-methyl]-cyclohexyl}-ethyl)-benzamide
(460) N-{2-[4-(dimethylamino-phenyl-methyl)-cyclohexyl]-ethyl}-2,3,4,5,6-pentafluoro-benzamide
(461) N-{2-[4-(dimethylamino-phenyl-methyl)-cyclohexyl]-ethyl}-2,6-difluoro-benzamide
(462) N-(2-{4-[dimethylamino-(3-fluoro-phenyl)-methyl]-cyclohexyl}-ethyl)-2,6-difluoro-benzamide
(463) 2-phenyl-thiazole-4-carboxylic acid {2-[4-(dimethylamino-phenylmethyl)-cyclohexyl]-ethyl}-amide
(464) 2-phenyl-thiazole-4-carboxylic acid (2-{4-[dimethylamino-(4-fluorophenyl)-methyl]-cyclohexyl}-ethyl)-amide
(465) benzo[b]thiophene-3-carboxylic acid {2-[4-(dimethylamino-thiophen-2-yl-methyl)-cyclohexyl]-ethyl}-amide
(466) N-{2-[4-(dimethylamino-phenyl-methyl)-cyclohexyl]-ethyl}-2-methylsulfanyl-nicotinamide
(467) 2-methyl-5-phenyl-furan-3-carboxylic acid {2-[4-(dimethylamino-phenylmethyl)-cyclohexyl]-ethyl}-amide
(468) 2-(2,3-dihydro-benzofuran-5-yl)-thiazole-4-carboxylic acid {2-[4-(dimethylamino-phenyl-methyl)-cyclohexyl]-ethyl}-amide
(469) 3-(2,6-dichloro-plienyl)-5-methyl-isoxazole-4-carboxylic acid {2-[4-(dimethylamino-thiophen-2-yl-methyl)-cyclohexyl]-ethyl}-amide
(470) 2-(4-chloro-phenylsulfanyl)-N-{2-[4-(dimethylamino-phenyl-methyl)-cyclohexyl]-ethyl}-nicotinamide (more polar diastereomer)
(471) 2-(4-chloro-phenylsulfanyl)-N-{2-[4-(dimethylamino-phenyl-methyl)-cyclohexyl]-ethyl}-nicotinamide (less polar diastereomer)
(472) benzo[1,2,3]thiadiazole-5-carboxylic acid {2-[4-(dimethylamino-phenylmethyl)-cyclohexyl]-ethyl}-amide

(473) 5-bromo-N-{2-[4-(dimethylamino-phenyl-methyl)-cyclohexyl]-ethyl}-nicotinamide
(474) 5-chloro-4-methoxy-thiophene-3-carboxylic acid {2-[4-(dimethylaminophenyl-methyl)-cyclohexyl]-ethyl}-amide
(475) 5-chloro-4-methoxy-thiophene-3-carboxylic acid (2-{4-[dimethylamino-(4-fluoro-phenyl)-methyl]-cyclohexyl}-ethyl)-amide
(476) 5-chloro-4-methoxy-thiophene-3-carboxylic acid {2-[4-(1-dimethylamino-3-phenyl-propyl)-cyclohexyl]-ethyl}-amide
(477) 3-cyano-N-{2-[4-(dimethylamino-phenyl-methyl)-cyclohexyl]-ethyl}-benzamide
(478) N-{2-[4-(dimethylamino-phenyl-methyl)-cyclohexyl]-ethyl}-2,4-dimethoxy-benzamide
(479) 2-chloro-N-((4-((dimethylamino)(phenyl)methyl)cyclohexyl)methyl)-benzamide
(480) N-((4-((dimethylamino)(phenyl)methyl)cyclohexyl)methyl)-4-fluoro-benzamide
(481) N-(2-(4-((dimethylamino)(phenyl)methyl)cyclohexyl)ethyl)-4-fluoro-benzamide
(482) N-((4-((dimethylamino)(phenyl)methyl)cyclohexyl)methyl)-2-fluoro-benzamide
(483) N-((4-((dimethylamino)(phenyl)methyl)cyclohexyl)methyl)-3-methyl-benzamide
(484) N-((4-((dimethylamino)(phenyl)methyl)cyclohexyl)methyl)-2-methoxybenzamide
(485) N-(2-(4-((dimethylamino)(phenyl)methyl)cyclohexyl)ethyl)-3,5-dimethoxy-benzamide
(486) N-((4-((dimethylamino)(3-fluorophenyl)methyl)cyclohexyl)methyl)-2,6-dimethoxybenzamide
(487) N-((4-((dimethylamino)(phenyl)methyl)cyclohexyl)methyl)-2,4-difluoro-benzamide
(488) N-((4-((dimethylamino)(thiophen-2-yl)methyl)cyclohexyl)methyl)-3-methoxy-benzamide
(489) N-((4-((dimethylamino)(thiophen-2-yl)methyl)cyclohexyl)methyl)-3,4,5-trimethoxybenzamide
(490) 4-((dimethylamino)(phenyl)methyl)-1-(4-fluoro-3-methylphenyl)cyclohexanol
(491) N-cyclohexyl-2-(4-(2-phenyl-1-(pyrrolidin-1-yl)ethyl)cyclohexyl)-acetamide
(492) N-(3-methoxyphenyl)-2-(4-(2-phenyl-1-(pyrrolidin-1-yl)ethyl)cyclohexyl)-acetamide
(493) N-(4-methoxyphemyl)-2-(4-(piperidin-1-yl(p-tolyl)methyl)cyclo-hexylidene)-acetamide
(494) N-phenethyl-2-(4-(piperidin-1-yl(p-tolyl)methyl)cyclohexylidene)-acetamide
(495) 2-(4-(2-phenyl-1-(pyrrolidin-1-yl)ethyl)cyclohexylidene)-N-(pyridin-2-ylmethyl)acetamide
(496) N-benzyl-N-methyl-2-(4-(piperidin-1-yl(p-tolyl)methyl)cyclohexylidene)-acetamide
(497) 3-thiophen-2-yl-[1,2,4]oxadiazole-5-carboxylic acid [4-(dimethylaminophenyl-methyl)-cyclohexyl]-amide
(498) 3-methyl-[1,2,4]oxadiazole-5-carboxylic acid {2-[4-(dimethylaminophenyl-methyl)-cyclohexyl]-ethyl}-amide
(499) 3-phenyl-[1,2,4]oxadiazole-5-carboxylic acid {4-[dimethylamino-(3-fluoro-phenyl)-methyl]-cyclohexyl}-amide
(500) 3-cyclopropylmethyl-[1,2,4]oxadiazole-5-carboxylic acid {4-[dimethylamino-(3-fluoro-phenyl)-methyl]-cyclohexyl}-amide and
(501) 3-methoxymethyl-[1,2,4]oxadiazole-5-carboxylic acid {2-[4-(1-dimethylamino-3-phenyl-propyl)-cyclohexyl]-ethyl}-amide.

**23.** A process for preparing an inventive cyclohexylmethyl derivative according to claim 1, wherein $R^1$ represents $(CH_2)_nC(O)H$, wherein ketones of the general formula G

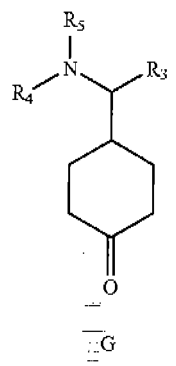

are reacted with (methoxymethyl)triphenylphosphonium chloride and a strong base, for example potassium tert-butoxide, at a temperature between -20°C and +30°C to give the corresponding aldehydes H,
wherein the reaction step is optionally repeated for the synthesis of aldehydes wherein n>0.

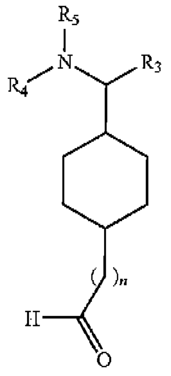

H

**24.** A process for preparing an inventive cyclohexylmethyl derivative according to claim 1, wherein $R^1$ represents $(CH_2)_mCHN\text{-}OH$, $=N\text{-}OH$, $(CH_2)_nNH_2$, wherein the ketone G or the aldehydes H

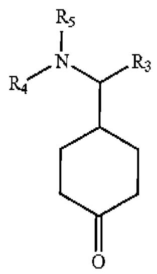

G

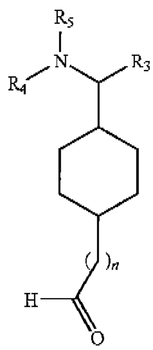

H

is or are converted into oximcs of the general formula K by reacting with hydroxylamine hydrochloride in an organic solvent, for example ethanol, with addition of a base, for example a basic ion exchanger Amb erlyst, and the amines of the general formula L are obtained by reacting with a reducing agent, for example $LiA<lH_4$.

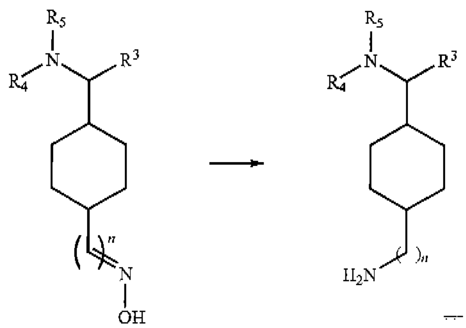

K L

**25.** A process for preparing an inventive cyclohexylmethyl derivative according to claim 1, wherein $R^1$ represents $(CH_2)_nNHC(O)R^{13}$ or $(CH_2)_nNHSO_2R^{12}$, wherein amines of the general formula L

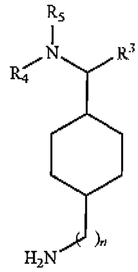

I,

are joined to carboxylic acids or sulfonic acids with the addition of coupling reagents or by activating an acid component, in particular by preparing the acid chloride.

**26.** A process for preparing an inventive cyclohexylmethyl derivative according to claim 1, wherein $R^1$ represents $(CH_2)_nNHC(O)NR^{10}R^{11}$ or $(CH_2)_nNHC(S)NR^{10}R^1$, wherein amines of the general formula L

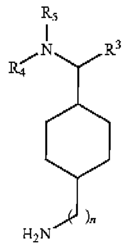

L

are reacted with suitable isocyanates of the general formula $R^{10}$-N=C=O or isothiocyanates of the general formula $R^{10}$-N=C=S, optionally in the presence of at least one base, to give a compound of the general formula I, wherein $R^1$ denotes $(CH_2)_nNHC(O)NR^{10}R^{11}$ or $(CH_2)_nNHC(S)NR^{10}R^{11}$ and $R^{11}$ denotes H, and this compound, optionally in the presence of at least one base, is converted into a compound of the general formula I wherein $R^1$ denotes $(CH_2)_nNHC(O)NR^{10}R^{11}$ or $(CH_2)_nNHC(S)NR^{10}R^{11}$, wherein $R^{11}$ docs not denote H, with a compound of the general formula LG-$R^{11}$, wherein LG represents a leaving group and $R^{11}$ is as defined above with the exception of hydrogen.

**27.** A process for preparing an inventive cyclohexylmethyl derivative according to claim 1, wherein $R^1$ represents C(O)OR$^9$ linked via a $C_{1-3}$-alkyl group, which may be saturated or unsaturated;
or $R^1$ and $R^2$ together represent , wherein a phosphonoacetic acid ester, preferably phosphonoacetic acid trimethyl ester or phosphonoacetic acid triethyl ester, is initially reacted with a strong base, preferably potassium tert-butoxide, sodium hydride or butyllithium, then with a ketone of the general formula G or with an aldehyde H

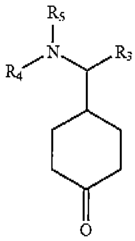

G

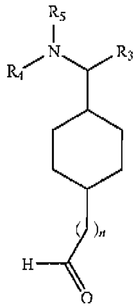

H

and the esters are optionally hydrolyzed with a suitable aqueous, basic solution, preferably with potassium hydroxide or lithium hydroxide solution, at room temperature or slightly elevated temperature to give the corresponding carboxylic acids, or the double bond is reduced, preferably by heterogeneous catalytic hydrogenation on palladium or platinum catalysts or by homogeneously catalysed hydrogenation with rhodium catalysts, in each case at temperatures of from room temperature to 60°C and under hydrogen pressures of from 1 bar to 6 bar, more preferably at room temperature under a hydrogen pressure from 2 to 3 bar on palladium on carbon. Then the ester hydrolysis is continued as described above. The esters can be reduced to the corresponding alcohols with a reducing agent, for example $LiAlH_4$.

**28.** A process for preparing an inventive cyclohexylmethyl derivative according to claim 1, wherein $R^1$ represents $(CH_2)_nC(O)NR^{10}R^{11}$, wherein carboxylic acids according to claim 27 are reacted with a primary or secondary amine in the presence of water-removing agents or after conversion into an acid chloride or an active ester.

**29.** A process for preparing an inventive step cyclohexylmethyl derivative according to claim 1, wherein $R^1$ represents $(CH_2)_nOR^8$, wherein the ketone G or the aldehydes H

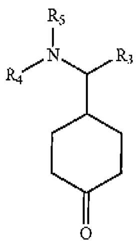

G

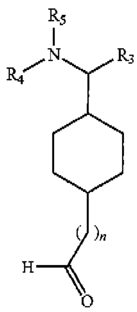

H

is or are converted into inventive compounds in which $R^1$ represents $(CH_2)_nOH$ by reaction with a reducing agent, for example sodium borohydride,

or

the esters of claim 27 are reduced to the corresponding alcohols with a reducing agent, for example $LiAlH_4$,

and these alcohols are reacted with addition of a base, for example NaH, with a compound of the general formula $R^8Hal$, wherein Hal is preferably Cl, to give compounds wherein $R^1$ represents $(CH_2)_nOR^8$, wherein $R^8$ is not H here.

**30.** A process for preparing an inventive cyclohexylmethyl derivative according to claim 1, wherein $R^1$ represents $(CH_2)_nNHR^6$, wherein the ketone G or the aldehydes H

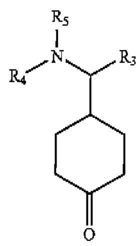

G

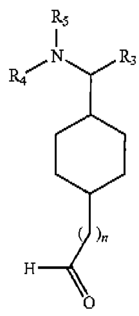

H

is or are dissolved in a polar aprotic solvent, for example THF, and reacted with the corresponding amines of the general formula $NH_2R^6$ with addition of a suitable reducing agent, for example sodium borohydride.

**31.** A process for preparing an inventive cyclohexylmethyl derivative according to claim 1, wherein $R^2$ represents OH and $R^1$ represents $C_{1-8}$-alkyl, in each case branched or unbranched, saturated or unsaturated, unsubstituted or mono- or poly-substituted; aryl or heteroaryl, unsubstituted or mono- or poly-substituted; $C_{3-10}$-cycloalkyl, saturated or unsaturated, unsubstituted or mono- or poly-substituted; an aryl or heteroaryl radical linked via a $C_{1-4}$-alkyl chain and in each case unsubstituted or mono- or poly-substituted, wherein ketones of the general formula G

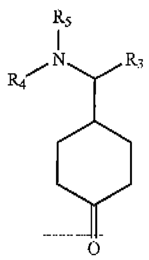

G

are reacted with organometallic compounds of the general formula $R^{Ia}MgHal$, wherein Hal=Cl or Br, or $R^{Ia}Li$, with cooling at from -30 to +10°C, in an organic solvent, for example diethyl ether or THF,
or
isopropylmagnesium chloride solution is added at a temperature of from -30°C to 0° to an aryl iodide in an organic solvent, for example THF, and after a stirring time of at least 10 minutes, the solution is reacted with the ketone of the general formula G to give a compound of the general formula I, wherein $R^2$ represents OH and $R^1$ represents aryl.

**32.** A pharmaceutical formulation comprising at least one substituted cyclohexylmethyl derivative according to claim 1 optionally in the form of the racemate; of the enantiomers, diastereomers, mixtures of the enantiomers or diastereomers or of a single enantiomer or diastereomer; the bases and/or salts of physiologically tolerated acids, and optionally comprising suitable additional and/or auxiliary substances and/or optionally further active compounds.

**33.** The use of a substituted cyclohexylmethyl derivative according to claim 1 optionally in the form of the racemate; of the enantiomers, diastereomers, mixtures of the enantiomers or diastereomers or of a single enantiomer or diastereomer; the bases and/or salts of physiologically tolerated acids, for production of a pharmaceutical formulation for treating pain, especially acute, neuropathic or chronic pain.

**34.** The use of a substituted cyclohexylmethyl derivative according to claim 1, optionally in the form of the racemate; of the enantiomers, diastereomers, mixtures of the enantiomers or diastereomers or of a single enantiomer or diastereomer; the bases and/or salts of physiologically tolerated acids, for preparing a pharmaceutical formulation for treating depression, urinary incontinence, diarrhoea, pruritus, alcohol and drug abuse, medicament dependency, lack of drive and/or for anxiolysis.

## Revendications

**1.** Dérivés cyclohexylméthylés substitués de formule générale I

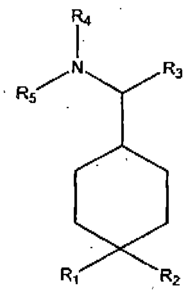

I

dans laquelle
$R^1$ est un groupe alkyle en $C_{1-8}$, chacun étant ramifié ou non ramifié, saturé ou insaturé, non substitué ou une ou plusieurs fois substitué ; aryle ou hétéroaryle, non substitué ou une ou plusieurs fois substitué ; cycloalkyle en $C_{3-10}$, saturé ou insaturé, non substitué ou une ou plusieurs fois substitué ; un radical aryle ou hétéroaryle, relié par l'intermédiaire d'une chaîne alkyle en $C_{1-4}$, chacun étant non substitué ou une ou plusieurs fois substitué ; $(CH_2)_mCHN\text{-}OH$, $(CH_2)_nNR^6R^7$ ou $(CH_2)_nOR^8$, n valant 0, 1, 2 ou 3, et m valant 0, 1 ou 2 ; ou $C(O)OR^9$, relié par l'intermédiaire d'un groupe alkyle en $C_{1-3}$ qui peut être saturé ou insaturé; $CONR^{10}R^{11}$;
$R^2$ est H ou OH ;
ou $R^1$ et $R^2$ représentent ensemble

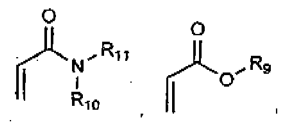

ou =N-OH ;
$R^3$ est un groupe aryle ou hétéroaryle, non substitué ou une ou plusieurs fois substitué ; ou un groupe aryle relié par l'intermédiaire d'un groupe alkyle en $C_{1-3}$, qui peut être non substitué ou une ou plusieurs fois substitué;
$R^4$ et $R^5$ représentent chacun indépendamment de l'autre H ; un groupe alkyle en $C_{1-3}$, où $R^4$ et $R^5$ ne représentent pas simultanément H, ou les radicaux $R^4$ et $R^5$ forment ensemble un radical $CH_2CH_2OCH_2CH_2$, ou $(CH_2)_{3-6}$ ;
$R^6$ est H ; un groupe alkyle en $C_{1-8}$, saturé ou insaturé, ramifié ou non ramifié, non substitué ou une ou plusieurs fois substitué ; aryle, hétéroaryle ou cycloalkyle en $C_{3-10}$, chacun étant non substitué ou une ou plusieurs fois substitué, ou un groupe aryle ou hétéroaryle, relié par l'intermédiaire d'une chaîne alkyle en $C_{1-4}$, dont chacun est non substitué ou une ou plusieurs fois substitué ;
$R^7$ est H ; un groupe alkyle en $C_{1-8}$, saturé ou insaturé, ramifié ou non ramifié, non substitué ou une ou plusieurs fois substitué ; aryle, hétéroaryle ou cycloalkyle en $C_{3-10}$, chacun étant non substitué ou une ou plusieurs fois substitué ; ou un groupe aryle ou hétéroaryle relié par l'intermédiaire d'une chaîne alkyle en $C_{1-4}$, chacun étant non substitué ou une ou plusieurs fois substitué ; $C(O)NR^{10}R^{11}$, $C(S)NR^{10}R^{11}$, $SO_2R^{12}$ ou $C(O)R^{13}$;
$R^8$ est H ; un groupe alkyle en $C_{1-8}$, chacun étant ramifié ou non ramifié, saturé ou insaturé, non substitué ou une ou plusieurs fois substitué ; cycloalkyle en $C_{3-10}$, saturé ou insaturé, non substitué ou une ou plusieurs fois substitué ; un groupe aryle ou hétéroaryle relié par l'intermédiaire d'un groupe alkyle en $C_{1-4}$, non substitué ou une ou plusieurs fois substitué ;
$R^9$ est H ; un groupe alkyle en $C_{1-6}$ saturé ou insaturé, ramifié ou non ramifié, non substitué ou une ou plusieurs fois substitué ;
$R^{10}$ et $R^{11}$ représentent chacun indépendamment de l'autre H ; un groupe cycloalkyle en $C_{3-10}$, saturé ou insaturé, non substitué ou une ou plusieurs fois substitué ; aryle ou hétéroaryle, chacun étant non substitué ou une ou plusieurs fois substitué ; ou un groupe aryle ou hétéroaryle relié par l'intermédiaire d'une chaîne alkyle en $C_{1-4}$, chacun étant non substitué ou une ou plusieurs fois substitué ;
$R^{12}$ est un groupe aryle ou hétéroaryle, chacun étant non substitué ou une ou plusieurs fois substitué ; alkyle en

$C_{1\ 8}$, chacun étant saturé ou insaturé, ramifié ou non ramifié, non substitué ou une ou plusieurs fois substitué ; cycloalkyle en $C_{3-10}$, saturé ou insaturé, non substitué ou une ou plusieurs fois substitué ; ou un groupe aryle ou hétéroaryle relié par l'intermédiaire d'une chaîne alkyle en $C_{1-3}$, chacun étant non substitué ou une ou plusieurs fois substitué ;

$R^{13}$ est un groupe alkyle en $C_{1-8}$, saturé ou insaturé, ramifié ou non ramifié, non substitué ou une ou plusieurs fois substitué ; cycloalkyle en $C_{3-10}$, aryle ou hétéroaryle, chacun étant non substitué ou une ou plusieurs fois substitué ; ou un groupe aryle ou hétéroaryle relié par l'intermédiaire d'une chaîne alkyle en $C_{1-4}$, chacun étant non substitué ou une ou plusieurs fois substitué, la chaîne alkyle pouvant être ramifiée ou non ramifiée, non substituée ou une ou plusieurs fois substituée ;

sous forme du racémate, des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles.

**2.** Dérivés cyclohexylméthylés substitués selon la revendication 1, dans lesquels $R^1$ est un groupe alkyle en $C_{1-8}$, chacun étant ramifié ou non ramifié, saturé ou insaturé, non substitué ou une ou plusieurs fois substitué ; aryle ou hétéroaryle, non substitué ou une ou plusieurs fois substitué ; cycloalkyle en $C_{3\ 10,}$ saturé ou insaturé, non substitué ou une ou plusieurs fois substitué ; un groupe aryle ou hétéroaryle relié par l'intermédiaire d'une chaîne alkyle en $C_{1-4}$, chacun étant non substitué ou une ou plusieurs fois substitué, et $R^2$ est OH.

**3.** Dérivés cyclohexylméthylés substitués selon la revendication 1, dans lesquels $R^1$ est $(CH_2)_mCHN\text{-}OH$, $(CH_2)_nNR^6R^7$ ou $(CH_2)_nOR^8$, n valant 0, 1, 2 ou 3, et m valant 0, 1 ou 2, ou $C(O)OR^9$ ou $CONR^{10}R^{11}$, relié par l'intermédiaire d'un groupe alkyle en $C_{1-3}$, qui peut être saturé ou insaturé, et $R^2$ est H.

**4.** Dérivés cyclohexylméthylés substitués selon la revendication 1 ou 2, dans lesquels $R^1$ est un groupe alkyle en $C_{1-8}$, chacun étant ramifié ou non ramifié, saturé ou insaturé, non substitués ou une ou plusieurs fois substitué par un ou des substituants méthyle, =0, phényle ou $CO_2CH_3$ ; phényle, naphtyle, benzyle, phénéthyle, 2-pyridyle ou 2-thiényle, non substitués ou une ou plusieurs fois substitués par un ou des substituants F, $CH_3$, Cl, tert-butyle, méthoxy ou $CF_3$ ; cyclohexyle ou cyclopentyle.

**5.** Dérivés cyclohexylméthylés substitués selon la revendication 1 ou 2, dans lesquels $R^1$ est le groupe 2,4-difluorophényle, 4-fluoro-3-méthylphényle, phényle, 3-méthoxybenzyle, 4-chlorophényle, benzyle, 2-méthylphényle, 4-tert-butylphényle, cyclopentyle, 4-fluorophényle, phénéthyle, 2-thiényle, 2,4-dichlorophenyle, 3-méthoxyphényle, 4-méthylphényle, 4-méthoxyphényle, 3,5-difluorophényle, isopropyle, butyle, éthyle, hexyle, sec-butyle, 2,4,6-triméthylphényle, pentyle, propyle, 3-fluorophényle, 3,5-dichlorophényle, 4-fluorobenzyle, 4-chloro-3-trifluorométhylphényle, cyclohexyle, isobutyle ou 2,5-diméthoxyphényle.

**6.** Dérivés cyclohexylméthylés substitués selon l'une des revendications 1-3, dans lesquels $R^3$ est le groupe phényle ou thiényle, chacun étant non substitué ou une ou plusieurs fois substitué par un ou des substituants F, Cl, CN, $NO_2$, SH, S-(alkyle en $C_{1-6}$), OH, O-(alkyle en $C_{1-6}$), $CO_2H$, $CO_2$-(alkyle en $C_{1-6}$), $CF_3$, alkyle en $C_{1-6}$ ; un groupe phényle lié par l'intermédiaire d'une chaîne alkyle en $C_{1-3,}$ chacun étant non substitué ou une ou plusieurs fois substitué par un ou des substituants F, Cl, CN, $NO_2$, SH, S-(alkyle en $C_{1-6}$), OH, O-(alkyle en $C_{1-6}$), $CO_2H$, $CO_2$-(alkyle en $C_{1-6}$), $CF_3$, alkyle en $C_{1-6}$.

**7.** Dérivés cyclohexylméthylés substitués selon la revendication 6, dans lesquels $R^3$ est un groupe phényle, non substitué ou une ou plusieurs fois substitué par un ou des substituants Cl ou F ; phénéthyle ou thiényle.

**8.** Dérivés cyclohexylméthylés substitués selon l'une des revendications 1-3, dans lesquels $R^4$ et $R^5$ représentent H ou $CH_3$, $R^4$ et $R^5$ ne représentant pas simultanément H.

**9.** Dérivés cyclohexylméthylés substitués selon l'une des revendications 1-3, dans lesquels $R^4$ et $R^5$ forment ensemble un radical $(CH_2)_{3-6}$.

**10.** Dérivés cyclohexylméthylés substitués selon l'une des revendications 1 ou 3, dans lesquels $R^6$ est H ; un groupe alkyle en $C_{1-8}$, saturé ou insaturé, ramifié ou non ramifié, non substitué ou une ou plusieurs fois substitué par un ou des substituants F, Cl, -CN, SH, $SCH_3$, $OCH_3$, OH, =O, $CO_2C_2H_5$, ou $CO_2CH_3$ ; aryle, non substitué ou une ou plusieurs fois substitué par un ou des substituants F, Cl, Br, CN, $NH_2$, $NO_2$, SH, $SCH_3$, OH, $OCH_3$, $CF_3$, méthyle, éthyle, propyle, butyle ou tert-butyle ; ou un groupe aryle ou hétéroaryle relié par l'intermédiaire d'une chaîne alkyle en $C_{1-4}$, chacun étant non substitué ou une ou plusieurs fois substitué par un ou des substituants F, Cl, Br, CN,

$NH_2$, $NO_2$, SH, $SCH_3$, OH, $OCH_3$, $CF_3$, méthyle, éthyle, propyle, butyle ou tert-butyle, la chaîne alkyle pouvant être ramifiée ou non ramifiée, non substituée ou une ou plusieurs fois substituée par un ou des substituants $CO_2C_2H_5$ ou $CO_2CH_3$.

**11.** Dérivés cyclohexylméthylés substitués selon la revendication 10, dans lesquels $R^6$ est le groupe 2-indolyléthyle, phénéthyle, 3-phénylpropyle, benzyle, phényle, 4-phénylbutyle, 1-(1H-indol-3-yl)propan-2-yle, ester méthylique de l'acide 4 2-(3-indolyl)propionique, chacun étant non substitué ou une ou plusieurs fois substitué par un ou des substituants F ou $OCH_3$,

**12.** Dérivés cyclohexylméthylés substitués selon la revendication 10, dans lesquels $R^6$ est H.

**13.** Dérivés cyclohexylméthylés substitués selon l'une des revendications 1 ou 3, dans lesquels $R^7$ est $C(O)R^{13}$.

**14.** Dérivés cyclohexylméthylés substitués selon l'une des revendications 1 ou 3, dans lesquels $R^8$ est H ; un groupe phényle relié par l'intermédiaire d'un groupe alkyle en $C_{1-4,}$ non substitué ou une ou plusieurs fois substitué par un ou des substituants F, Cl, Br, CN, $NH_2$, $NO_2$, SH, $SCH_3$, OH, $OCH_3$, $CF_3$, méthyle, éthyle, propyle, butyle ou tert-butyle.

**15.** Dérivés cyclohexylméthylés substitués selon la revendication 14, dans lesquels $R^8$ est le groupe benzyle, non substitué ou une ou plusieurs fois substitué par F.

**16.** Dérivés cyclohexylméthylés substitués selon l'une des revendications 1 ou 3, dans lesquels $R^9$ est un groupe alkyle en $C_{1-8}$, ramifié ou non ramifié.

**17.** Dérivés cyclohexylméthylés substitués selon l'une des revendications 1 ou 3, dans lesquels $R^{10}$ et $R^{11}$ représentent chacun indépendamment de l'autre H ; un groupe phényle, naphtyle, ou un groupe phényle ou un indolyle relié par l'intermédiaire d'une chaîne alkyle en $C_{1-4}$, chacun étant non substitué ou substitué par un ou des substituants F, Cl, Br, CN, $NH_2$, $NO_2$, SH, $SCH_3$, OH, $OCH_3$, $CF_3$, méthyle, éthyle, propyle, butyle ou tert-butyle.

**18.** Dérivés cyclohexylméthylés selon la revendication 17, dans lesquels $R^{10}$ et $R^{11}$ représentent chacun indépendamment de l'autre H ; un groupe naphtyle, phényle ou benzyle, chacun étant non substitué ou une ou plusieurs fois substitué par un ou des substituants $CF_3$, F, $NO_2$ ou Br ; ou cyclohexyle, $R^{10}$ et $R^{11}$ ne représentant pas simultanément H.

**19.** Dérivés cyclohexylméthylés selon l'une des revendications 1 ou 3, dans lesquels $R^{12}$ est le groupe naphtyle, phényle ou benzyle, chacun étant non substitué ou une ou plusieurs fois substitué par un ou des substituants F, Cl, Br, CN, $NH_2$, $NO_2$, SH, $SCH_3$, OH, $OCH_3$, $CF_3$, méthyle, éthyle, propyle, butyle ou tert-bulyle.

**20.** Dérivés cyclohexylméthylés substitués selon l'une des revendications 1 ou 3, dans lesquels $R^{13}$ est H ; un groupe alkyle en $C_{1-8}$, saturé ou insaturé, ramifié ou non ramifié, non substitué ou une ou plusieurs fois substitué par un ou des substituants F, Cl, -CN, NH-(alkyle en $C_{1-6}$), alkyle en $C_{1-6}$, N(alkyle en $C_{1\ 6}$)$_2$, SH, S-(alkyle en $C_{1-6}$), S-benzyle, O-(alkyle en $C_{1-6}$), OH, =O, O-benzyle, C(=O)alkyle en $C_{1-6}$, $CO_2H$, $CO_2$-(alkyle en $C_{1-6}$), phényle ou benzyle ; cycloalkyle en $C_{3-10}$, non substitué ou une ou plusieurs fois substitué par un ou des substituants F, Cl, -CN, NH-(alkyle en $C_{1-6}$), alkyle en $C_{1-6}$, N(alkyle en $C_{1-6}$)$_2$, SH, S-(alkyle en $C_{1-6}$), alkyle en $C_{1-6}$, S-benzyle, O-(alkyle en $C_{1-6}$), OH, =O, O-benzyle, C(=O) (alkyle en $C_{1-6}$), $CO_2H$, $CO_2$-(alkyle en $C_{1-6}$), phényle ou benzyle ; aryle ou hétéroaryle, chacun étant non substitué ou une ou plusieurs fois substitué par un ou des substituants F, Cl, Br, CN, $NH_2$, NH-(alkyle en $C_{1-6}$), N(alkyle en $C_{1-6}$)$_2$, $NO_2$, SH, pyridyle, S-(alkyle en $C_{1-6}$), S-phényle, OH, $(CH_2)_{0-3}$O-(alkyle en $C_{1-6}$), (alkyle en $C_{1-3}$)-(cycloalkyle en $C_{3-6}$), O-(alkyle en $C_{1-6}$)-OH, O-phényle, phényle, benzyle, C(=O)alkyle en $C_{1-6}$, $CO_2H$, $CO_2$-(alkyle en $C_{3-6}$), $CF_3$, alkyle en $C_{1-6}$, dihydrobenzofuranne, $SO_2$phényle ou $SO_2$(alkyle en $C_{1-6}$) ; ou un groupe aryle ou hétéroaryle relié par l'intermédiaire d'une chaîne alkyle en $C_{1-4}$, chacun étant non substitué ou une ou plusieurs fois substitué par un ou des substituants F, Cl, Br, CN, $NH_2$, NH-(alkyle en $C_{1-6}$), N(alkyle en $C_{1-6}$)$_2$, $NO_2$, SH, pyridyle, S-(alkyle en $C_{1-6}$), S-phényle, OH, O-(alkyle en $C_{1-6}$), O-(alkyle en $C_{1-6}$)-OH, O-phényle, phényle, benzyle, C(=O) (alkyle en $C_{1-6}$), $CO_2H$, $CO_2$-(alkyle en $C_{1-6}$), $CF_3$, alkyle en $C_{1-6}$, $SO_2$phényle ou $SO_2$(alkyle en $C_{1-6}$), la chaîne alkyle pouvant être ramifiée ou non ramifiée, non substituée ou une ou plusieurs fois substituée par un ou des substituants F, Cl, -CN, SH, S-(alkyle en $C_{1-6}$), S-benzyle, O-(alkyle en $C_{1-6}$), OH, O-(alkyle en $C_{1-6}$)-OH, O-benzyle, $CO_2$-(alkyle en $C_{1-6}$), phényle ou benzyle.

**21.** Dérivés cyclohexylméthylés substitués selon la revendication 20, dans lesquels $R^{13}$ est le groupe méthyle, éthyle,

phényle, benzyle, 3-pentyle, n-propyle, benzothiényle, 1-(4-chlorophényl)-cyclopentyle, 4-propylphényle, 3-cyanophényle, 3-chlorophényle, 5-chloro-4-méthoxy-thiophèn-3-yle, 3-fluoro-5-trifluorométhylphényle, 4-flucro-5-triflucrométhylphényle, 2-thiényle, 3,5-dichlorophényle, 2,4,5-trifluorophényle, 3-bromophényle, 4-méthylphényle, 3-méthoxyphényle, 2,2-diméthylpropyle, 2-tert-butyl-5-méthyl-pyrazol-3-yle, 2,4-diméthoxyphényle, 3-trifluorométhylphényle, 3,5-difluorophényle, 2-fluoro-5-trifluorométhylphényle, 4-chlorobenzyle, 2-méthoxyphényle, 2-méthylsulfanyl-3-pyridyle, 3,4,5-triméthoxyphényle, 2-éthylsulfanyl-3-pyridyle, 2-méthyl-5-phényl-furann-3-yle, 1-phénoxy-éthyle, tert-butylphényle, 2-(4-chloro-phénylsulfanyl)-3-pyridyle, 2-p-tolyloxy-3-pyridyle, 3-chloro-4-(sulfonyl-2-propyl)-thiophén-2-yle, 5-méthylisoxazol-3-yle, 5-bromo-3-pyridyle, naphtyle, 2-méthyl-5-(4-chloro-phényl)-furann-3-yle, 4-(4-chloro-phényl-sulfonyl)-3-méthyl-thiophèn-2-yle, 1-phénylpropyle, adamantyle, 2-phényl-thiazol-4-yle, 4-méthyl-2-phényl-thiazol-5-yle, 2-(2,3-dihydro-benzofurann-5-yle)-thiazol-4-yle, 3-méthylphényle, 3-chloro-4-méthyl-sulfonyl-thiophèn-2-yle, benzyloxyméthyle, méthyl-thiényle, 4-bromo-2-éthyl-5-méthyl-pyrazol-3-yle, 2,5-diméthylsulfuryle, 5-pyridin-2-yl-thiophène, 3-chloro-4-fluorophényle, cyclohexyle, 3-nitrophényle, 2,5-difluorophényle, 2,6-difluorophényle, 2-trifluorométhyl-5-fluoro-phényle, 4-chlorophénoxy-méthyle, 2-bromophényle, cyclopentyle, benzothiadiazolyle, diphényl-méthyle, 2-méthylphényle, 3-méthoxybenzyle, 2,4,6-trichlorophényle, 2-butyle, 2-chlorophényle, 3,5-dinitrophényle, 4-cyanophényle, 2,4-dichloro-5-fluorophényle, 2-chloro-3-pyridyle, 4-nitrophényle, 2,3,4,5,6-penLafluorophényle ou 3-(2,5-dichloro-phényl)-5-méthyl-isoxazol-4-yle, 5-chloro-4-méthylthiophén-3-yle, 4-fluorophényle, 4-chlorophényle, 2-fluorophényle, 3-méthylphényle, 3-bromophényle, 2,6-dichlorophényle, 3,4-dichlorophényle, 4-cyanophényle, 2,4-difluorophényle, 2,4-dichlorophényle, 2,4-dichloro-5-fluorophényle, 2-chloropyridin-3-yle, 3,5-diméthoxyphényle, 2,6-diméthoxyphényle, 2,3,6-trifluorophényle, 2-(4-chlorophénoxy)-3-pyridyle, 3,4-difluorophényle, 2-(2,3-dihydro-benzofurann-5-yl)-4-méthyl-thiazol-5-yle, 3-méthyl-oxadiazolyle, 3-phényl-oxadiazolyle, 3-cyclopropylméthyl-oxadiazolyle, 3-méthoxyméthyl-oxadiazolyle ou 2,4-diméthoxyphényle.

**22.** Dérivés cyclohexylméthylés substitués selon la revendication 1, appartenant au groupe :

(16) 4-(diméthylamino-phényl-méthyl)-cyclohexanone-oxime
(17) 4-(diméthylamino-phényl-méthyl)-cyclohexylamine
(18) 4-[diméthylamino-(4-fluorophényl)-méthyl]-cyclohexanone-oxime
(19) 4-[diméthylamino-(4-fluorophényl)-méthyl]-cyclohexylamine
(20) 4-[diméthylamino-(3-fluorophényl)-méthyl]-cyclohexanone-oxime
(21) 4-[diméthylamino-(3-fluorophényl)-méthyl]-cyclohexylamine
(22) 4-[(4-chlorophényl)-diméthylamino-méthyl]-cyclohexanone-oxime
(23) 4-[(4-chlorophényl)-diméthylamino-méthyl]-cyclohexylamine
(24) 4-(diméthylamino-thiophèn-2-yl-méthyl)-cyclohexanone-oxime
(25) 4-(diméthylamino-thiophèn-2-yl-méthyl)-cyclohexylamine
(26) 4-(1-diméthylamino-3-phényl-propyl)-cyclohexanone-oxime
(27) 4-(1-diméthylamino-3-phényl-propyl)-cyclohexyl-amine
(29) 4-(diméthylamino-phényl-méthyl)-cyclohexane-carbaldéhyde-oxime
(30) [(4-aminométhyl-cyclohexyl)-phényl-méthyl] - diméthylamine
(32) 4-[diméthylamino-(4-fluorophényl)-méthyl]-cyclohexanecarbaldéhyde-oxime
(33) [(4-aminométhyl-cyclohexyl)-(4-fluorophényl)-méthyl]-diméthylamine
(35) 4-[diméthylamino-(3-fluorophényl)-méthyl]-cyclohexanecarbaldéhyde-oxime
(36) [(4-aminométhyl-cyclohexyl)-(3-fluorophényl)-méthyl]-diméthylamine
(38) 4-[(4-chlorophényl)-diméthylamino-méthyl]-cyclohexanecarbaldéhyde-oxime
(39) [(4-aminométhyl-cyclohexyl)-(4-chlorophényl)-méthyl]-diméthylamine
(41) 4-(diméthylamino-thiophèn-2-yl-méthyl)-cyclohexanecarbaldéhyde-oxime
(42) [(4-aminométhyl-cyclohexyl)-thiophén-2-yl-méthyl]-diméthylamine
(44) 4-(1-diméthylamino-3-phényl-propyl)-cyclohexane-carbaldéhyde-oxime
(45) [1-(4-aminométhyl-cyclohexyl)-3-phényl-propyl]-diméthylamine
(47) [4-(diméthylamino-phényl-méthyl)-cyclohexyl]-acétaldéhyde-oxime
(48) 2-[4-diméthylamino-phényl-méthyl)-cyclohexyl]-éthylamine
(50) {4-[diméthylamino-(4-fluorophényl)-méthyl]-cyclohexyl}-acétaldéhyde-oxime
(51) 2-{4-[diméthylamino-(4-fluorophényl)-méthyl]-cyclohexyl}-éthylamine
(53) {4-[diméthylamino-(3-fluorophényl)-méthyl]-cyclohexyl}-acétaldéhyde-oxime
(54) 2-{4-[diméthylamino-(3-fluorophényl)-méthyl] cyclohexyl}-éthylamine
(56) {4-[diméthylamino-(4-chlorophényl)-méthyl]-cyclohexyl}-acétaldéhyde-oxime
(66) 2-{4-[diméthylamino-(9-chlorophényl)-méthyl]-cyclohexyl}-éthylamine
(68) 2-(4-((diméthylamino)(thiophèn-2-yl)méthyl)cyclohexyl)acétaldéhyde-oxime
(69) 2-[4-(diméthylamino-thiophén-2-yl-méthyl)-cyclohexyl]-éthylamine

(71) [4-(1-diméthylamino-3-phényl-propyl)-cyclohexyl]-acétaldéhyde-oxime
(72) {1-[4-(2-amino-éthyl)-cyclohexyl]-3-phényl-propyl}-diméthylamine
(111) 4-[diméthylamino-phényl-méthyl]-cyclohexanol
(112) 4-[diméthylamino-(4-fluorophényl)-méthyl]-cyclohexanol
(113) 4-[diméthylamino-(3-fluorophényl)-méthyl]-cyclohexanol
(114) 4-[(4-chlorophényl)-diméthylamino-méthyl]-cyclohexanol
(115) 4-(diméthylamino-thiophèn-2-yl-méthyl)-cyclohexanol
(116) 4-(1-diméthylamino-3-phényl-propyl)-cyclohexanol
(117) [4-(diméthylamino-phényl-méthyl)-cyclohexyl]-méthanol
(118) {4-[diméthylamino-(4-fluorophényl)-méthyl]-cyclohexyl}-méthanol
(119) {4-[diméthylamino-(3-fluorophényl)-méthyl]-cyclohexyl}-méthanol
(120) {4-[(4-chlorophényl)-diméthylamino-méthyl]-cyclohexyl}-méthanol
(121) [4-(diméthylamino-thiophèn-2-yl-méthyl)-cyclohexyl]-méthanol
(122) [4-(1-diméthylamino-3-phényl-propyl)-cyclohexyl]-méthanol
(123) ester éthylique de l'acide [4-(diméthylamino-phényl-méthyl)-cyclohexylidène]-acétique
(124) ester éthylique de l'acide [4-(diméthylamino-phényl-méthyl)-cyclohexyl]-acétique
(125) 2-[4-(diméthylamino-phényl-méthyl)-cyclohexyl]-éthanol
(126) ester éthylique de l'acide {4-[diméthylamino-(4-fluorophényl)-méthyl]-cyclohexylidène}-acétique
(127) ester éthylique de l'acide {4-[diméthylamino-(4-fluorophényl)-méthyl]-cyclohexyl}-acétique
(128) 2-{4-[diméthylamino-(4-fluorophényl)-méthyl]-cyclohexyl}-éthanol
(129) ester éthylique de l'acide {4-[diméthylamino-(3-fluorophényl)-méthyl]-cyclohexylidène}-acétique
(130) ester éthylique de l'acide {4-[diméthylamino-(3-fluorophényl)-méthyl]-cyclohexyl}-acétique
(131) 2-{4-[diméthylamino-(3-fluorophényl)-méthyl]-cyclohexyl}-éthanol
(132) ester éthylique de l'acide 3-[4-(diméthylamino-phényl-méthyl)-cyclohexyl]-acrylique
(133) ester éthylique de l'acide 3-[4-(diméthylamino-phényl-méthyl)-cyclohexyl]-propionique
(134) 3-[4-(diméthylamino-phényl-méthyl)-cyclohexyl]-propan-1-ol.
(135) ester éthylique de l'acide 3-{4-[diméthylamino-(4-fluorophényl)-méthyl]-cyclohexyl}-acrylique
(136) ester éthylique de l'acide 3-{4-[diméthylamino-(4-fluorophényl)-méthyl]-cyclohexyl}-propionique
(137) 3-{4-[diméthylamino-(4-fluorophényl)-méthyl]-cyclohexyl}-propan-1-ol
(138) ester éthylique de l'acide 3-{4-[diméthylamino-(3-fluorophényl)-méthyl]-cyclohexyl}-acrylique
(139) ester éthylique de l'acide 3-{4-[diméthylamino-(3-fluorophényl)-méthyl]-cyclohexyl}-propionique
(140) 3-{4-[diméthylamino-(3-fluorophényl)-méthyl]-cyclohexyl}-propan-1-ol
(141) ester éthylique de l'acide 3-[4-(1-diméthylamino-3-phényl-propyl)-cyclohexyl]-acrylique
(142) ester éthylique de l'acide 3-[4-(1-diméthylamino-3-phényl-propyl)-cyclohexyl]-propionique
(143) 3-[4-(1-diméthylamino-3-phényl-propyl)-cyclohexyl]-propan-1-ol
(73) 1-(4-((diméthylamino)(phényl)méthyl)cyclohexyl)-3-(naphtalèn-1-yl)urée
(74) chlorhydrate de 1-(2,4-difluorophényl)-3-(4-((diméthylamino)(phényl)méthyl)cyclohexyl)urée
(75) chlorhydrate de 1-(4-((diméthylamino)(phényl)méthyl)cyclohexyl)-3-(3-(trifluorométhyl)phényl)urée
(76) chlorhydrate de 1-(4-((diméthylamino)(phényl)-méthyl)cyclohexyl)-3-(2-nitrophényl)urée
(77) chlorhydrate de 1-(3-bromophényl)-3-(4-((diméthylamino)(phényl)méthyl)cyclohexyl)urée
(78) chlorhydrate de 1-(4-((diméthylamino) (phényl)-méthyl)cyclohexyl)-3-phényl)urée
(79) 1-benzyl-3-(4-((diméthylamino)(phényl)méthyl)-cyclohexyl)urée
(80) 1-cyclohexyl-3-(4-((diméthylamino)(phényl)méthyl)-cyclohexyl)urée
(81) 1-(4-bromophényl)-3-(4-((diméthylamino)(phényl)-méthyl)cyclohexyl)urée
(82) 1-(4-((diméthylamino)(phényl)méthyl)cyclohexyl)-3-(4-méthoxyphényl)urée
(83) chlorhydrate de N-(2-(1H-indol-3-yl)éthyl)-4-((diméthylamino)(phényl)méthyl)cyclohexanamine
(84) chlorhydrate de 4-((diméthylamino)(phényl)méthyl)-N-phénéthylcyclohexanamine
(85) dichlorhydrate de 4-((diméthylamino)(phényl)-méthyl)-N-(3-phénylpropyl)cyclohexanamine
(86) chlorhydrate de N-benzyl-4-((diméthylamino)-(phényl)méthyl)cyclohexanamine
(87) chlorhydrate de 4-((diméthylamino)(phényl)méthyl)-N-(4-phénylbutyl)cyclohexanamine
(88) chlorhydrate de N-(1-(1H-indol-3-yl)propan-2-yl)-4-((diméthylamino)(phényl)méthyl)-cyclohexanamine
(89) chlorhydrate de N-(4-((diméthylamino)(phényl)-méthyl)cyclohexyl)-4-méthoxybenzènamine
(90) dichlorhydrate de 4-((diméthylamino)(phényl)-méthyl)-N-(4-méthoxybenzyl)cyclohexanamine
(91) chlorhydrate de 4-((diméthylamino)(phényl)méthyl)-N-(4-fluorobenzyl)cyclohexanamine
(92) chlorhydrate de N-(4-((diméthylamino)(phényl)-méthyl)cyclohexyl)benzènamine
(93) chlorhydrate de N-(4-((diméthylamino)(phényl)-méthyl)cyclohexyl)-2-éthylbutanamide
(94) chlorhydrate de N-(4-((diméthylamino)(phényl)-méthyl)cyclohexyl)benzamide
(95) chlorhydrate de N-(4-((diméthylamino)(phényl)-méthyl)cyclohexyl)-N-(3-pénylpropyl)acétamide

(96) chlorhydrate de N-(4-((diméthylamino)(phényl)-méthyl)cyclohexyl)-N-phénylacétamide
(97) chlorhydrate de N-(4-((diméthylamino)(phényl)-méthyl)cyclohexyl)-N-(4-phénylbutyl)propionamide
(98) chlorhydrate de N-(4-((diméthylamino)(phényl)-méthyl)cyclohexyl)-N-(4-phénylbutyl)acétamide
(99) chlorhydrate de N-(4-((diméthylamino)(phényl)-méthyl)cyclohexyl)-N-(4-méthoxyphényl)acétamide
(100) chlorhydrate de N-benzyl-N-(4-((diméthylamino)-(phényl)-méthyl)cyclohexyl)acétamide
(101) chlorhydrate de N-benzyl-N-(4-((diméthylamino)-(phényl)méthyl)cyclohexyl)-2-éthylbutanamide
(102) chlorhydrate de N-benzyle-N-(4-((diméthylamino)-(phényl)méthyl)cyclohexyl)butyramide
(103) chlorhydrate de N-benzyl-N-(4-((diméthylamino)-(phényl)méthyl)cyclohexyl)-4-fluorobenzamide
(104) chlorhydrate de N-benzyl-N-(4-((diméthylamino)-(phényl)méthyl)cyclohexyl)benzamide
(105) chlorhydrate de N-(4-((diméthylamino)(phényl)-méthyl)cyclohexyl)-2-éthyl-N-phénylbutanamide
(106) chlorhydrate de 4-chloro-N-(4-((diméthylamino)-(phéhyl)méthyl)cyclohexyl)benzènesulfonamide
(107) chlorhydrate de N-(4-((diméthylamino)(phényl)-méthyl)cyclohexyl)-4-méthoxybenzénesulfonamide
(108) chlorhydrate de 4-tert-butyl-N-(4-((diméthylamino)-(phényl)méthyl)cyclohexyl)benzènesulfonamide
(109) chlorhydrate de N-(4-((diméthylamino)(phényl)-méthyl)cyclohexyl)-2-nitrobenzènesulfonamide
(110) chlorhydrate de N-(4-((diméthylamino)(phényl)-méthyl)cyclohexyl)benzénesulfonamide
(144) chlorhydrate de 4-(benzyloxy)cyclohexyl)-N,N-diméthyl(phényl)méthanamine
(145) chlorhydrate de 4-(4-fluorobenzyloxy)cyclohexyl)-N,N-diméthyl(phényl)méthanamine
(146) chlorhydrate de trans-N,N-diméthyl(4-phénéthyl-cycloxyl)(phényl)méthanamine
(147) chlorhydrate de 1-benzyl-4-(diméthylamino)-(phényl)méthyl)cyclohexanol
(148) chlorhydrate de 4-((diméthylamino)(phényl)méthyl)-1-(4-fluorobenzyl)cyclohexanol
(149) 1-(2,5-diméthoxyphényl)-4-((diméthylamino)-(phényl)méthyl)cyclohexanol
(150) 4-((diméthylamino)(phényl)méthyl)-1-(4-fluoro-3-méthylphényl)cyclohexanol
(151) 4-(diméthylamino-phényl-méthyl)-1-(4-fluoro-3-méthyl-phényl)-cyclohexanol
(152) 1-benzyl-4-[diméthylamino-(3-fluoro-phényl)-méthyl]-cyclohexanol
(153) 4-[diméthylamino-(3-fluoro-phényl)-méthyl]-1-phénéthyl-cyclohexanol
(154) 4-[diméthylamino-(3-fluoro-phényl)-méthyl]-1-pentyl-cyclohexanol
(155) 1-(3,5-dichloro-phényl)-4-[diméthylamino-(3-fluoro-phényl)-méthyl]-cyclohexanol
(156) 4-[diméthylamino-(3-fluoro-phényl)-méthyl]-1-(3-méthoxy-benzyl)-cyclohexanol
(157) 1-(4-chloro-3-trifluorométhyl-phényl)-4-[diméthylamino-(3-fluoro-phényl)-méthyl]-cyclohexanol
(158) 4-[(4-chloro-phényl)-diméthylamino-méthyl]-1-phényl-cyclohexanol
(159) 1-benzyl-4-[(4-chloro-phényl)-diméthylamino-méthyl]-cyclohexanol.
(160) 4-[(4-chloro-phényl)-diméthylamino-méthyl]-1-(4-fluoro-3-méthyl-phényl)-cyclohexanol
(161) 4-[(4-chloro-phényl)-diméthylamino-méthyl]-1-o-tolyl-cyclohexanol
(162) 4-[(4-chloro-phényl)-diméthylamino-méthyl]-1-(4-fluoro-phényl)-cyclohezanol
(163) 4-[(4-chloro-phényl)-diméthylamino-méthyl]-1-phénéthyl-cyclohezanol
(164) 4-[(4-chloro-phényl)-diméthylamino-méthyl]-1-(3-méthoxy-phényl)-cyclohexanol
(165) 4-[(4-chloro-phényl)-diméthylamino-méthyl]-1-p-tolyl-cyclohexanol
(166) 4-[(4-chloro-phényl)-diméthylamino-méthyl]-1-(3,5-difluoro-phényl)-cyclohexanol
(167) 1-butyl-4-[(4-chloro-phényl)-diméthylamino-méthyl]-cyclohexanol
(168) 4-[(4-chloro-phényl)-diméthylamino-méthyl]-1-hexyl-cyclohexanol
(169) 4-[(4-chloro-phényl)-diméthylamino-méthyl]-1-pentyl-cyclohexanol (le diastéréoisomère le plus polaire)
(170) 4-[(4-chloro-phényl)-diméthylamino-méthyl]-1-pentyl-cyclohexanol (le diastéréoisomère le plus apolaire)
(171) 4-[(4-chloro-phényl)-diméthylamino-méthyl]-1-(3-fluoro-phényl)-cyclohexanol
(172) 4-[(4-chloro-phényl)-diméthylamino-méthyl]-1-(4-fluoro-benzyl)-cyclohexanol
(173) 4-[(4-chloro-phényl)-diméthylamino-méthyl]-1-(3-méthoxy-benzyl)-cyclohexanol
(174) 2-(4-((diméthylamino)(phényl)méthyl)-cyclohexylamino)-3-(1H-indol-3-yl)propanoate de méthyle (le diastéréoisomère le plus polaire)
(175) 2-(4-((diméthylamino)(phényl)méthyl)-cyclohexylamino)-3-(1H-indol-3-yl)propanoate de méthyle (le diastéréoisomère le plus apolaire)
(176) N-(4-((diméthylamino)(phényl)méthyl)cyclohexyl)-N-(4-méthoxybenzyl)acétamide
(177) N-(1-(1H-indol-3-yl)propan-2-yl)-N-(4-((diméthylamino)(phényl)méthyl)cyclohexyl)acétamide (le diastéréoisomère le plus polaire)
(178) N-(1-(1H-indol-3-yl)propan-2-yl)-N-(4-((diméthylamino)(phényl)méthyl)cyclohexyl)acétamide (le diastéréoisomère le plus apolaire)
(179) N-(4-((diméthylamino)(phényl)méthyl)cyclohexyl)-N-(4-fluorobenzyl)acétamide
(180) N-(4-((diméthylamino)(phényl)méthyl)cyclohexyl)-N-phénylbutyramide
(181) N-(2-(1H-indol-3-yl)éthyl)-N-(4-((diméthylamino)-(phényl)méthyl)-cyclohexyl)butyramide
(182) N-(2-(1H-indol-3-yl)éthyl)-N-(4-((diméthylamino)-(phényl)méthyl)cyclohexyl)acétamide

(183) [4-(diméthylamino-phényl-méthyl)-cyclohexyl]-amide de l'acide benzo[b]thiophène-3-carboxylique
(184) [4-(diméthylamino-phényl-méthyl)-cyclohexyl]-amide de l'acide 1-(4-chloro-phényl)-cyclopentanecarboxylique
(185) N-[4-(diméthylamino-thiophèn-2-yl-méthyl)-cyclohexyl]-4-propyl-benzamide
(186) 3-cyano-N-[4-(diméthylamino-thiophèn-2-yl-méthyl)-cyclohexyl]-benzamide
(187) 3-chloro-N-[4-(diméthylamino-phényl-méthyl)-cyclohezyl]benzamide
(188) [4-(diméthylamino-phényl-méthyl)-cyclohexyl]-amide de l'acide 5-chloro-4-méthoxy-thiophène-3-carboxylique
(189) 3,4-dichloro-N-[4-(diméthylamino-phényl-méthyl)-cyclohexyl]-benzamide
(190) N-[4-(diméthylamino-phényl-méthyl)-cyclohexyl]-3-fluoro-5-trifluorométhyl-benzamide
(191) [4-[diméthylamino-(3-fluoro-phényl)-méthyl]-cyclohexyl}-amide de l'acide 5-chloro-4-méthoxy-thiophène-3-carboxylique
(192) N-[4-(diméthylamino-thiophèn-2-yl-méthyl)-cyclohexyl]-4-fluoro-3-trifluorométhyl-benzamide
(193) [4-(diméthylamino-thiophèn-2-yl-méthyl)-cyclohexyl]-amide de l'acide thiophène-2-carboxylique
(194) 3,5-dichloro-N-[4-(diméthylamino-thiophèn-2-yl-méthyl)-cyclohexyl]-benzamide
(195) [4-(diméthylamino-thiophèn-2-yl-méthyl)-cyclohexyl]-amide de l'acide 5-chloro-4-méthoxy-thiophène-3-carboxylique
(196) N-[4-(diméthylamino-phényl-méthyl)-cyclohexyl]-2,4,5-trifluoro-benzamide
(197) 3-bromo-N-[4-(diméthylamino-phényl-méthyl)-cyclohexyl)-benzamide
(198) N-[4-(diméthylamino-thiophèn-2-yl-méthyl)-cyclohexyl]-4-méthyl-benzamide
(199) N-[4-(diméthylamino-thiophèn-2-yl-méthyl)-cyclohexyl]-3-méthoxy-benzamide
(200) N-[4-(diméthylamino-thiophèn-2-yl-méthyl)-cyclohexyl]-3,3-diméthyl-butyramide
(201) [4-(diméthylamino-thiophèn-2-yl-méthyl)-cyclohexyl]-amide de l'acide 2-tert-butyl-5-méthyl-2H-pyrazole-3-carboxylique
(202) N-[4-(diméthylamino-thiophèn-2-yl-méthyl)-cyclohexyl]-2,4-diméthoxy-benzamide
(203) N-[4-(diméthylamino-phényl-méthyl)-cyclohexyl]-3-trifluorométhyl-benzamide
(204) N-{4-[diméthylamino-(3-fluoro-phényl)-méthyl]-cyclohexyl}-3,5-difluoro-benzamide
(205) N-[4-(diméthylamino-phényl-méthyl)-cyclohexyl]-2-fluoro-5-trifluorométhyl-benzamide
(206) 2-(4-chloro-phényl)-N-[4-(diméthylamino-phényl-méthyl)-cyclohexyl]-acétamide
(207) N-[4-(diméthylamino-thiophèn-2-yl-méthyl)-cyclohexyl]-2-méthoxy-benzamide
(208) N-[4-(diméthylamino-thiophèn-2-yl-méthyl)-cyclohexyl]-2-méthylsulfanyl-nicotinamide
(209) 3,4-dichloro-N-{4-[diméthylamino-(3-fluoro-phényl)-méthyl]-cyclohexyl}-benzamide
(210) N-[4-(1-diméthylamino-3-phényl-propyl)-cyclohexyl]-4-fluoro-3-trifluorométhyl-benzamide (le diastéréoisomère le plus polaire)
(211) (4-[diméthylamino-(4-fluoro-phényl)-méthyl]-cyclohexyl)-amide de l'acide 5-chloro-4-méthoxy-thiophène-3-carboxylique
(212) N-[4-(diméthylamino-thiophèn-2-yl-méthyl)-cyclohexyl]-3,4,5-triméthoxy-benzamide
(213) N-[4-(diméthylamino-thiophèn-2-yl-méthyl)-cyclohexyl]-2-éthylsulfanyl-nicotinamide
(214) [4-(diméthylamino-phényl-méthyl)-cyclohexyl]-amide de l'acide 2-méthyl-5-phényl-furanne-3-carboxylique
(215) N-{4-[diméthylamino-(3-fluroro-phényl)-méthyl]-cyclohexyl}-2-phénoxy-propionamide (le diastéréoisomère le plus apolaire)
(216) N-[4-(diméthylamino-phényl-méthyl)-cyclohexyl]-2,4-diméthoxy-benzamide
(217) 4-tert-butyl-N-[4-(diméthylamino-phényl-méthyl)-cyclohexyl]-benzamide
(218) 2-(4-chloro-phénylsulfanyl)-N-[4-(diméthylamino-thiophèn-2-yl-méthyl)-cyclohexyl]-nicotinamide
(219) 2-(4-chloro-phényl)-N-[4-(diméthylamino-thiophèn-2-yl-méthyl)-cyclohexyl]-acétamide
(220) N-[4-(diméthylamino-thiophèn-2-yl-méthyl)-cyclohexyl]-2-p-tolyloxy-nicotinamide
(221) [4-(diméthylamino-thiophèn-2-yl-méthyl)-cyclohexyl]-amide de l'acide 3-chloro-4-(propan-2-sulfonyl)-thiophène-2-carboxylique
(222) N-{4-[diméthylamino-(3-fluoro-phényl)-méthyl]-cyclohexyl}-2-phénoxy-propionamide (le diastéréoisomère le plus polaire)
(223) [4-(diméthylamino-phényl-méthyl)-cyclohexyl]-amide de l'acide 2-tert-butyl-5-méthyl-2H-pyrazole-3-carboxylique
(224) [4-(diméthylamino-thiophèn-2-yl-méthyl) - cyclohexyl]-amide de l'acide 5-méthyl-isoxazole-3-carboxyliques
(225) 5-bromo-N-[4-(diméthylamino-thiophèn-2-yl-méthyl)-cyclohexyl]-nicotinamide
(226) [4-(diméthylamino-phényl-méthyl)-cyclohexyl]-amide de l'acide naphtyl-1-carboxylique
(227) N-[4-(1-dimêthylamino-3-phényl-propyl)-cyclohexyl]-4-fluoro-3-trifluorométhyl-benzamide (le diastéréoisomère le plus apolaire)
(228) N-[4-(diméthylamino-phényl-méthyl)-cyclohexyl]-3,3-diméthyl-butyramide (le diastéréoisomère le plus polaire)
(229) [4-(dimethylamino-thiophèn-2-yl-méthyl)-cyclohexyl]-amide de l'acide 5-(4-chloro-phényl)-2-méthyl-furanne-3-carboxylique
(230) N-[4-(diméthylamino-phényl-méthyl)-cyclohexyl]-2-phénoxy-propionamide
(231) {4-[diméthylamino-(3-fluoro-phényl)-méthyl]-cyclohexyl}-amide de l'acide benzo[b]thiophène-2-carboxylique
(232) [4-(diméthylamino-phényl-méthyl)-cyclohexyl]-amide de l'acide 5-(4-chloro-phényl)-2-méthyl-furanne-3-carboxylique
(233) [4-(dimethylamino-phényl-méthyl)-cyclohexyl]-amide de l'acide 4-(4-chloro-benzènesulfonyl)-3-méthyl-thiophène-2-carboxylique
(234) N-[4-(diméthylamino-phényl-méthyl)-cyclohexyl]-2-phényl-butyramide (le diastéréoisomère le plus apolaire)
(235) 5-bromo-N-[4-(diméthylamino-phényl-méthyl)-cyclohexyl]-nicotinamide
(236) [4-(diméthylamino-phényl-méthyl)-cyclohexyl]-amide de l'acide adamantane-1-carboxylique
(237) [4-(diméthylamino-phényl-méthyl)-cyclohexyl]-amide de l'acide 2-phényl-thiazole-4-carboxylique
(238) [4-(diméthylamino-phényl-méthyl)-cyclohexyl]-amide de l'acide 4-méthyl-2-phényl-thiazole-5-carboxylique
(239) [4-(diméthylamino-thiophèn-2-yl-méthyl)-cyclohexyl]-amide de l'acide 2-(2,3-dihydro-benzofurann-5-yl)-thiazole-4-carboxylique
(240) N-{4-[diméthylamino-(3-fluoro-phényl)-méthyl]-cyclohexyl}-2-phényl-acétamide
(241) 3-chloro-N-[4-(1-diméthylamino-3-phényl-propyl) - cyclohexyl]-benzamide
(242) N-[4-(dimethylamino-phényl-méthyl)-cyclohexyl]-4-méthyl-benzamide
(243) 3,5-dichloro-N-{4-[diméthylamino-(3-fluoro-phényl)-méthyl]-cyclohexyl}-benzamide
(244) N-[4-(diméthylamino-phényl-méthyl)-cyclohexyl]-2,3,6-trifluoro-benzamide
(245) [4-(diméthylamino-phényl-méthyl)-cyclohexyl]-amide de l'acide thiophène-2-carboxylique (le diastéréoisomère le plus apolaire)
(246) N-[4-(diméthylamino-phényl-méthyl)-cyclohexyl]-3,3-diméthyl-butyramide (le diastéréoisomère le plus apolaire
(247) [4-(diméthylamino-phényl-méthyl)-cyclohexyl]-amide de l'acide 2-tert-butyl-5-méthyl-2H-pyrazole-3-carboxylique
(248) N-[4-(1-diméthylamino-3-phényl-propyl)-cyclohexyl]-3-méthyl-benzamide
(249) [4-(diméthylamino-phényl-méthyl)-cyclohexyl]-amide de l'acide thiophène-2-carboxylique (le diastéréoisomère le plus polaire)
(250) N-[4-(diméthylamino-phényl-méthyl)-cyclohexyl]-2-phényl-butyramide (le diastéréoisomère le plus polaire)
(251) N-{4-[diméthylamino-(3-fluoro-phényl)-méthyl]-cyclohexyl}-3,3-diméthyl-butyramide
(252) [4-(diméthylamino-phényl-méthyl)-cyclohexyl]-amide de l'acide 3-chloro-4-méthanesulfonyl-thiophène-2-carboxylique
(253) [4-(diméthylamino-phényl-méthyl)-cyclohexyl]-amide de l'acide 4-(4-chloro-benzènesulfonyl)-3-méthyl-thiophène-2-carboxylique
(254) 2-benzyloxy-N-[4-(diméthylamino-phényl-méthyl)-cyclohexyl]-acétamide
(255) N-[4-(diméthylamino-phényl-méthyl)-cyclohexyl]-2-thiophèn-2-yl-acétamide
(256) {4-[diméthylamino-(3-fluoro-phényl)-méthyl]-cyclohexyl}-amide de l'acide 4-méthyl-2-phényl-thiazole-5-carboxylique
(257) 2-(4-chloro-phénoxy)-N-[4-(diméthylamino-thiophèn-2-yl-méthyl)-cyclohexyl]-nicotinamide
(258) N-[4-(1-diméthylamino-3-phényl-propyl)-cyclohexyl]-4-fluoro-benzamide
(259) 5-bromo-N-[4-(diméthylamino-phényl-méthyl)-cyclohexyl]-nicotinamide
(260) [4-(diméthylamino-thiophèn-2-yl-méthyl)-cyclohexyl]-amide de l'acide 4-bromo-2-éthyl-5-méthyl-2H-pyrazole-3-carboxylique
(261) 3-cyano-N-[4-(diméthylamino-phényl-méthyl)-cyclohexyl]-benzamide
(262) N-{4-[diméthylamino-(4-fluoro-phényl)-méthyl]-cyclohexyl}-4-fluoro-benzamide
(263) 3-bromo-N-{4-[diméthylamino-(4-fluoro-phényl)-méthyl]-cyclohexyl}-benzamide
(264) {4-[diméthylamino-(3-fluoro-phényl)-méthyl]-cyclohexyl}-amide de l'acide 2-phényl-thiazole-4-carboxylique
(265) {4-[diméthylamino-(3-fluoro-phényl)-méthyl]-cyclohexyl}-amide de l'acide 2,5-diméthyl-furanne-3-carboxylique

(266) {4-[diméthylamino-(3-fluoro-phényl)-méthyl]-cyclohexyl}-amide de l'acide 2-méthyl-5-phényl-furanne-3-carboxylique
(267) {4-[diméthylamino-(3-fluoro-phényl)-méthyl]-cyclohexyl}-amide de l'acide 5-pyridin-2-yl-thiophène-2-carboxylique
(268) [4-(diméthylamino-phényl-méthyl)-cyclohexyl]-amide de l'acide 4-bromo-2-éthyl-5-méthyl-2H-pyrazole-3-carboxylique
(269) 3-chloro-N-{4-[diméthylamino-(3-fluoro-phényl)-méthyl]-cyclohexyl}-4-fluoro-benzamide
(270) 3,4-dichloro-N-{4-[diméthylamino-(4-fluoro-phényl)-méthyl]-cyclohexyl}-benzamide
(271) N-{4-[diméthylamino-(4-fluoro-phényl)-méthyl]-cyclohexyl}-2,4,5-trifluoro-benzamide
(272) [4-(diméthylamino-phényl-méthyl)-cyclohexyl]-amide de l'acide cyclohexanecarboxylique
(273) N-{4-[diméthylamino-(3-fluoro-phényl)-méthyl]-cyclohexyl}-2-phényl-butyramide
(274) 2-(4-chloro-phényl)-N-{4-[diméthylamino-(3-fluoro-phényl)-méthyl]-cyclohexyl-acétamide
(275) N-[4-(diméthylamino-phényl-méthyl)-cyclohexyl]-3-nitro-benzamide
(276) N-[4-(1-diméthylamino-3-phényl-propyl)-cyclohexyl]-2,5-difluoro-benzamide
(277) 3-bromo-N-[4-(1-diméthylamino-3-phényl-propyl)-cyclohexyl]-benzamide
(278) N-{4-[diméthylamino-(3-fluoro-phényl)-méthyl]-cyclohexyl}-2,6-difluoro-benzamide
(279) [4-(diméthylamino-phényl-méthyl)-cyclohexyl]-amide de l'acide 2,5-diméthyl-furanne-3-carboxylique
(280) 3-chloro-N-{4-[diméthylamino-(4-fluoro-phényl)-méthyl]-cyclohexyl}-4-fluoro-benzamide
(281) N- [4-(diméthylamino-phényl-méthyl)-cyclohexyl]-5-fluoro-2-trifluorométhyl-benzamide
(282) [4-(diméthylamino-phényl-méthyl)-cyclohexyl]-amide de l'acide 5-méthyl-isoxazole-3-carboxylique
(283) [4-(diméthylamino-thiophèn-2-yl-méthyl)-cyclohexyl]-amide de l'acide 2-(2,3-dihydro-benzofurann-5-yl)-4-méthyl-thiazole-5-carboxylique
(284) 2-(4-chloro-phénoxy)-N-[4-(diméthylamino-phényl-méthyl)-cyclohexyl]-acétamide
(285) {4-[diméthylamino-(3-fluoro-phényl)-méthyl]-cyclohexyl}-amide de l'acide 5-(4-chloro-phényl)-2-méthyl-furanne-3-carboxylique
(286) 2-bromo-N-[4-(diméthylamino-phényl-méthyl)-cyclohexyl]-benzamide
(287) N-[4-(diméthylamino-phényl-méthyl)-cyclohexyl]-2,6-diméthoxy-benzamide
(288) [4-(diméthylamino-phényl-méthyl)-cyclohexyl]-amide de l'acide cyclopentanecarboxylique
(289) [4-(diméthylamino-phényl-methyl)-cyclohexyl]-amide de l'acide 2-(2,3-dihydro-benzofurann-5-yl)-thiazole-4-carboxylique
(290) {4-[diméthylamino-(3-fluoro-phényl)-méthyl]-cyclohexyl}-amide de l'acide benzo[1,2,5]thiadiazole-5-carboxylique
(291) N-[4-(diméthylamino-thiophèn-2-yl-méthyl)-cyclohexyliuêthyl] -2-thiophèn-2-yl-acétamide
(292) [4-(diméthylamino-phényl-méthyl)-cyclohexyl-méthyl]-amide de l'acide benzo[b]thiophène-3-carboxylique
(293) [4-(diméthylamino-phényl-méthyl)-cyclohexyl-méthyl]-amide de l'acide 5-chloro-4-méthoxy-thiophène-3-carboxylique
(294) N-[4-(diméthylamino-phényl-méthyl)-cyclohexyl-méthyl]-3,4-difluoro-benzamide (le diastéréoisomère le plus apolaire)
(295) N-{4-[diméthylamino-(3-fluoro-phényl)-méthyl]-cyclohexylméthyl}-3,3-diméthyl-butyramide
(296) 2-bromo-N-[4-(diméthylamino-thiophèn-2-yl-méthyl)-cyclohexylméthyl]-benzamide
(297) N-[4-(ciméthylamino-thiophèn-2-yl-méthyl)-cyclohexylméthyl]-2,2-diphényl-acétamide
(298) N-[4-(diméthylamino-phényl-méthyl)-cyclohexylméthyl]-3,3-diméthyl-butyramide
(299) N-[4-(diméthylamino-phényl-méthyl)-cyclohexyl-méthyl]-2-méthylsulfanyl-nicotinamide
(300) N-[4-(diméthylamino-thiophèn-2-yl-méthyl)-cyclohexylméthyl]-2,6-diméthoxy-benzamide
(301) N-[4-(diméthylamino-thiophèn-2-yl-méthyl)-cyclohexylméthyl]-3,3-diméthyl-butyramide
(302) [4-(diméthylamino-thiophèn-2-yl-méthyl)-cyclohexylméthyl]-amide de l'acide benzo[b]thiophène-3-carboxylique
(303) {4-[diméthylamino-(3-fluoro-phényl)-méthyl]-cyclohexylméthyl}-amide de l'acide 5-chloro-4-méthoxy-thiophène-3-carboxyligue
(304) N-[4-(diméthylamino-thiophèn-2-yl-méthyl)-cyclohexylméthyl]-2-phénoxy-propionamide
(305) N-[4-(diméthylamino-thiophèn-2-yl-méthyl)-cyclohexylméthyl]-2-méthoxy-benzamide
(306) N-[4-(diméthylamino-phényl-méthyl)-cyclohexyl-méthyl]-2-phényl-acétamide
(307) 3-bromo-N-[4-(diméthylamino-thiophèn-2-yl-méthyl)-cyclohexylméthyl]-benzamide
(308) N-[4-(diméthylamino-thiophèn-2-yl-méthyl)-cyclohexylméthy]-3-fluoro-5-trifluorométhyl-benzamide
(309) N-{4-[(4-chloro-phényl)-diméthylamino-méthyl]-cyclohexylméthyl}-3,3-diméthyl-butyramide
(310) N-[4-(diméthylamino-phényl-méthyl)-cyclohexylméthyl]-2-éthylsulfanyl-nicotinamide
(311) 2-(4-chloro-phényl)-N-[4-(diméthylamino-thiophèn-2-yl-méthyl)-cyclohexylméthyl]-acétamide

(312) N-{4-[(4-chloro-phényl)-diméthylamino-méthyl]-cyclohexylméthyl}-2,2-diphényl-acétamide
(313) N-[4-(diméthylamino-thiophèn-2-yl-méthyl)-cyclohexylméthyl]-2,6-difluoro-benzamide
(314) {4-[diméthylamino-(3-fluoro-phényl)-méthyl]-cyclohexylméthyl}-amide de l'acide benzo[b]thiophène-3-carboxylique
(315) N-{4-[(4-chloro-phényl)-diméthylamino-méthyl]-cyclohexylméthyl}-2-méthylsulfanyl-nicotinamide
(316) N-{4-[(4-chloro-phényl)-diméthylamino-méthyl]-cyclohexylméthyl}-2-thiophèn-2-yl-acétamide
(317) N-[4-(diméthylamino-phényl-méthyl)-cyclohexyl-méthyl]-2-méthyl-benzamide (le diastéréoisomère le plus apolaire)
(318) [4-(diméthylamino-phényl-methyl)-cyclohexyl-méthyl]-amide de l'acide 1-(4-chloro-phényl)-cyclopentane-caboxylique
(319) N-{4-[diméthylamino-(3-fluoro-phényl)-méthyl]-cyclohexylméthyl}-2-phényl-acétamide (le diastéréoisomère le plus polaire)
(320) N-{4-[(4-chloro-phényl)-diméthylamino-méthyl]-cyclohexylméthyl}-2-(3-méthoxy-phényl)-acétamide
(321) N-[4-(diméthylamino-phényl-methyl)-cyclohexylméthyll-2-phényl-butyramide
(322) N-{4-[diméthylamino-(4-fluoro-phényl)-méthyl]-cyclohexylméthyl}-3,3-diméthyl-butyramide
(323) N-[4-(diméthylamino-phényl-méthyl)-cyclohexyl-méthyl]-2-phénoxy-propionamide
(324) 2-(4-chloro-phényl)-N-[4-(dimethylamino-phényl-méthyl)-cyclohexylméthyl]-acétamide
(325) N-[4-(diméthylamino-phényl-méthyl)-cyclohexyl-méthyl]-2-méthyl-benzamide (le diastéréoisomère le plus polaire)
(326) N-[4-(diméthylamino-phényl-méthyl)-cyclohexyl-méthyl] -3-trifluorométhyl-benzamide (le diastéréoisomère le plus apolaire)
(327) {4-[diméthylamino-(3-fluoro-phényl)-méthyl]-cyclohexylméthyl}-amide de l'acide 1-(4-chloro-phényl)-cyclopentane-carboxylique
(328) [4-(diméthylamino-phényl-méthyl)-cyclohexyl-méthyl]-amide de l'acide thiophène-2-carboxylique
(329) 3,5-dichloro-N-[4-(diméthylamino-phényl-méthyl)-cyclohexylméthyl]-benzamide
(330) {4-[(4-chloro-phényl)-diméthylamino-méthyl]-cyclohexylméthyl}-amide de l'acide 2-méthyl-5-phényl-furanne-3-carboxylique
(331) 3-chloro-N-[4-(diméthylamino-phényl-méthyl)-cyclohexylméthyl]-benzamide
(332) N-{4-[diméthylamino-(3-fluoro-phényl)-méthyl]-cyclohexylméthyl}-2-phénoxy-propionamide (le diastéréoisomère le plus polaire)
(333) N-[4-(diméthylamino-phényl-méthyl)-cyclohexyl-méthyl]-3-trifluorométhyl-benzamide (le diastéréoisomère le plus polaire)
(334) {4-[diméthylamino-(3-fluoro-phényl)-méthyl]-cyclohexylméthyl}-amide de l'acide thiophène-2-carboxylique (le diastéréoisomère le plus polaire)
(335) [4-(diméthylamino-phényl-méthyl)-cyclohexyl-méthyl]-amide de l'acide 2-phényl]-thiazole-4-carboxylique
(336) {4-[(4-chloro-phényl)-diméthylamino-méthyl]-cyclohexylméthyl}-amide de l'acide benzo[b]thiophène-3-carboxylique (le diastéréoisomère le plus apolaire)
(337) N-{4-[diméthylamino-(3-fluoro-phényl)-méthyl]-cyclohexylméthyl}-2-p-tolyloxy-nicotamide
(338) 2,4,6-trichloro-N-[4-(diméthylamino-phényl-méthyl)-cyclohexylméthyl]-benzamide
(339) [4-(diméthylamino-phényl-méthyl)-cyclohexyl-méthyl]-amide de l'acide 1-(4-chloro-phényl)-cyclopentane-carboxylique
(340) {4-[diméthylamino-(3-fluoro-phényl)-méthyl]-cyclohexylméthyl}-amide de l'acide thiophène-2-carboxylique (le diastéréoisomère le plus apolaire)
(341) N-{4-[diméthylamino-(3-fluoro-phényl)-méthyl]-cyclohexylméthyl}-2-phénoxy-propionamide (le diastéréoisomère le plus apolaire)
(342) N-{4-[diméthylamino-(3-fluoro-phényl)-méthyl]-cyclohexylméthyl}-2-méthyl-butyramide (le diastéréoisomère le plus polaire)
(343) N-{4-[diméthylamino-(3-fluoro-phényl)-méthyl]-cyclohexylméthyl}-2-thiophèn-2-yl-acétalmide
(344) {4-[diméthylamino-(4-fluoro-phényl)-méthyl]-cyclohexylméthyl}-amide de l'acide benzo[b]thiophéne-3-carboxylique
(345) [4-(diméthylamino-phényl-méthyl)-cyclohexyl-méthyl]-amide de l'acide 2-méthyl-5-phényl-furanne-3-carboxylique
(346) N-{4-[diméthylamino-(3-fluoro-phényl)-méthyl]-cyclohexylméthyl}-2-phénoxy-propionamide
(347) 3-cyano-N-[4-(diméthylamino-thiophèn-2-yl-méthyl)-cyclohexylméthyl]-benzamide
(348) N-{4-[diméthylamino-(3-fluoro-phényl)-méthyl]-cyclohexylméthyl}-2-phényl-acétamide (le diastéréoisomére le plus apolaire)
(349) N-[4-(diméthylamino-phényl-méthyl)-cyclohexylméthyl]-2-(3-méthoxy-phényl)-acétamide
(350) N-{4-[diméthylamino-(3-fluoro-phényl)-méthyl]-cyclohexylméthyl}-4-fluoro-3-trifluorométhyl-benzamide

(351) N-{4-[(4-chloro-phényl)-diméthylamino-méthyl]-cyclohexylméthyl}-2-éthylsulfanyl-nicotinamide
(352) N-[4-(diméthylamino-thiophèn-2-yl-méthyl)-cyclohexylméthyl]-2-p-tolyloxy-nicotinamide (le diastéréoisomère le plus polaire)
(353) {4-[diméthylamino-(3-fluoro-phényl)-méthyl]-cyclohexylméthyl}-amide de l'acide 2-méthyl-5-phényl-furanne-3-carboxylique
(354) 2-chloro-N-{4-[(4-chloro-phényl)-diméthylamino-méthyl]-cyclchaxylméthyl}-benzamide
(355) 2-chloro-N-[4-(diméthylamino-phényl-méthyl)-cyclohexylméthyl]-nicotinamide
(356) N-[4-(diméthylamino-thiophèn-2-yl-méthyl)-cyclohexylméthyl]-4-propyl-benzamide
(357) N-[4-(diméthylamino-phényl-méthyl)-cyclohexylméthyl]-3,4-difluoro-benzamide (le diastéréoisomère le plus polaire)
(358) 3-bromo-N-[4-(diméthylamino-phényl-méthyl)-cyclohexylméthyl]-benzamide
(359) N-{4-[diméthylamino-(4-fluoro-phényl)-méthyl]-cyclohexylméthyl}-2-thiophèn-2-yl-acétamide
(360) 2-(4-chloro-phénoxy)-N-[4-(diméthylamino-phényl-méthyl)-cyclohexylméthyl]-nicotinamide (le diastéréoisomère le plus apolaire)
(361) 2,4-dichloro-N-[4-(diméthylamino-phényl-méthyl)-cyclohexylméthyl]-benzamide
(362) N-[4-(diméthylamino-phényl-méthyl)-cyclohexyl-méthyl]-3-méthyl-benzamide
(363) 2-bromo-N-{4-[diméthylamino-(3-fluoro-phényl)-méthyl]-cyclohexylméthyl}-benzamide
(364) N-{4-[diméthylamino-(3-fluoro-phényl)-méthyl]-cyclohexylméthyl}-3-trifluorométhyl-benzamide
(365) {4-[diméthylamino-(3-fluoro-phényl)-méthyl]-cyclohexylméthyl}-amide de l'acide 2-phényl-thiazole-4-carboxylique
(366) [4-(diméthylamino-thiophèn-2-yl-méthyl)-cyclohexylméthyl]-amide de l'acide 2-tert-butyl-5-méthyl-2H-pyrazole-3-carboxylique
(367) [4-(diméthylamino-thiophèn-2-yl-méthyl)-cyclohexylméthyl]-amide de l'acide 3-chloro-4-(propan-2-sulfonyl)-thiophène-2-carboxylique
(368) N-{4-[diméthylamino-(3-fluoro-phényl)-méthyl]-cyclohexylméthyl}-2-méthoxy-benzamide
(369) N-{4-[(4-chloro-phényl)-diméthylamino-méthyl]-cyclohexylméthyl}-3-trifluorométhyl-benzamide
(370) {4-[diméthylamino-(4-fluoro-phényl)-methyl]-cyclohexylméthyl}-amide de l'acide 1-(4-chloro-phényl)-cyclopentane-carboxylique
(371) 3,5-dichloro-N-{4-[diméthylamino-(3-fluoro-phényl)-méthyl]-cyclohexylméthyl}-benzamide
(372) {4-[(4-chloro-phényl)-diméthylamino-méthyl]-cyclohexylméthyl}-amide de l'acide benzo[b]thiophène-3-carboxylique (le diastéréoisomère le plus polaire)
(373) {4-[diméthylamino-(3-fluoro-phényl)-méthyl]-cyclohexylméthyl}-amide de l'acide 2-méthyl-5-phényl-furanne-3-carboxylique
(374) 2-(4-chloro-phénylsulfanyl)-N-[4-(diméthylamino-phényl-méthyl)-cyclohexylméthyl]-nicctinamide
(375) [4-(diméthylamino-thiophèn-2-yl-méthyl) - cyclohexylméthyl]-amide de l'acide 4-bromo-2-éthyl-5-méthyl-2H-pyrazole-3-carboxylique
(376) {4-[diméthylamino-(4-fluoro-phényl)-méthyl]-cyclohexylméthyl}-amide de l'acide 5-chloro-4-méthoxy-thiophène-3-carboxylique
(378) N-{4-[diméthylamino-(3-fluoro-phényl)-méthyl]-cyclohexylméthyl}-2-méthyl-butyramide (le diastéréoisomère le plus apolaire)
(379) [4-(diméthylamino-phényl-méthyl)-cyclohexyl-méthyl]-amide de l'acide 5-méthyl-isoxazole-3-carboxylique
(380) [4-(1-diméthylamino-3-phényl-propyl)-cyclohexyl-méthyl]-amide de l'acide benzo[b]thiophène-3-carboxylique
(381) N-{4-[diméthylamino-(4-fluoro-phényl)-méthyl]-cyclohexylméthyl}-2-méthylsulfanyl-nicotinamide
(382) N-{4-[(4-chloro-phényl)-diméthylamino-méthyl]-cyclohexylméthyl}-2-p-tolyloxy-nicotinamide
(383) 2-(4-chloro-phénoxy)-N-[4-(diméthylamino-phényl-méthyl)-cyclohexylméthyl]-nicotinamide (le diastéréoisomère le plus polaire)
(384) N-{4-[diméthylamino-(3-fluoro-phényl)-méthyl]-cyclohexylméthyl}-2-méthyl-benzamide
(385) {4-[(4-chloro-phény-)-diméthylamino-méthyl]-cyclohexylméthyl}-amide de l'acide 5-méthyl-isoxazole-3-carboxylique
(386) 5-bromo-N-[4-(diméthylamino-phényl-méthyl)-cyclohexylméthyl]-nicotinamide
(387) N-[4-(diméthylamino-phényl-méthyl)-cyclohexyl-méthyl]-2-méthyl-butyramide
(388) N-{4-[diméthylamino-(3-fluoro-phényl)-méthyl]-cyclohexylméthyl}-2-éthylsulfanyl-nicotinamide
(389) N-[9-(diméthylamino-thiophèn-2-yl-méthyl)-cyclohexylméthyl]-2-p-tolyloxy-nicotinamide (le diastéréoisomère le plus apolaire)
(390) [4-(diméthylamino-phényl-méthyl)-cyclohexyl-méthyl]-amide de l'acide 4-bromo-2-éthyl-5-méthyl-2H-pyrazole-3-carboxylique

(391) N-{4-[(4-chloro-phényl)-dimethylamino-méthyl]-cyclohexylméthyl}-2-phénoxy-propionamide
(392) N-[4-(diméthylamino-phényl-méthyl)-cyclohexyl-méthyl]-3,5-dinitro-benzamide
(393) N-{4-[(4-chloro-phényl)-diméthylamino-méthyl]-cyclohexylméthyl}-3-méthoxy-benzamide
(394) 2-bromo-N-{4-[(4-chloro-phényl)-diméthylamino-méthyl]-cyclohexylméthyl}-benzamide
(395) 2-bromo-N-(2-{4-[(4-chloro-phényl)-diméthylamino-méthyl]-cyclohexyl}-éthyl)-benzamide
(396) 2-bromo-N-(2-{4-[diméthylamino-(3-fluoro-phényl)-méthyl]-cyclohexyl}-éthyl)-benzamide
(397) 3-chloro-N-{2-[4-(diméthylamino-phényl-méthyl)-cyclohexyl]-éthyl}-benzamide (le diastéréoisomère le plus polaire)
(398) 3-chloro-N-{2-[4-(diméthylamino-phényl-méthyl)-cyclohexyl]-éthyl}-benzamide (le diastéréoisomère le plus apolaire)
(399) 3-chloro-N-(2-{4-[(4-chloro-phényl)-diméthylamino-méthyl]-cyclohexyl}éthyl)-benzamide
(400) 3-chloro-N-(2-{4-[diméthylamino-(3-fluoro-phényl)-méthyl]-cyclohexyl}-éthyl)-benzamide (le diastéréoisomère le plus apolaire)
(401) 3-chloro-N-(2-{4-[diméthylamino-(3-fluoro-phényl)-méthyl]-cyclohexyl}-éthyl)-benzamide (le diastéréoisomère le plus polaire)
(402) 2-chloro-N-{2-[4-(diméthylamino-phényl-méthyl)-cyclohexyl]-éthyl}-benzamide
(403) 2-chloro-N-(2-{4-[(4-chloro-phényl)-diméthylamino-méthyl]-cyclohexyl}-éthyl)-benzamide
(404) 2-chloro-N-(2-{4-[diméthylamino-(3-fluoro-phényl)-méthyl]-cyclohexyl}-éthyl)-benzamide
(405) 4-chloro-N-{2-[4-(diméthylamino-phényl-méthyl)-cyclohexyl]-éthyl}-benzamide
(406) 4-chloro-N-(2-{4-[diméthylamino-(3-fluro-phényl)-méthyl]-cyclohexyl}-éthyl)-benzamide
(407) N-{2-[4-(diméthylamino-phényl-méthyl)-cyclohexyl]-éthyl}-4-fluoro-benzamide
(408) N-(2-{4-[(4-chloro-phényl)-diméthylamino-méthyl]-cyclohexyl}-éthyl)-4-fluro-benzamide
(409) N-(2-{4-[diméthylamino-(3-fluoro-phényl)-méthyl]-cyclohexyl}-éthyl)-4-fluoro-benzamide
(410) N-{2-[4-(diméthylamino-phényl-méthyl)-cyclohexyl]-éthyl}-2-fluoro-benzamide
(411) N-(2-{4-[diméthylamino-(3-fluoro-phényl)-méthyl]-cyclohexyl}-éthyl)-2-fluoro-benzamide
(412) N-(2-{4-[diméthylamino-(3-fluoro-phényl)-méthyl]-cyclohexyl}-éthyl)-3-méthyl-benzamide
(413) 2,6-dichloro-N-{2-[4-(diméthylamino-phényl-méthyl)-cyclohexyl]-éthyl-benzamide
(414) N-{2-[4-(diméthylamino-phényl-méthyl)-cyclohexyl]-éthyl}-2-méthoxy-benzamide
(415) N-(2-{4-[diméthylamino-(3-fluoro-phényl)-méthyl]-cyclohexyl}-éthyl)-2-méthoxy-benzamide
(416) 3,4-dichloro-N-{2-[4-(diméthylamino-phényl-méthyl)-cyclohexyl]-éthyl}-benzamide
(417) N-{2-[4-(diméthylamino-phényl-méthyl)-cyclohexyl]-éthyl}-2-méthyl-benzamide (le diastéréoisomère le plus polaire)
(418) N-{2-[4-(diméthylamino-phényl-méthyl)-cyclohexyl]-éthyl}-2-méthyl-benzamide (le diastéréoisomère le plus apolaire)
(419) N-(2-{4-[(4-chloro-phényl)-diméthylamino-méthyl]-cyclohexyl}-éthyl)-2-méthyl-benzamide
(420) N-(2-{4-[diméthylamino-(3-fluoro-phényl)-méthyl]-cyclohexyl}-éthyl)-2-méthyl-benzamide
(421) 4-cyano-N-{2-[4-(diméthylamino-phényl-méthyl)-cyclohexyl]-éthyl}-benzamide
(422) 3-chloro-N-(2-{4-[diméthylamino-(4-fluoro-phényl)-méthyl]-cyclohexyl}-éthyl)-benzamide (le diastéréoisomère le plus polaire)
(423) 3-chloro-N-(2-{4-[diméthylamino-(4-fluoro-phényl)-méthyl]-cyclohexyl}-éthyl)-benzamide (le diastéréoisomère le plus apolaire)
(424) 3-chloro-N-{2-[4-(diméthylamino-thiophèn-2-yl-méthyl)-cyclohexyl]-éthyl}-benzamide
(425) 2-chloro-N-{2-[4-(diméthylamino-thiophèn-2-yl-méthyl)-cyclohexyl]-éthyl}-benzamide
(426) N-{2-[4-(diméthylamino-thiophèn-2-yl-méthyl)-cyclohexyl]-éthyl}-4-fluoro-benzamide
(427) N-(2-{4-[diméthylamino-(4-fluoro-phényl)-méthyl]-cyclohexyl}-éthyl)-2-fluoro-benzamide
(428) N-{2-[4-(diméthylamino-thiophèn-2-yl-méthyl)-cyclohexyl]-éthyl}-2-fluoro-benzamide
(429) N-(2-{4-[diméthylamino-(4-fluoro-phényl)-méthyl]-cyclohexyl}-éthyl)-3-méthyl-benzamide
(430) N-{2-[4-(diméthylamino-thiophèn-2-yl-méthyl)-cyclohexyl]-éthyl}-3-méthyl-benzamide
(431) 2,6-dichloro-N-{2-[4-(diméthylamino-thiophèn-2-yl-méthyl)-cyclohexyl]-éthyl}-benzamide
(432) N-(2-{4-[diméthylamino-(4-fluoro-phényl)-méthyl]-cyclohexyl}-éthyl)-2-méthoxy-benzamide
(433) N-{2-[4-(diméthylamino-phényl-méthyl)-cyclohexyl]-éthyl}-3,5-difluoro-benzamide
(434) N-(2-{4-[(4-chloro-phényl)-diméthylamino-méthyl]-cyclohexyl}-éthyl)-3,5-difluoro-benzamide
(435) N-(2-{4-[diméthylamino-(3-fluoro-phényl)-méthyl]-cyclohexyl}-éthyl)-3,5-difluoro-benzamide
(436) N-(2-{4-[(4-chloro-phényl)-diméthylamino-méthyl]-cyclohexyl}-éthyl)-2,4-difluoro-benzamide
(437) 2,4-dichloro-N-(2-{4-[(4-chloro-phényl)-diméthylamino-méthyl]-cyclohexyl}-éthyl)-5-fluoro-benzamide
(438) 2,4-dichloro-N-(2-{4-[diméthylamino-(3-fluoro-phényl)-méthyl]-cyclohexyl}-éthyl)-5-fluoro-benzamide (le diastéréoisomère le plus polaire)
(439) 2,4-dichloro-N-(2-{4-[diméthylamino-(3-fluoro-phényl)-méthyl]-cyclohexyl}-éthyl)-5-fluro-benzamide (le

diastéréoisomère le plus apolaire)
(440) 2,4-dichloro-N-{2-[4-(diméthylamino-thiophèn-2-yl-méthyl)-cyclohexyl]-éthyl}-5-fluoro-benzamide
(441) 2-chloro-N-(2-{4-[(4-chloro-phényl)-diméthylamino-méthyl]-cyclohexyl}-éthyl)-nicotinamide
(442) (2-{4-[diméthylamino-(4-fluoro-phényl)-méthyl]-cyclohexyl}-éthyl)-amide de l'acide naphtalène-2-carboxylique
(443) N-{2-[4-(diméthylamino-thiophèn-2-yl-méthyl)-cyclohexyl]-éthyl}-4-propyl-benzamide
(444) N-{2-[4-(diméthylamino-phényl-méthyl)-cyclohexyl]-éthyl}-3,4-difluoro-benzamide
(445) N-(2-{4-[(4-chloro-phényl)-diméthylamino-méthyl]-cyclohexyl}-éthyl)-3,4-difluoro-benzamide
(446) N-{2-[4-(diméthylamino-thiophèn-2-yl-méthyl)-cyclohexyl]-éthyl}-3,4-difluoro-benzamide
(447) N-(2-{4-[diméthylamino-{4-fluoro-phényl)-méthyl]-cyclohexyl}-éthyl)-3-méthoxy-benzamide
(448) N-{2-[4-(diméthylamino-phényl-méthyl)-cyclohexyl]-éthyl}-2,2-diphényl-acétamide
(449) {2-[4-(diméthylamino-phényl-méthyl)-cyclohexyl]-éthyl}-amide de l'acide 1-(4-chloro-phényl)-cyclopentane-carboxylique
(450) 2-benzyloxy-N-{2-[4-(diméthylamino-phényl-méthyl)-cyclohexyl]-éthyl}-acétamide
(451) N-{2-[4-(diméthylamino-phényl-méthyl)-cyclohexyl]-éthyl}-2-phényl-acétamide
(452) {2-[4-(diméthylamino-phényl-méthyl)-cyclohexyl]-éthyl}-amide de l'acide thiophène-2-carboxylique
(453) N-(2-{4-[diméthylamino-(3-fluoro-phényl)-méthyl]-cyclohexyl}-éthyl)-2-(3-méthoxy-phényl)-acétamide
(454) N-{2-[4-(diméthylamino-thiophèn-2-yl-méthyl)-cyclohexyl]-éthyl}-2-(3-méthoxy-phényl)-acétamide
(455) N-(2-{4-[(4-chloro-phényl)-diméthylamino-méthyl]-cyclohexyl}-éthyl)-2-phényl-butyramide
(456) N-{2-[4-(diméthylamino-thiophèn-2-yl-méthyl)-cyclohexyl]-éthyl}-2-phényl-butyramide
(457) {2-[4-(diméthylamino-phényl-méthyl)-cyclohexyl]-éthyl}-amide de l'acide benzo[b]thiophène-2-carboxylique
(458) N-{2-[4-(diméthylamino-phényl-méthyl)-cyclohexyl]-éthyl}-4-nitro-benzamide
(459) 3-bromo-N-(2-{4-[diméthylamino-(4-fluoro-phényl)-méthyl]-cyclohexyl}-éthyl)-benzamide
(460) N-{2-[4-(diméthylamino-phényl-méthyl)-cyclohexyl]-éthyl}-2,3,4,5,6-pentafluoro-benzamide
(461) N-{2-[4-(diméthylamino-phényl-méthyl)-cyclohexyl]-éthyl}-2,6-difluoro-benzamide
(462) N-(2-{4-[diméthylamino-(3-fluoro-phényl)-méthyl]-cyclohexyl}-éthyl)-2,6-difluoro-benzamide
(463) {2-[4-(diméthylamino-phényl-méthyl)-cyclohexyl]-éthyl}-amide de l'acide 2-phényl-thiazole-4-carboxylique
(464) (2-{4-[diméthylamino-(4-fluoro-phényl)-méthyl]-cyclohexyl}-éthyl)-amide de l'acide 2-phényl-thiazole-4-carboxylique
(465) {2-[4-(diméthylamino-thiophèn-2-yl-méthyl)-cyclohexyl]-éthyl}-amide de l'acide benzo[b]thiophène-3-carboxylique
(466) N-{2-[4-(diméthylamino-phényl-méthyl)-cyclohexyl]-éthyl}-2-méthylsulfanyl-nicotinamide
(467) {2-[4-(diméthylamino-phényl-méthyl)-cyclohexyl]-éthyl}-amide de l'acide 2-méthyl-5-phényl-furanne-3-carboxylique
(468) {2-[4-(diméthylamino-phényl-méthyl)-cyclohexyl]-éthyl}-amide de l'acide 2-(2,3-dihydro-benzofurann-5-yl)-thiazole-4-carboxylique
(469) {2-[4-(diméthylamino-thiophèn-2-yl-méthyl)-cyclohexyl]-éthyl}-amide de l'acide 3-(2,6-dichloro-phényl)-5-phényl-isoxazole-4-carboxylique
(470) 2-(4-chloro-phénylsulfanyl)-N-{2-[4-(diméthylamino-phényl-méthyl)-cyclohexyl]-éthyl}-nicotinamide (le diastéréoisomère le plus polaire)
(471) 2-(4-chloro-phénylsulfanyl)-N-{2-[4-(diméthylamino-phényl-méthyl)-cyclohexyl]-éthyl}-nicotinamide (le diastéréoisomère le plus apolaire)
(472) {2-[4-(diméthylamino-phényl-méthyl)-cyclohexyl]-éthyl}-amide de l'acide benzo[1,2,3]thiadiazole-5-carboxylique
(473) 5-bromo-N-{2-[4-(diméthylamino-phényl-méthyl)-cyclohexyl]-éthyl}-nicotinamide
(474) {2-[4-(diméthylamino-phényl-méthyl)-cyclohexyl]-éthyl}-amide de l'acide 5-chloro-4-méthoxy-thiophène--3-carboxylique
(475) (2-{4-[diméthylamino-(4-fluoro-phényl)-méthyl]-cyclohexyl}-éthyl)-amide de l'acide 5-chloro-4-méthoxy-thiophène-3-carboxylique
(476) {2-[4-(1-diméthylamino-3-phényl-propyl)-cyclohexyl]-éthyl}-amide de l'acide 5-chloro-4-méthoxy-thiophène-3-carboxylique
(477) 3-cyano-N-{2-[4-(diméthylamino-phényl-méthyl)-cyclohexyl]éthyl}-benzamide
(478) N-{2-[4-(diméthylamino-phényl-méthyl)-cyclohexyl]-éthyl}-2,4-diméthoxy-benzamide
(479) 2-chloro-N-((4-((diméthylamino) (phényl)méthyl)-cyclohexyl)méthyl)benzamide
(480) N-((4-((diméthylamino) (phényl)méthyl)cyclohexyl)-méthyl)-4-fluorobenzamide
(481) N-(2-(4-((diméthylamino)(phényl)méthyl)-cyclohexyl)éthyl)-4-fluorobenzamide

(482) N-((4-((diméthylamino) (phényl)méthyl) cyclohexyl)-méthyl)-2-fluorobenzamide
(483) N-((4-((diméthylamino)(phényl)méthyl)cyclohexyl)-méthyl)-3-méthylbenzamide
(484) N-((4-((diméthylamino)(phényl)méthyl)cyclohexyl)-méthyl)-2-méthoxybenzamide
(485) N-(2-(4-((diméthylamino)(phényl)méthyl)-cyclohexyl)éthyl)-3,5-diméthoxybenzamide
(486) N-((4-((diméthylamino)(3-fluorophényl)méthyl)-cyclohexyl)méthyl)-2,6-diméthoxybenzamide
(487) N-((4-((diméthylamino)(phényl)méthyl)cyclohexyl)-méthyl)-2,4-difluorobenzamide
(488) N-((4- ((diméthylamino) (thiophén-2-yl) méthyl)-cyclohexyl)méthyl)-3-méthoxybenzamide
(489) N-((4-((diméthylamino)(thiophèn-2-y1)méthyl)-cyclohexyl)méthyl)-3,4,5-triméthoxybenzamide
(490) 4-((diméthylamino)(phényl)méthyl)-1-(4-fluoro-3-méthylphényl)cyclohexanol
(491) N-cyclohexyl-2-(4-(2-phényl-1-(pyrrolidin-1-yl)éthyl)cyclohexyl)acétamide
(492) N-(3-méthoxyphényl)-2-(4-(2-phényl-1-(pyrrolidin-1-yl)éthyl)cyclohexyl)acétamide
(493) N-(4-méthoxyphényl)-2-(4-(pipéridin-1-yl(p-tolyl)méthyl)cyclohexylidène)acétamide
(494) N-phénéthyl-2-(4-(pipéridin-1-yl(p-tolyl)méthyl)-cyclohexylidène)acétamide
(495) 2-(4-(2-phényl-1-(pyrrolidin-1-yl)éthyl)-cyclohexylidène)-N-(pyridin-2-ylméthyl)acétamide
(496) N-benzyl-N-méthyl-2-(4-(pipéridin-1-yl(p-tolyl)méthyl)cyclohexylidène)acétamide
(497) [4-(diméthylamino-phényl-méthyl)-cyclohexyl]-amido de l'acide 3-thiophèn-2-yl-[1,2,4]oxadiazole-5-carboxylique
(498) {2-[4-(diméthylamino-phényl-méthyl)-cyclohexyl]-éthyl}-amide de l'acide 3-méthyl-[1,2,4]oxadiazole-5-carboxylique
(499) {4-[diméthylamino-(3-fluoro-phényl)-méthyl]-cyalohexyl}-amide de l'acide 3-phényl-[1,2,4]oxadiazole-5-carboxylique
(500) {4-[diméthylamino-(3-fluoro-phényl)-méthyl]-cyclohexyl}-amide de l'acide 3-cyclopropylméthyl-[1,2,4] oxadiazol-5-carboxylique
(501) {2-[4-(1-diméthylamino-3-phényl-propyl)-cyclohexyl]-éthyl}-amide de l'acide 3-méthoxy-méthyl-[1,2,4] oxadiazole-5-carboxylique

**23.** Procédé de préparation d'un dérivé cyclohexylméthylé selon l'invention selon la revendication 1, dans lequel $R^1$ est $(CH_2)_nC(O)H$, dans lequel on fait réagir des cétones de formule générale G

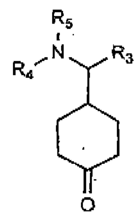

**G**

avec du chlorure de (méthoxyméthyl)triphénylphosphonium et une base forte, par exemple le tert-butylate de potassium à une température comprise entre -20°C et +30°C, pour donner les aldéhydes correspondants **H**, l'étape de réaction étant éventuellement répétée pour la synthèse d'aldéhydes dans lesquels n > 0.

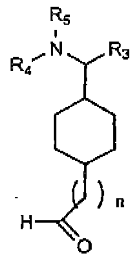

**H**

**24.** Procédé de préparation d'un dérivé cyclohexylméthylé selon l'invention selon la revendication 1, dans lequel $R^1$ est $(CH_2)_mCHN\text{-}OH$, =N-OH, $(CH_2)_nNH_2$, dans lequel on fait réagir la cétone **G** ou l'aldéhyde **H**

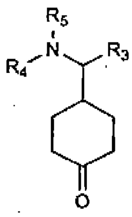

G

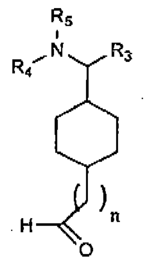

H

par réaction avec du chlorhydrate d'hydroxylamine dans un solvant organique, par exemple l'éthanol, avec addition d'une base, par exemple un échangeur d'ions basiques Amberlyst, pour donner des oximes de formule générale **K**, et on obtient les amines de formule générale **L** par réaction avec un réducteur, par exemple $LiAlH_4$.

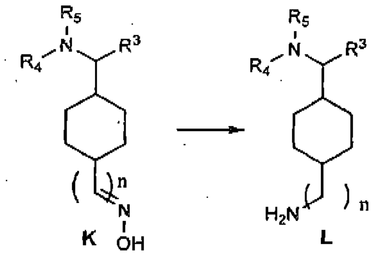

K L

**25.** Procédé de préparation d'un dérivé cyclohexylméthylé selon l'invention selon la revendication 1, dans lequel $R^1$ est $(CH_2)_nNHC(O)R^{13}$ ou $(CH_2)_nNHSO_2R^{12}$, dans lequel on relie des amines de formule générale **L**

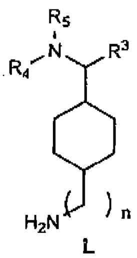

L

à des acides carboxyliques ou des acides sulfoniques par addition de réactifs de couplage ou par activation du composant acide, en particulier par préparation du chlorure d'acyle.

**26.** Procédé de préparation d'un dérivé cyclohexylméthylé selon l'invention selon la revendication 1, dans lequel $R^1$ est $(CH_2)_nNHC(O)NR^{10}R^{11}$ ou $(CH_2)_nNHC(S)NR^{10}R^{11}$, dans lequel on fait réagir des amines de formule générale **L**

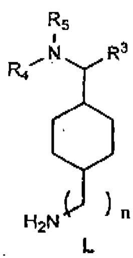

L

avec des isocyanates appropriés de formule générale $R^{10}$-N=C=O ou des isothiocyanates de formule générale

$R^{10}$-N=C=S, éventuellement en présence d'au moins une base, pour donner un composé de formule générale I, dans laquelle $R^1$ est $(CH_2)_nNHC(O)NR^{10}R^{11}$ ou $(CH2)_nNHC(S)NR^{10}R^{11}$ et $R^{11}$ est H, et on fait réagir ce composé, éventuellement en présence d'au moins une base, avec un composé de formule générale LG-$R^{11}$, dans laquelle LG est un groupe partant et $R^{11}$ a les significations données ci-dessus, à l'exception de l'hydrogène, pour donner un composé de formule générale I, dans laquelle $R^1$ est $(CH_2)_nNHC(O)NR^{10}R^{11}$ ou $(CH_2)_nNHC(S)NR^{10}R^{11}$, où $R^{11}$ n'est pas H.

**27.** Procédé de préparation d'un dérivé cyclohexylméthylé selon l'invention selon la revendication 1, dans lequel $R^1$ est un radical $C(O)OR^9$, relié par l'intermédiaire d'un groupe alkyle en $C_{1-3}$, qui peut être saturé ou insaturé ; ou $R^1$ et $R^2$ forment ensemble

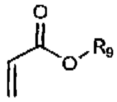

où on fait réagir un ester de l'acide phosphonoacétique, de préférence le phosphonoacétate de triméthyle ou le phosphonoacétate de triéthyle, d'abord avec une base forte, de préférence le tert-butylate de

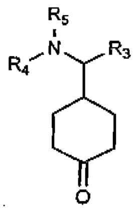

G

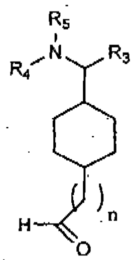

H

et on hydrolyse éventuellement les esters avec une solution basique aqueuse appropriée, de préférence avec une solution d'hydroxyde de potassium ou d'hydroxyde de lithium, à la température ambiante ou à une température légèrement élevée, pour donner les acides carboxyliques correspondants, ou on réduit la double liaison, de préférence par hydrogénation catalytique hétérogène sur des catalyseurs au palladium ou au platine ou par hydrogénation, catalysée par un catalyseur homogène, avec des catalyseurs au rhodium, dans chaque cas à des températures comprises entre la température ambiante et 60°C et sous des pressions d'hydrogène comprises entre 1 bar et 6 bar, d'une manière particulièrement préférée à la température ambiante et sous une pression d'hydrogène comprise entre 2 et 3 bar, sur du palladium sur charbon. Puis on continue, comme décrit ci-dessus, avec l'hydrolyse des esters. Les esters peuvent être réduits à l'aide d'un réducteur, par exemple $LiAlH_4$, pour donner les alcools correspondants.

**28.** Procédé de préparation d'un dérivé cyclohexylméthylé selon l'invention selon la revendication 1, dans lequel $R^1$ est $(CH_2)_nC(O)NR^{10}R^{11}$, où on fait réagir des acides carboxyliques selon la potassium, l'hydrure de sodium ou le butyllithium, puis avec une cétone de formule générale **G** ou un aldéhyde **H** revendication 27 en présence d'agents déshydratants ou après conversion en un chlorure d'acyle ou un ester actif à l'aide d'une amine primaire ou secondaire.

**29.** Procédé de préparation d'un dérivé cyclohexylméthylé selon l'invention selon la revendication 1, dans lequel $R^1$ est $(CH2)_nOR^9$, où on fait réagir la cétone G ou l'aldéhyde H

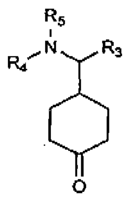

G

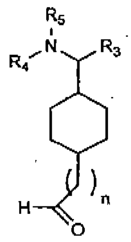

H

par réaction avec un réducteur, par exemple le borohydrure de sodium, pour donner des composés selon l'invention dans lesquels $R^1$ est $(CH_2)_nOH$, ou
on réduit les esters selon la revendication 27 avec un réducteur, par exemple $LiAlH_4$, pour donner les alcools correspondants,
et on fait réagir ces alcools, par addition d'une base, par exemple NaH, avec un composé de formule générale $R^8Hal$ dans laquelle Hal est de préférence Cl, pour donner des composés dans lesquels $R^1$ est $(CH_2)_nOR^8$, $R^8$ ici ne représentant pas H.

**30.** Procédé de préparation d'un dérivé cyclohexylméthylé selon l'invention selon la revendication 1, dans lequel $R^1$ est $(CH_2)_nNHR^6$, dans lequel on dissout la cétone G ou l'aldéhyde H

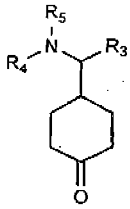

G

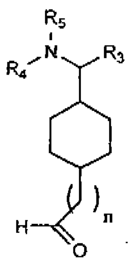

H

dans des solvants aprotiques polaires, par exemple THF, et on le fait réagir avec les amines correspondantes de formule générale $NH_2R^6$, par addition d'un réducteur approprié, par exemple le borohydrure de sodium.

**31.** Procédé de préparation d'un dérivé cyclohexylméthylé selon l'invention selon la revendication 1, dans lequel $R^2$ est OH et $R^1$ est un groupe alkyle en $C_{1-8}$, chacun étant ramifié ou non ramifié, saturé ou insaturé, non substitué ou une ou plusieurs fois substitué ; aryle ou hétéroaryle, non substitué ou une ou plusieurs fois substitué ; cycloalkyle en $C_{3-10}$, saturé ou insaturé, non substitué ou une ou plusieurs fois substitué ; un groupe aryle ou hétéroaryle relié par l'intermédiaire d'une chaîne alkyle en $C_{1-4}$, chacun étant non substitué ou une ou plusieurs fois substitué, où on fait réagir des cétones de formule générale G

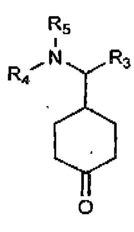

G

avec des composés organométalliques de formule générale $R^{1a}MgHal$, dans laquelle Hal = Cl ou Br, ou $R^{1a}Li$, par refroidissement à une température de -30 à +10°C dans un solvant organique, par exemple le diéthyléther ou le THF, ou
on ajoute à un iodure d'aryle dans un solvant organique, par exemple le THF, à une température comprise entre

-30°C et 0°C, une solution de chlorure d'isopropylmagnésium et, après au moins 10 minutes d'agitation, on le fait réagir avec une cétone de formule générale G pour donner un composé de formule générale I dans laquelle $R^2$ est OH et $R^1$ est un groupe aryle.

**32.** Médicament contenant au moins un dérivé cyclohexylméthylé substitué selon la revendication 1, éventuellement sous forme du racémate ; des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles, ainsi qu'éventuellement des additifs et/ou adjuvants éventuellement présents, et/ou éventuellement d'autres principes actifs.

**33.** Utilisation d'un dérivé cyclohexylméthylé substitué selon la revendication 1, éventuellement sous forme du racémale; des énantiomères, des diastéréoisomeres, des mélanges des énantiomères ou des diastéréoisomères, ou d'un énantiomère ou d'un diastéréoisomère unique ; des bases et/ou des sels d'acides physiologiquement compatibles, pour préparer un médicament destiné au traitement de la douleur, en particulier de la douleur aiguë, neuropathique ou chronique.

**34.** Utilisation d'un dérivé cyclohexylméthylé substitué selon la revendication 1, éventuellement sous forme du racémate ; des énantioméres, des diastéréoisomères, des mélanges des énantiomères ou des diastéréoisoméres, ou d'un énantiomère ou d'un diastéréoisomère unique ; des bases et/ou des sels d'acides physiologiquement compatibles, pour préparer un médicament destiné au traitement des dépressions, de l'incontinence urinaire, de la diarrhée, du prurit, de l'alcoolisme et de la toxicomanie, de la dépendance aux médicaments, de l'avolition et/ou pour l'anxiolyse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0290317 A
- EP 1043307 A2
- WO 02066432 A1
- WO 04043899 A1


### In der Beschreibung aufgeführte Nicht-Patentliteratur


- Chemistry and Pharmacology to Clinical Application. Wiley, 2002, 265-284
- **E.G. Gray ; V.P. Whittaker.** *J. Anat.,* 1962, vol. 76, 79-88
- **M.Ch. Frink ; H.-H. Hennies ; W. Englberger ; M. Haurand ; B. Wilffert.** *Arzneim.-Forsch./Drug Res.,* 1996, vol. 46 (III), 1029-1036